# EUROPEAN PATENT SPECIFICATION

(11) **EP 3 467 894 B1**
(45) Date of publication and mention of the grant of the patent: **02.08.2023**
(21) Application number: 18195568.3
(22) Date of filing: 19.09.2018
(51) Int. Cl.: C07D 209/86, C07D 209/88, C07D 213/06, C07D 213/22, C07D 235/18, C07D 239/26, C07D 251/24, C07D 307/91, C07D 333/76, C07D 251/00

(54) **ORGANIC LIGHT-EMITTING DEVICE**
ORGANISCHE LICHTEMITTIERENDE VORRICHTUNG
DISPOSITIF ÉLECTROLUMINESCENT ORGANIQUE

(30) Priority: 26.09.2017 KR 20170124530; 10.08.2018 KR 20180093994
(43) Date of publication of application: 10.04.2019
(73) Proprietor: Samsung Display Co., Ltd., Gyeonggi-do (KR)
(72) Inventor: KIM, Mikyung, Gyeonggi-do (KR); SEO, Jihyun, Gyeonggi-do (KR); YANG, Seunggak, Gyeonggi-do (KR); LEE, Kwanhee, Gyeonggi-do (KR); LIM, Jino, Gyeonggi-do (KR)
(74) Representative: Marks & Clerk LLP

(56) References cited:
- WO-A1-2014/171779
- ALEXANDRA YA. FREIDZON, ET AL.: "Predicting the Operational Stability of Phosphorescent OLED HostMolecules from First Principles: A Case Study", J. PHYS. CHEM. C, vol. 121, 20 September 2017 (2017-09-20), pages 22422-22433, XP002788845,
- HIROHIKO FUKAGAWA, TAKAHISA SHIMIZU , YUKIKO IWASAKI & TOSHIHIRO YAMAMOTO: "Operational lifetimes of organiclight-emitting diodes dominated byFörster resonance energy transfer", NATURE SCIENTIFIC REPORTS, vol. 7, 1735, 11 May 2017 (2017-05-11), pages 1-8, XP002788846, DOI: 10.1038/s41598-017-02033-3

## Description

### BACKGROUND

### 1. Field

One or more embodiments of the present disclosure relate to an organic light-emitting device.

### 2. Description of the Related Art

Organic light-emitting devices are self-emission devices that produce full-color images, and also have wide viewing angles, high contrast ratios, short response times, as well as excellent characteristics in terms of brightness, driving voltage, and response speed.

In an organic light-emitting device, a first electrode is arranged on a substrate, and a hole transport region, an emission layer, an electron transport region, and a second electrode are sequentially formed on the first electrode. Holes provided from the first electrode may move toward the emission layer through the hole transport region, and electrons provided from the second electrode may move toward the emission layer through the electron transport region. Carriers, such as the holes and the electrons, recombine in the emission layer to produce excitons. These excitons transit (e.g., transition or relax) from an excited state to a ground state, thereby generating light.

### SUMMARY

Aspects of embodiments of the present disclosure provide an organic light-emitting device.

Additional aspects of embodiments will be set forth in part in the description which follows and, in part, will be apparent from the description, or may be learned by practice of the presented embodiments.

An aspect of an embodiment provides an organic light-emitting device including:
a first electrode;
a second electrode; and
an organic layer between the first electrode and the second electrode and including an emission layer,
wherein the organic layer includes a first compound, a second compound, and a third compound, and
the first compound, the second compound, and the third compound are each independently an azine-based compound represented by one selected from Formulae 1-1 to 1-5, except for a case where the first compound, the second compound, and the third compound concurrently (e.g., simultaneously) include an azine-based compound represented by Formula 1-3: 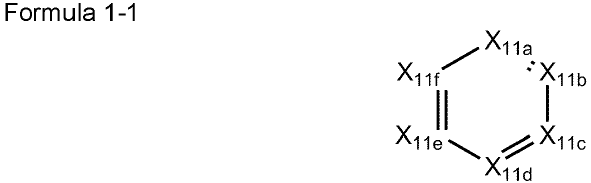 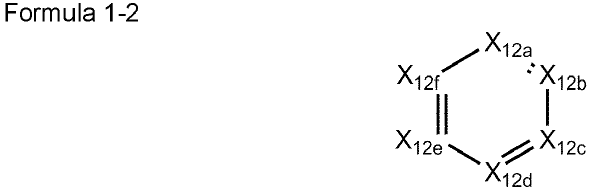 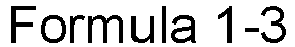 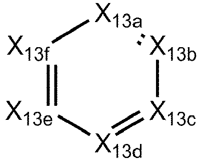 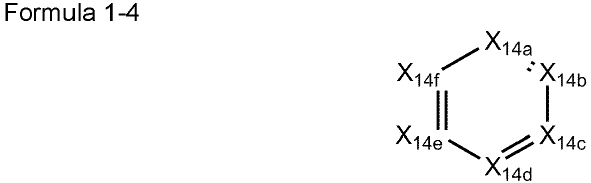 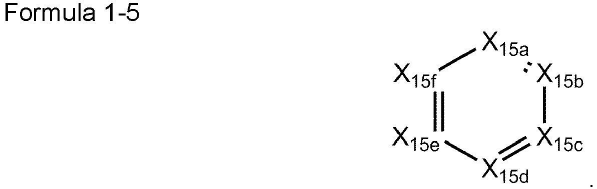
In Formulae 1-1 to 1-5,
X₁₁ₐ is selected from N and C[(L₁₁ₐ)ₐ₁₁ₐ-R₁₁ₐ]; X_{11b} is selected from N and C[(L_{11b})_{a11b}-R_{11b}]; X_{11c} is selected from N and C[(L_{11c})_{a11c}-R_{11c}]; X_{11d} is selected from N and C[(L_{11d})_{a11d}-R_{11d}]; X₁₁ₑ is selected from N and C[(L₁₁ₑ)ₐ₁₁ₑ-R₁₁ₑ]; X_{11f} is selected from N and C[(L_{11f})_{a11f}-R_{11f}]; and one of X₁₁ₐ to X_{11f} is N,
X₁₂ₐ is selected from N and C[(L₁₂ₐ)ₐ₁₂ₐ-R₁₂ₐ]; X_{12b} is selected from N and C[(L_{12b})_{a12b}-R_{12b}]; X_{12c} is selected from N and C[(L_{12c})_{a12c}-R_{12c}]; X_{12d} is selected from N and C[(L_{12d})_{a12d}-R_{12d}]; X₁₂ₑ is selected from N and C[(L₁₂ₑ)ₐ₁₂ₑ-R₁₂ₑ]; X_{12f} is selected from N and C[(L_{12f})_{a12f}-R_{12f}]; and two of X₁₂ₐ to X_{12f} are N,
X₁₃ₐ is selected from N and C[(L₁₃ₐ)ₐ₁₃ₐ-R₁₃ₐ], X_{13b} is selected from N and C[(L_{13b})_{a13b}-R_{13b}]; X_{13c} is selected from N and C[(L_{13c})_{a13c}-R_{13c}]; X_{13d} is selected from N and C[(L_{13d})_{a13d}-R_{13d}]; X₁₃ₑ is selected from N and C[(L₁₃ₑ)ₐ₁₃ₑ-R₁₃ₑ]; X_{13f} is selected from N and C[(L_{13f})_{a13f}-R_{13f}]; and three of X₁₃ₐ to X_{13f} are N,
X₁₄ₐ is selected from N and C[(L₁₄ₐ)ₐ₁₄ₐ-R₁₄ₐ], X_{14b} is selected from N and C[(L_{14b})_{a14b}-R_{14b}]; X_{14c} is selected from N and C[(L_{14c})_{a14c}-R_{14c}]; X_{14d} is selected from N and C[(L_{14d})_{a14d}-R_{14d}]; X₁₄ₑ is selected from N and C[(L₁₄ₑ)ₐ₁₄ₑ-R₁₄ₑ]; X_{14f} is selected from N and C[(L_{14f})_{a14f}-R_{14f}]; and four of X₁₄ₐ to X_{14f} are N,
X₁₅ₐ is selected from N and C[(L₁₅ₐ)ₐ₁₅ₐ-R₁₅ₐ]; X_{15b} is selected from N and C[(L_{15b})_{a15b}-R_{15b}]; X_{15c} is selected from N and C[(L_{15c})_{a15c}-R_{15c}]; X_{15d} is selected from N and C[(L_{15d})_{a15d}-R_{15d}]; X₁₅ₑ is selected from N and C[(L₁₅ₑ)ₐ₁₅ₑ-R₁₅ₑ]; X_{15f} is selected from N and C[(L_{15f})_{a15f}-R_{15f}]; and five of X₁₅ₐ to X_{15f} are N,
L₁₁ₐ to L_{11f}, L₁₂ₐ to L_{12f}, L₁₃ₐ to L_{13f}, L₁₄ₐ to L_{14f}, and L₁₅ₐ to L_{15f} are each independently selected from:
   a C₃-C₁₀ cycloalkenylene group, a C₁-C₁₀ heterocycloalkenylene group, a C₆-C₆₀ arylene group, a C₁-C₆₀ heteroarylene group, a divalent non-aromatic condensed polycyclic group, and a divalent non-aromatic condensed heteropolycyclic group; and
   a C₃-C₁₀ cycloalkenylene group, a C₁-C₁₀ heterocycloalkenylene group, a C₆-C₆₀ arylene group, a C₁-C₆₀ heteroarylene group, a divalent non-aromatic condensed polycyclic group, and a divalent non-aromatic condensed heteropolycyclic group, each substituted with at least one selected from deuterium, -F, -Cl, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₂₀ alkyl group, a C₁-C₂₀ alkoxy group, a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₃₀ aryl group, a C₆-C₃₀ aryloxy group, a C₆-C₃₀ arylthio group, a C₁-C₃₀ heteroaryloxy group, a C₁-C₃₀ heteroarylthio group, a monovalent non-aromatic condensed polycyclic group, a monovalent non-aromatic condensed heteropolycyclic group, a pyrrolyl group, a thiophenyl group, a furanyl group, an imidazolyl group, a pyrazolyl group, a thiazolyl group, an isothiazolyl group, an oxazolyl group, an isoxazolyl group, a pyridinyl group, a pyrazinyl group, a pyrimidinyl group, a pyridazinyl group, an isoindolyl group, an indolyl group, an indazolyl group, a purinyl group, a quinolinyl group, an isoquinolinyl group, a benzoquinolinyl group, a phthalazinyl group, a naphthyridinyl group, a quinoxalinyl group, a quinazolinyl group, a cinnolinyl group, a carbazolyl group, a phenanthridinyl group, an acridinyl group, a phenanthrolinyl group, a phenazinyl group, a benzimidazolyl group, a benzofuranyl group, a benzothiophenyl group, an isobenzothiazolyl group, a benzoxazolyl group, an isobenzoxazolyl group, a triazolyl group, a tetrazolyl group, an oxadiazolyl group, a dibenzofuranyl group, a dibenzothiophenyl group, a benzocarbazolyl group, a dibenzocarbazolyl group, a thiadiazolyl group, and an imidazopyridinyl group,
   wherein L₁₁ₐ to L_{11f}, L₁₂ₐ to L_{12f}, L₁₃ₐ to L_{13f}, L₁₄ₐ to L_{14f}, and L₁₅ₐ to L_{15f} are not each independently selected from:
      a triazinylene group; and
      a triazinylene group substituted with at least one selected from deuterium, - F, -Cl, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₂₀ alkyl group, a C₁-C₂₀ alkoxy group, a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₃₀ aryl group, a C₆-C₃₀ aryloxy group, a C₆-C₃₀ arylthio group, a C₁-C₃₀ heteroaryloxy group, a C₁-C₃₀ heteroarylthio group, a monovalent non-aromatic condensed polycyclic group, a monovalent non-aromatic condensed heteropolycyclic group, a pyrrolyl group, a thiophenyl group, a furanyl group, an imidazolyl group, a pyrazolyl group, a thiazolyl group, an isothiazolyl group, an oxazolyl group, an isoxazolyl group, a pyridinyl group, a pyrazinyl group, a pyrimidinyl group, a pyridazinyl group, an isoindolyl group, an indolyl group, an indazolyl group, a purinyl group, a quinolinyl group, an isoquinolinyl group, a benzoquinolinyl group, a phthalazinyl group, a naphthyridinyl group, a quinoxalinyl group, a quinazolinyl group, a cinnolinyl group, a carbazolyl group, a phenanthridinyl group, an acridinyl group, a phenanthrolinyl group, a phenazinyl group, a benzimidazolyl group, a benzofuranyl group, a benzothiophenyl group, an isobenzothiazolyl group, a benzoxazolyl group, an isobenzoxazolyl group, a triazolyl group, a tetrazolyl group, an oxadiazolyl group, a dibenzofuranyl group, a dibenzothiophenyl group, a benzocarbazolyl group, a dibenzocarbazolyl group, a thiadiazolyl group, and an imidazopyridinyl group,
      a11a to a11f, a12a to a12f, a13a to a13f, a14a to a14f, and a15a to a15f are each independently selected from 0, 1, 2, 3, and 4,
      R₁₁ₐ to R_{11f}, R₁₂ₐ to R_{12f}, R₁₃ₐ to R_{13f}, R₁₄ₐ to R_{14f}, and R₁₅ₐ to R_{15f} are each independently selected from:
         hydrogen, deuterium, -F, -Cl, -Br, -I, a methyl group, an ethyl group, an n-propyl group, an isopropyl group, an n-butyl group, an isobutyl group, a sec-butyl group, a tert-butyl group, a methoxy group, an ethoxy group, an n-propoxy group, an isopropoxy group, an n-butoxy group, an isobutoxy group, a sec-butoxy group, and a tert-butoxy group;
         a methyl group, an ethyl group, an n-propyl group, an isopropyl group, an n-butyl group, an isobutyl group, a sec-butyl group, and a tert-butyl group, each substituted with at least one selected from deuterium, -F, -Cl, -Br, and -I;
         a phenyl group, a biphenyl group, a terphenyl group, a naphthyl group, a fluorenyl group, a carbazolyl group, a dibenzofuranyl group, a dibenzothiophenyl group, a pyridinyl group, a pyrimidinyl group, a quinolinyl group, an isoquinolinyl group, a quinoxalinyl group, a quinazolinyl group, a benzofuropyridinyl group, a benzothienopyridinyl group, a benzofuropyrimidinyl group, a benzothienopyrimidinyl group, a monovalent non-aromatic condensed polycyclic group, and a monovalent non-aromatic condensed heteropolycyclic group;
         a phenyl group, a biphenyl group, a terphenyl group, a naphthyl group, a fluorenyl group, a carbazolyl group, a dibenzofuranyl group, a dibenzothiophenyl group, a pyridinyl group, a pyrimidinyl group, a quinolinyl group, an isoquinolinyl group, a quinoxalinyl group, a quinazolinyl group, a benzofuropyridinyl group, a benzothienopyridinyl group, a benzofuropyrimidinyl group, a benzothienopyrimidinyl group, a monovalent non-aromatic condensed polycyclic group, and a monovalent non-aromatic condensed heteropolycyclic group, each substituted with at least one selected from deuterium, -F, -Cl, -Br, -I, a methyl group, an ethyl group, an n-propyl group, an isopropyl group, an n-butyl group, an isobutyl group, a sec-butyl group, a tert-butyl group, a methoxy group, an ethoxy group, an n-propoxy group, an isopropoxy group, an n-butoxy group, an isobutoxy group, a sec-butoxy group, a tert-butoxy group, a phenyl group, a biphenyl group, a terphenyl group, a naphthyl group, a fluorenyl group, a carbazolyl group, a dibenzofuranyl group, a dibenzothiophenyl group, a pyridinyl group, a pyrimidinyl group, a quinolinyl group, an isoquinolinyl group, a quinoxalinyl group, a quinazolinyl group, a benzofuropyridinyl group, a benzothienopyridinyl group, a benzofuropyrimidinyl group, and a benzothienopyrimidinyl group; and
         a phenyl group, a biphenyl group, a terphenyl group, a naphthyl group, a fluorenyl group, a carbazolyl group, a dibenzofuranyl group, a dibenzothiophenyl group, a pyridinyl group, a pyrimidinyl group, a quinolinyl group, an isoquinolinyl group, a quinoxalinyl group, a quinazolinyl group, a benzofuropyridinyl group, a benzothienopyridinyl group, a benzofuropyrimidinyl group, a benzothienopyrimidinyl group, a monovalent non-aromatic condensed polycyclic group, and a monovalent non-aromatic condensed heteropolycyclic group, each substituted with at least one selected from a methyl group, an ethyl group, an n-propyl group, an isopropyl group, an n-butyl group, an isobutyl group, a sec-butyl group, a tert-butyl group, a phenyl group, a biphenyl group, a terphenyl group, a naphthyl group, a fluorenyl group, a carbazolyl group, a dibenzofuranyl group, a dibenzothiophenyl group, a pyridinyl group, a pyrimidinyl group, a quinolinyl group, an isoquinolinyl group, a quinoxalinyl group, a quinazolinyl group, a benzofuropyridinyl group, a benzothienopyridinyl group, a benzofuropyrimidinyl group, and a benzothienopyrimidinyl group that are each substituted with at least one selected from deuterium, -F, -Cl, -Br, and -I, and
         neighboring atoms of R₁₁ₐ to R_{11f}, neighboring atoms of R₁₂ₐ to R_{12f}, neighboring R₁₃ₐ to R_{13f}, neighboring atoms of R₁₄ₐ to R_{14f}, and/or neighboring atoms of R₁₅ₐ to R_{15f} optionally form a saturated ring or an unsaturated ring.

At least some of the above and other embodiments of the invention are set out in the claims.

### BRIEF DESCRIPTION OF THE DRAWINGS

These and/or other aspects of embodiments will become apparent and more readily appreciated from the following description of the embodiments, taken in conjunction with the accompanying drawings in which:
FIG. 1 is a schematic cross-sectional view of an organic light-emitting device according to an embodiment;
FIG. 2 is a schematic cross-sectional view of an organic light-emitting device according to another embodiment;
FIG. 3 is a schematic cross-sectional view of an organic light-emitting device according to another embodiment; and
FIG. 4 is a schematic cross-sectional view of an organic light-emitting device according to another embodiment.

### DETAILED DESCRIPTION

The present disclosure will now be described more fully with reference to embodiments.

Hereinafter, embodiments are described in more detail by referring to the attached drawings, and in the drawings, like reference numerals denote like elements, and a redundant explanation thereof will not be repeated herein.

As used herein, the singular forms "a," "an" and "the" are intended to include the plural forms as well, unless the context clearly indicates otherwise.

It will be further understood that the terms "comprises" and/or "comprising" used herein specify the presence of stated features or components, but do not preclude the presence or addition of one or more other features or components.

It will be understood that when a layer, region, or component is referred to as being "on" or "onto" another layer, region, or component, it may be directly or indirectly formed on the other layer, region, or component. For example, intervening layers, regions, or components may be present.

Sizes of components in the drawings may be exaggerated for convenience of explanation. In other words, since sizes and thicknesses of components in the drawings may be arbitrarily illustrated for convenience of explanation, the following embodiments of the present disclosure are not essentially limited thereto.

An organic light-emitting device according to an embodiment includes a first electrode; a second electrode; and an organic layer between the first electrode and the second electrode and including an emission layer, wherein the organic layer includes a first compound, a second compound, and a third compound, and the first compound, the second compound, and the third compound are each independently an azine-based compound represented by one selected from Formulae 1-1 to 1-5, except for a case where the first compound, the second compound, and the third compound concurrently (e.g., simultaneously) include an azine-based compound represented by Formula 1-3 (e.g., only one or two selected from the first compound, the second compound, and the third compound is represented by Formula 1-3):

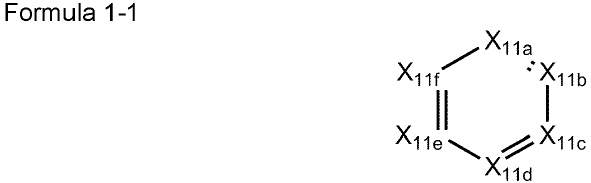

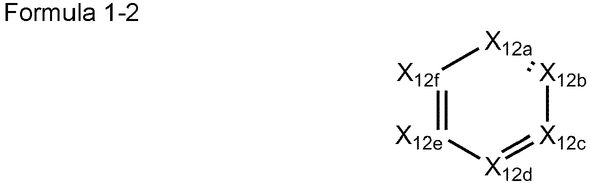

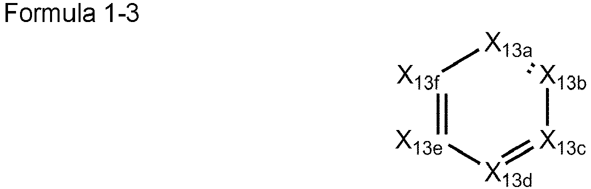

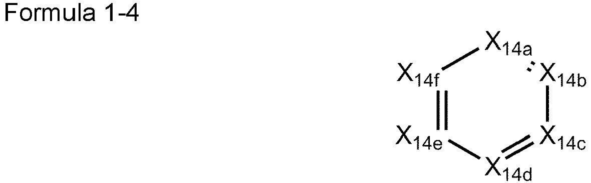

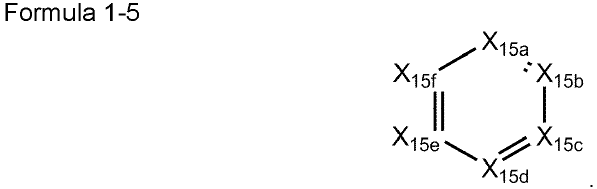

In Formula 1-1, X₁₁ₐ is selected from N and C[(L₁₁ₐ)ₐ₁₁ₐ-R₁₁ₐ]; X_{11b} is selected from N and C[(L_{11b})_{a11b}-R_{11b}]; Xnc is selected from N and C[(L_{11c})_{a11c}-R_{11c}]; X_{11d} is selected from N and C[(L_{11d})_{a11d}-R_{11d}]; X₁₁ₑ is selected from N and C[(L₁₁ₑ)ₐ₁₁ₑ-R₁₁ₑ]; X_{11f} is selected from N and C[(L_{11f})_{a11f}-R_{11f}]; and one of X₁₁ₐ to X_{11f} is N. L₁₁ₐ to L_{11f}, a11a to a11f, and R₁₁ₐ to R_{11f} are each independently the same as described below.

In Formula 1-2, X₁₂ₐ is selected from N and C[(L₁₂ₐ)ₐ₁₂ₐ-R₁₂ₐ]; X_{12b} is selected from N and C[(L_{12b})_{a12b}-R_{12b}]; X_{12c} is selected from N and C[(L_{12c})_{a12c}-R_{12c}]; X_{12d} is selected from N and C[(L_{12d})_{a12d}-R_{12d}]; X₁₂ₑ is selected from N and C[(L₁₂ₑ)ₐ₁₂ₑ-R₁₂ₑ]; X_{12f} is selected from N and C[(L_{12f})_{a12f}-R_{12f}]; and two of X₁₂ₐ to X_{12f} are N. L₁₂ₐ to L_{12f}, a12a to a12f, and R₁₂ₐ to R_{12f} are each independently the same as described below.

In Formula 1-3, X₁₃ₐ is selected from N and C[(L₁₃ₐ)ₐ₁₃ₐ-R₁₃ₐ]; X_{13b} is selected from N and C[(L_{13b})_{a13b}-R_{13b}]; X_{13c} is selected from N and C[(L_{13c})_{a13c}-R_{13c}]; X_{13d} is selected from N and C[(L_{13d})_{a13d}-R_{13d}]; X₁₃ₑ is selected from N and C[(L₁₃ₑ)ₐ₁₃ₑ-R₁₃ₑ], X_{13f} is selected from N and C[(L_{13f})_{a13f}-R_{13f}]; and three of X₁₃ₐ to X_{13f} are N. L₁₃ₐ to L_{13f}, a13a to a13f, and R₁₃ₐ to R_{13f} are each independently the same as described below.

In Formula 1-4, X₁₄ₐ is selected from N and C[(L₁₄ₐ)ₐ₁₄ₐ-R₁₄ₐ]; X_{14b} is selected from N and C[(L_{14b})_{a14b}-R_{14b}]; X_{14c} is selected from N and C[(L_{14c})_{a14c}-R_{14c}]; X_{14d} is selected from N and C[(L_{14d})_{a14d}-R_{14d}]; X₁₄ₑ is selected from N and C[(L₁₄ₑ)ₐ₁₄ₑ-R₁₄ₑ]; X_{14f} is selected from N and C[(L_{14f})_{a14f}-R_{14f}]; and four of X₁₄ₐ to X_{14f} are N. L₁₄ₐ to L_{14f}, a14a to a14f, and R₁₄ₐ to R_{14f} are each independently the same as described below.

In Formula 1-5, X₁₅ₐ is selected from N and C[(L₁₅ₐ)ₐ₁₅ₐ-R₁₅ₐ], X_{15b} is selected from N and C[(L_{15b})_{a15b}-R_{15b}]; X_{15c} is selected from N and C[(L_{15c})_{a15c}-R_{15c}]; X_{15d} is selected from N and C[(L_{15d})_{a15d}-R_{15d}]; X₁₅ₑ is selected from N and C[(L₁₅ₑ)ₐ₁₅ₑ-R₁₅ₑ]; X_{15f} is selected from N and C[(L_{15f})_{a15f}-R_{15f}]; and five of X₁₅ₐ to X_{15f} are N. L₁₅ₐ to L_{15f}, a15a to a15f, and R₁₅ₐ to R_{15f} are each independently the same as described below.

In Formulae 1-1 to 1-5, L₁₁ₐ to L_{11f}, L₁₂ₐ to L_{12f}, L₁₃ₐ to L_{13f}, L₁₄ₐ to L_{14f}, and L₁₅ₐ to L_{15f} are each independently be selected from:
a C₃-C₁₀ cycloalkenylene group, a C₁-C₁₀ heterocycloalkenylene group, a C₆-C₆₀ (*e.g.* C₆-C₃₂) arylene group, a C₁-C₆₀ (*e*.*g*. C₁-C₂₀) heteroarylene group, a divalent non-aromatic condensed polycyclic group, and a divalent non-aromatic condensed heteropolycyclic group; and
a C₃-C₁₀ cycloalkenylene group, a C₁-C₁₀ heterocycloalkenylene group, a C₆-C₆₀ (*e*.*g*. C₆-C₃₂) arylene group, a C₁-C₆₀ (*e*.*g*. C₁-C₂₀) heteroarylene group, a divalent non-aromatic condensed polycyclic group, and a divalent non-aromatic condensed heteropolycyclic group, each substituted with at least one selected from deuterium, -F, -Cl, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₂₀ (*e*.*g*. C₁-C₁₀) alkyl group, a C₁-C₂₀ (*e*.*g*. C₁-C₁₀) alkoxy group, a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₃₀ aryl group, a C₆-C₃₀ aryloxy group, a C₆-C₃₀ arylthio group, a C₁-C₃₀ (*e.g*. C₁-C₂₀) heteroaryloxy group, a C₁-C₃₀ (*e*.*g*. C₁-C₂₀) heteroarylthio group, a monovalent non-aromatic condensed polycyclic group, a monovalent non-aromatic condensed heteropolycyclic group, a pyrrolyl group, a thiophenyl group, a furanyl group, an imidazolyl group, a pyrazolyl group, a thiazolyl group, an isothiazolyl group, an oxazolyl group, an isoxazolyl group, a pyridinyl group, a pyrazinyl group, a pyrimidinyl group, a pyridazinyl group, an isoindolyl group, an indolyl group, an indazolyl group, a purinyl group, a quinolinyl group, an isoquinolinyl group, a benzoquinolinyl group, a phthalazinyl group, a naphthyridinyl group, a quinoxalinyl group, a quinazolinyl group, a cinnolinyl group, a carbazolyl group, a phenanthridinyl group, an acridinyl group, a phenanthrolinyl group, a phenazinyl group, a benzimidazolyl group, a benzofuranyl group, a benzothiophenyl group, an isobenzothiazolyl group, a benzoxazolyl group, an isobenzoxazolyl group, a triazolyl group, a tetrazolyl group, an oxadiazolyl group, a dibenzofuranyl group, a dibenzothiophenyl group, a benzocarbazolyl group, a dibenzocarbazolyl group, a thiadiazolyl group, and an imidazopyridinyl group, and
L₁₁ₐ to L_{11f}, L₁₂ₐ to L_{12f}, L₁₃ₐ to L_{13f}, L₁₄ₐ to L_{14f}, and L₁₅ₐ to L_{15f} are not each independently be selected from: a triazinylene group; and a triazinylene group substituted with at least one selected from deuterium, -F, -Cl, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₂₀ alkyl group, a C₁-C₂₀ alkoxy group, a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₃₀ aryl group, a C₆-C₃₀ aryloxy group, a C₆-C₃₀ arylthio group, a C₁-C₃₀ heteroaryloxy group, a C₁-C₃₀ heteroarylthio group, a monovalent non-aromatic condensed polycyclic group, a monovalent non-aromatic condensed heteropolycyclic group, a pyrrolyl group, a thiophenyl group, a furanyl group, an imidazolyl group, a pyrazolyl group, a thiazolyl group, an isothiazolyl group, an oxazolyl group, an isoxazolyl group, a pyridinyl group, a pyrazinyl group, a pyrimidinyl group, a pyridazinyl group, an isoindolyl group, an indolyl group, an indazolyl group, a purinyl group, a quinolinyl group, an isoquinolinyl group, a benzoquinolinyl group, a phthalazinyl group, a naphthyridinyl group, a quinoxalinyl group, a quinazolinyl group, a cinnolinyl group, a carbazolyl group, a phenanthridinyl group, an acridinyl group, a phenanthrolinyl group, a phenazinyl group, a benzimidazolyl group, a benzofuranyl group, a benzothiophenyl group, an isobenzothiazolyl group, a benzoxazolyl group, an isobenzoxazolyl group, a triazolyl group, a tetrazolyl group, an oxadiazolyl group, a dibenzofuranyl group, a dibenzothiophenyl group, a benzocarbazolyl group, a dibenzocarbazolyl group, a thiadiazolyl group, and an imidazopyridinyl group.

For example, in Formulae 1-1 to 1-5, L₁₁ₐ to L_{11f}, L₁₂ₐ to L_{12f}, L₁₃ₐ to L_{13f}, L₁₄ₐ to L_{14f}, and L₁₅ₐ to L_{15f} may each independently be selected from:
a phenylene group, a pentalenylene group, an indenylene group, a naphthylene group, an azulenylene group, a heptalenylene group, an indacenylene group, an acenaphthylene group, a fluorenylene group, a spiro-fluorenylene group, a benzofluorenylene group, a dibenzofluorenylene group, a phenalenylene group, a phenanthrenylene group, an anthracenylene group, a fluoranthenylene group, a triphenylenylene group, a pyrenylene group, a chrysenylene group, a naphthacenylene group, a picenylene group, a perylenylene group, a pentaphenylene group, a hexacenylene group, a pentacenylene group, a rubicenylene group, a coronenylene group, an ovalenylene group, a pyrrolylene group, a thiophenylene group, a furanylene group, an imidazolylene group, a pyrazolylene group, a thiazolylene group, an isothiazolylene group, an oxazolylene group, an isoxazolylene group, a pyridinylene group, a pyrazinylene group, a pyrimidinylene group, a pyridazinylene group, an isoindolylene group, an indolylene group, an indazolylene group, a purinylene group, a quinolinylene group, an isoquinolinylene group, a benzoquinolinylene group, a phthalazinylene group, a naphthyridinylene group, a quinoxalinylene group, a quinazolinylene group, a cinnolinylene group, a carbazolylene group, a phenanthridinylene group, an acridinylene group, a phenanthrolinylene group, a phenazinylene group, a benzimidazolylene group, a benzofuranylene group, a benzothiophenylene group, an isobenzothiazolylene group, a benzoxazolylene group, an isobenzoxazolylene group, a triazolylene group, a tetrazolylene group, an oxadiazolylene group, a dibenzofuranylene group, a dibenzothiophenylene group, a benzofuropyridinylene group, a benzothienopyridinylene group, a benzofuropyrimidinylene group, a benzothienopyrimidinylene group, a benzocarbazolylene group, and a dibenzocarbazolylene group; and
a phenylene group, a pentalenylene group, an indenylene group, a naphthylene group, an azulenylene group, a heptalenylene group, an indacenylene group, an acenaphthylene group, a fluorenylene group, a spiro-fluorenylene group, a benzofluorenylene group, a dibenzofluorenylene group, a phenalenylene group, a phenanthrenylene group, an anthracenylene group, a fluoranthenylene group, a triphenylenylene group, a pyrenylene group, a chrysenylene group, a naphthacenylene group, a picenylene group, a perylenylene group, a pentaphenylene group, a hexacenylene group, a pentacenylene group, a rubicenylene group, a coronenylene group, an ovalenylene group, a pyrrolylene group, a thiophenylene group, a furanylene group, an imidazolylene group, a pyrazolylene group, a thiazolylene group, an isothiazolylene group, an oxazolylene group, an isoxazolylene group, a pyridinylene group, a pyrazinylene group, a pyrimidinylene group, a pyridazinylene group, an isoindolylene group, an indolylene group, an indazolylene group, a purinylene group, a quinolinylene group, an isoquinolinylene group, a benzoquinolinylene group, a phthalazinylene group, a naphthyridinylene group, a quinoxalinylene group, a quinazolinylene group, a cinnolinylene group, a carbazolylene group, a phenanthridinylene group, an acridinylene group, a phenanthrolinylene group, a phenazinylene group, a benzimidazolylene group, a benzofuranylene group, a benzothiophenylene group, an isobenzothiazolylene group, a benzoxazolylene group, an isobenzoxazolylene group, a triazolylene group, a tetrazolylene group, an oxadiazolylene group, a dibenzofuranylene group, a dibenzothiophenylene group, a benzofuropyridinylene group, a benzothienopyridinylene group, a benzofuropyrimidinylene group, a benzothienopyrimidinylene group, a benzocarbazolylene group, and a dibenzocarbazolylene group, each substituted with at least one selected from deuterium, -F, -Cl, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₂₀ alkyl group, a C₁-C₂₀ alkoxy group, a cyclopentyl group, a cyclohexyl group, a cycloheptyl group, a cyclopentenyl group, a cyclohexenyl group, a phenyl group, a pentalenyl group, an indenyl group, a naphthyl group, an azulenyl group, a heptalenyl group, an indacenyl group, an acenaphthyl group, a fluorenyl group, a spiro-fluorenyl group, a benzofluorenyl group, a dibenzofluorenyl group, a phenalenyl group, a phenanthrenyl group, an anthracenyl group, a fluoranthenyl group, a triphenylenyl group, a pyrenyl group, a chrysenyl group, a naphthacenyl group, a picenyl group, a perylenyl group, a pentaphenyl group, a hexacenyl group, a pentacenyl group, a rubicenyl group, a coronenyl group, an ovalenyl group, a pyrrolyl group, a thiophenyl group, a furanyl group, an imidazolyl group, a pyrazolyl group, a thiazolyl group, an isothiazolyl group, an oxazolyl group, an isoxazolyl group, a pyridinyl group, a pyrazinyl group, a pyrimidinyl group, a pyridazinyl group, an isoindolyl group, an indolyl group, an indazolyl group, a purinyl group, a quinolinyl group, an isoquinolinyl group, a benzoquinolinyl group, a phthalazinyl group, a naphthyridinyl group, a quinoxalinyl group, a quinazolinyl group, a cinnolinyl group, a carbazolyl group, a phenanthridinyl group, an acridinyl group, a phenanthrolinyl group, a phenazinyl group, a benzimidazolyl group, a benzofuranyl group, a benzothiophenyl group, an isobenzothiazolyl group, a benzoxazolyl group, an isobenzoxazolyl group, a triazolyl group, a tetrazolyl group, an oxadiazolyl group, a dibenzofuranyl group, a dibenzothiophenyl group, a benzocarbazolyl group, a dibenzocarbazolyl group, a thiadiazolyl group, and an imidazopyridinyl group, but embodiments of the present disclosure are not limited thereto.

In one embodiment, in Formulae 1-1 to 1-5, L₁₁ₐ to L_{11f}, L₁₂ₐ to L_{12f}, L₁₃ₐ to L_{13f}, L₁₄ₐ to L_{14f}, and L₁₅ₐ to L_{15f} may each independently be selected from:
a phenylene group, a naphthylene group, a fluorenylene group, a phenanthrenylene group, an anthracenylene group, a triphenylenylene group, a pyrrolylene group, a thiophenylene group, a furanylene group, a pyridinylene group, a pyrazinylene group, a pyrimidinylene group, an indolylene group, a quinolinylene group, an isoquinolinylene group, a benzoquinolinylene group, a naphthyridinylene group, a quinoxalinylene group, a quinazolinylene group, a cinnolinylene group, a carbazolylene group, a phenanthridinylene group, a benzimidazolylene group, a benzofuranylene group, a benzothiophenylene group, a triazolylene group, a dibenzofuranylene group, a dibenzothiophenylene group, a benzofuropyridinylene group, a benzothienopyridinylene group, a benzofuropyrimidinylene group and a benzothienopyrimidinylene group; and
a phenylene group, a naphthylene group, a fluorenylene group, a phenanthrenylene group, an anthracenylene group, a triphenylenylene group, a pyrrolylene group, a thiophenylene group, a furanylene group, a pyridinylene group, a pyrazinylene group, a pyrimidinylene group, an indolylene group, a quinolinylene group, an isoquinolinylene group, a benzoquinolinylene group, a naphthyridinylene group, a quinoxalinylene group, a quinazolinylene group, a cinnolinylene group, a carbazolylene group, a phenanthridinylene group, a benzimidazolylene group, a benzofuranylene group, a benzothiophenylene group, a triazolylene group, a dibenzofuranylene group, a dibenzothiophenylene group, a benzofuropyridinylene group, a benzothienopyridinylene group, a benzofuropyrimidinylene group, and a benzothienopyrimidinylene group, each substituted with at least one selected from hydrogen, deuterium, -F, -Cl, -Br, -I, a hydroxyl group, a cyano group, a nitro group, an amino group, an amidino group, a hydrazine group, a hydrazone group, a carboxyl group or a salt thereof, a sulfonic acid group or a salt thereof, a phosphoric acid group or a salt thereof, a C₁-C₂₀ alkyl group, a C₁-C₂₀ alkoxy group, a phenyl group, a naphthyl group, a fluorenyl group, a spiro-fluorenyl group, a benzofluorenyl group, a dibenzofluorenyl group, a phenanthrenyl group, an anthracenyl group, a pyrenyl group, a chrysenyl group, a pyridinyl group, a pyrazinyl group, a pyrimidinyl group, a pyridazinyl group, a quinolinyl group, an isoquinolinyl group, a quinoxalinyl group, a quinazolinyl group, and a carbazolyl group, but embodiments of the present disclosure are not limited thereto.

In Formulae 1-1 to 1-5, a11a to a11f, a12a to a12f, a13a to a13f, a14a to a14f, and a15a to a15f are each independently selected from 0, 1, 2, 3, and 4. In Formulae 1-1 to 1-5, when a11a to a11f, a12a to a12f, a13a to a13f, a14a to a14f, and a15a to a15f are each 0, (L₁₁ₐ)ₐ₁₁ₐ to (L_{11f})_{a11f}, (L₁₂ₐ)ₐ₁₂ₐ to (L_{12f})_{a12f}, (L₁₃ₐ)ₐ₁₃ₐ to (L_{13f})_{a13f} to (L₁₄ₐ)ₐ₁₄ₐ to (L_{14f})_{a14f}, and (L₁₅ₐ)ₐ₁₅ₐ to (L_{15f})_{a15f} are each a single bond. When a11a to a11f, a12a to a12f, a13a to a13f, a14a to a14f, and a15a to a15f are each two or more, a plurality of L₁₁ₐ to L_{11f}, L₁₂ₐ to L_{12f}, L₁₃ₐ to L₁₃f, L₁₄ₐ to L_{14f}, and L₁₅ₐ to L_{15f} may be identical to or different from each other.

For example, in Formulae 1-1 to 1-5, a11a to a11f, a12a to a12f, a13a to a13f, a14a to a14f, and a15a to a15f may each independently be selected from 0, 1, and 2, but embodiments of the present disclosure are not limited thereto.

In Formulae 1-1 to 1-5, R₁₁ₐ to R_{11f}, R₁₂ₐ to R_{12f}, R₁₃ₐ to R_{13f}, R₁₄ₐ to R_{14f}, and R₁₅ₐ to R_{15f} are each independently selected from:
hydrogen, deuterium, -F, -Cl, -Br, -I, a methyl group, an ethyl group, an n-propyl group, an isopropyl group, an n-butyl group, an isobutyl group, a sec-butyl group, a tert-butyl group, a methoxy group, an ethoxy group, an n-propoxy group, an isopropoxy group, an n-butoxy group, an isobutoxy group, a sec-butoxy group, and a tert-butoxy group;
a methyl group, an ethyl group, an n-propyl group, an isopropyl group, an n-butyl group, an isobutyl group, a sec-butyl group, and a tert-butyl group, each substituted with at least one selected from deuterium, -F, -Cl, -Br, and -I;
a phenyl group, a biphenyl group, a terphenyl group, a naphthyl group, a fluorenyl group, a carbazolyl group, a dibenzofuranyl group, a dibenzothiophenyl group, a pyridinyl group, a pyrimidinyl group, a quinolinyl group, an isoquinolinyl group, a quinoxalinyl group, a quinazolinyl group, a benzofuropyridinyl group, a benzothienopyridinyl group, a benzofuropyrimidinyl group, a benzothienopyrimidinyl group, a monovalent non-aromatic condensed polycyclic group, and a monovalent non-aromatic condensed heteropolycyclic group;
a phenyl group, a biphenyl group, a terphenyl group, a naphthyl group, a fluorenyl group, a carbazolyl group, a dibenzofuranyl group, a dibenzothiophenyl group, a pyridinyl group, a pyrimidinyl group, a quinolinyl group, an isoquinolinyl group, a quinoxalinyl group, a quinazolinyl group, a benzofuropyridinyl group, a benzothienopyridinyl group, a benzofuropyrimidinyl group, a benzothienopyrimidinyl group, a monovalent non-aromatic condensed polycyclic group, and a monovalent non-aromatic condensed heteropolycyclic group, each substituted with at least one selected from deuterium, -F, -Cl, -Br, -I, a methyl group, an ethyl group, an n-propyl group, an isopropyl group, an n-butyl group, an isobutyl group, a sec-butyl group, a tert-butyl group, a methoxy group, an ethoxy group, an n-propoxy group, an isopropoxy group, an n-butoxy group, an isobutoxy group, a sec-butoxy group, a tert-butoxy group, a phenyl group, a biphenyl group, a terphenyl group, a naphthyl group, a fluorenyl group, a carbazolyl group, a dibenzofuranyl group, a dibenzothiophenyl group, a pyridinyl group, a pyrimidinyl group, a quinolinyl group, an isoquinolinyl group, a quinoxalinyl group, a quinazolinyl group, a benzofuropyridinyl group, a benzothienopyridinyl group, a benzofuropyrimidinyl group, and a benzothienopyrimidinyl group; and
a phenyl group, a biphenyl group, a terphenyl group, a naphthyl group, a fluorenyl group, a carbazolyl group, a dibenzofuranyl group, a dibenzothiophenyl group, a pyridinyl group, a pyrimidinyl group, a quinolinyl group, an isoquinolinyl group, a quinoxalinyl group, a quinazolinyl group, a benzofuropyridinyl group, a benzothienopyridinyl group, a benzofuropyrimidinyl group, a benzothienopyrimidinyl group, a monovalent non-aromatic condensed polycyclic group, and a monovalent non-aromatic condensed heteropolycyclic group, each substituted with at least one selected from a methyl group, an ethyl group, an n-propyl group, an isopropyl group, an n-butyl group, an isobutyl group, a sec-butyl group, a tert-butyl group, a phenyl group, a biphenyl group, a terphenyl group, a naphthyl group, a fluorenyl group, a carbazolyl group, a dibenzofuranyl group, a dibenzothiophenyl group, a pyridinyl group, a pyrimidinyl group, a quinolinyl group, an isoquinolinyl group, a quinoxalinyl group, a quinazolinyl group, a benzofuropyridinyl group, a benzothienopyridinyl group, a benzofuropyrimidinyl group, and a benzothienopyrimidinyl group that are each substituted with at least one selected from deuterium, -F, -Cl, -Br, and -I, and
neighboring atoms of R₁₁ₐ to R_{11f}, neighboring atoms of R₁₂ₐ to R_{12f}, neighboring R₁₃ₐ to R_{13f}, neighboring atoms of R₁₄ₐ to R_{14f}, and/or atoms of neighboring R₁₅ₐ to R_{15f} optionally form a saturated ring or an unsaturated ring.

For example, in Formulae 1-1 to 1-5, R₁₁ₐ to R_{11f}, R₁₂ₐ to R_{12f}, R₁₃ₐ to R_{13f}, R₁₄ₐ to R_{14f}, and R₁₅ₐ to R_{15f} may each independently be selected from:
hydrogen, deuterium, -F, -Cl, -Br, -I, a methyl group, an ethyl group, an n-propyl group, an isopropyl group, an n-butyl group, an isobutyl group, a sec-butyl group, a tert-butyl group, a methoxy group, an ethoxy group, an n-propoxy group, an isopropoxy group, an n-butoxy group, an isobutoxy group, a sec-butoxy group, and a tert-butoxy group;
a methyl group, an ethyl group, an n-propyl group, an isopropyl group, an n-butyl group, an isobutyl group, a sec-butyl group, and a tert-butyl group, each substituted with at least one selected from deuterium, -F, -Cl, -Br and -I;
a phenyl group, a biphenyl group, a terphenyl group, a naphthyl group, a fluorenyl group, a carbazolyl group, a dibenzofuranyl group, a dibenzothiophenyl group, a pyridinyl group, a pyrimidinyl group, a quinolinyl group, an isoquinolinyl group, a quinoxalinyl group, a quinazolinyl group, a benzofuropyridinyl group, a benzothienopyridinyl group, a benzofuropyrimidinyl group and a benzothienopyrimidinyl group;
a phenyl group, a biphenyl group, a terphenyl group, a naphthyl group, a fluorenyl group, a carbazolyl group, a dibenzofuranyl group, a dibenzothiophenyl group, a pyridinyl group, a pyrimidinyl group, a quinolinyl group, an isoquinolinyl group, a quinoxalinyl group, a quinazolinyl group, a benzofuropyridinyl group, a benzothienopyridinyl group, a benzofuropyrimidinyl group and a benzothienopyrimidinyl group, each substituted with at least one selected from deuterium, -F, -Cl, -Br, -I, a methyl group, an ethyl group, an n-propyl group, an isopropyl group, an n-butyl group, an isobutyl group, a sec-butyl group, a tert-butyl group, a methoxy group, an ethoxy group, an n-propoxy group, an isopropoxy group, an n-butoxy group, an isobutoxy group, a sec-butoxy group, a tert-butoxy group, a phenyl group, a biphenyl group, a terphenyl group, a naphthyl group, a fluorenyl group, a carbazolyl group, a dibenzofuranyl group, a dibenzothiophenyl group, a pyridinyl group, a pyrimidinyl group, a quinolinyl group, an isoquinolinyl group, a quinoxalinyl group, a quinazolinyl group, a benzofuropyridinyl group, a benzothienopyridinyl group, a benzofuropyrimidinyl group, and a benzothienopyrimidinyl group;
a phenyl group, a biphenyl group, a terphenyl group, a naphthyl group, a fluorenyl group, a carbazolyl group, a dibenzofuranyl group, a dibenzothiophenyl group, a pyridinyl group, a pyrimidinyl group, a quinolinyl group, an isoquinolinyl group, a quinoxalinyl group, a quinazolinyl group, a benzofuropyridinyl group, a benzothienopyridinyl group, a benzofuropyrimidinyl group and a benzothienopyrimidinyl group, each substituted with at least one selected from a methyl group, an ethyl group, an n-propyl group, an isopropyl group, an n-butyl group, an isobutyl group, a sec-butyl group, a tert-butyl group, a phenyl group, a biphenyl group, a terphenyl group, a naphthyl group, a fluorenyl group, a carbazolyl group, a dibenzofuranyl group, a dibenzothiophenyl group, a pyridinyl group, a pyrimidinyl group, a quinolinyl group, an isoquinolinyl group, a quinoxalinyl group, a quinazolinyl group, a benzofuropyridinyl group, a benzothienopyridinyl group, a benzofuropyrimidinyl group and a benzothienopyrimidinyl group that are each substituted with at least one selected from deuterium, -F, -Cl, -Br, and -I; and
a partial structure represented by one selected from Formulae 2-1 to 2-17, but embodiments of the present disclosure are not limited thereto: 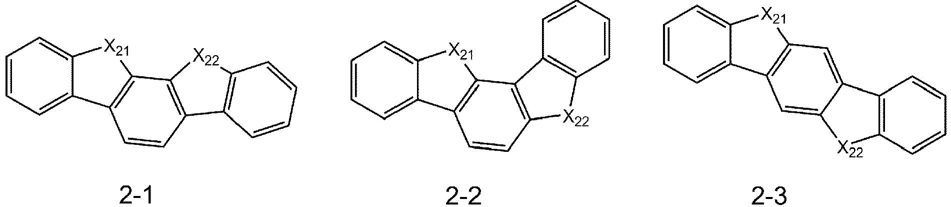 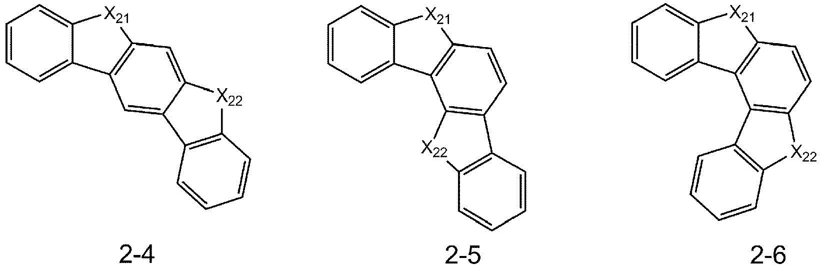 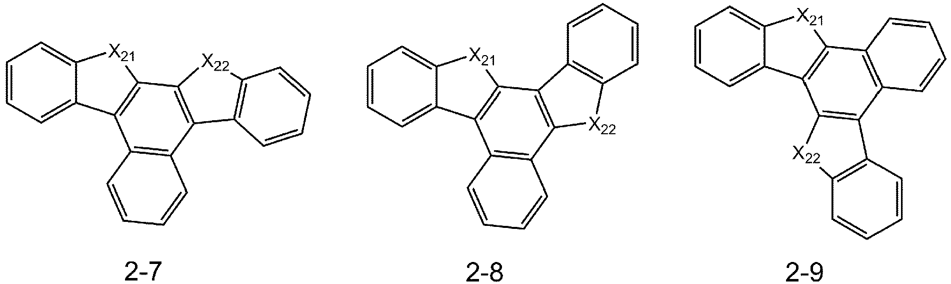 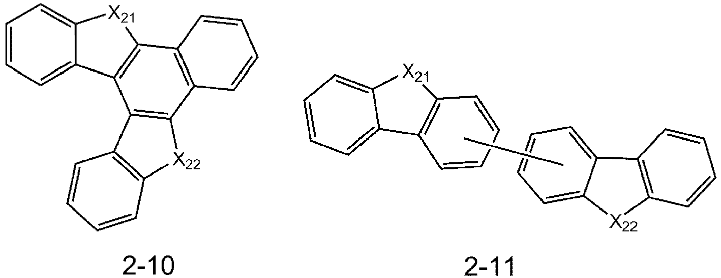 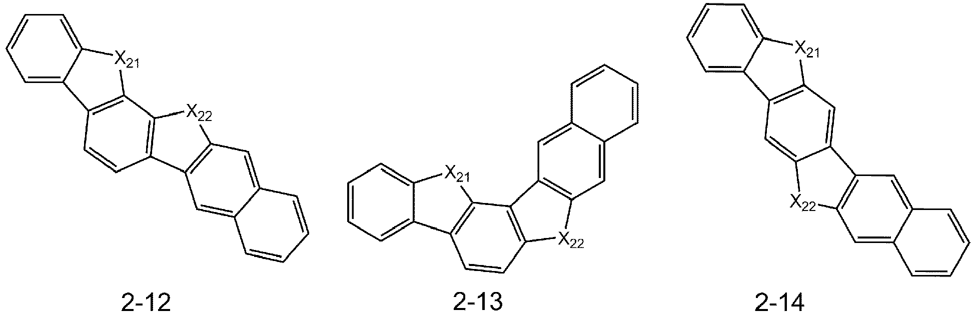 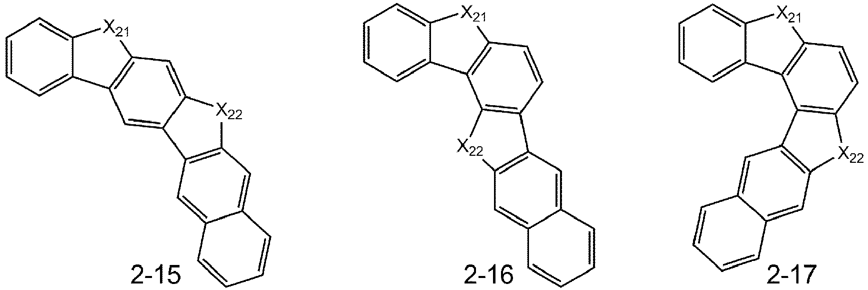
In Formulae 2-1 to 2-17,
X₂₁ is selected from C(R₂₁)(R₂₂), N(R₂₁), O, and S,
X₂₂ is selected from C(R₂₃)(R₂₄), N(R₂₃), O, and S,
R₂₁ to R₂₄ are each independently selected from:
   a methyl group, an ethyl group, an n-propyl group, an isopropyl group, an n-butyl group, an isobutyl group, a sec-butyl group, and a tert-butyl group;
   a methyl group, an ethyl group, an n-propyl group, an isopropyl group, an n-butyl group, an isobutyl group, a sec-butyl group, and a tert-butyl group, each substituted with at least one selected from deuterium, -F, -Cl, -Br, and -I;
   a phenyl group, a biphenyl group, a terphenyl group, a naphthyl group, a fluorenyl group, a carbazolyl group, a dibenzofuranyl group, a dibenzothiophenyl group, a pyridinyl group, a pyrimidinyl group, a quinolinyl group, an isoquinolinyl group, a quinoxalinyl group, a quinazolinyl group, a benzofuropyridinyl group, a benzothienopyridinyl group, a benzofuropyrimidinyl group and a benzothienopyrimidinyl group; and
   a phenyl group, a biphenyl group, a terphenyl group, a naphthyl group, a fluorenyl group, a carbazolyl group, a dibenzofuranyl group, a dibenzothiophenyl group, a pyridinyl group, a pyrimidinyl group, a quinolinyl group, an isoquinolinyl group, a quinoxalinyl group, a quinazolinyl group, a benzofuropyridinyl group, a benzothienopyridinyl group, a benzofuropyrimidinyl group, and a benzothienopyrimidinyl group, each substituted with at least one selected from deuterium, -F, -Cl, -Br, -I, a methyl group, an ethyl group, an n-propyl group, an isopropyl group, an n-butyl group, an isobutyl group, a sec-butyl group, a tert-butyl group, a methoxy group, an ethoxy group, an n-propoxy group, an isopropoxy group, an n-butoxy group, an isobutoxy group, a sec-butoxy group, a tert-butoxy group, a phenyl group, a biphenyl group, a terphenyl group, a naphthyl group, a fluorenyl group, a carbazolyl group, a dibenzofuranyl group, a dibenzothiophenyl group, a pyridinyl group, a pyrimidinyl group, a quinolinyl group, an isoquinolinyl group, a quinoxalinyl group, a quinazolinyl group, a benzofuropyridinyl group, a benzothienopyridinyl group, a benzofuropyrimidinyl group and a benzothienopyrimidinyl group; and
   one selected from a carbon atom and a nitrogen atom that constitute a partial structure represented by one selected from Formulae 2-1 to 2-17 indicates a binding site to (e.g., is chemically bonded to) a neighboring atom (e.g., a neighboring atom selected from Formula 1-1 to 1-5).

In the specification, the term "a compound is represented by a partial structure" and/or "a compound includes a partial structure" as used herein refers to a case where a compound includes a substituent (for example, a monovalent or divalent substituent) having the partial structure, or a case where the partial structure further includes an additional substituent. For example, in some embodiments, the compounds described herein may include the partial structure as a substituent (noting that the partial structure may be further substituted), where the partial structure is chemically bonded to the remaining portion of the compound (e.g., is chemically bonded to a neighboring atom selected from Formula 1-1 to 1-5) through a carbon atom or nitrogen atom of the partial structure.

In one embodiment, in Formulae 1-1 to 1-5, R₁₁ₐ to R_{11f}, R₁₂ₐ to R_{12f}, R₁₃ₐ to R_{13f}, R₁₄ₐ to R_{14f}, and R₁₅ₐ to R_{15f} may each independently be selected from:
hydrogen, deuterium, -F, -Cl, -Br, -I, a methyl group, an ethyl group, an n-propyl group, an isopropyl group, an n-butyl group, an isobutyl group, a sec-butyl group, and a tert-butyl group;
a phenyl group, a biphenyl group, a terphenyl group, a naphthyl group, a fluorenyl group, a carbazolyl group, a dibenzofuranyl group, a dibenzothiophenyl group, a pyridinyl group, a pyrimidinyl group, a quinolinyl group, an isoquinolinyl group, a quinoxalinyl group, and a quinazolinyl group;
a phenyl group, a biphenyl group, a terphenyl group, a naphthyl group, a fluorenyl group, a carbazolyl group, a dibenzofuranyl group, a dibenzothiophenyl group, a pyridinyl group, a pyrimidinyl group, a quinolinyl group, an isoquinolinyl group, a quinoxalinyl group, and a quinazolinyl group, each substituted with at least one selected from deuterium, -F, -Cl, -Br, -I, a methyl group, an ethyl group, an n-propyl group, an isopropyl group, an n-butyl group, an isobutyl group, a sec-butyl group, a tert-butyl group, a phenyl group, a biphenyl group, a terphenyl group, a naphthyl group, a fluorenyl group, a carbazolyl group, a dibenzofuranyl group, a dibenzothiophenyl group, a pyridinyl group, a pyrimidinyl group, a quinolinyl group, an isoquinolinyl group, a quinoxalinyl group, and a quinazolinyl group; and
a partial structure represented by one selected from Formulae 2-1 to 2-17, but embodiments of the present disclosure are not limited thereto: 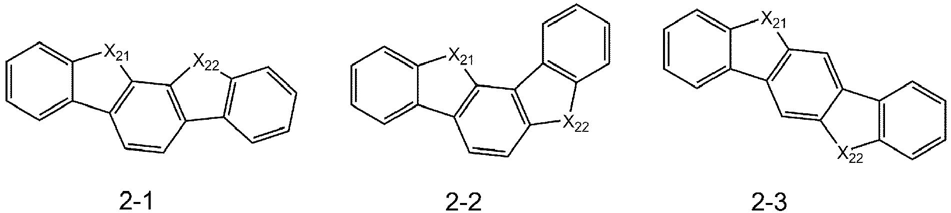 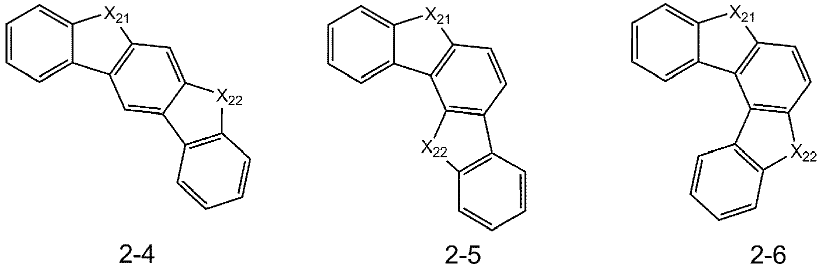 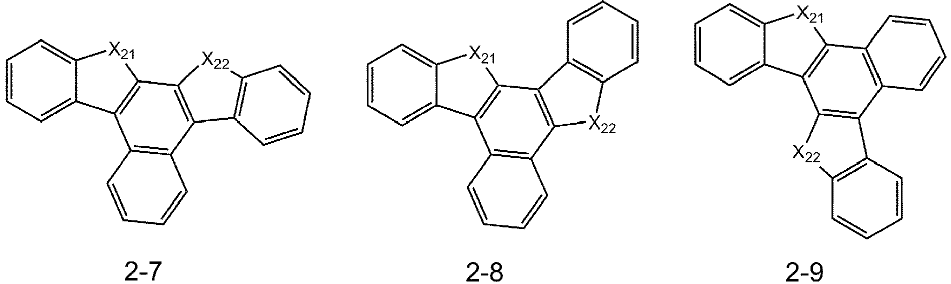 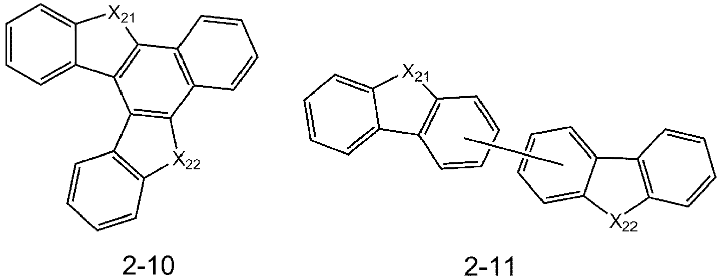 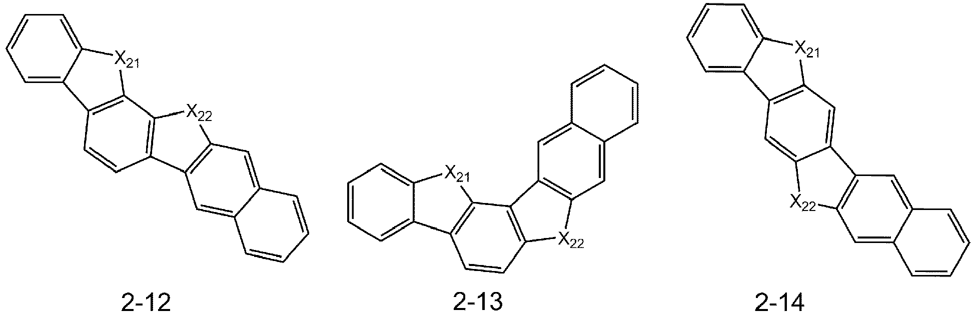 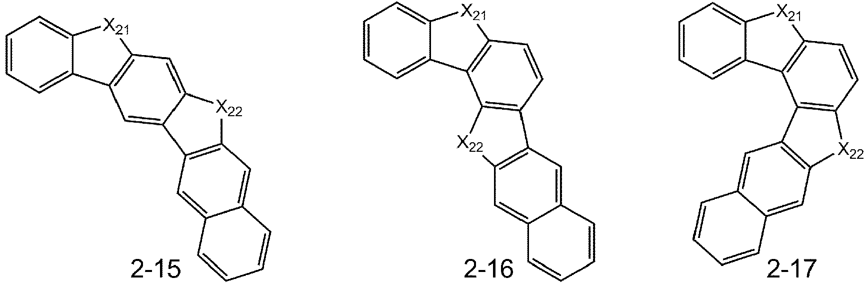
In Formulae 2-1 to 2-17,
X₂₁ is selected from C(R₂₁)(R₂₂), N(R₂₁), O, and S,
X₂₂ is selected from C(R₂₃)(R₂₄), N(R₂₃), O, and S,
R₂₁ to R₂₄ are each independently selected from:
a methyl group, an ethyl group, an n-propyl group, an isopropyl group, an n-butyl group, an isobutyl group, a sec-butyl group, and a tert-butyl group;
a methyl group, an ethyl group, an n-propyl group, an isopropyl group, an n-butyl group, an isobutyl group, a sec-butyl group, and a tert-butyl group, each substituted with at least one selected from deuterium, -F, -Cl, -Br, and -I; and
a phenyl group, a biphenyl group, a terphenyl group, a naphthyl group, a fluorenyl group, a carbazolyl group, a dibenzofuranyl group, a dibenzothiophenyl group, a pyridinyl group, a pyrimidinyl group, a quinolinyl group, an isoquinolinyl group, a quinoxalinyl group, and a quinazolinyl group; and
one selected from a carbon atom and a nitrogen atom that constitute a partial structure represented by one selected from Formulae 2-1 to 2-17 indicates a binding site to (e.g., is chemically bonded to) a neighboring atom (e.g., a neighboring atom selected from Formula 1-1 to 1-5).

In an embodiment, when (L₁₁ₐ)ₐ₁₁ₐ to (L_{11f})_{a11f} (when present) are each a single bond, at least one of R₁₁ₐ to R_{11f} (when present) is not hydrogen. In an embodiment, when (L₁₂ₐ)ₐ₁₂ₐ to (L_{12f})_{a12f} (when present) are each a single bond, at least one of R₁₂ₐ to R_{12f} (when present) is not hydrogen. In an embodiment, when (L₁₃ₐ)ₐ₁₃ₐ to (L_{13f})_{a13f} (when present) are each a single bond, at least one of R₁₃ₐ to R₁₃f (when present) is not hydrogen. In an embodiment, when (L₁₄ₐ)ₐ₁₄ₐ to (L_{14f})_{a14f} (when present) are each a single bond, at least one of R₁₄ₐ to R_{14f} (when present) is not hydrogen. In an embodiment, when (L₁₅ₐ)ₐ₁₅ₐ to (L_{15f})_{a15f} (when present) are each a single bond, at least one of R₁₅ₐ to R_{15f} (when present) is not hydrogen.

In one embodiment, the first compound, the second compound, and the third compound may each independently be an azine-based compound represented by one selected from Formulae 1-11, 1-21 to 1-25, 1-31 to 1-33, 1-41, 1-42, and 1-51, except for a case where the first compound, the second compound, and the third compound concurrently (e.g., simultaneously) include an azine-based compound represented by one selected from Formulae 1-31 to 1-33 (e.g., only one or two selected from the first compound, the second compound, and the third compound is represented by one selected from Formulae 1-31 to 1-33): 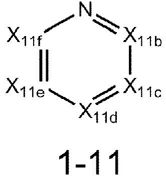 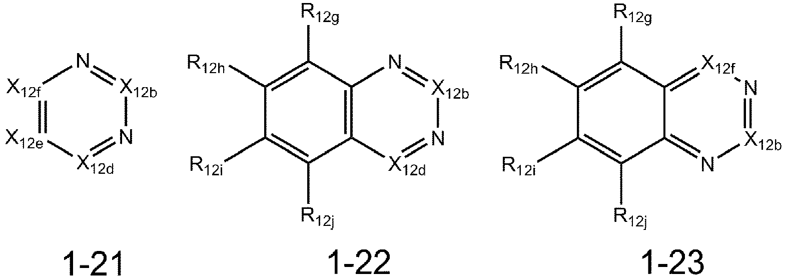 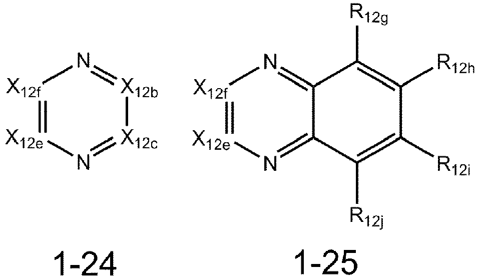 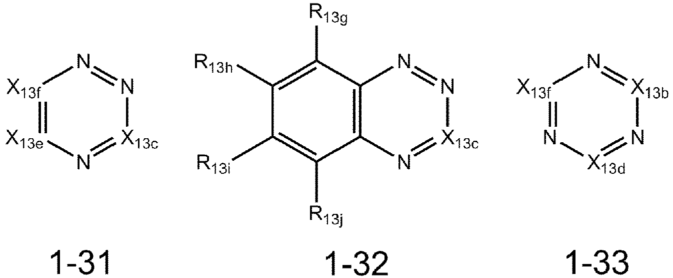 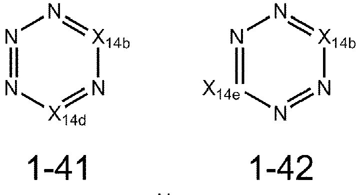 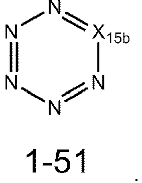
In Formulae 1-11, 1-21 to 1-25, 1-31 to 1-33, 1-41, 1-42, and 1-51,
X_{11b} to X_{11f} are each independently defined the same as Formula 1-1,
X_{12b} to X_{12f} are each independently defined the same as Formula 1-2,
X_{13b} to X_{13f} are each independently defined the same as Formula 1-3,
X_{14b}, X_{14d}, and X₁₄ₑ are each independently defined the same as Formula 1-4,
X_{15b} is defined the same as Formula 1-5, and
R_{12g} to R₁₂ⱼ and R_{13g} to R₁₃ⱼ are each independently selected from:
   hydrogen, deuterium, -F, -Cl, -Br, -I, a methyl group, an ethyl group, an n-propyl group, an isopropyl group, an n-butyl group, an isobutyl group, a sec-butyl group, a tert-butyl group, a methoxy group, an ethoxy group, an n-propoxy group, an isopropoxy group, an n-butoxy group, an isobutoxy group, a sec-butoxy group, a tert-butoxy group, a phenyl group, a biphenyl group, a terphenyl group, a naphthyl group, a fluorenyl group, a carbazolyl group, a dibenzofuranyl group, a dibenzothiophenyl group, a pyridinyl group, a pyrimidinyl group, a quinolinyl group, an isoquinolinyl group, a quinoxalinyl group, a quinazolinyl group, a benzofuropyridinyl group, a benzothienopyridinyl group, a benzofuropyrimidinyl group, and a benzothienopyrimidinyl group; and
   a methyl group, an ethyl group, an n-propyl group, an isopropyl group, an n-butyl group, an isobutyl group, a sec-butyl group, a tert-butyl group, a phenyl group, a biphenyl group, a terphenyl group, a naphthyl group, a fluorenyl group, a carbazolyl group, a dibenzofuranyl group, a dibenzothiophenyl group, a pyridinyl group, a pyrimidinyl group, a quinolinyl group, an isoquinolinyl group, a quinoxalinyl group, a quinazolinyl group, a benzofuropyridinyl group, a benzothienopyridinyl group, a benzofuropyrimidinyl group, and a benzothienopyrimidinyl group that are each substituted with at least one selected from deuterium, -F, -Cl, -Br, and -I.

In one embodiment, the first compound may further include a partial structure represented by one selected form Formulae 2-1 to 2-17, but embodiments of the present disclosure are not limited thereto: 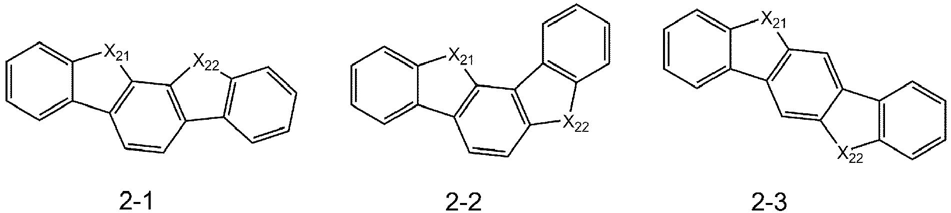 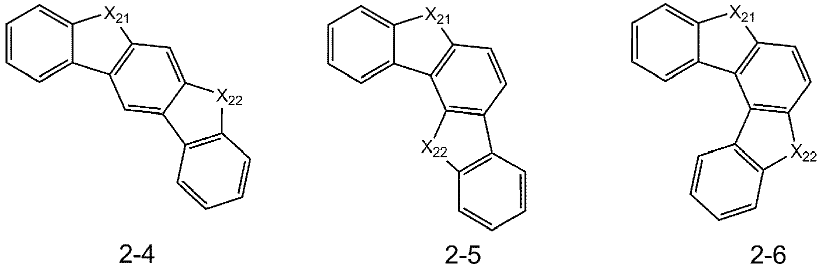 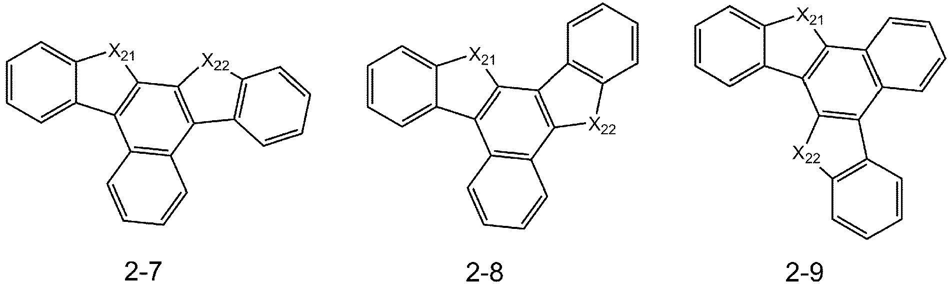 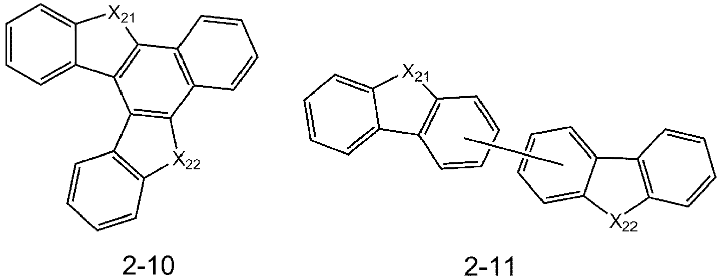 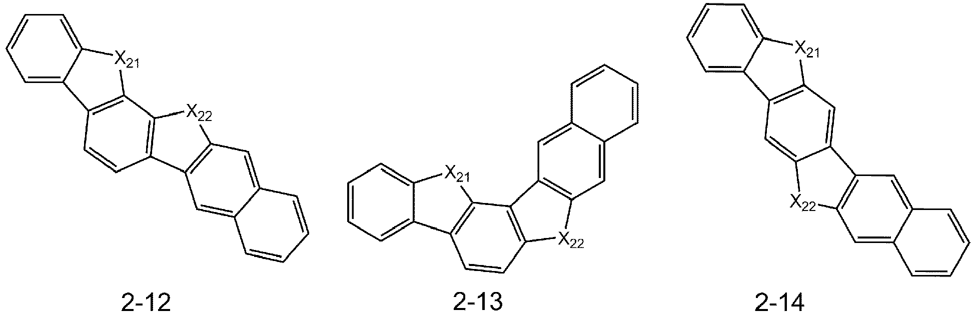 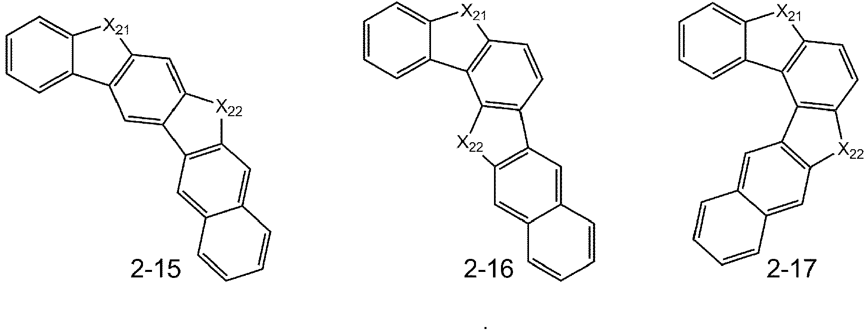
In Formulae 2-1 to 2-17,
X₂₁ is selected from C(R₂₁)(R₂₂), N(R₂₁), O, and S,
X₂₂ is selected from C(R₂₃)(R₂₄), N(R₂₃), O, and S,
R₂₁ to R₂₄ are each independently selected from:
   a methyl group, an ethyl group, an n-propyl group, an isopropyl group, an n-butyl group, an isobutyl group, a sec-butyl group, and a tert-butyl group;
   a methyl group, an ethyl group, an n-propyl group, an isopropyl group, an n-butyl group, an isobutyl group, a sec-butyl group, and a tert-butyl group, each substituted with at least one selected from deuterium, -F, -Cl, -Br, and -I;
   a phenyl group, a naphthyl group, a fluorenyl group, a carbazolyl group, a dibenzofuranyl group, a dibenzothiophenyl group, a pyridinyl group, a pyrimidinyl group, a quinolinyl group, an isoquinolinyl group, a quinoxalinyl group, a quinazolinyl group, a benzofuropyridinyl group, a benzothienopyridinyl group, a benzofuropyrimidinyl group and a benzothienopyrimidinyl group; and
   a phenyl group, a biphenyl group, a terphenyl group, a naphthyl group, a fluorenyl group, a carbazolyl group, a dibenzofuranyl group, a dibenzothiophenyl group, a pyridinyl group, a pyrimidinyl group, a quinolinyl group, an isoquinolinyl group, a quinoxalinyl group, a quinazolinyl group, a benzofuropyridinyl group, a benzothienopyridinyl group, a benzofuropyrimidinyl group, and a benzothienopyrimidinyl group, each substituted with at least one selected from deuterium, -F, -Cl, -Br, -I, a methyl group, an ethyl group, an n-propyl group, an isopropyl group, an n-butyl group, an isobutyl group, a sec-butyl group, a tert-butyl group, a methoxy group, an ethoxy group, an n-propoxy group, an isopropoxy group, an n-butoxy group, an isobutoxy group, a sec-butoxy group, a tert-butoxy group, a phenyl group, a biphenyl group, a terphenyl group, a naphthyl group, a fluorenyl group, a carbazolyl group, a dibenzofuranyl group, a dibenzothiophenyl group, a pyridinyl group, a pyrimidinyl group, a quinolinyl group, an isoquinolinyl group, a quinoxalinyl group, a quinazolinyl group, a benzofuropyridinyl group, a benzothienopyridinyl group, a benzofuropyrimidinyl group and a benzothienopyrimidinyl group; and
   one selected from a carbon atom and a nitrogen atom that constitute a partial structure represented by one selected from Formulae 2-1 to 2-17 indicates a binding site to (e.g., is chemically bonded to) a neighboring atom (e.g., a neighboring atom selected from Formula 1-1 to 1-5).

In one embodiment, the first compound may be selected from Compounds H-1 to H-11, but embodiments of the present disclosure are not limited thereto:

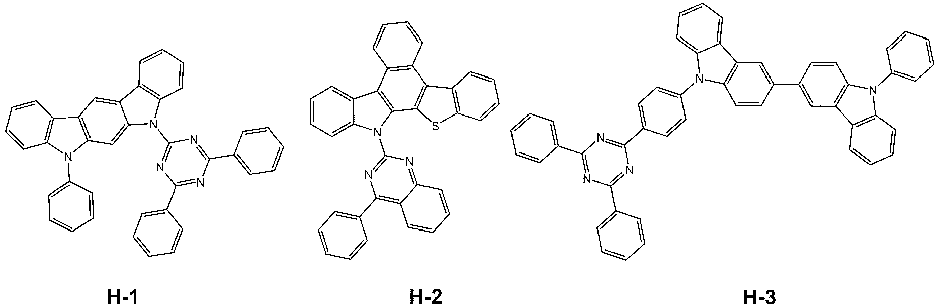

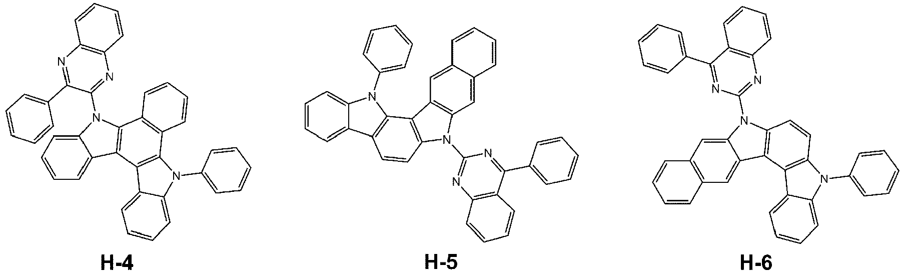

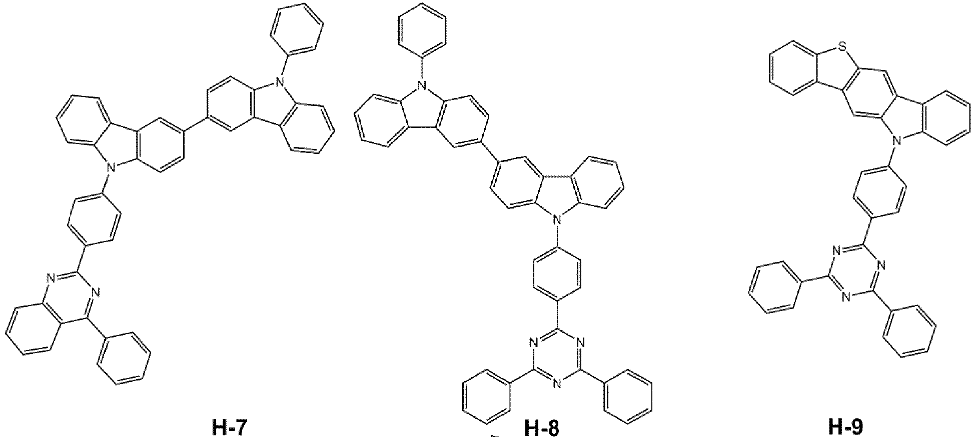

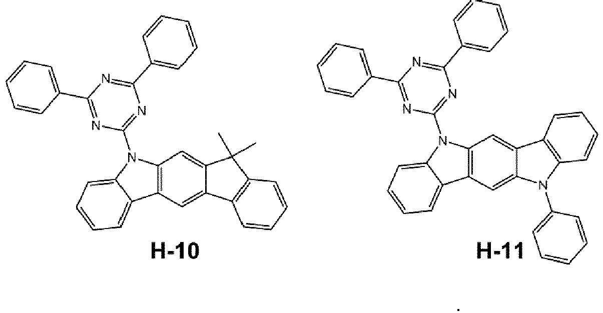

In one embodiment, the second compound may be selected from Compounds HB-1 to HB-3, but embodiments of the present disclosure are not limited thereto:

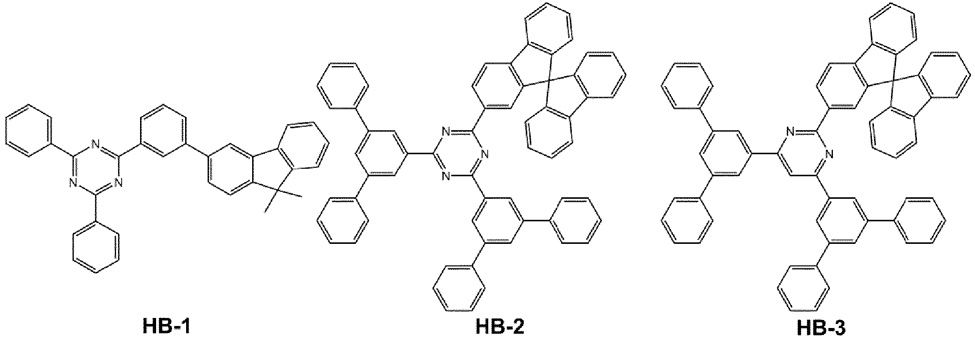

In one embodiment, the third compound may be selected from Compounds ET-1 and ET-2, but embodiments of the present disclosure are not limited thereto:

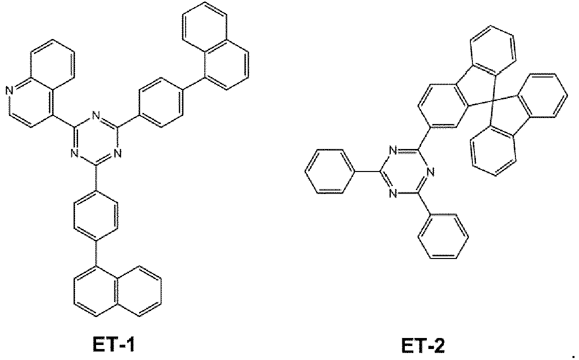

In one embodiment, the first compound, the second compound, and the third compound may each independently be an azine-based compound represented by one selected from Formulae 1-1 to 1-3, except for a case where the first compound, the second compound, and the third compound concurrently (e.g., simultaneously) include an azine-based compound represented by Formula 1-3 (e.g., only one or two selected from the first compound, the second compound, and the third compound is represented by Formula 1-3). In more detail, when the first compound, the second compound, and the third compound concurrently (e.g., simultaneously) include an azine-based compound represented by Formula 1-3, an organic light-emitting device requiring great electron transportability more than necessary may be provided, and in this regard, the lifespan of such an organic light-emitting device may be shortened.

For example, the first compound may be an azine-based compound represented by one selected from Formulae 1-1 and 1-2, and the second compound and the third compound may each independently be an azine-based compound represented by one selected from Formulae 1-2 and 1-3, but embodiments of the present disclosure are not limited thereto.

In one embodiment, the second compound may be an azine-based compound represented by one selected from Formulae 1-1 and 1-2, and the first compound and the third compound may each independently be an azine-based compound represented by one selected from Formulae 1-2 and 1-3, but embodiments of the present disclosure are not limited thereto.

In one embodiment, the third compound may be an azine-based compound represented by one selected from Formulae 1-1 and 1-2, and the first compound and the second compound may each independently be an azine-based compound represented by one selected from Formulae 1-2 and 1-3, but embodiments of the present disclosure are not limited thereto.

In one embodiment, the first compound, the second compound, and the third compound may be different from each other.

In one embodiment, the organic layer may include an electron transport region disposed between the emission layer and the second electrode, the electron transport region may include a buffer layer and an electron transport layer, the buffer layer may be disposed between the emission layer and the electron transport layer, the emission layer may include the first compound, the buffer layer may include the second compound, and the electron transport layer may include the third compound, but embodiments of the present disclosure are not limited thereto.

For example, the emission layer may directly contact the buffer layer, and the buffer layer may directly contact the electron transport layer, but embodiments of the present disclosure are not limited thereto.

In one embodiment, the first electrode may be an anode, and the second electrode may be a cathode.

In one embodiment, the emission layer may include an organometallic compound.

For example, the organometallic compound may emit phosphorescence (e.g., may phosphoresce), but embodiments of the present disclosure are not limited thereto.

In more detail, the organometallic compound may be represented by Formula 401:

Formula 401 M(L₄₀₁)_{xc1}(L₄₀₂)_{xc2}

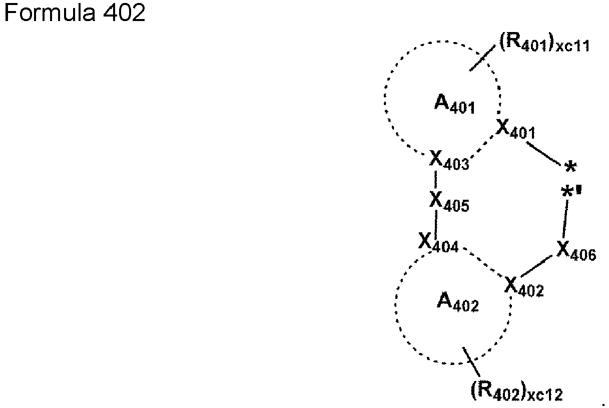

In Formulae 401 and 402,
M may be selected from iridium (Ir), platinum (Pt), palladium (Pd), osmium (Os), titanium (Ti), zirconium (Zr), hafnium (Hf), europium (Eu), terbium (Tb), rhodium (Rh), and thulium (Tm),
L₄₀₁ may be selected from ligands represented by Formula 402, and xc1 may be 1, 2, or 3, wherein, when xc1 is two or more, two or more L₄₀₁(s) may be identical to or different from each other,
L₄₀₂ may be an organic ligand, and xc2 may be an integer from 0 to 4, wherein, when xc2 is two or more, two or more L₄₀₂(s) may be identical to or different from each other,
X₄₀₁ to X₄₀₄ may each independently be nitrogen or carbon,
X₄₀₁ and X₄₀₃ may be linked via a single bond or a double bond, and X₄₀₂ and X₄₀₄ may be linked via a single bond or a double bond,
A₄₀₁ and A₄₀₂ may each independently be selected from a C₅-C₆₀ (*e.g*. C₅-C₃₀) carbocyclic group or a C₁-C₆₀ (e.g. C₁-C₂₀) heterocyclic group,
X₄₀₅ may be a single bond, *-O-*', *-S-*', *-C(=O)-*', *-N(Q₄₁₁)-*', *-C(Q₄₁₁)(Q₄₁₂)-*', *-C(Q₄₁₁)=C(Q₄₁₂)-*', *-C(Q₄₁₁)=*', or *=C(Q₄₁₁)=*', wherein Q₄₁₁ and Q₄₁₂ may each independently be hydrogen, deuterium, a C₁-C₂₀ alkyl group, a C₁-C₂₀ alkoxy group, a phenyl group, a biphenyl group, a terphenyl group or a naphthyl group,
X₄₀₆ may be a single bond, O, or S,
R₄₀₁ and R₄₀₂ may each independently be selected from hydrogen, deuterium, -F, -Cl, -Br, -I, a hydroxyl group, a cyano group, a nitro group, an amidino group, a hydrazino group, a hydrazono group, a substituted or unsubstituted C₁-C₂₀ alkyl group, a substituted or unsubstituted C₁-C₂₀ alkoxy group, a substituted or unsubstituted C₃-C₁₀ cycloalkyl group, a substituted or unsubstituted C₁-C₁₀ heterocycloalkyl group, a substituted or unsubstituted C₃-C₁₀ cycloalkenyl group, a substituted or unsubstituted C₁-C₁₀ heterocycloalkenyl group, a substituted or unsubstituted C₆-C₆₀ (*e*.*g*. C₆-C₃₀) aryl group, a substituted or unsubstituted C₆-C₆₀ (*e*.*g*. C₆-C₃₀) aryloxy group, a substituted or unsubstituted C₆-C₆₀ (*e*.*g*. C₆-C₃₀) arylthio group, a substituted or unsubstituted C₁-C₆₀ (*e*.*g*. C₁-C₂₀) heteroaryl group, a substituted or unsubstituted monovalent non-aromatic condensed polycyclic group, and a substituted or unsubstituted monovalent non-aromatic condensed heteropolycyclic group, -Si(Q₄₀₁)(Q₄₀₂)(Q₄₀₃), -N(Q₄₀₁)(Q₄₀₂), -B(Q₄₀₁)(Q₄₀₂), -C(=O)(Q₄₀₁), -S(=O)₂(Q₄₀₁), and -P(=O)(Q₄₀₁)(Q₄₀₂), wherein Q₄₀₁ to Q₄₀₃ may each independently be selected from a C₁-C₁₀ alkyl group, a C₁-C₁₀ alkoxy group, a C₆-C₂₀ aryl group, and a C₁-C₂₀ heteroaryl group,
xc11 and xc12 may each independently be an integer from 0 to 10, and
* and *' in Formula 402 each indicate a binding site to M in Formula 401.

In one embodiment, A₄₀₁ and A₄₀₂ in Formula 402 may each independently be selected from a benzene group, a naphthalene group, a fluorene group, a spiro-bifluorene group, an indene group, a pyrrole group, a thiophene group, a furan group, an imidazole group, a pyrazole group, a thiazole group, an isothiazole group, an oxazole group, an isoxazole group, a pyridine group, a pyrazine group, a pyrimidine group, a pyridazine group, a quinoline group, an isoquinoline group, a benzoquinoline group, a quinoxaline group, a quinazoline group, a carbazole group, a benzimidazole group, a benzofuran group, a benzothiophene group, an isobenzothiophene group, a benzoxazole group, an isobenzoxazole group, a triazole group, a tetrazole group, an oxadiazole group, a triazine group, a dibenzofuran group, and a dibenzothiophene group.

In one or more embodiments, in Formula 402, i) X₄₀₁ may be nitrogen, and X₄₀₂ may be carbon, or ii) X₄₀₁ and X₄₀₂ may each be nitrogen concurrently (e.g., at the same time).

In one or more embodiments, in Formula 402, R₄₀₁ and R₄₀₂ may each independently be selected from:
hydrogen, deuterium, -F, -Cl, -Br, -I, a hydroxyl group, a cyano group, a nitro group, an amidino group, a hydrazino group, a hydrazono group, a C₁-C₂₀ alkyl group, and a C₁-C₂₀ alkoxy group;
a C₁-C₂₀ alkyl group and a C₁-C₂₀ alkoxy group, each substituted with at least one selected from deuterium, -F, -Cl, -Br, -I, a hydroxyl group, a cyano group, a nitro group, an amidino group, a hydrazino group, a hydrazono group, a phenyl group, a naphthyl group, a cyclopentyl group, a cyclohexyl group, an adamantanyl group, a norbornanyl group, and a norbornenyl group;
a cyclopentyl group, a cyclohexyl group, an adamantanyl group, a norbornanyl group, a norbornenyl group, a phenyl group, a biphenyl group, a terphenyl group, a naphthyl group, a fluorenyl group, a pyridinyl group, a pyrazinyl group, a pyrimidinyl group, a pyridazinyl group, a triazinyl group, a quinolinyl group, an isoquinolinyl group, a quinoxalinyl group, a quinazolinyl group, a carbazolyl group, a dibenzofuranyl group, and a dibenzothiophenyl group;
a cyclopentyl group, a cyclohexyl group, an adamantanyl group, a norbornanyl group, a norbornenyl group a phenyl group, a biphenyl group, a terphenyl group, a naphthyl group, a fluorenyl group, a pyridinyl group, a pyrazinyl group, a pyrimidinyl group, a pyridazinyl group, a triazinyl group, a quinolinyl group, an isoquinolinyl group, a quinoxalinyl group, a quinazolinyl group, a carbazolyl group, a dibenzofuranyl group, and a dibenzothiophenyl group, each substituted with at least one selected from deuterium, -F, -Cl, -Br, -I, a hydroxyl group, a cyano group, a nitro group, an amidino group, a hydrazino group, a hydrazono group, a C₁-C₂₀ alkyl group, a C₁-C₂₀ alkoxy group, a cyclopentyl group, a cyclohexyl group, an adamantanyl group, a norbornanyl group, a norbornenyl group, a phenyl group, a biphenyl group, a terphenyl group, a naphthyl group, a fluorenyl group, a pyridinyl group, a pyrazinyl group, a pyrimidinyl group, a pyridazinyl group, a triazinyl group, a quinolinyl group, an isoquinolinyl group, a quinoxalinyl group, a quinazolinyl group, a carbazolyl group, a dibenzofuranyl group, and a dibenzothiophenyl group; and
-Si(Q₄₀₁)(Q₄₀₂)(Q₄₀₃), -N(Q₄₀₁)(Q₄₀₂), -B(Q₄₀₁)(Q₄₀₂), -C(=O)(Q₄₀₁), - S(=O)₂(Q₄₀₁), and -P(=O)(Q₄₀₁)(Q₄₀₂), and
Q₄₀₁ to Q₄₀₃ may each independently be selected from a C₁-C₁₀ alkyl group, a C₁-C₁₀ alkoxy group, a phenyl group, a biphenyl group, and a naphthyl group, but embodiments of the present disclosure are not limited thereto.
In one or more embodiments, when xc1 in Formula 401 is two or more, two A₄₀₁(s) in two or more L₄₀₁(s) may optionally be linked via X₄₀₇, which is a linking group, or two A₄₀₂(s) in two or more L₄₀₁(s) may optionally be linked via X₄₀₈, which is a linking group (see Compounds PD1 to PD4 and PD7). X₄₀₇ and X₄₀₆ may each independently be a single bond, *-O-*', *-S-*', *-C(=O)-*', *-N(Q₄₁₃)-*', *-C(Q₄₁₃)(Q₄₁₄)-*', or *-C(Q₄₁₃)=C(Q₄₁₄)-*' (wherein Q₄₁₃ and Q₄₁₄ may each independently be hydrogen, deuterium, a C₁-C₂₀ alkyl group, a C₁-C₂₀ alkoxy group, a phenyl group, a biphenyl group, a terphenyl group, or a naphthyl group), but embodiments of the present disclosure are not limited thereto.

In Formula 401, L₄₀₂ may be a monovalent, divalent, or trivalent organic ligand. For example, L₄₀₂ may be selected from halogen, diketone (for example, acetylacetonate), carboxylic acid (for example, picolinate), -C(=O), isonitrile, -CN, and phosphorus (for example, phosphine, or phosphite), but embodiments of the present disclosure are not limited thereto.

In one or more embodiments, the phosphorescent dopant may be selected from, for example, Compounds PD1 to PD27, but embodiments of the present disclosure are not limited thereto:

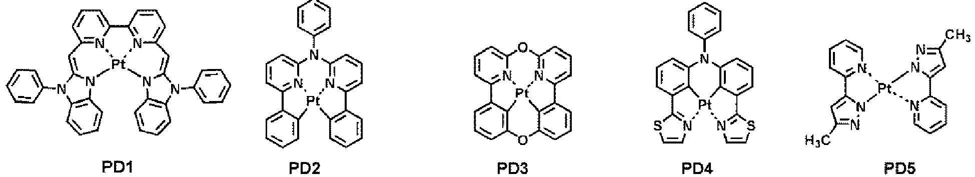

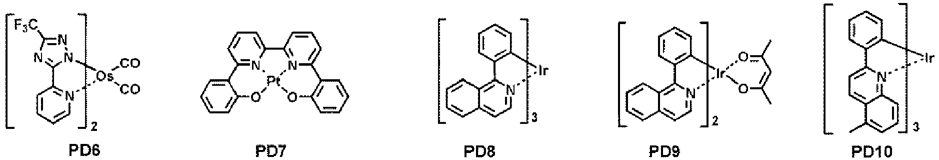

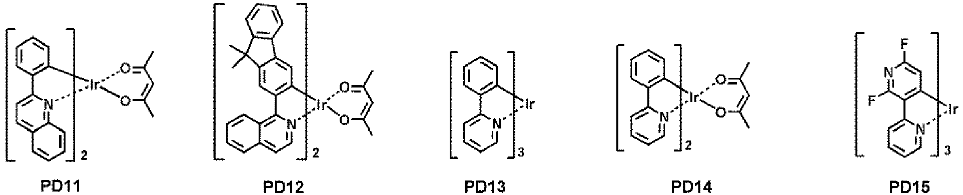

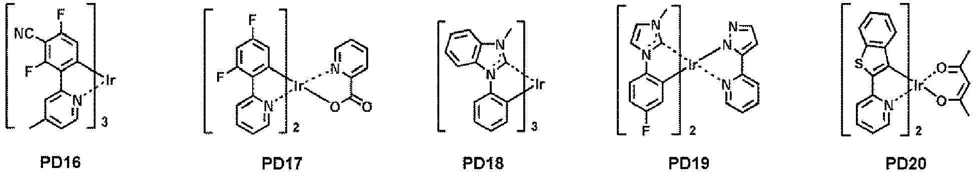

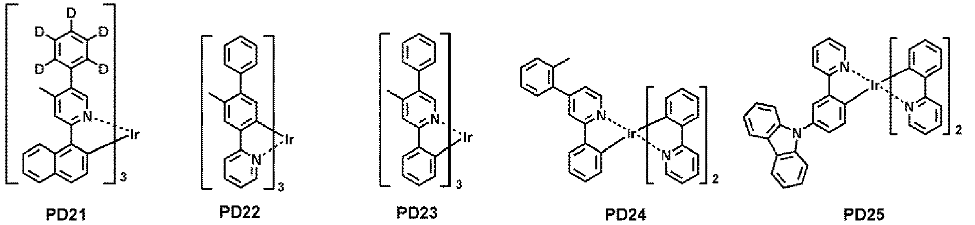

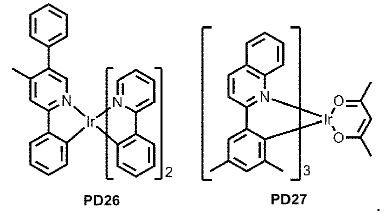

In one or more embodiments, the organic light-emitting device may further include a substrate divided into a first sub-pixel region, a second sub-pixel region, and a third sub-pixel region;
the first electrode may comprise a plurality of first electrodes respectively arranged in the first sub-pixel region, the second sub-pixel region, and the third sub-pixel region of the substrate;
the second electrode may face the plurality of first electrodes; and
the organic layer may be between the plurality of first electrodes and the second electrode.

In one embodiment, the first compound, the second compound, and the third compound may each independently be an azine-based compound represented by one selected from Formulae 1-1 to 1-3, where only one or two selected from the first compound, the second compound, and the third compound is represented by Formula 1-3, but embodiments of the present disclosure are not limited thereto.

In one embodiment, the organic layer may include an electron transport region disposed between the emission layer and the second electrode; the electron transport region may include a buffer layer and an electron transport layer; the buffer layer may be disposed between the emission layer and the electron transport layer; the emission layer may include the first compound; the buffer layer may include the second compound; and the electron transport layer may include the third compound, but embodiments of the present disclosure are not limited thereto.

For example, the emission layer may directly contact the buffer layer, and the buffer layer may directly contact the electron transport layer, but embodiments of the present disclosure are not limited thereto.

In one embodiment, the plurality of first electrodes may be an anode, and the second electrode may be a cathode, but embodiments of the present disclosure are not limited thereto.

In one embodiment, the emission layer may include an organometallic compound.

For example, the organometallic compound may emit phosphorescence (e.g., may phosphoresce), but embodiments of the present disclosure are not limited thereto.

For example, the organometallic compound may be represented by Formula 401. The organometallic compound represented by Formula 401 may be the same as described above.

In one embodiment, the emission layer may include a first emission layer, a second emission layer, and a third emission layer respectively arranged in the first sub-pixel region, the second sub-pixel region, and the third sub-pixel region of the substrate, and at least one of the first emission layer and the second emission layer may include the first compound and the organometallic compound, but embodiments of the present disclosure are not limited thereto.

For example, the first emission layer may emit red light, the second emission layer may emit green light, and the third emission layer may emit blue light, but embodiments of the present disclosure are not limited thereto.

In one embodiment, the first compound, the second compound, and the third compound may be different from each other.

Since the organic light-emitting device includes the first compound, the second compound, and the third compound, an organic light-emitting device having a low driving voltage and high efficiency may be provided. For example, the organic light-emitting device excludes a case where the first compound, the second compound, and the third compound concurrently (e.g., simultaneously) include a triazine moiety. Therefore, an organic light-emitting device having a long lifespan may be provided.

The term "an organic layer" used herein refers to a single layer and/or a plurality of layers disposed between the first electrode and the second electrode of an organic light-emitting device. A material included in the "organic layer" is not limited to an organic material.

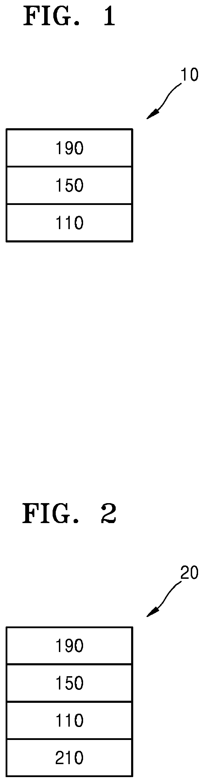

### Description of FIG. 1

FIG. 1 is a schematic cross-sectional view of an organic light-emitting device 10 according to an embodiment. The organic light-emitting device 10 includes a first electrode 110, an organic layer 150, and a second electrode 190.

Hereinafter, the structure of the organic light-emitting device 10 according to an embodiment and a method of manufacturing the organic light-emitting device 10 will be described in connection with FIG. 1.

### Description of first electrode 110

In FIG. 1, a substrate may be additionally disposed under the first electrode 110 or above the second electrode 190. The substrate may be a glass substrate or a plastic substrate, each having excellent mechanical strength, thermal stability, transparency, surface smoothness, ease of handling, and water resistance.

The first electrode 110 may be formed by depositing or sputtering a material for forming the first electrode 110 on the substrate. When the first electrode 110 is an anode, the material for forming a first electrode may be selected from materials with a high work function to facilitate hole injection.

The first electrode 110 may be a reflective electrode, a semi-transmissive electrode, or a transmissive electrode. When the first electrode 110 is a transmissive electrode, a material for forming a first electrode may be selected from indium tin oxide (ITO), indium zinc oxide (IZO), tin oxide (SnO₂), zinc oxide (ZnO), and any combinations thereof, but embodiments of the present disclosure are not limited thereto. In one or more embodiments, when the first electrode 110 is a semi-transmissive electrode or a reflectable electrode, a material for forming a first electrode may be selected from magnesium (Mg), silver (Ag), aluminium (Al), aluminium-lithium (Al-Li), calcium (Ca), magnesium-indium (Mg-In), magnesium-silver (Mg-Ag), and any combinations thereof, but embodiments of the present disclosure are not limited thereto

The first electrode 110 may have a single-layered structure, or a multi-layered structure including two or more layers. For example, the first electrode 110 may have a three-layered structure of ITO/Ag/ITO, but the structure of the first electrode 110 is not limited thereto.

### Organic layer 150

The organic layer 150 is disposed on the first electrode 110. The organic layer 150 may include an emission layer.

The organic layer 150 may further include a hole transport region between the first electrode 110 and the emission layer, and an electron transport region between the emission layer and the second electrode 190.

### Hole transport region in organic layer 150

The hole transport region may have i) a single-layered structure including a single layer including a single material, ii) a single-layered structure including a single layer including a plurality of different materials, or iii) a multi-layered structure having a plurality of layers including a plurality of different materials.

The hole transport region may include at least one layer selected from a hole injection layer, a hole transport layer, an emission auxiliary layer, and an electron blocking layer.

For example, the hole transport region may have a single-layered structure including a single layer including a plurality of different materials, or a multi-layered structure having a hole injection layer/hole transport layer structure, a hole injection layer/hole transport layer/emission auxiliary layer structure, a hole injection layer/emission auxiliary layer structure, a hole transport layer/emission auxiliary layer structure, or a hole injection layer/hole transport layer/electron blocking layer structure, wherein for each structure, constituting layers are sequentially stacked from the first electrode 110 in this stated order, but the structure of the hole transport region is not limited thereto.

The hole transport region may include at least one selected from m-MTDATA, TDATA, 2-TNATA, NPB(NPD), β-NPB, TPD, Spiro-TPD, Spiro-NPB, methylated-NPB, TAPC, HMTPD, 4,4',4"-tris(N-carbazolyl)triphenylamine (TCTA), polyaniline/dodecylbenzenesulfonic acid (PANI/DBSA), poly(3,4-ethylenedioxythiophene)/poly(4-styrenesulfonate) (PEDOT/PSS), polyaniline/camphor sulfonic acid (PANI/CSA), polyaniline/poly(4-styrenesulfonate) (PANI/PSS), a compound represented by Formula 201, and a compound represented by Formula 202:

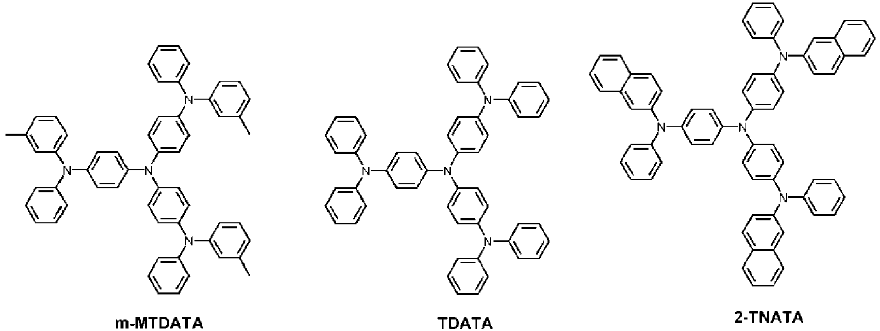

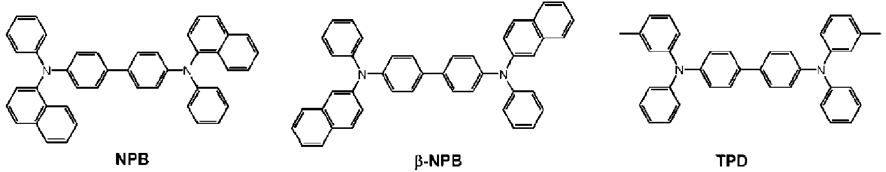

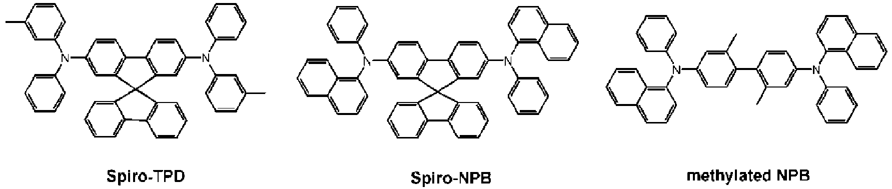

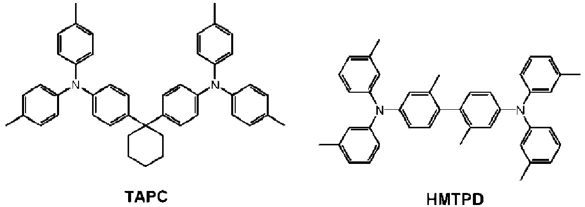

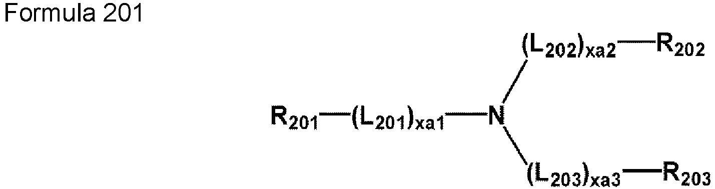

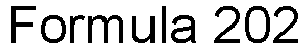

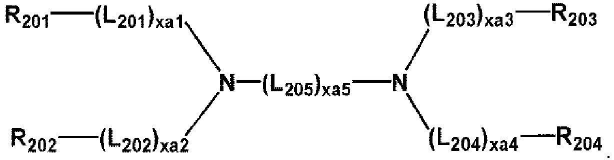

In Formulae 201 and 202,
L₂₀₁ to L₂₀₄ may each independently be selected from a substituted or unsubstituted C₃-C₁₀ cycloalkylene group, a substituted or unsubstituted C₁-C₁₀ heterocycloalkylene group, a substituted or unsubstituted C₃-C₁₀ cycloalkenylene group, a substituted or unsubstituted C₁-C₁₀ heterocycloalkenylene group, a substituted or unsubstituted C₆-C₆₀ (e.g. C₆-C₃₂) arylene group, a substituted or unsubstituted C₁-C₆₀ (e.g. C₁-C₂₀) heteroarylene group, a substituted or unsubstituted divalent non-aromatic condensed polycyclic group, and a substituted or unsubstituted divalent non-aromatic condensed heteropolycyclic group,
L₂₀₅ may be selected from *-O-*', *-S-*', *-N(Q₂₀₁)-*', a substituted or unsubstituted C₁-C₂₀ alkylene group, a substituted or unsubstituted C₂-C₂₀ alkenylene group, a substituted or unsubstituted C₃-C₁₀ cycloalkylene group, a substituted or unsubstituted C₁-C₁₀ heterocycloalkylene group, a substituted or unsubstituted C₃-C₁₀ cycloalkenylene group, a substituted or unsubstituted C₁-C₁₀ heterocycloalkenylene group, a substituted or unsubstituted C₆-C₆₀ (*e*.*g*. C₆-C₃₂) arylene group, a substituted or unsubstituted C₁-C₆₀ (*e*.*g*. C₁-C₂₀) heteroarylene group, a substituted or unsubstituted divalent non-aromatic condensed polycyclic group, and a substituted or unsubstituted divalent non-aromatic condensed heteropolycyclic group,
xa1 to xa4 may each independently be an integer from 0 to 3,
xa5 may be an integer from 1 to 10,
R₂₀₁ to R₂₀₄ and Q₂₀₁ may each independently be selected from a substituted or unsubstituted C₃-C₁₀ cycloalkyl group, a substituted or unsubstituted C₁-C₁₀ heterocycloalkyl group, a substituted or unsubstituted C₃-C₁₀ cycloalkenyl group, a substituted or unsubstituted C₁-C₁₀ heterocycloalkenyl group, a substituted or unsubstituted C₆-C₆₀ (*e*.*g*. C₆-C₃₂) aryl group, a substituted or unsubstituted C₆-C₆₀ (*e*.*g*. C₆-C₃₂) aryloxy group, a substituted or unsubstituted C₆-C₆₀ (*e*.*g*. C₆-C₃₂) arylthio group, a substituted or unsubstituted C₁-C₆₀ (*e*.*g*. C₁-C₂₀) heteroaryl group, a substituted or unsubstituted monovalent non-aromatic condensed polycyclic group, and a substituted or unsubstituted monovalent non-aromatic condensed heteropolycyclic group.

For example, in Formula 202, R₂₀₁ and R₂₀₂ may optionally be linked each other via a single bond, a dimethyl-methylene group, or a diphenyl-methylene group, and R₂₀₃ and R₂₀₄ may optionally be linked each other via a single bond, a dimethyl-methylene group, or a diphenyl-methylene group.

In one embodiment, in Formulae 201 and 202,

L₂₀₁ to L₂₀₅ may each independently be selected from:
a phenylene group, a pentalenylene group, an indenylene group, a naphthylene group, an azulenylene group, a heptalenylene group, an indacenylene group, an acenaphthylene group, a fluorenylene group, a spiro-bifluorenylene group, a benzofluorenylene group, a dibenzofluorenylene group, a phenalenylene group, a phenanthrenylene group, an anthracenylene group, a fluoranthenylene group, a triphenylenylene group, a pyrenylene group, a chrysenylene group, a naphthacenylene group, a picenylene group, a perylenylene group, a pentaphenylene group, a hexacenylene group, a pentacenylene group, a rubicenylene group, a coronenylene group, an ovalenylene group, a thiophenylene group, a furanylene group, a carbazolylene group, an indolylene group, an isoindolylene group, a benzofuranylene group, a benzothiophenylene group, a dibenzofuranylene group, a dibenzothiophenylene group, a benzocarbazolylene group, a dibenzocarbazolylene group, a dibenzosilolylene group, and a pyridinylene group; and
a phenylene group, a pentalenylene group, an indenylene group, a naphthylene group, an azulenylene group, a heptalenylene group, an indacenylene group, an acenaphthylene group, a fluorenylene group, a spiro-bifluorenylene group, a benzofluorenylene group, a dibenzofluorenylene group, a phenalenylene group, a phenanthrenylene group, an anthracenylene group, a fluoranthenylene group, a triphenylenylene group, a pyrenylene group, a chrysenylene group, a naphthacenylene group, a picenylene group, a perylenylene group, a pentaphenylene group, a hexacenylene group, a pentacenylene group, a rubicenylene group, a coronenylene group, an ovalenylene group, a thiophenylene group, a furanylene group, a carbazolylene group, an indolylene group, an isoindolylene group, a benzofuranylene group, a benzothiophenylene group, a dibenzofuranylene group, a dibenzothiophenylene group, a benzocarbazolylene group, a dibenzocarbazolylene group, a dibenzosilolylene group, and a pyridinylene group, each substituted with at least one selected from deuterium, -F, -Cl, -Br, -I, a hydroxyl group, a cyano group, a nitro group, an amidino group, a hydrazino group, a hydrazono group, a C₁-C₂₀ alkyl group, a C₁-C₂₀ alkoxy group, a cyclopentyl group, a cyclohexyl group, a cycloheptyl group, a cyclopentenyl group, a cyclohexenyl group, a phenyl group, a biphenyl group, a terphenyl group, a phenyl group substituted with a C₁-C₁₀ alkyl group, a phenyl group substituted with -F, a pentalenyl group, an indenyl group, a naphthyl group, an azulenyl group, a heptalenyl group, an indacenyl group, an acenaphthyl group, a fluorenyl group, a spiro-bifluorenyl group, a benzofluorenyl group, a dibenzofluorenyl group, a phenalenyl group, a phenanthrenyl group, an anthracenyl group, a fluoranthenyl group, a triphenylenyl group, a pyrenyl group, a chrysenyl group, a naphthacenyl group, a picenyl group, a perylenyl group, a pentaphenyl group, a hexacenyl group, a pentacenyl group, a rubicenyl group, a coronenyl group, an ovalenyl group, a thiophenyl group, a furanyl group, a carbazolyl group, an indolyl group, an isoindolyl group, a benzofuranyl group, a benzothiophenyl group, a dibenzofuranyl group, a dibenzothiophenyl group, a benzocarbazolyl group, a dibenzocarbazolyl group, a dibenzosilolyl group, a pyridinyl group, -Si(Q₃₁)(Q₃₂)(Q₃₃), and -N(Q₃₁)(Q₃₂), and
Q₃₁ to Q₃₃ may each independently be selected from a C₁-C₁₀ alkyl group, a C₁-C₁₀ alkoxy group, a phenyl group, a biphenyl group, a terphenyl group, and a naphthyl group.

In one or more embodiments, xa1 to xa4 may each independently be 0, 1, or 2.

In one or more embodiments, xa5 may be 1, 2, 3, or 4.

In one or more embodiments, R₂₀₁ to R₂₀₄ and Q₂₀₁ may each independently be selected from:
a phenyl group, a biphenyl group, a terphenyl group, a pentalenyl group, an indenyl group, a naphthyl group, an azulenyl group, a heptalenyl group, an indacenyl group, an acenaphthyl group, a fluorenyl group, a spiro-bifluorenyl group, a benzofluorenyl group, a dibenzofluorenyl group, a phenalenyl group, a phenanthrenyl group, an anthracenyl group, a fluoranthenyl group, a triphenylenyl group, a pyrenyl group, a chrysenyl group, a naphthacenyl group, a picenyl group, a perylenyl group, a pentaphenyl group, a hexacenyl group, a pentacenyl group, a rubicenyl group, a coronenyl group, an ovalenyl group, a thiophenyl group, a furanyl group, a carbazolyl group, an indolyl group, an isoindolyl group, a benzofuranyl group, a benzothiophenyl group, a dibenzofuranyl group, a dibenzothiophenyl group, a benzocarbazolyl group, a dibenzocarbazolyl group, a dibenzosilolyl group, and a pyridinyl group; and
a phenyl group, a biphenyl group, a terphenyl group, a pentalenyl group, an indenyl group, a naphthyl group, an azulenyl group, a heptalenyl group, an indacenyl group, an acenaphthyl group, a fluorenyl group, a spiro-bifluorenyl group, a benzofluorenyl group, a dibenzofluorenyl group, a phenalenyl group, a phenanthrenyl group, an anthracenyl group, a fluoranthenyl group, a triphenylenyl group, a pyrenyl group, a chrysenyl group, a naphthacenyl group, a picenyl group, a perylenyl group, a pentaphenyl group, a hexacenyl group, a pentacenyl group, a rubicenyl group, a coronenyl group, an ovalenyl group, a thiophenyl group, a furanyl group, a carbazolyl group, an indolyl group, an isoindolyl group, a benzofuranyl group, a benzothiophenyl group, a dibenzofuranyl group, a dibenzothiophenyl group, a benzocarbazolyl group, a dibenzocarbazolyl group, a dibenzosilolyl group, and a pyridinyl group, each substituted with at least one selected from deuterium, -F, -Cl, -Br, -I, a hydroxyl group, a cyano group, a nitro group, an amidino group, a hydrazino group, a hydrazono group, a C₁-C₂₀ alkyl group, a C₁-C₂₀ alkoxy group, a cyclopentyl group, a cyclohexyl group, a cycloheptyl group, a cyclopentenyl group, a cyclohexenyl group, a phenyl group, a biphenyl group, a terphenyl group, a phenyl group substituted with a C₁-C₁₀ alkyl group, a phenyl group substituted with -F, a pentalenyl group, an indenyl group, a naphthyl group, an azulenyl group, a heptalenyl group, an indacenyl group, an acenaphthyl group, a fluorenyl group, a spiro-bifluorenyl group, a benzofluorenyl group, a dibenzofluorenyl group, a phenalenyl group, a phenanthrenyl group, an anthracenyl group, a fluoranthenyl group, a triphenylenyl group, a pyrenyl group, a chrysenyl group, a naphthacenyl group, a picenyl group, a perylenyl group, a pentaphenyl group, a hexacenyl group, a pentacenyl group, a rubicenyl group, a coronenyl group, an ovalenyl group, a thiophenyl group, a furanyl group, a carbazolyl group, an indolyl group, an isoindolyl group, a benzofuranyl group, a benzothiophenyl group, a dibenzofuranyl group, a dibenzothiophenyl group, a benzocarbazolyl group, a dibenzocarbazolyl group, a dibenzosilolyl group, a pyridinyl group, -Si(Q₃₁)(Q₃₂)(Q₃₃), and -N(Q₃₁)(Q₃₂), and
Q₃₁ to Q₃₃ may be the same as described above

In one or more embodiments, at least one selected from R₂₀₁ to R₂₀₃ in Formula 201 may each independently be selected from:
a fluorenyl group, a spiro-bifluorenyl group, a carbazolyl group, a dibenzofuranyl group, and a dibenzothiophenyl group; and
a fluorenyl group, a spiro-bifluorenyl group, a carbazolyl group, a dibenzofuranyl group, and a dibenzothiophenyl group, each substituted with at least one selected from deuterium, -F, -Cl, -Br, -I, a hydroxyl group, a cyano group, a nitro group, an amidino group, a hydrazino group, a hydrazono group, a C₁-C₂₀ alkyl group, a C₁-C₂₀ alkoxy group, a cyclopentyl group, a cyclohexyl group, a cycloheptyl group, a cyclopentenyl group, a cyclohexenyl group, a phenyl group, a biphenyl group, a terphenyl group, a phenyl group substituted with a C₁-C₁₀ alkyl group, a phenyl group substituted with -F, a naphthyl group, a fluorenyl group, a spiro-bifluorenyl group, a carbazolyl group, a dibenzofuranyl group, and a dibenzothiophenyl group, but embodiments of the present disclosure are not limited thereto.

In one or more embodiments, in Formula 202, i) R₂₀₁ and R₂₀₂ may be linked each other via a single bond, and/or ii) R₂₀₃ and R₂₀₄ may be linked each other via a single bond.

In one or more embodiments, in Formula 202, at least one selected from R₂₀₁ to R₂₀₄ may be selected from:
a carbazolyl group; and
a carbazolyl group substituted with at least one selected from deuterium, -F, -Cl, -Br, -I, a hydroxyl group, a cyano group, a nitro group, an amidino group, a hydrazino group, a hydrazono group, a C₁-C₂₀ alkyl group, a C₁-C₂₀ alkoxy group, a cyclopentyl group, a cyclohexyl group, a cycloheptyl group, a cyclopentenyl group, a cyclohexenyl group, a phenyl group, a biphenyl group, a terphenyl group, a phenyl group substituted with a C₁-C₁₀ alkyl group, a phenyl group substituted with -F, a naphthyl group, a fluorenyl group, a spiro-bifluorenyl group, a carbazolyl group, a dibenzofuranyl group, and a dibenzothiophenyl group, but embodiments of the present disclosure are not limited thereto.

The compound represented by Formula 201 may be represented by Formula 201A:

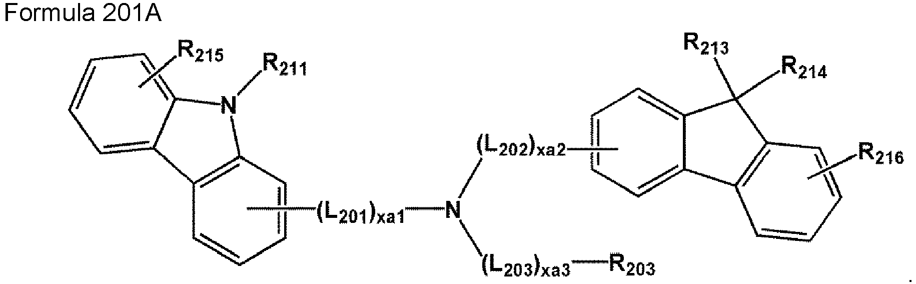

For example, the compound represented by Formula 201 may be represented by Formula 201A, but embodiments of the present disclosure are not limited thereto:

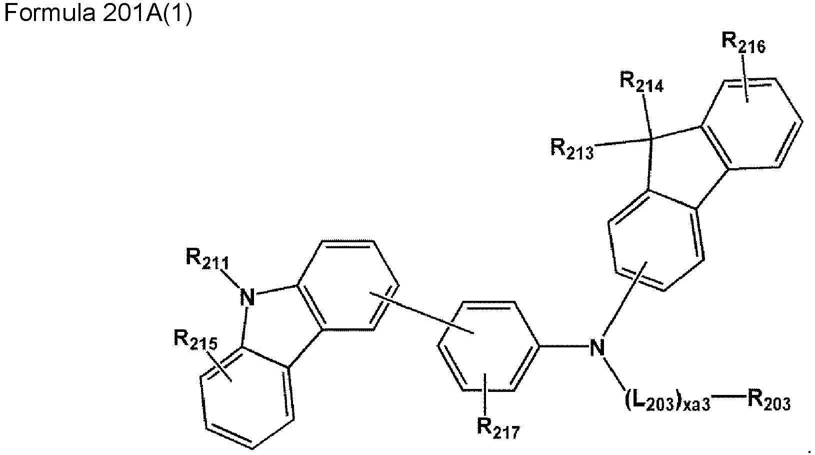

In one embodiment, the compound represented by Formula 201 may be represented by Formula 201A(1), but embodiments of the present disclosure are not limited thereto:

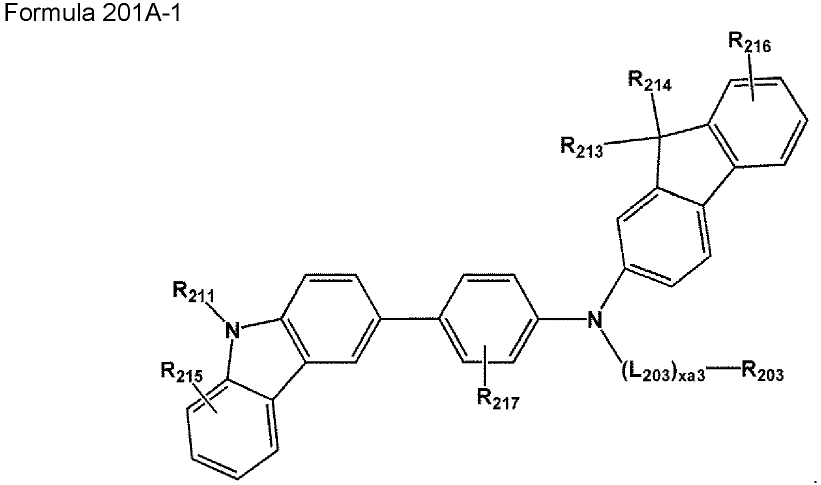

In one embodiment, the compound represented by Formula 202 may be represented by Formula 202A:

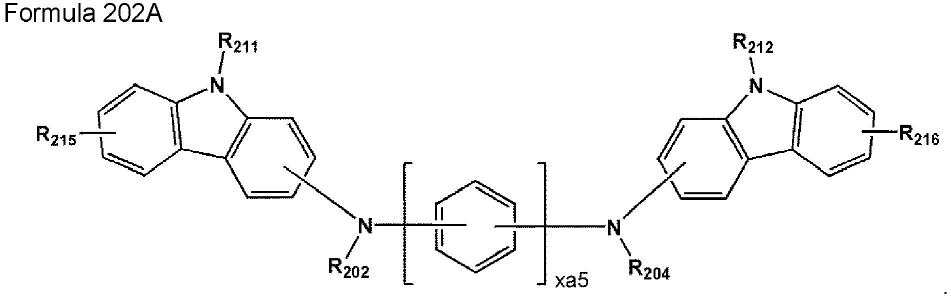

In one or more embodiments, the compound represented by Formula 202 may be represented by Formula 202A-1:

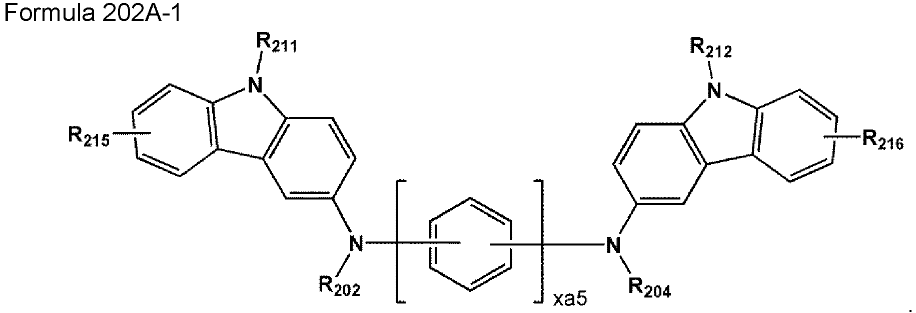

In Formulae 201A, 201A(1), 201A-1, 202A, and 202A-1,
L₂₀₁ to L₂₀₃, xa1 to xa3, xa5, and R₂₀₂ to R₂₀₄ may each independently be the same as described above,
R₂₁₁ and R₂₁₂ may each independently be the same as described in connection with R₂₀₃,
R₂₁₃ to R₂₁₇ may each independently be selected from hydrogen, deuterium, -F, -Cl, -Br, -I, a hydroxyl group, a cyano group, a nitro group, an amidino group, a hydrazino group, a hydrazono group, a C₁-C₂₀ alkyl group, a C₁-C₂₀ alkoxy group, a cyclopentyl group, a cyclohexyl group, a cycloheptyl group, a cyclopentenyl group, a cyclohexenyl group, a phenyl group, a biphenyl group, a terphenyl group, a phenyl group substituted with a C₁-C₁₀ alkyl group, a phenyl group substituted with -F, a pentalenyl group, an indenyl group, a naphthyl group, an azulenyl group, a heptalenyl group, an indacenyl group, an acenaphthyl group, a fluorenyl group, a spiro-bifluorenyl group, a benzofluorenyl group, a dibenzofluorenyl group, a phenalenyl group, a phenanthrenyl group, an anthracenyl group, a fluoranthenyl group, a triphenylenyl group, a pyrenyl group, a chrysenyl group, a naphthacenyl group, a picenyl group, a perylenyl group, a pentaphenyl group, a hexacenyl group, a pentacenyl group, a rubicenyl group, a coronenyl group, an ovalenyl group, a thiophenyl group, a furanyl group, a carbazolyl group, an indolyl group, an isoindolyl group, a benzofuranyl group, a benzothiophenyl group, a dibenzofuranyl group, a dibenzothiophenyl group, a benzocarbazolyl group, a dibenzocarbazolyl group, a dibenzosilolyl group, and a pyridinyl group.

The hole transport region may include at least one compound selected from Compounds HT1 to HT40, but embodiments of the present disclosure are not limited thereto:

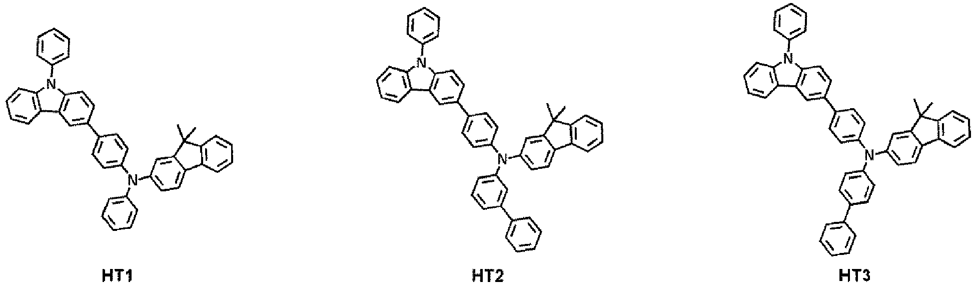

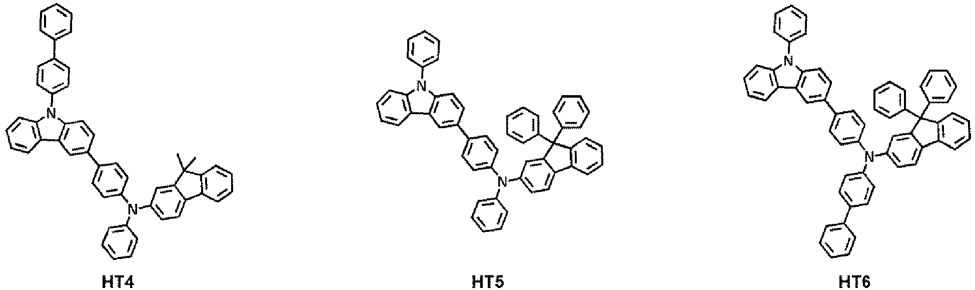

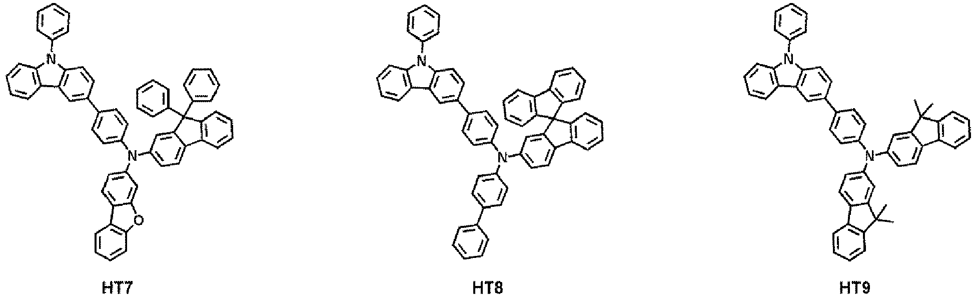

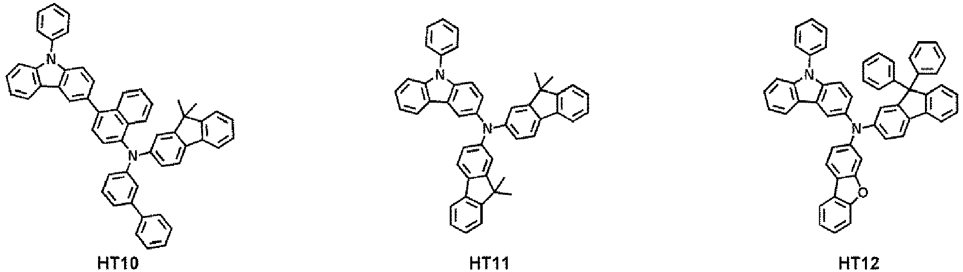

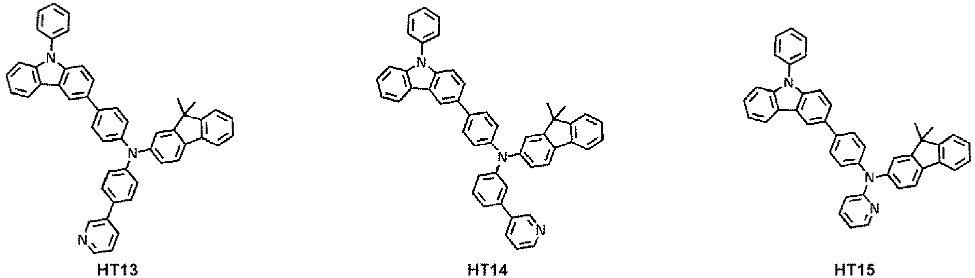

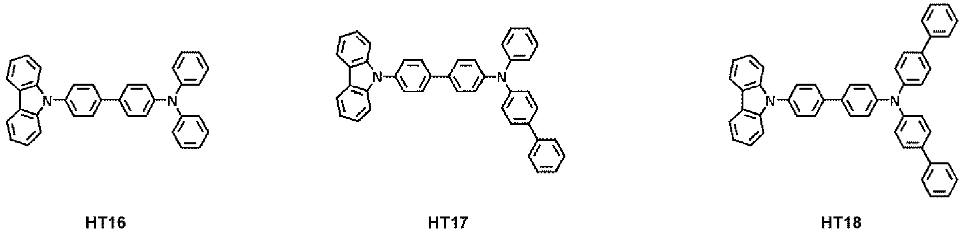

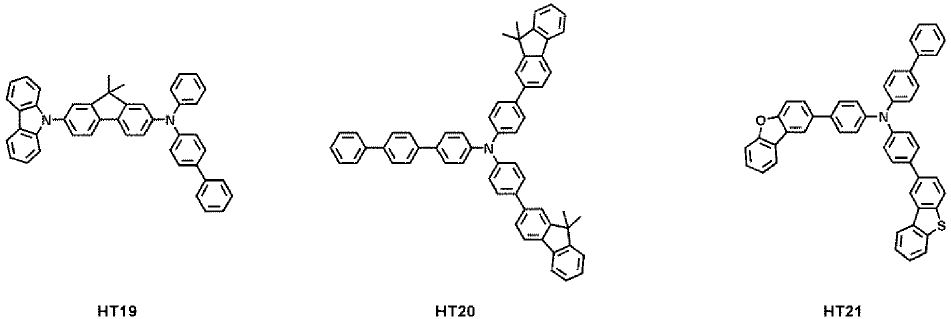

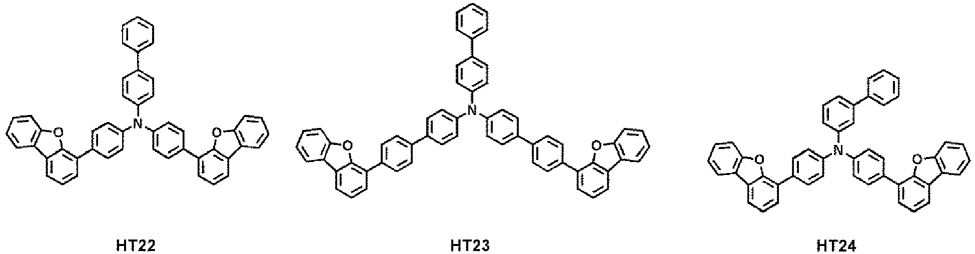

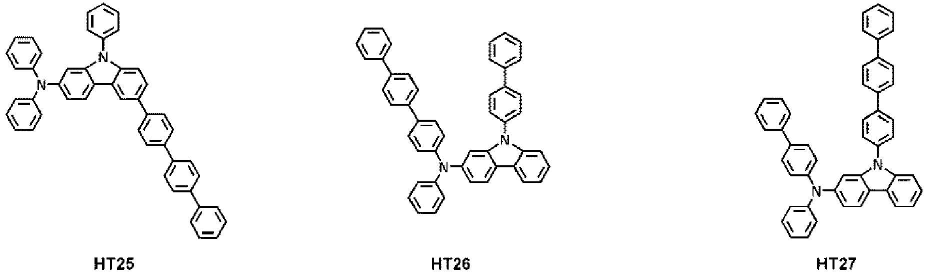

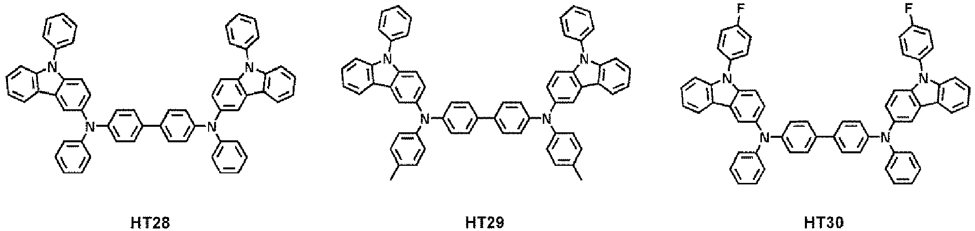

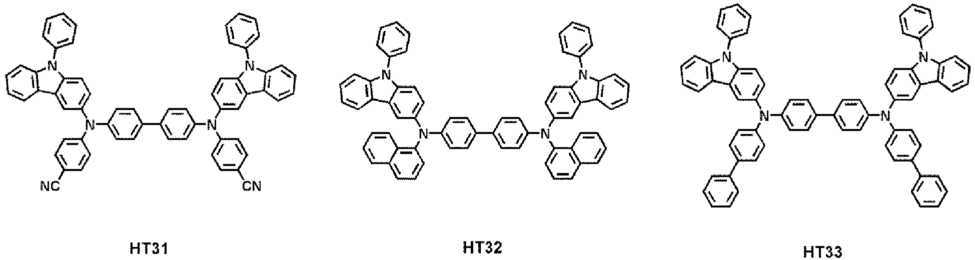

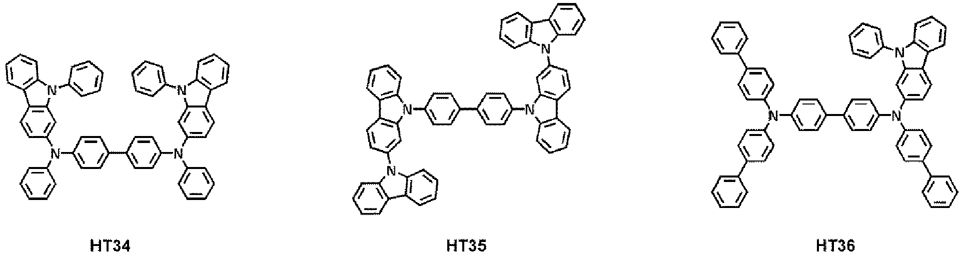

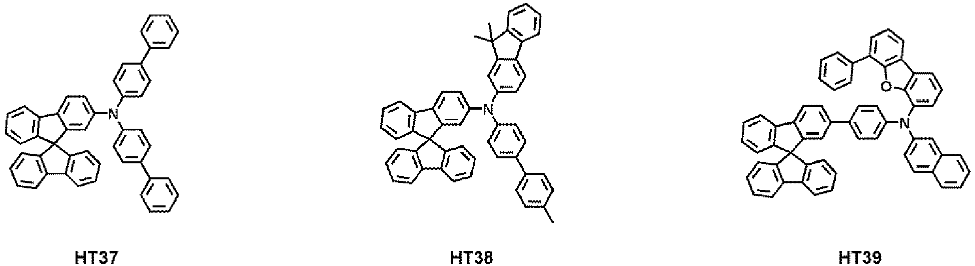

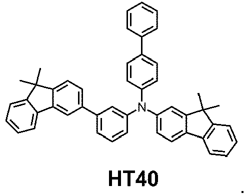

A thickness of the hole transport region may be in a range of about 100 Å to about 10,000 Å, for example, about 100 Å to about 7000 Å, about 100 Å to about 5000 Å, about 100 Å to about 3000 Å, or about 100 Å to about 1,000 Å. When hole transport region includes at least one selected from a hole injection layer and a hole transport layer, a thickness of the hole injection layer may be in a range of about 100 Å to about 9,000 Å, for example, about 100 Å to about 7,000 Å, about 100 Å to about 5,000 Å, about 100 Å to about 3,000 Å, about 100 Å to about 2,000 Å or about 100 Å to about 1,000 Å, and a thickness of the hole transport layer may be in a range of about 50 Å to about 2,000 Å, for example, about 100 Å to about 1,500 Å. When the thicknesses of the hole transport region, the hole injection layer, and the hole transport layer are within the foregoing ranges, suitable or satisfactory hole transporting characteristics may be obtained without a substantial increase in driving voltage.

The emission auxiliary layer may increase light-emission efficiency by compensating for an optical resonance distance according to the wavelength of light emitted by an emission layer, and the electron blocking layer may block the flow of electrons from an electron transport region. The emission auxiliary layer and the electron blocking layer may include the materials as described above.

### p-dopant

The hole transport region may further include, in addition to these materials, a charge-generation material for the improvement of conductive properties. The charge-generation material may be homogeneously or non-homogeneously dispersed in the hole transport region.

The charge-generation material may be, for example, a p-dopant.

In one embodiment, a lowest unoccupied molecular orbital (LUMO) energy level of the p-dopant may be -3.5 eV or less.

The p-dopant may include at least one selected from a quinone derivative, a metal oxide, and a cyano group-containing compound, but embodiments of the present disclosure are not limited thereto.

For example, the p-dopant may include at least one selected from:
a quinone derivative, such as tetracyanoquinodimethane (TCNQ) or 2,3,5,6-tetrafluoro-7,7,8,8-tetracyanoquinodimethane (F4-TCNQ);
a metal oxide, such as tungsten oxide or molybdenum oxide;
1,4,5,8,9,12-hexaazatriphenylene-hexacarbonitrile (HAT-CN); and
a compound represented by Formula 221:
   but embodiments of the present disclosure are not limited thereto: 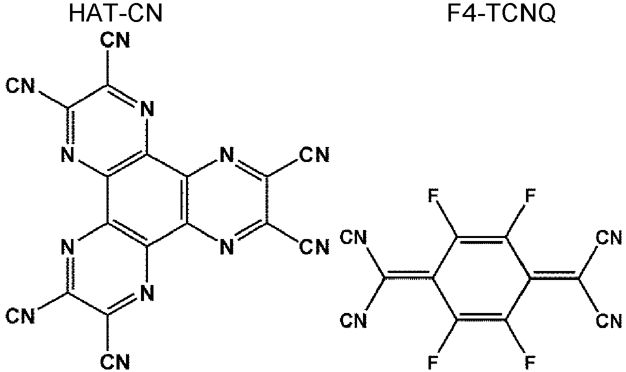 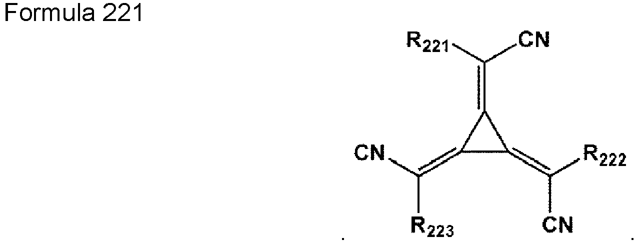
In Formula 221,
R₂₂₁ to R₂₂₃ may each independently be selected from a substituted or unsubstituted C₃-C₁₀ cycloalkyl group, a substituted or unsubstituted C₁-C₁₀ heterocycloalkyl group, a substituted or unsubstituted C₃-C₁₀ cycloalkenyl group, a substituted or unsubstituted C₁-C₁₀ heterocycloalkenyl group, a substituted or unsubstituted C₆-C₆₀ (e.g. C₆-C₃₂) aryl group, a substituted or unsubstituted C₁-C₆₀ (e.g. C₁-C₂₀) heteroaryl group, a substituted or unsubstituted monovalent non-aromatic condensed polycyclic group, and a substituted or unsubstituted monovalent non-aromatic condensed heteropolycyclic group, wherein at least one selected from R₂₂₁ to R₂₂₃ includes at least one substituent selected from a cyano group, -F, -Cl, -Br, -I, a C₁-C₂₀ alkyl group substituted with -F, a C₁-C₂₀ alkyl group substituted with -Cl, a C₁-C₂₀ alkyl group substituted with -Br, and a C₁-C₂₀ alkyl group substituted with -I.

### Emission layer in organic layer 150

When the organic light-emitting device 10 is a full-color organic light-emitting device, the emission layer may be patterned into a red emission layer, a green emission layer, or a blue emission layer, according to a sub-pixel. In one or more embodiments, the emission layer may have a stacked structure of two or more layers selected from a red emission layer, a green emission layer, and a blue emission layer, in which the two or more layers contact each other or are separated from each other. In one or more embodiments, the emission layer may include two or more materials selected from a red light-emitting material, a green light-emitting material, and a blue light-emitting material, in which the two or more materials are mixed with each other in a single layer to emit white light.

The emission layer may include a host and a dopant. The dopant may include at least one selected from a phosphorescent dopant and a fluorescent dopant.

An amount of the dopant in the emission layer may be, in general, in a range of about 0.01 parts by weight to about 15 parts by weight based on 100 parts by weight of the host, but is not limited thereto.

A thickness of the emission layer may be in a range of about 100 Å to about 1,000 Å, for example, about 100 Å to about 800 Å, about 150 Å to about 800 Å, about 150 Å to about 700 Å, about 150 Å to about 650 Å, about 200 Å to about 600 Å. When the thickness of the emission layer is within the foregoing range, excellent light-emission characteristics may be obtained without a substantial increase in driving voltage.

### Host in emission layer

The host may be the first compound. In one or more embodiments, the host may further include a compound represented by Formula 301 below.

Formula 301 [Ar₃₀₁]_{xb11}-[(L₃₀₁)_{xb1}-R₃₀₁]_{xb21}.

In Formula 301,
Ar₃₀₁ may be a substituted or unsubstituted C₅-C₆₀ carbocyclic group or a substituted or unsubstituted C₁-C₆₀ heterocyclic group,
xb11 may be 1, 2, or 3,
L₃₀₁ may be selected from a substituted or unsubstituted C₃-C₁₀ cycloalkylene group, a substituted or unsubstituted C₁-C₁₀ heterocycloalkylene group, a substituted or unsubstituted C₃-C₁₀ cycloalkenylene group, a substituted or unsubstituted C₁-C₁₀ heterocycloalkenylene group, a substituted or unsubstituted C₆-C₆₀ arylene group, a substituted or unsubstituted C₁-C₆₀ heteroarylene group, a substituted or unsubstituted divalent non-aromatic condensed polycyclic group, and a substituted or unsubstituted divalent non-aromatic condensed heteropolycyclic group,
xb1 may be an integer from 0 to 5,
R₃₀₁ may be selected from deuterium, -F, -Cl, -Br, -I, a hydroxyl group, a cyano group, a nitro group, an amidino group, a hydrazino group, a hydrazono group, a substituted or unsubstituted C₁-C₆₀ alkyl group, a substituted or unsubstituted C₂-C₆₀ alkenyl group, a substituted or unsubstituted C₂-C₆₀ alkynyl group, a substituted or unsubstituted C₁-C₆₀ alkoxy group, a substituted or unsubstituted C₃-C₁₀ cycloalkyl group, a substituted or unsubstituted C₁-C₁₀ heterocycloalkyl group, a substituted or unsubstituted C₃-C₁₀ cycloalkenyl group, a substituted or unsubstituted C₁-C₁₀ heterocycloalkenyl group, a substituted or unsubstituted C₆-C₆₀ aryl group, a substituted or unsubstituted C₆-C₆₀ aryloxy group, a substituted or unsubstituted C₆-C₆₀ arylthio group, a substituted or unsubstituted C₁-C₆₀ heteroaryl group, a substituted or unsubstituted monovalent non-aromatic condensed polycyclic group, a substituted or unsubstituted monovalent non-aromatic condensed heteropolycyclic group, - Si(Q₃₀₁)(Q₃₀₂)(Q₃₀₃), -N(Q₃₀₁)(Q₃₀₂), -B(Q₃₀₁)(Q₃₀₂), -C(=O)(Q₃₀₁), -S(=O)₂(Q₃₀₁), and - P(=O)(Q₃₀₁)(Q₃₀₂),
xb21 may be an integer from 1 to 5,
Q₃₀₁ to Q₃₀₃ may each independently be selected from a C₁-C₁₀ alkyl group, a C₁-C₁₀ alkoxy group, a phenyl group, a biphenyl group, a terphenyl group, and a naphthyl group, but embodiments of the present disclosure are not limited thereto.

For example, Ar₃₀₁ may be a substituted or unsubstituted C₆-C₃₂ carbocyclic group or a substituted or unsubstituted C₁-C₁₂ heterocyclic group, but embodiments are not limited thereto.

In one embodiment, Ar₃₀₁ in Formula 301 may be selected from:
a naphthalene group, a fluorene group, a spiro-bifluorene group, a benzofluorene group, a dibenzofluorene group, a phenalene group, a phenanthrene group, an anthracene group, a fluoranthene group, a triphenylene group, a pyrene group, a chrysene group, a naphthacene group, a picene group, a perylene group, a pentaphene group, an indenoanthracene group, a dibenzofuran group, and a dibenzothiophene group; and
a naphthalene group, a fluorene group, a spiro-bifluorene group, a benzofluorene group, a dibenzofluorene group, a phenalene group, a phenanthrene group, an anthracene group, a fluoranthene group, a triphenylene group, a pyrene group, a chrysene group, a naphthacene group, a picene group, a perylene group, a pentaphene group, an indenoanthracene group, a dibenzofuran group, and a dibenzothiophene group, each substituted with at least one selected from deuterium, - F, -Cl, -Br, -I, a hydroxyl group, a cyano group, a nitro group, an amidino group, a hydrazino group, a hydrazono group, a C₁-C₂₀ alkyl group, a C₁-C₂₀ alkoxy group, a phenyl group, a biphenyl group, a terphenyl group, a naphthyl group, - Si(Q₃₁)(Q₃₂)(Q₃₃), -N(Q₃₁)(Q₃₂), -B(Q₃₁)(Q₃₂), -C(=O)(Q₃₁), -S(=O)₂(Q₃₁), and - P(=O)(Q₃₁)(Q₃₂), and
Q₃₁ to Q₃₃ may each independently be selected from a C₁-C₁₀ alkyl group, a C₁-C₁₀ alkoxy group, a phenyl group, a biphenyl group, a terphenyl group, and a naphthyl group, but embodiments of the present disclosure are not limited thereto.

In Formula 301, when xb11 is two or more, two or more Ar₃₀₁(s) may be linked each other via a single bond.

In one or more embodiments, the compound represented by Formula 301 may be represented by Formula 301-1 or 301-2:

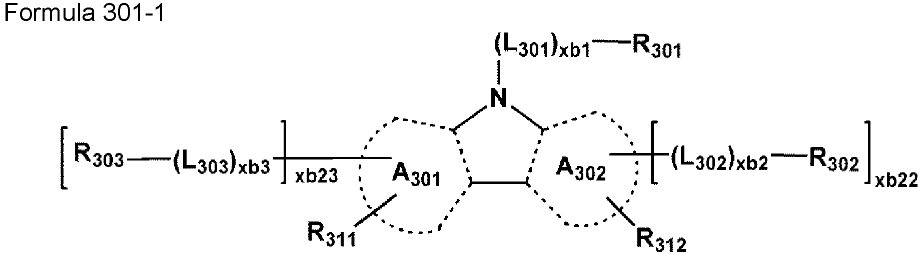

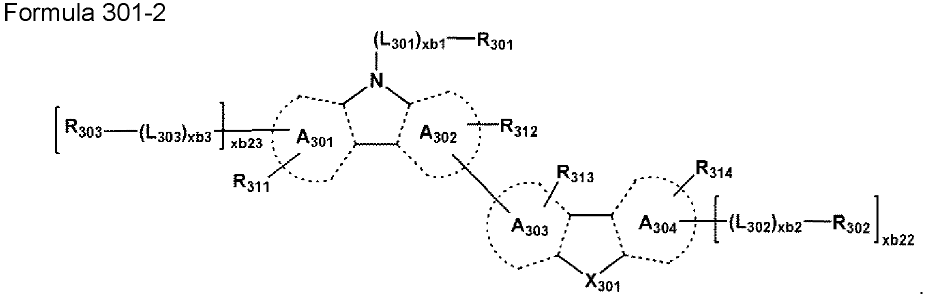

In Formulae 301-1 to 301-2,
A₃₀₁ to A₃₀₄ may each independently be selected from a benzene group, a naphthalene group, a phenanthrene group, a fluoranthene group, a triphenylene group, a pyrene group, a chrysene group, a pyridine group, a pyrimidine group, an indene group, a fluorene group, a spiro-bifluorene group, a benzofluorene group, a dibenzofluorene group, an indole group, a carbazole group, a benzocarbazole group, a dibenzocarbazole group, a furan group, a benzofuran group, a dibenzofuran group, a naphthofuran group, a benzonaphthofuran group, a dinaphthofuran group, a thiophene group, a benzothiophene group, a dibenzothiophene group, a naphthothiophene group, a benzonaphthothiophene group, and dinaphthothiophene group,
X₃₀₁ may be O, S, or N-[(L₃₀₄)_{xb4}-R₃₀₄],
R₃₁₁ to R₃₁₄ may each independently be selected from hydrogen, deuterium, -F, -Cl, -Br, -I, a hydroxyl group, a cyano group, a nitro group, an amidino group, a hydrazino group, a hydrazono group, a C₁-C₂₀ alkyl group, a C₁-C₂₀ alkoxy group, a phenyl group, a biphenyl group, a terphenyl group, a naphthyl group -Si(Q₃₁)(Q₃₂)(Q₃₃), -N(Q₃₁)(Q₃₂), -B(Q₃₁)(Q₃₂), -C(=O)(Q₃₁), -S(=O)₂(Q₃₁), and -P(=O)(Q₃₁)(Q₃₂),
xb22 and xb23 may each independently be 0, 1, or 2,
L₃₀₁, xb1, R₃₀₁, and Q₃₁ to Q₃₃ may each independently be the same as described above,
L₃₀₂ to L₃₀₄ may each independently be the same as described in connection with L₃₀₁,
xb2 to xb4 may each independently be the same as described in connection with xb1, and
R₃₀₂ to R₃₀₄ may each independently be the same as described in connection with R₃₀₁.

For example, L₃₀₁ to L₃₀₄ in Formulae 301, 301-1, and 301-2 may each independently be selected from: a substituted or unsubstituted C₃-C₁₀ cycloalkylene group, a substituted or unsubstituted C₁-C₁₀ heterocycloalkylene group, a substituted or unsubstituted C₃-C₁₀ cycloalkenylene group, a substituted or unsubstituted C₁-C₁₀ heterocycloalkenylene group, a substituted or unsubstituted C₆-C₃₀ arylene group, a substituted or unsubstituted C₁-C₂₀ heteroarylene group, a substituted or unsubstituted divalent non-aromatic condensed polycyclic group, and a substituted or unsubstituted divalent non-aromatic condensed heteropolycyclic group, but embodiments are not limited thereto.

For example, in Formulae 301, 301-1, and 301-2, L₃₀₁ to L₃₀₄ may each independently be selected from:
a phenylene group, a naphthylene group, a fluorenylene group, a spirobifluorenylene group, a benzofluorenylene group, a dibenzofluorenylene group, a phenanthrenylene group, an anthracenylene group, a fluoranthenylene group, a triphenylenylene group, a pyrenylene group, a chrysenylene group, a perylenylene group, a pentaphenylene group, a hexacenylene group, a pentacenylene group, a thiophenylene group, a furanylene group, a carbazolylene group, an indolylene group, an isoindolylene group, a benzofuranylene group, a benzothiophenylene group, a dibenzofuranylene group, a dibenzothiophenylene group, a benzocarbazolylene group, a dibenzocarbazolylene group, a dibenzosilolylene group, a pyridinylene group, an imidazolylene group, a pyrazolylene group, a thiazolylene group, an isothiazolylene group, an oxazolylene group, an isoxazolylene group, a thiadiazolylene group, an oxadiazolylene group, a pyrazinylene group, a pyrimidinylene group, a pyridazinylene group, a triazinylene group, a quinolinylene group, an isoquinolinylene group, a benzoquinolinylene group, a phthalazinylene group, a naphthyridinylene group, a quinoxalinylene group, a quinazolinylene group, a cinnolinylene group, a phenanthridinylene group, an acridinylene group, a phenanthrolinylene group, a phenazinylene group, a benzimidazolylene group, an isobenzothiazolylene group, a benzoxazolylene group, an isobenzoxazolylene group, a triazolylene group, a tetrazolylene group, an imidazopyridinylene group, an imidazopyrimidinylene group, and an azacarbazolylene group; and
a phenylene group, a naphthylene group, a fluorenylene group, a spirobifluorenylene group, a benzofluorenylene group, a dibenzofluorenylene group, a phenanthrenylene group, an anthracenylene group, a fluoranthenylene group, a triphenylenylene group, a pyrenylene group, a chrysenylene group, a perylenylene group, a pentaphenylene group, a hexacenylene group, a pentacenylene group, a thiophenylene group, a furanylene group, a carbazolylene group, an indolylene group, an isoindolylene group, a benzofuranylene group, a benzothiophenylene group, a dibenzofuranylene group, a dibenzothiophenylene group, a benzocarbazolylene group, a dibenzocarbazolylene group, a dibenzosilolylene group, a pyridinylene group, an imidazolylene group, a pyrazolylene group, a thiazolylene group, an isothiazolylene group, an oxazolylene group, an isoxazolylene group, a thiadiazolylene group, an oxadiazolylene group, a pyrazinylene group, a pyrimidinylene group, a pyridazinylene group, a triazinylene group, a quinolinylene group, an isoquinolinylene group, a benzoquinolinylene group, a phthalazinylene group, a naphthyridinylene group, a quinoxalinylene group, a quinazolinylene group, a cinnolinylene group, a phenanthridinylene group, an acridinylene group, a phenanthrolinylene group, a phenazinylene group, a benzimidazolylene group, an isobenzothiazolylene group, a benzoxazolylene group, an isobenzoxazolylene group, a triazolylene group, a tetrazolylene group, an imidazopyridinylene group, an imidazopyrimidinylene group, and an azacarbazolylene group, each substituted with at least one selected from deuterium, -F, -Cl, -Br, -I, a hydroxyl group, a cyano group, a nitro group, an amidino group, a hydrazino group, a hydrazono group, a C₁-C₂₀ alkyl group, a C₁-C₂₀ alkoxy group, a phenyl group, a biphenyl group, a terphenyl group, a naphthyl group, a fluorenyl group, a spiro-bifluorenyl group, a benzofluorenyl group, a dibenzofluorenyl group, a phenanthrenyl group, an anthracenyl group, a fluoranthenyl group, a triphenylenyl group, a pyrenyl group, a chrysenyl group, a perylenyl group, a pentaphenyl group, a hexacenyl group, a pentacenyl group, a thiophenyl group, a furanyl group, a carbazolyl group, an indolyl group, an isoindolyl group, a benzofuranyl group, a benzothiophenyl group, a dibenzofuranyl group, a dibenzothiophenyl group, a benzocarbazolyl group, a dibenzocarbazolyl group, a dibenzosilolyl group, a pyridinyl group, an imidazolyl group, a pyrazolyl group, a thiazolyl group, an isothiazolyl group, an oxazolyl group, an isoxazolyl group, a thiadiazolyl group, an oxadiazolyl group, a pyrazinyl group, a pyrimidinyl group, a pyridazinyl group, a triazinyl group, a quinolinyl group, an isoquinolinyl group, a benzoquinolinyl group, a phthalazinyl group, a naphthyridinyl group, a quinoxalinyl group, a quinazolinyl group, a cinnolinyl group, a phenanthridinyl group, an acridinyl group, a phenanthrolinyl group, a phenazinyl group, a benzimidazolyl group, an isobenzothiazolyl group, a benzoxazolyl group, an isobenzoxazolyl group, a triazolyl group, a tetrazolyl group, an imidazopyridinyl group, an imidazopyrimidinyl group, an azacarbazolyl group, -Si(Q₃₁)(Q₃₂)(Q₃₃), -N(Q₃₁)(Q₃₂), - B(Q₃₁)(Q₃₂), -C(=O)(Q₃₁), -S(=O)₂(Q₃₁), and -P(=O)(Q₃₁)(Q₃₂), and
Q₃₁ to Q₃₃ may each independently be the same as described above.

As another example, R₃₀₁ to R₃₀₄ in Formulae 301, 301-1, and 301-2 may each independently be selected from: deuterium, -F, -Cl, -Br, -I, a hydroxyl group, a cyano group, a nitro group, an amidino group, a hydrazino group, a hydrazono group, a substituted or unsubstituted C₁-C₆₀ (e.g. C₁-C₁₀) alkyl group, a substituted or unsubstituted C₂-C₆₀ (e.g. C₂-C₁₀) alkenyl group, a substituted or unsubstituted C₂-C₆₀ (e.g. C₂-C₁₀) alkynyl group, a substituted or unsubstituted C₁-C₆₀ (e.g. C₁-C₁₀) alkoxy group, a substituted or unsubstituted C₃-C₁₀ cycloalkyl group, a substituted or unsubstituted C₁-C₁₀ heterocycloalkyl group, a substituted or unsubstituted C₃-C₁₀ cycloalkenyl group, a substituted or unsubstituted C₁-C₁₀ heterocycloalkenyl group, a substituted or unsubstituted C₆-C₃₀ aryl group, a substituted or unsubstituted C₆-C₃₀ aryloxy group, a substituted or unsubstituted C₆-C₃₀ arylthio group, a substituted or unsubstituted C₁-C₂₀ heteroaryl group, a substituted or unsubstituted monovalent non-aromatic condensed polycyclic group, a substituted or unsubstituted monovalent non-aromatic condensed heteropolycyclic group, -Si(Q₃₀₁)(Q₃₀₂)(Q₃₀₃), -N(Q₃₀₁)(Q₃₀₂), - B(Q₃₀₁)(Q₃₀₂), -C(=O)(Q₃₀₁), -S(=O)₂(Q₃₀₁), and -P(=O)(Q₃₀₁)(Q₃₀₂), but embodiments are not limited thereto.

In one embodiment, in Formulae 301, 301-1, and 301-2, R₃₀₁ to R₃₀₄ may each independently be selected from:
a phenyl group, a biphenyl group, a terphenyl group, a naphthyl group, a fluorenyl group, a spiro-bifluorenyl group, a benzofluorenyl group, a dibenzofluorenyl group, a phenanthrenyl group, an anthracenyl group, a fluoranthenyl group, a triphenylenyl group, a pyrenyl group, a chrysenyl group, a perylenyl group, a pentaphenyl group, a hexacenyl group, a pentacenyl group, a thiophenyl group, a furanyl group, a carbazolyl group, an indolyl group, an isoindolyl group, a benzofuranyl group, a benzothiophenyl group, a dibenzofuranyl group, a dibenzothiophenyl group, a benzocarbazolyl group, a dibenzocarbazolyl group, a dibenzosilolyl group, a pyridinyl group, an imidazolyl group, a pyrazolyl group, a thiazolyl group, an isothiazolyl group, an oxazolyl group, an isoxazolyl group, a thiadiazolyl group, an oxadiazolyl group, a pyrazinyl group, a pyrimidinyl group, a pyridazinyl group, a triazinyl group, a quinolinyl group, an isoquinolinyl group, a benzoquinolinyl group, a phthalazinyl group, a naphthyridinyl group, a quinoxalinyl group, a quinazolinyl group, a cinnolinyl group, a phenanthridinyl group, an acridinyl group, a phenanthrolinyl group, a phenazinyl group, a benzimidazolyl group, an isobenzothiazolyl group, a benzoxazolyl group, an isobenzoxazolyl group, a triazolyl group, a tetrazolyl group, an imidazopyridinyl group, an imidazopyrimidinyl group, and an azacarbazolyl group; and
a phenyl group, a biphenyl group, a terphenyl group, a naphthyl group, a fluorenyl group, a spiro-bifluorenyl group, a benzofluorenyl group, a dibenzofluorenyl group, a phenanthrenyl group, an anthracenyl group, a fluoranthenyl group, a triphenylenyl group, a pyrenyl group, a chrysenyl group, a perylenyl group, a pentaphenyl group, a hexacenyl group, a pentacenyl group, a thiophenyl group, a furanyl group, a carbazolyl group, an indolyl group, an isoindolyl group, a benzofuranyl group, a benzothiophenyl group, a dibenzofuranyl group, a dibenzothiophenyl group, a benzocarbazolyl group, a dibenzocarbazolyl group, a dibenzosilolyl group, a pyridinyl group, an imidazolyl group, a pyrazolyl group, a thiazolyl group, an isothiazolyl group, an oxazolyl group, an isoxazolyl group, a thiadiazolyl group, an oxadiazolyl group, a pyrazinyl group, a pyrimidinyl group, a pyridazinyl group, a triazinyl group, a quinolinyl group, an isoquinolinyl group, a benzoquinolinyl group, a phthalazinyl group, a naphthyridinyl group, a quinoxalinyl group, a quinazolinyl group, a cinnolinyl group, a phenanthridinyl group, an acridinyl group, a phenanthrolinyl group, a phenazinyl group, a benzimidazolyl group, an isobenzothiazolyl group, a benzoxazolyl group, an isobenzoxazolyl group, a triazolyl group, a tetrazolyl group, an imidazopyridinyl group, an imidazopyrimidinyl group, and an azacarbazolyl group, each substituted with at least one selected from deuterium, -F, -Cl, -Br, -I, a hydroxyl group, a cyano group, a nitro group, an amidino group, a hydrazino group, a hydrazono group, a C₁-C₂₀ alkyl group, a C₁-C₂₀ alkoxy group, a phenyl group, a biphenyl group, a terphenyl group, a naphthyl group, a fluorenyl group, a spiro-bifluorenyl group, a benzofluorenyl group, a dibenzofluorenyl group, a phenanthrenyl group, an anthracenyl group, a fluoranthenyl group, a triphenylenyl group, a pyrenyl group, a chrysenyl group, a perylenyl group, a pentaphenyl group, a hexacenyl group, a pentacenyl group, a thiophenyl group, a furanyl group, a carbazolyl group, an indolyl group, an isoindolyl group, a benzofuranyl group, a benzothiophenyl group, a dibenzofuranyl group, a dibenzothiophenyl group, a benzocarbazolyl group, a dibenzocarbazolyl group, a dibenzosilolyl group, a pyridinyl group, an imidazolyl group, a pyrazolyl group, a thiazolyl group, an isothiazolyl group, an oxazolyl group, an isoxazolyl group, a thiadiazolyl group, an oxadiazolyl group, a pyrazinyl group, a pyrimidinyl group, a pyridazinyl group, a triazinyl group, a quinolinyl group, an isoquinolinyl group, a benzoquinolinyl group, a phthalazinyl group, a naphthyridinyl group, a quinoxalinyl group, a quinazolinyl group, a cinnolinyl group, a phenanthridinyl group, an acridinyl group, a phenanthrolinyl group, a phenazinyl group, a benzimidazolyl group, an isobenzothiazolyl group, a benzoxazolyl group, an isobenzoxazolyl group, a triazolyl group, a tetrazolyl group, an imidazopyridinyl group, an imidazopyrimidinyl group, an azacarbazolyl group, -Si(Q₃₁)(Q₃₂)(Q₃₃), -N(Q₃₁)(Q₃₂), - B(Q₃₁)(Q₃₂), -C(=O)(Q₃₁), -S(=O)₂(Q₃₁), and -P(=O)(Q₃₁)(Q₃₂), and
Q₃₁ to Q₃₃ may each independently be the same as described above.

In one embodiment, the host may further include an alkaline earth metal complex. For example, the host may include a complex selected from a Be complex (for example, Compound H55), a Mg complex, and a Zn complex. For example, the host may be selected from a Be complex (for example, Compound H55), a Mg complex, and a Zn complex.

The host may be further include at least one selected from (9,10-di(2-naphthyl)anthracene (ADN), 2-methyl-9,10-bis(naphthalen-2-yl)anthracene (MADN), 9,10-di-(2-naphthyl)-2-t-butyl-anthracene (TBADN), 4,4'-bis(N-carbazolyl)-1,1'-biphenyl (CBP), 1,3-di-9-carbazolylbenzene (mCP), 1,3,5-tri(carbazol-9-yl)benzene (TCP), bis(4-(9H-carbazol-9-yl)phenyl)diphenylsilane (BCPDS), 4-(1-(4-(diphenylamino)phenyl)cyclohexyl)phenyl)diphenyl-phosphine oxide (POPCPA), and Compounds H1 to H55, but embodiments of the present disclosure are not limited thereto:

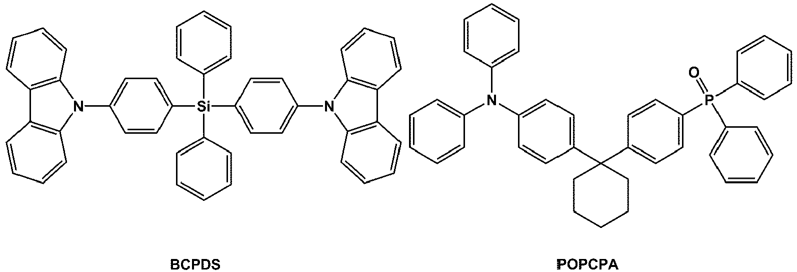

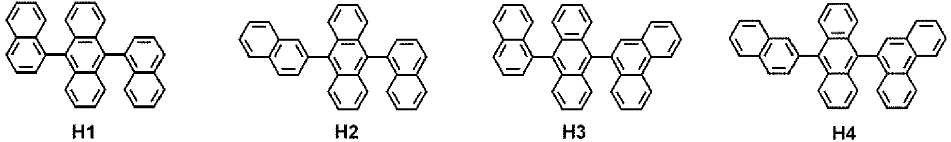

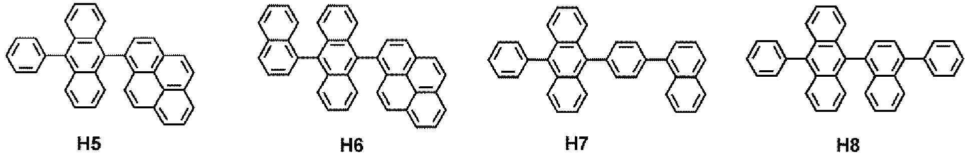

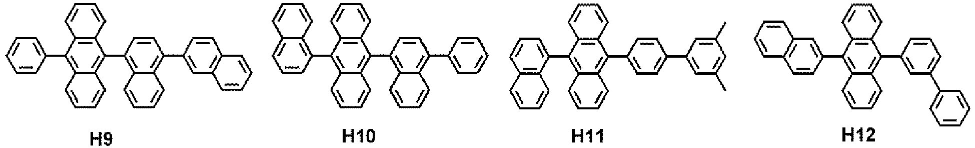

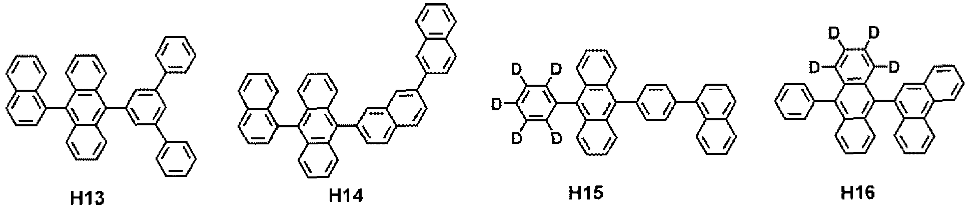

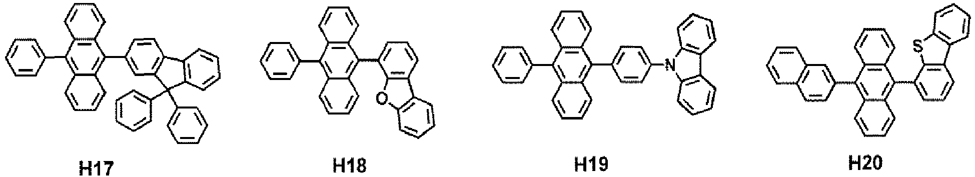

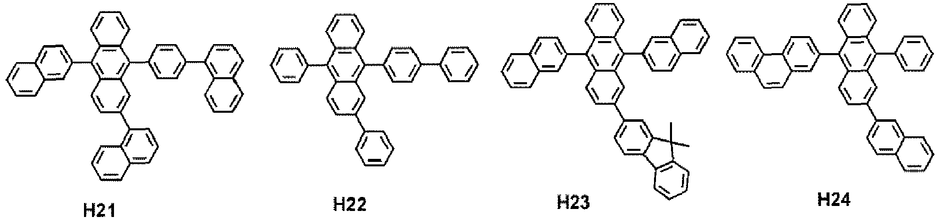

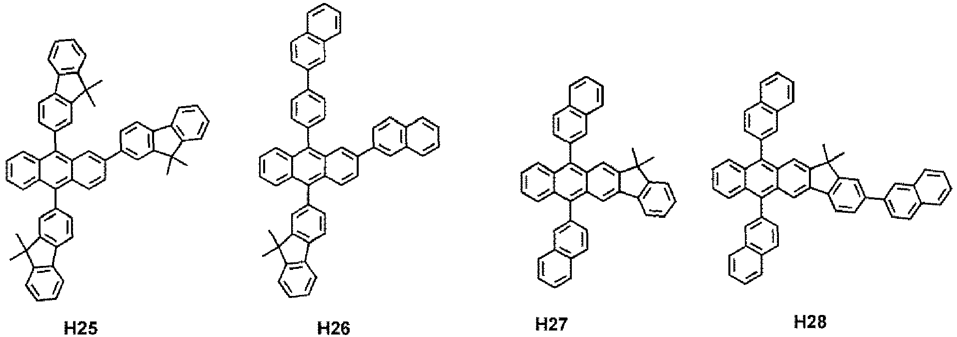

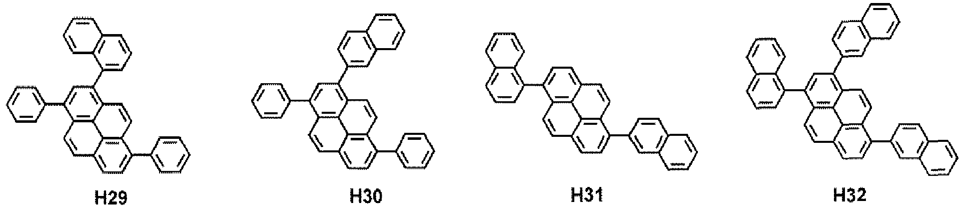

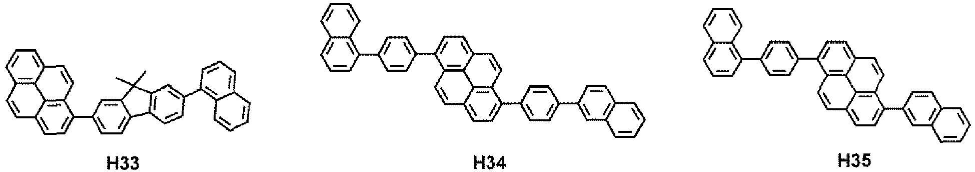

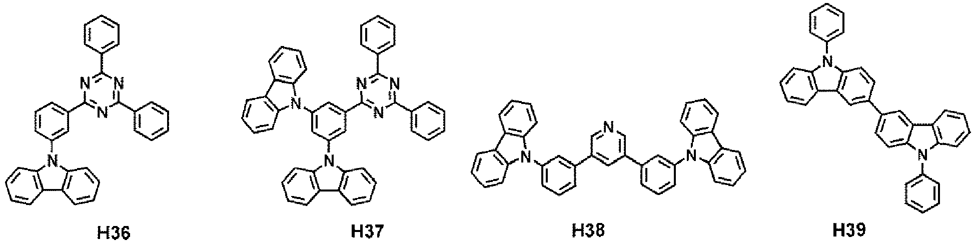

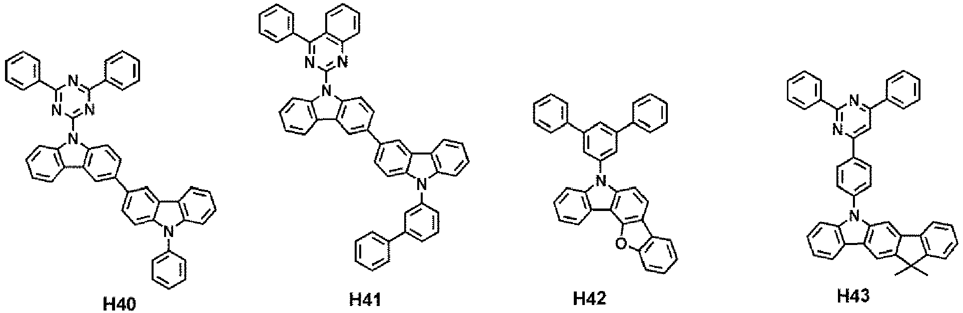

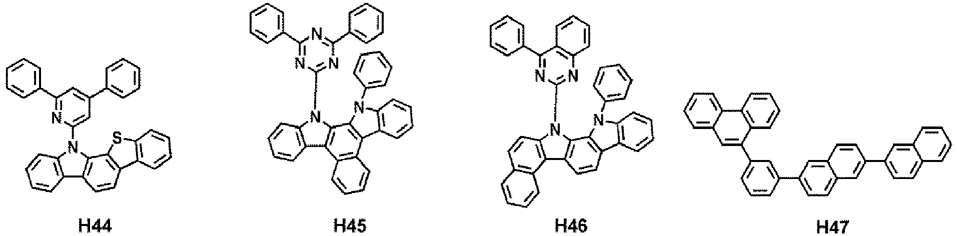

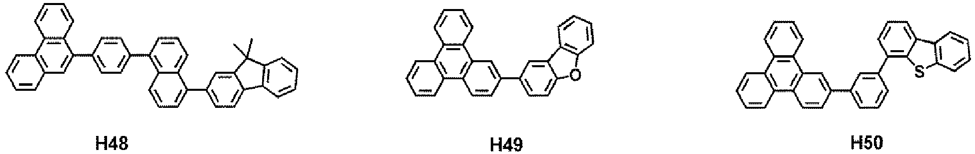

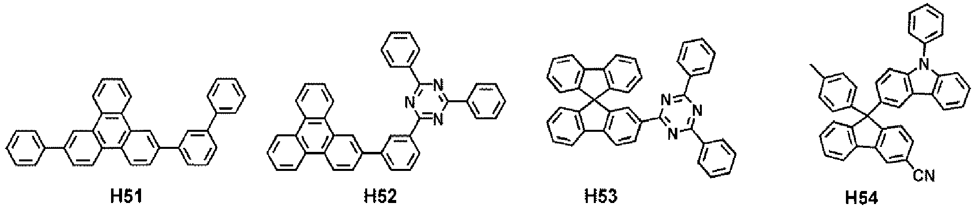

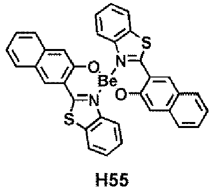

### Fluorescent dopant in emission layer

The fluorescent dopant may include an arylamine compound or a styrylamine compound.

The fluorescent dopant may include a compound represented by Formula 501:

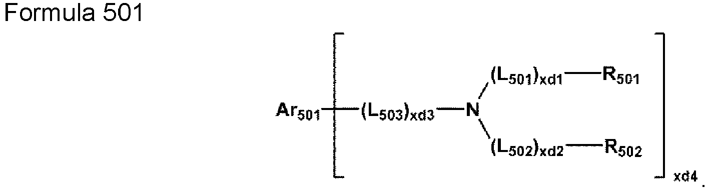

In Formula 501,
Ar₅₀₁ may be a substituted or unsubstituted C₆-C₆₀ carbocyclic group or a substituted or unsubstituted C₁-C₆₀ heterocyclic group,
L₅₀₁ to L₅₀₃ may each independently be selected from a substituted or unsubstituted C₃-C₁₀ cycloalkylene group, a substituted or unsubstituted C₁-C₁₀ heterocycloalkylene group, a substituted or unsubstituted C₃-C₁₀ cycloalkenylene group, a substituted or unsubstituted C₁-C₁₀ heterocycloalkenylene group, a substituted or unsubstituted C₆-C₆₀ (e.g. C₆-C₃₀) arylene group, a substituted or unsubstituted C₁-C₆₀ (e.g. C₁-C₂₀) heteroarylene group, a substituted or unsubstituted divalent non-aromatic condensed polycyclic group, and a substituted or unsubstituted divalent non-aromatic condensed heteropolycyclic group,
xd1 to xd3 may each independently be an integer from 0 to 3,
R₅₀₁ and R₅₀₂ may each independently be selected from a substituted or unsubstituted C₃-C₁₀ cycloalkyl group, a substituted or unsubstituted C₁-C₁₀ heterocycloalkyl group, a substituted or unsubstituted C₃-C₁₀ cycloalkenyl group, a substituted or unsubstituted C₁-C₁₀ heterocycloalkenyl group, a substituted or unsubstituted C₆-C₆₀ (e.g. C₆-C₃₀) aryl group, a substituted or unsubstituted C₆-C₆₀ (e.g. C₆-C₃₀) aryloxy group, a substituted or unsubstituted C₆-C₆₀ (e.g. C₆-C₃₀) arylthio group, a substituted or unsubstituted C₁-C₆₀ (e.g. C₁-C₂₀) heteroaryl group, a substituted or unsubstituted monovalent non-aromatic condensed polycyclic group, and a substituted or unsubstituted monovalent non-aromatic condensed heteropolycyclic group, and
xd4 may be an integer from 1 to 6.

For example, Ar₅₀₁ may be a substituted or unsubstituted C₆-C₃₀ carbocyclic group or a substituted or unsubstituted C₁-C₂₀ heterocyclic group, but embodiments are not limited thereto.

In one embodiment, Ar₅₀₁ in Formula 501 may be selected from:
a naphthalene group, a heptalene group, a fluorene group, a spiro-bifluorene group, a benzofluorene group, a dibenzofluorene group, a phenalene group, a phenanthrene group, an anthracene group, a fluoranthene group, a triphenylene group, a pyrene group, a chrysene group, a naphthacene group, a picene group, a perylene group, a pentaphene group, an indenoanthracene group, and an indenophenanthrene group; and
a naphthalene group, a heptalene group, a fluorene group, a spiro-bifluorene group, a benzofluorene group, a dibenzofluorene group, a phenalene group, a phenanthrene group, an anthracene group, a fluoranthene group, a triphenylene group, a pyrene group, a chrysene group, a naphthacene group, a picene group, a perylene group, a pentaphene group, an indenoanthracene group, and an indenophenanthrene group, each substituted with at least one selected from deuterium, -F, -Cl, -Br, -I, a hydroxyl group, a cyano group, a nitro group, an amidino group, a hydrazino group, a hydrazono group, a C₁-C₂₀ alkyl group, a C₁-C₂₀ alkoxy group, a phenyl group, a biphenyl group, a terphenyl group, and a naphthyl group.

In one or more embodiments, L₅₀₁ to L₅₀₃ in Formula 501 may each independently be selected from:
a phenylene group, a naphthylene group, a fluorenylene group, a spirobifluorenylene group, a benzofluorenylene group, a dibenzofluorenylene group, a phenanthrenylene group, an anthracenylene group, a fluoranthenylene group, a triphenylenylene group, a pyrenylene group, a chrysenylene group, a perylenylene group, a pentaphenylene group, a hexacenylene group, a pentacenylene group, a thiophenylene group, a furanylene group, a carbazolylene group, an indolylene group, an isoindolylene group, a benzofuranylene group, a benzothiophenylene group, a dibenzofuranylene group, a dibenzothiophenylene group, a benzocarbazolylene group, a dibenzocarbazolylene group, a dibenzosilolylene group, a pyridinylene group; and
a phenylene group, a naphthylene group, a fluorenylene group, a spirobifluorenylene group, a benzofluorenylene group, a dibenzofluorenylene group, a phenanthrenylene group, an anthracenylene group, a fluoranthenylene group, a triphenylenylene group, a pyrenylene group, a chrysenylene group, a perylenylene group, a pentaphenylene group, a hexacenylene group, a pentacenylene group, a thiophenylene group, a furanylene group, a carbazolylene group, an indolylene group, an isoindolylene group, a benzofuranylene group, a benzothiophenylene group, a dibenzofuranylene group, a dibenzothiophenylene group, a benzocarbazolylene group, a dibenzocarbazolylene group, a dibenzosilolylene group, and a pyridinylene group, each substituted with at least one selected from deuterium, -F, -Cl, -Br, -I, a hydroxyl group, a cyano group, a nitro group, an amidino group, a hydrazino group, a hydrazono group, a C₁-C₂₀ alkyl group, a C₁-C₂₀ alkoxy group, a phenyl group, a biphenyl group, a terphenyl group, a naphthyl group, a fluorenyl group, a spiro-bifluorenyl group, a benzofluorenyl group, a dibenzofluorenyl group, a phenanthrenyl group, an anthracenyl group, a fluoranthenyl group, a triphenylenyl group, a pyrenyl group, a chrysenyl group, a perylenyl group, a pentaphenyl group, a hexacenyl group, a pentacenyl group, a thiophenyl group, a furanyl group, a carbazolyl group, an indolyl group, an isoindolyl group, a benzofuranyl group, a benzothiophenyl group, a dibenzofuranyl group, a dibenzothiophenyl group, a benzocarbazolyl group, a dibenzocarbazolyl group, a dibenzosilolyl group, and a pyridinyl group.

In one or more embodiments, R₅₀₁ and R₅₀₂ in Formula 501 may each independently be selected from:
a phenyl group, a biphenyl group, a terphenyl group, a naphthyl group, a fluorenyl group, a spiro-bifluorenyl group, a benzofluorenyl group, a dibenzofluorenyl group, a phenanthrenyl group, an anthracenyl group, a fluoranthenyl group, a triphenylenyl group, a pyrenyl group, a chrysenyl group, a perylenyl group, a pentaphenyl group, a hexacenyl group, a pentacenyl group, a thiophenyl group, a furanyl group, a carbazolyl group, an indolyl group, an isoindolyl group, a benzofuranyl group, a benzothiophenyl group, a dibenzofuranyl group, a dibenzothiophenyl group, a benzocarbazolyl group, a dibenzocarbazolyl group, a dibenzosilolyl group, and a pyridinyl group; and
a phenyl group, a biphenyl group, a terphenyl group, a naphthyl group, a fluorenyl group, a spiro-bifluorenyl group, a benzofluorenyl group, a dibenzofluorenyl group, a phenanthrenyl group, an anthracenyl group, a fluoranthenyl group, a triphenylenyl group, a pyrenyl group, a chrysenyl group, a perylenyl group, a pentaphenyl group, a hexacenyl group, a pentacenyl group, a thiophenyl group, a furanyl group, a carbazolyl group, an indolyl group, an isoindolyl group, a benzofuranyl group, a benzothiophenyl group, a dibenzofuranyl group, a dibenzothiophenyl group, a benzocarbazolyl group, a dibenzocarbazolyl group, a dibenzosilolyl group, and a pyridinyl group, each substituted with at least one selected from deuterium, -F, -Cl, -Br, -I, a hydroxyl group, a cyano group, a nitro group, an amidino group, a hydrazino group, a hydrazono group, a C₁-C₂₀ alkyl group, a C₁-C₂₀ alkoxy group, a phenyl group, a biphenyl group, a terphenyl group, a naphthyl group, a fluorenyl group, a spiro-bifluorenyl group, a benzofluorenyl group, a dibenzofluorenyl group, a phenanthrenyl group, an anthracenyl group, a fluoranthenyl group, a triphenylenyl group, a pyrenyl group, a chrysenyl group, a perylenyl group, a pentaphenyl group, a hexacenyl group, a pentacenyl group, a thiophenyl group, a furanyl group, a carbazolyl group, an indolyl group, an isoindolyl group, a benzofuranyl group, a benzothiophenyl group, a dibenzofuranyl group, a dibenzothiophenyl group, a benzocarbazolyl group, a dibenzocarbazolyl group, a dibenzosilolyl group, a pyridinyl group, and - Si(Q₃₁)(Q₃₂)(Q₃₃), and
Q₃₁ to Q₃₃ may each independently be selected from a C₁-C₁₀ alkyl group, a C₁-C₁₀ alkoxy group, a phenyl group, a biphenyl group, a terphenyl group, and a naphthyl group.

In one or more embodiments, xd4 in Formula 501 may be 2, but embodiments of the present disclosure are not limited thereto.

For example, the fluorescent dopant may be selected from Compounds FD1 to FD22:

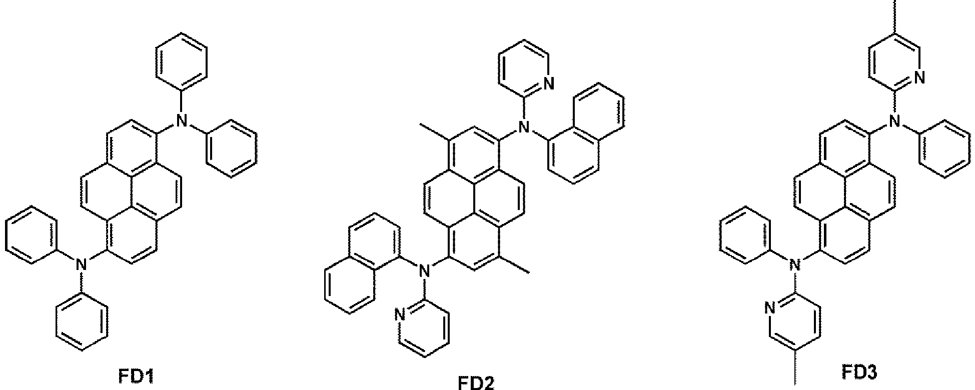

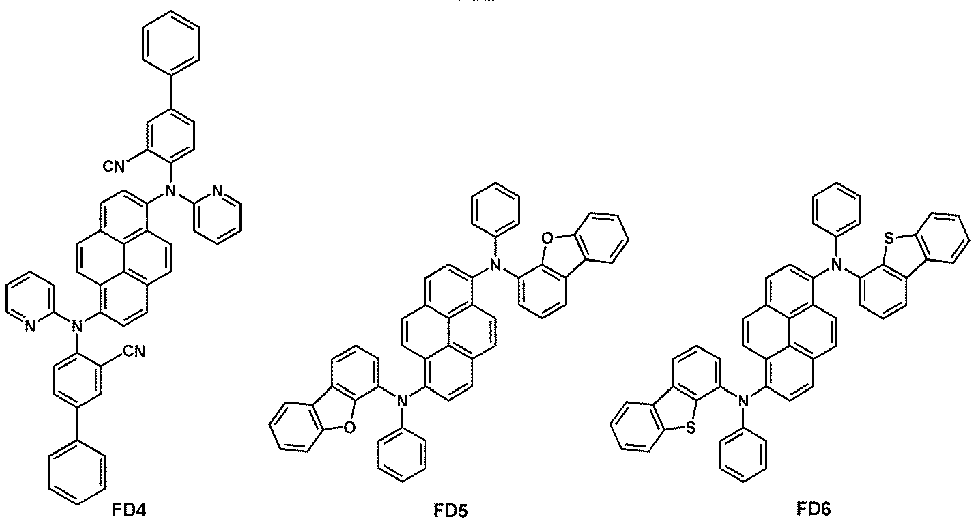

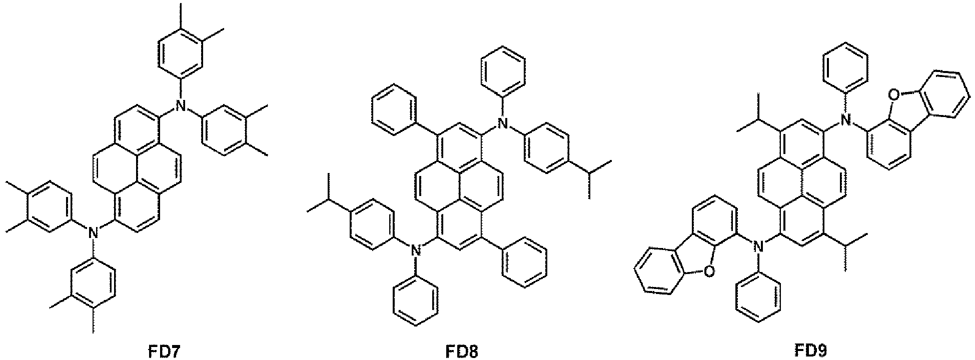

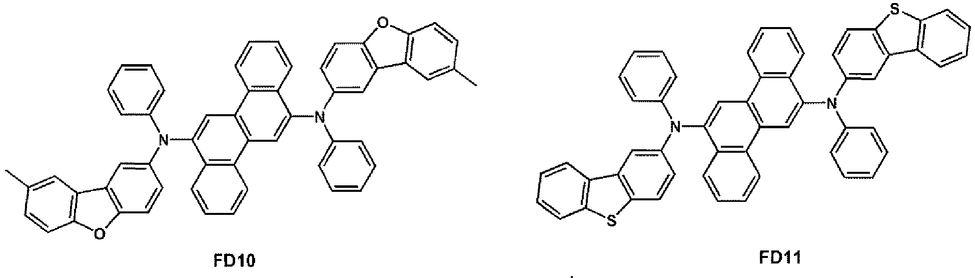

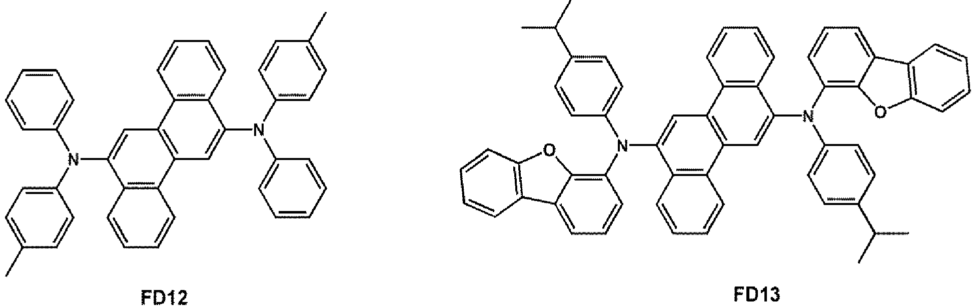

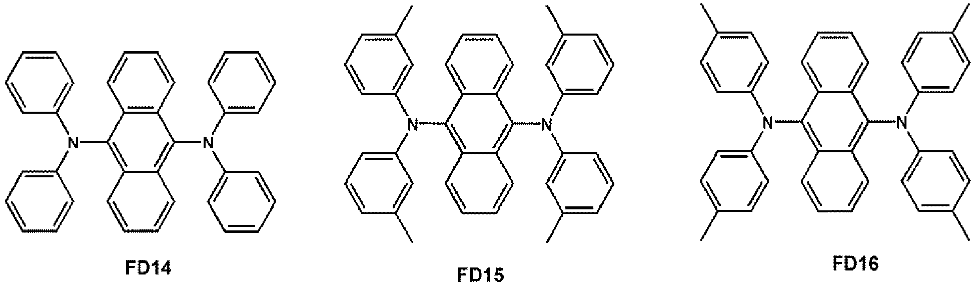

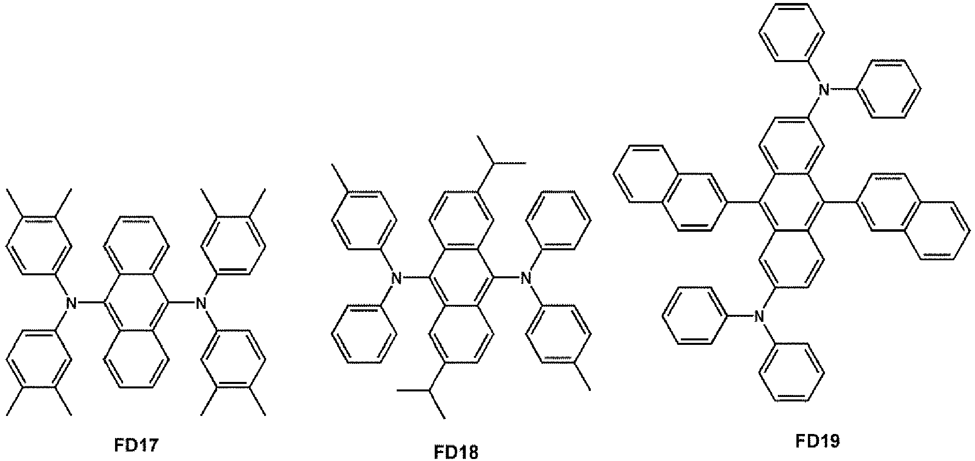

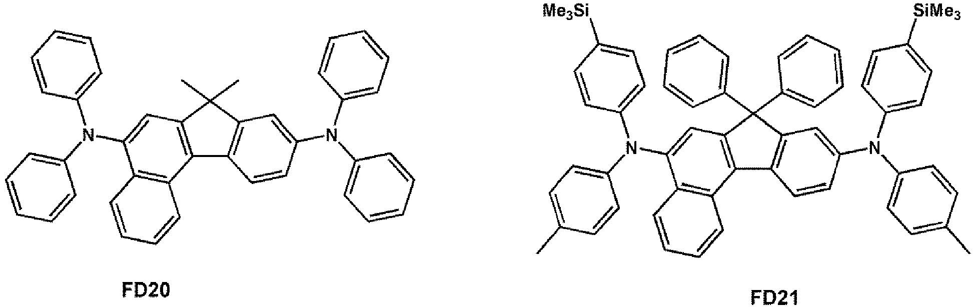

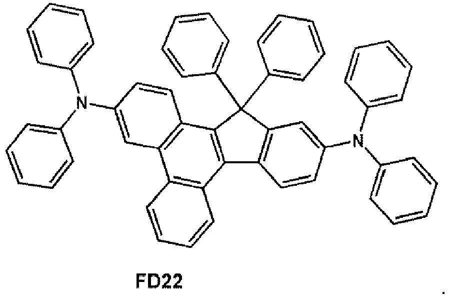

In one or more embodiments, the fluorescent dopant may be selected from the following compounds, but embodiments of the present disclosure are not limited thereto:

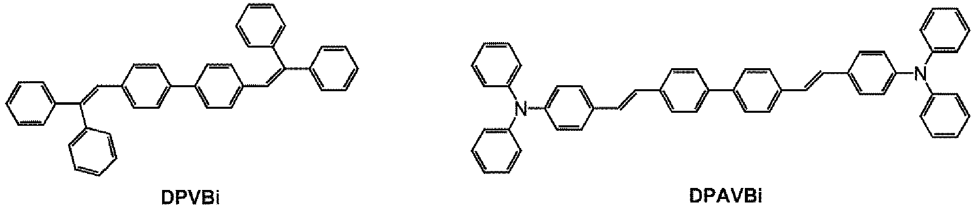

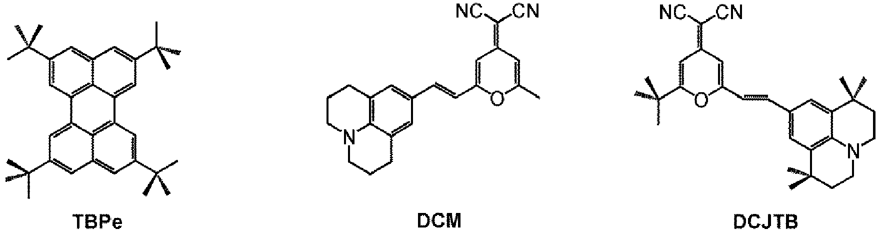

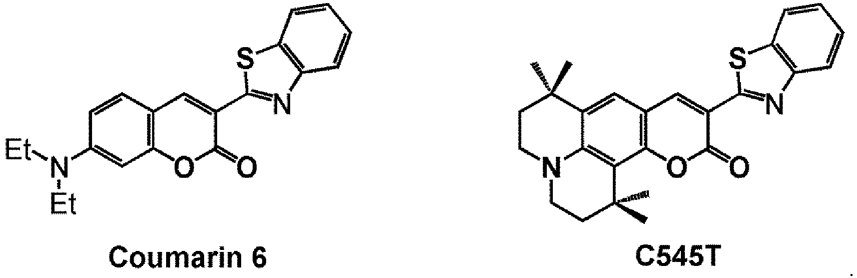

### Electron transport region in organic layer 150

The electron transport region may have i) a single-layered structure including a single layer including a single material, ii) a single-layered structure including a single layer including a plurality of different materials, or iii) a multi-layered structure having a plurality of layers including a plurality of different materials.

The electron transport region may include at least one selected from a buffer layer, a hole blocking layer, an electron control layer, an electron transport layer, and an electron injection layer, but embodiments of the present disclosure are not limited thereto.

For example, the electron transport region may have an electron transport layer/electron injection layer structure, a hole blocking layer/electron transport layer/electron injection layer structure, an electron control layer/electron transport layer/electron injection layer structure, or a buffer layer/electron transport layer/electron injection layer structure, wherein for each structure, constituting layers are sequentially stacked from an emission layer. However, embodiments of the structure of the electron transport region are not limited thereto.

The electron transport region (for example, a buffer layer, a hole blocking layer, an electron control layer, or an electron transport layer in the electron transport region) may include a metal-free compound containing at least one π electron-depleted nitrogen-containing ring.

The term "π electron-depleted nitrogen-containing ring," as used herein, indicates a C₁-C₆₀ (e.g. a C₁-C₃₀) heterocyclic group having at least one *-N=*' moiety as a ring-forming moiety.

For example, the "π electron-depleted nitrogen-containing ring" may be i) a 5-membered to 7-membered heteromonocyclic group having at least one *-N=*' moiety, ii) a heteropolycyclic group in which two or more 5-membered to 7-membered heteromonocyclic groups each having at least one *-N=*' moiety are condensed with each other (e.g., combined together), or iii) a heteropolycyclic group in which at least one of 5-membered to 7-membered heteromonocyclic groups, each having at least one *-N=*' moiety, is condensed with (e.g., combined with) at least one C₆-C₆₀ (e.g. a C₅-C₃₀) carbocyclic group.

Examples of the π electron-depleted nitrogen-containing ring include an imidazole, a pyrazole, a thiazole, an isothiazole, an oxazole, an isoxazole, a pyridine, a pyrazine, a pyrimidine, a pyridazine, indazole, a purine, a quinoline, an isoquinoline, a benzoquinoline, a phthalazine, a naphthyridine, a quinoxaline, a quinazoline, a cinnoline, a phenanthridine, an acridine, phenanthroline, phenazine, a benzimidazole, an isobenzothiazole, a benzoxazole, an isobenzoxazole, a triazole, a tetrazole, an oxadiazole, a triazine, thiadiazole, an imidazopyridine, an imidazopyrimidine, and an azacarbazole, but embodiments of the present disclosure are not limited thereto.

For example, the electron transport region may include a compound represented by Formula 601:

Formula 601 [Ar₆₀₁]ₓₑ₁₁-[(L₆₀₁)ₓₑ₁-R₆₀₁]ₓₑ₂₁.

In Formula 601,
Ar₆₀₁ may be a substituted or unsubstituted C₆-C₆₀ carbocyclic group or a substituted or unsubstituted C₁-C₆₀ heterocyclic group,
xe11 may be 1, 2, or 3,
L₆₀₁ may be selected from a substituted or unsubstituted C₃-C₁₀ cycloalkylene group, a substituted or unsubstituted C₁-C₁₀ heterocycloalkylene group, a substituted or unsubstituted C₃-C₁₀ cycloalkenylene group, a substituted or unsubstituted C₁-C₁₀ heterocycloalkenylene group, a substituted or unsubstituted C₆-C₆₀ arylene group, a substituted or unsubstituted C₁-C₆₀ heteroarylene group, a substituted or unsubstituted divalent non-aromatic condensed polycyclic group, and a substituted or unsubstituted divalent non-aromatic condensed heteropolycyclic group,
xe1 may be an integer from 0 to 5,
R₆₀₁ may be selected from a substituted or unsubstituted C₃-C₁₀ cycloalkyl group, a substituted or unsubstituted C₁-C₁₀ heterocycloalkyl group, a substituted or unsubstituted C₃-C₁₀ cycloalkenyl group, a substituted or unsubstituted C₁-C₁₀ heterocycloalkenyl group, a substituted or unsubstituted C₆-C₆₀ aryl group, a substituted or unsubstituted C₆-C₆₀ aryloxy group, a substituted or unsubstituted C₆-C₆₀ arylthio group, a substituted or unsubstituted C₁-C₆₀ heteroaryl group, a substituted or unsubstituted monovalent non-aromatic condensed polycyclic group, a substituted or unsubstituted monovalent non-aromatic condensed heteropolycyclic group, - Si(Q₆₀₁)(Q₆₀₂)(Q₆₀₃), -C(=O)(Q₆₀₁), -S(=O)₂(Q₆₀₁), and -P(=O)(Q₆₀₁)(Q₆₀₂), and
Q₆₀₁ to Q₆₀₃ may each independently be a C₁-C₁₀ alkyl group, a C₁-C₁₀ alkoxy group, a phenyl group, a biphenyl group, a terphenyl group, or a naphthyl group, and
xe21 may be an integer from 1 to 5.

In one embodiment, at least one of Ar₆₀₁(s) in the number of xe11 and R₆₀₁(s) in the number of xe21 may include the π electron-depleted nitrogen-containing ring.

For example, Ar₆₀₁ may be a substituted or unsubstituted C₆-C₃₀ carbocyclic group or a substituted or unsubstituted C₁-C₂₀ heterocyclic group, but embodiments are not limited thereto.

In one embodiment, ring Ar₆₀₁ in Formula 601 may be selected from:
a benzene group, a naphthalene group, a fluorene group, a spiro-bifluorene group, a benzofluorene group, a dibenzofluorene group, a phenalene group, a phenanthrene group, an anthracene group, a fluoranthene group, a triphenylene group, a pyrene group, a chrysene group, a naphthacene group, a picene group, a perylene group, a pentaphene group, an indenoanthracene group, a dibenzofuran group, a dibenzothiophene group, a carbazole group, an imidazole group, a pyrazole group, a thiazole group, an isothiazole group, an oxazole group, an isoxazole group, a pyridine group, a pyrazine group, a pyrimidine group, a pyridazine group, an indazole group, a purine group, a quinoline group, an isoquinoline group, a benzoquinoline group, a phthalazine group, a naphthyridine group, a quinoxaline group, a quinazoline group, a cinnoline group, a phenanthridine group, an acridine group, a phenanthroline group, a phenazine group, a benzimidazole group, an isobenzothiazole group, a benzoxazole group, an isobenzoxazole group, a triazole group, a tetrazole group, an oxadiazole group, a triazine group, a thiadiazole group, an imidazopyridine group, an imidazopyrimidine group, and an azacarbazole group; and
a benzene group, a naphthalene group, a fluorene group, a spiro-bifluorene group, a benzofluorene group, a dibenzofluorene group, a phenalene group, a phenanthrene group, an anthracene group, a fluoranthene group, a triphenylene group, a pyrene group, a chrysene group, a naphthacene group, a picene group, a perylene group, a pentaphene group, an indenoanthracene group, a dibenzofuran group, a dibenzothiophene group, a carbazole group, an imidazole group, a pyrazole group, a thiazole group, an isothiazole group, an oxazole group, an isoxazole group, a pyridine group, a pyrazine group, a pyrimidine group, a pyridazine group, an indazole group, a purine group, a quinoline group, an isoquinoline group, a benzoquinoline group, a phthalazine group, a naphthyridine group, a quinoxaline group, a quinazoline group, a cinnoline group, a phenanthridine group, an acridine group, a phenanthroline group, a phenazine group, a benzimidazole group, an isobenzothiazole group, a benzoxazole group, an isobenzoxazole group, a triazole group, a tetrazole group, an oxadiazole group, a triazine group, a thiadiazole group, an imidazopyridine group, an imidazopyrimidine group, and an azacarbazole group, each substituted with at least one selected from deuterium, -F, -Cl, -Br, -I, a hydroxyl group, a cyano group, a nitro group, an amidino group, a hydrazino group, a hydrazono group, a C₁-C₂₀ alkyl group, a C₁-C₂₀ alkoxy group, a phenyl group, a biphenyl group, a terphenyl group, a naphthyl group, -Si(Q₃₁)(Q₃₂)(Q₃₃), -S(=O)₂(Q₃₁), and -P(=O)(Q₃₁)(Q₃₂), and
Q₃₁ to Q₃₃ may each independently be selected from a C₁-C₁₀ alkyl group, a C₁-C₁₀ alkoxy group, a phenyl group, a biphenyl group, a terphenyl group, and a naphthyl group.

When xe11 in Formula 601 is two or more, two or more Ar₆₀₁(s) may be linked via a single bond.

In one or more embodiments, Ar₆₀₁ in Formula 601 may be an anthracene group.

In one or more embodiments, a compound represented by Formula 601 may be represented by Formula 601-1:

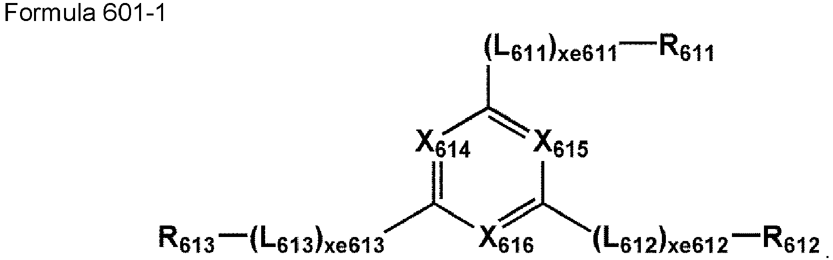

In Formula 601-1,
X₆₁₄ may be N or C(R₆₁₄), X₆₁₅ may be N or C(R₆₁₆), X₆₁₆ may be N or C(R₆₁₆), and at least one selected from X₆₁₄ to X₆₁₆ may be N,
L₆₁₁ to L₆₁₃ may each independently be the same as described in connection with L₆₀₁,
xe611 to xe613 may each independently be the same as described in connection with xe1,
R₆₁₁ to R₆₁₃ may each independently be the same as described in connection with R₆₀₁,
R₆₁₄ to R₆₁₆ may each independently be selected from hydrogen, deuterium, -F, -Cl, -Br, -I, a hydroxyl group, a cyano group, a nitro group, an amidino group, a hydrazino group, a hydrazono group, a C₁-C₂₀ alkyl group, a C₁-C₂₀ alkoxy group, a phenyl group, a biphenyl group, a terphenyl group, and a naphthyl group.

For example, L₆₀₁ and L₆₁₁ to L₆₁₃ in Formulae 601 and 601-1 may each independently be selected from: a substituted or unsubstituted C₃-C₁₀ cycloalkylene group, a substituted or unsubstituted C₁-C₁₀ heterocycloalkylene group, a substituted or unsubstituted C₃-C₁₀ cycloalkenylene group, a substituted or unsubstituted C₁-C₁₀ heterocycloalkenylene group, a substituted or unsubstituted C₆-C₃₀ arylene group, a substituted or unsubstituted C₁-C₂₀ heteroarylene group, a substituted or unsubstituted divalent non-aromatic condensed polycyclic group, and a substituted or unsubstituted divalent non-aromatic condensed heteropolycyclic group, but embodiments are not limited thereto.

In one embodiment, L₆₀₁ and L₆₁₁ to L₆₁₃ in Formulae 601 and 601-1 may each independently be selected from:
a phenylene group, a naphthylene group, a fluorenylene group, a spirobifluorenylene group, a benzofluorenylene group, a dibenzofluorenylene group, a phenanthrenylene group, an anthracenylene group, a fluoranthenylene group, a triphenylenylene group, a pyrenylene group, a chrysenylene group, a perylenylene group, a pentaphenylene group, a hexacenylene group, a pentacenylene group, a thiophenylene group, a furanylene group, a carbazolylene group, an indolylene group, an isoindolylene group, a benzofuranylene group, a benzothiophenylene group, a dibenzofuranylene group, a dibenzothiophenylene group, a benzocarbazolylene group, a dibenzocarbazolylene group, a dibenzosilolylene group, a pyridinylene group, an imidazolylene group, a pyrazolylene group, a thiazolylene group, an isothiazolylene group, an oxazolylene group, an isoxazolylene group, a thiadiazolylene group, an oxadiazolylene group, a pyrazinylene group, a pyrimidinylene group, a pyridazinylene group, a triazinylene group, a quinolinylene group, an isoquinolinylene group, a benzoquinolinylene group, a phthalazinylene group, a naphthyridinylene group, a quinoxalinylene group, a quinazolinylene group, a cinnolinylene group, a phenanthridinylene group, an acridinylene group, a phenanthrolinylene group, a phenazinylene group, a benzimidazolylene group, an isobenzothiazolylene group, a benzoxazolylene group, an isobenzoxazolylene group, a triazolylene group, a tetrazolylene group, an imidazopyridinylene group, an imidazopyrimidinylene group, and an azacarbazolylene group; and
a phenylene group, a naphthylene group, a fluorenylene group, a spirobifluorenylene group, a benzofluorenylene group, a dibenzofluorenylene group, a phenanthrenylene group, an anthracenylene group, a fluoranthenylene group, a triphenylenylene group, a pyrenylene group, a chrysenylene group, a perylenylene group, a pentaphenylene group, a hexacenylene group, a pentacenylene group, a thiophenylene group, a furanylene group, a carbazolylene group, an indolylene group, an isoindolylene group, a benzofuranylene group, a benzothiophenylene group, a dibenzofuranylene group, a dibenzothiophenylene group, a benzocarbazolylene group, a dibenzocarbazolylene group, a dibenzosilolylene group, a pyridinylene group, an imidazolylene group, a pyrazolylene group, a thiazolylene group, an isothiazolylene group, an oxazolylene group, an isoxazolylene group, a thiadiazolylene group, an oxadiazolylene group, a pyrazinylene group, a pyrimidinylene group, a pyridazinylene group, a triazinylene group, a quinolinylene group, an isoquinolinylene group, a benzoquinolinylene group, a phthalazinylene group, a naphthyridinylene group, a quinoxalinylene group, a quinazolinylene group, a cinnolinylene group, a phenanthridinylene group, an acridinylene group, a phenanthrolinylene group, a phenazinylene group, a benzimidazolylene group, an isobenzothiazolylene group, a benzoxazolylene group, an isobenzoxazolylene group, a triazolylene group, a tetrazolylene group, an imidazopyridinylene group, an imidazopyrimidinylene group, and an azacarbazolylene group, each substituted with at least one selected from deuterium, -F, -Cl, -Br, -I, a hydroxyl group, a cyano group, a nitro group, an amidino group, a hydrazino group, a hydrazono group, a C₁-C₂₀ alkyl group, a C₁-C₂₀ alkoxy group, a phenyl group, a biphenyl group, a terphenyl group, a naphthyl group, a fluorenyl group, a spiro-bifluorenyl group, a benzofluorenyl group, a dibenzofluorenyl group, a phenanthrenyl group, an anthracenyl group, a fluoranthenyl group, a triphenylenyl group, a pyrenyl group, a chrysenyl group, a perylenyl group, a pentaphenyl group, a hexacenyl group, a pentacenyl group, a thiophenyl group, a furanyl group, a carbazolyl group, an indolyl group, an isoindolyl group, a benzofuranyl group, a benzothiophenyl group, a dibenzofuranyl group, a dibenzothiophenyl group, a benzocarbazolyl group, a dibenzocarbazolyl group, a dibenzosilolyl group, a pyridinyl group, an imidazolyl group, a pyrazolyl group, a thiazolyl group, an isothiazolyl group, an oxazolyl group, an isoxazolyl group, a thiadiazolyl group, an oxadiazolyl group, a pyrazinyl group, a pyrimidinyl group, a pyridazinyl group, a triazinyl group, a quinolinyl group, an isoquinolinyl group, a benzoquinolinyl group, a phthalazinyl group, a naphthyridinyl group, a quinoxalinyl group, a quinazolinyl group, a cinnolinyl group, a phenanthridinyl group, an acridinyl group, a phenanthrolinyl group, a phenazinyl group, a benzimidazolyl group, an isobenzothiazolyl group, a benzoxazolyl group, an isobenzoxazolyl group, a triazolyl group, a tetrazolyl group, an imidazopyridinyl group, an imidazopyrimidinyl group, and an azacarbazolyl group, but embodiments of the present disclosure are not limited thereto.

In one or more embodiments, xe1 and xe611 to xe613 in Formulae 601 and 601-1 may each independently be 0, 1, or 2.

For example, R₆₀₁ and R₆₁₁ to R₆₁₃ in Formulae 601 and 601-1 may each independently be selected from: a substituted or unsubstituted C₃-C₁₀ cycloalkyl group, a substituted or unsubstituted C₁-C₁₀ heterocycloalkyl group, a substituted or unsubstituted C₃-C₁₀ cycloalkenyl group, a substituted or unsubstituted C₁-C₁₀ heterocycloalkenyl group, a substituted or unsubstituted C₆-C₃₀ aryl group, a substituted or unsubstituted C₆-C₃₀ aryloxy group, a substituted or unsubstituted C₆-C₃₀ arylthio group, a substituted or unsubstituted C₁-C₂₀ heteroaryl group, a substituted or unsubstituted monovalent non-aromatic condensed polycyclic group, a substituted or unsubstituted monovalent non-aromatic condensed heteropolycyclic group, - Si(Q₆₀₁)(Q₆₀₂)(Q₆₀₃), -C(=O)(Q₆₀₁), -S(=O)₂(Q₆₀₁), and -P(=O)(Q₆₀₁)(Q₆₀₂).

In one or more embodiments, R₆₀₁ and R₆₁₁ to R₆₁₃ in Formulae 601 and 601-1 may each independently be selected from:
a phenyl group, a biphenyl group, a terphenyl group, a naphthyl group, a fluorenyl group, a spiro-bifluorenyl group, a benzofluorenyl group, a dibenzofluorenyl group, a phenanthrenyl group, an anthracenyl group, a fluoranthenyl group, a triphenylenyl group, a pyrenyl group, a chrysenyl group, a perylenyl group, a pentaphenyl group, a hexacenyl group, a pentacenyl group, a thiophenyl group, a furanyl group, a carbazolyl group, an indolyl group, an isoindolyl group, a benzofuranyl group, a benzothiophenyl group, a dibenzofuranyl group, a dibenzothiophenyl group, a benzocarbazolyl group, a dibenzocarbazolyl group, a dibenzosilolyl group, a pyridinyl group, an imidazolyl group, a pyrazolyl group, a thiazolyl group, an isothiazolyl group, an oxazolyl group, an isoxazolyl group, a thiadiazolyl group, an oxadiazolyl group, a pyrazinyl group, a pyrimidinyl group, a pyridazinyl group, a triazinyl group, a quinolinyl group, an isoquinolinyl group, a benzoquinolinyl group, a phthalazinyl group, a naphthyridinyl group, a quinoxalinyl group, a quinazolinyl group, a cinnolinyl group, a phenanthridinyl group, an acridinyl group, a phenanthrolinyl group, a phenazinyl group, a benzimidazolyl group, an isobenzothiazolyl group, a benzoxazolyl group, an isobenzoxazolyl group, a triazolyl group, a tetrazolyl group, an imidazopyridinyl group, an imidazopyrimidinyl group, and an azacarbazolyl group;
a phenyl group, a biphenyl group, a terphenyl group, a naphthyl group, a fluorenyl group, a spiro-bifluorenyl group, a benzofluorenyl group, a dibenzofluorenyl group, a phenanthrenyl group, an anthracenyl group, a fluoranthenyl group, a triphenylenyl group, a pyrenyl group, a chrysenyl group, a perylenyl group, a pentaphenyl group, a hexacenyl group, a pentacenyl group, a thiophenyl group, a furanyl group, a carbazolyl group, an indolyl group, an isoindolyl group, a benzofuranyl group, a benzothiophenyl group, a dibenzofuranyl group, a dibenzothiophenyl group, a benzocarbazolyl group, a dibenzocarbazolyl group, a dibenzosilolyl group, a pyridinyl group, an imidazolyl group, a pyrazolyl group, a thiazolyl group, an isothiazolyl group, an oxazolyl group, an isoxazolyl group, a thiadiazolyl group, an oxadiazolyl group, a pyrazinyl group, a pyrimidinyl group, a pyridazinyl group, a triazinyl group, a quinolinyl group, an isoquinolinyl group, a benzoquinolinyl group, a phthalazinyl group, a naphthyridinyl group, a quinoxalinyl group, a quinazolinyl group, a cinnolinyl group, a phenanthridinyl group, an acridinyl group, a phenanthrolinyl group, a phenazinyl group, a benzimidazolyl group, an isobenzothiazolyl group, a benzoxazolyl group, an isobenzoxazolyl group, a triazolyl group, a tetrazolyl group, an imidazopyridinyl group, an imidazopyrimidinyl group, and an azacarbazolyl group, each substituted with at least one selected from deuterium, -F, -Cl, -Br, -I, a hydroxyl group, a cyano group, a nitro group, an amidino group, a hydrazino group, a hydrazono group, a C₁-C₂₀ alkyl group, a C₁-C₂₀ alkoxy group, a phenyl group, a biphenyl group, a terphenyl group, a naphthyl group, a fluorenyl group, a spiro-bifluorenyl group, a benzofluorenyl group, a dibenzofluorenyl group, a phenanthrenyl group, an anthracenyl group, a fluoranthenyl group, a triphenylenyl group, a pyrenyl group, a chrysenyl group, a perylenyl group, a pentaphenyl group, a hexacenyl group, a pentacenyl group, a thiophenyl group, a furanyl group, a carbazolyl group, an indolyl group, an isoindolyl group, a benzofuranyl group, a benzothiophenyl group, a dibenzofuranyl group, a dibenzothiophenyl group, a benzocarbazolyl group, a dibenzocarbazolyl group, a dibenzosilolyl group, a pyridinyl group, an imidazolyl group, a pyrazolyl group, a thiazolyl group, an isothiazolyl group, an oxazolyl group, an isoxazolyl group, a thiadiazolyl group, an oxadiazolyl group, a pyrazinyl group, a pyrimidinyl group, a pyridazinyl group, a triazinyl group, a quinolinyl group, an isoquinolinyl group, a benzoquinolinyl group, a phthalazinyl group, a naphthyridinyl group, a quinoxalinyl group, a quinazolinyl group, a cinnolinyl group, a phenanthridinyl group, an acridinyl group, a phenanthrolinyl group, a phenazinyl group, a benzimidazolyl group, an isobenzothiazolyl group, a benzoxazolyl group, an isobenzoxazolyl group, a triazolyl group, a tetrazolyl group, an imidazopyridinyl group, an imidazopyrimidinyl group, and an azacarbazolyl group; and
-S(=O)₂(Q₆₀₁) and -P(=O)(Q₆₀₁)(Q₆₀₂), and
Q₆₀₁ and Q₆₀₂ may each independently be the same as described above.

The electron transport region may include, in addition to the second compound and the third compound, at least one compound selected from Compounds ET1 to ET36, but embodiments of the present disclosure are not limited thereto:

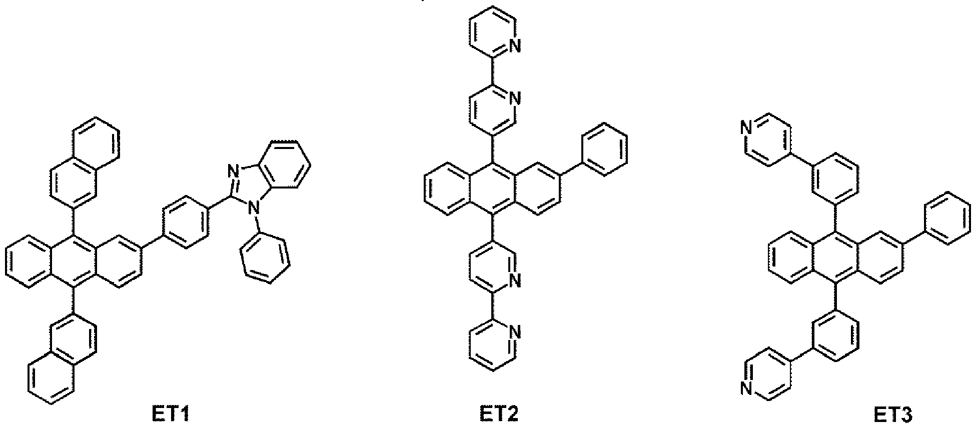

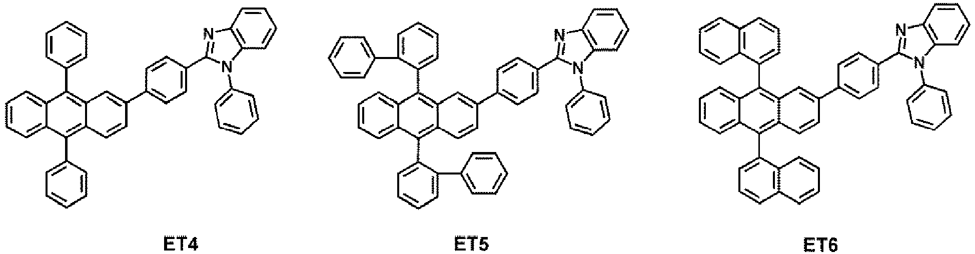

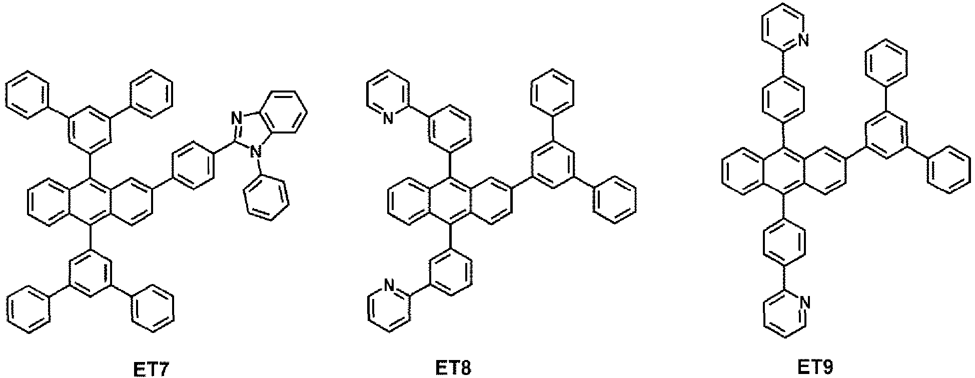

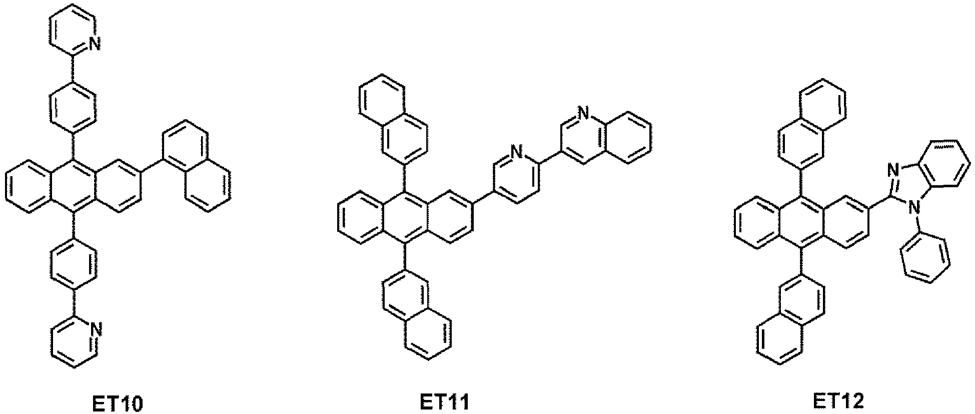

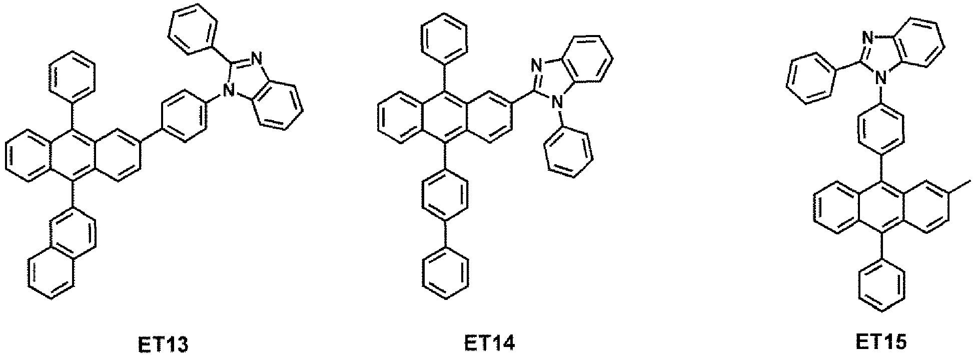

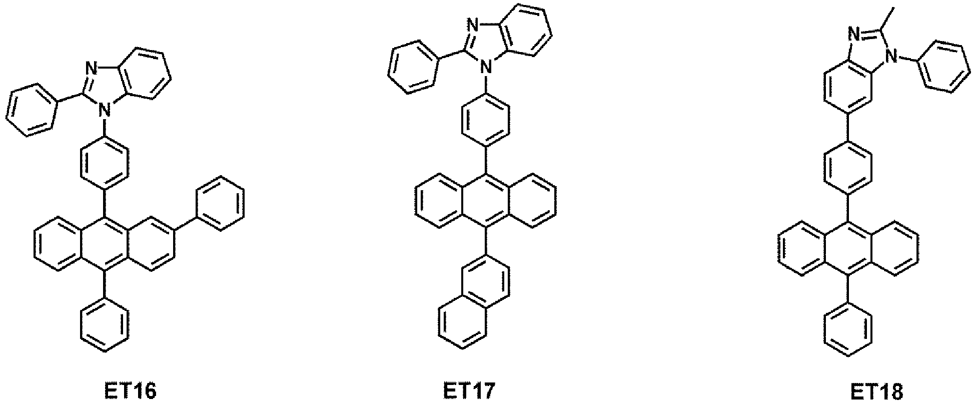

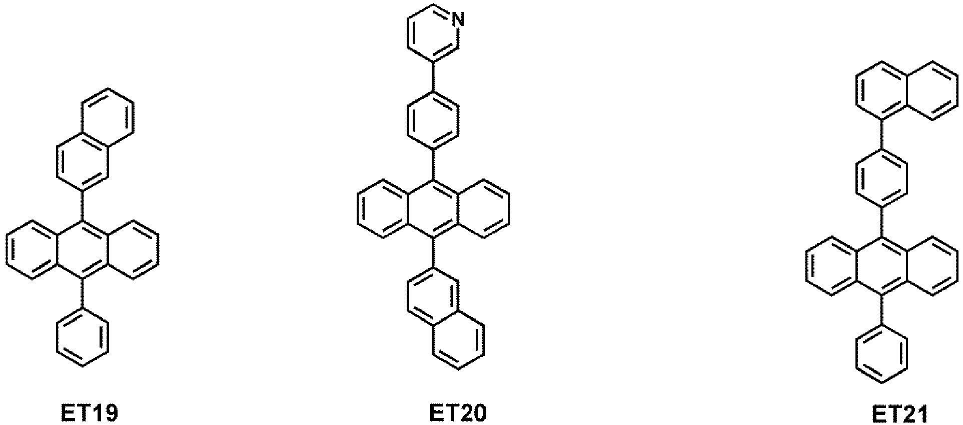

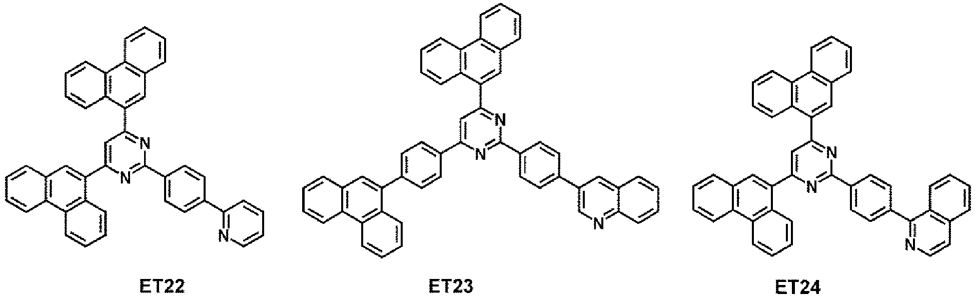

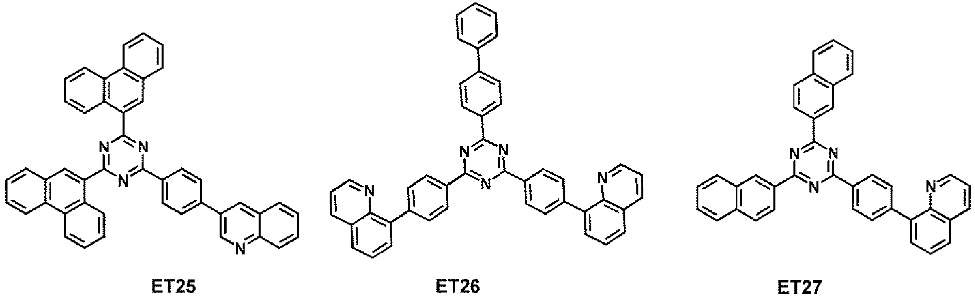

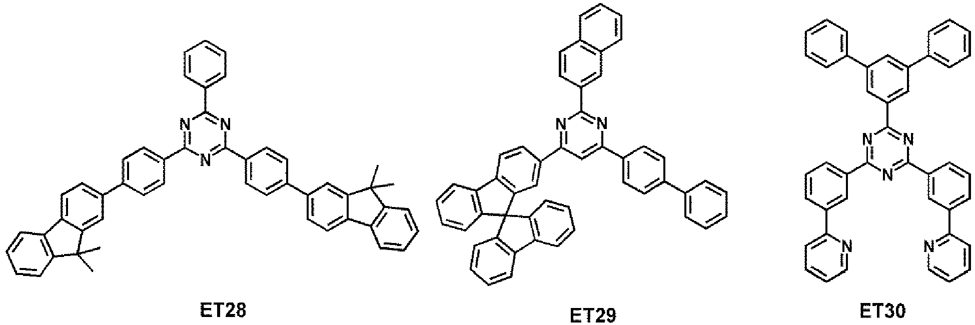

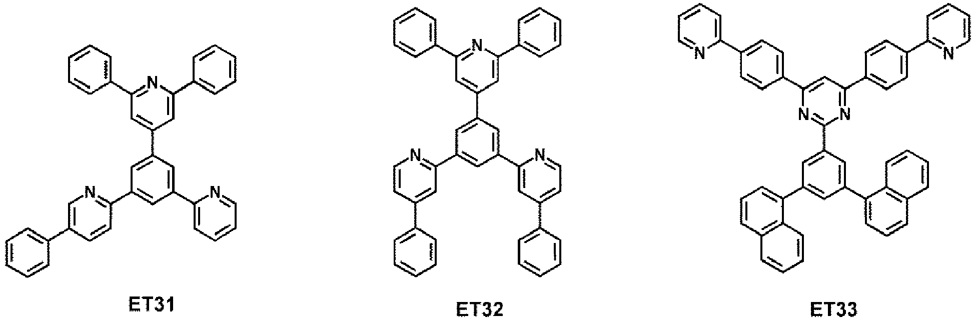

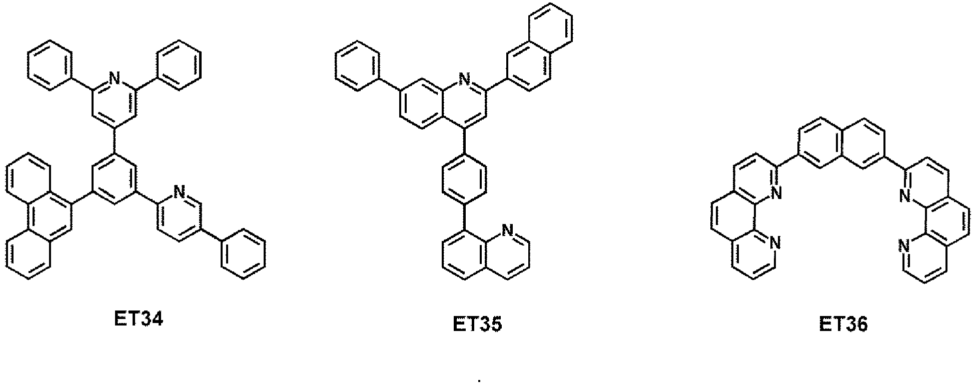

In one or more embodiments, the electron transport region may include at least one selected from 2,9-dimethyl-4,7-diphenyl-1,10-phenanthroline (BCP), 4,7-diphenyl-1,10-phenanthroline (Bphen), Alq₃, BAlq, 3-(biphenyl-4-yl)-5-(4-tert-butylphenyl)-4-phenyl-4H-1,2,4-triazole (TAZ), NTAZ, and diphenyl(4-(triphenylsilyl)phenyl)-phosphine oxide (TSPO1):

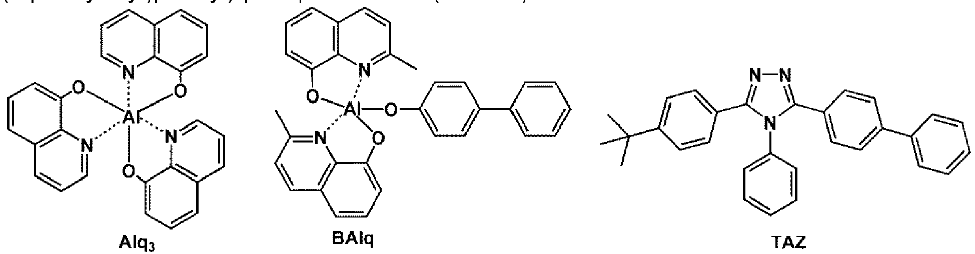

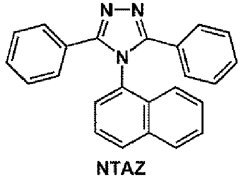

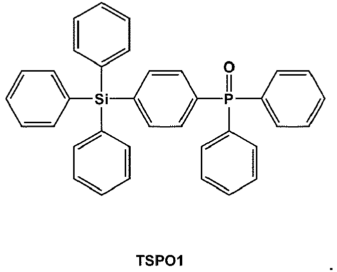

The thickness of the buffer layer, the hole blocking layer, or the electron controlling layer may each independently be in a range of about 20 Å to about 1,000 Å, for example, about 20 Å to about 700 Å, about 20 Å to about 500 Å, about 20 Å to about 400 Å, about 30 Å to about 400 Å or about 30 Å to about 300 Å. When the thicknesses of the buffer layer, the hole blocking layer, and the electron control layer are within the foregoing ranges, the electron blocking layer may have excellent electron blocking characteristics or electron control characteristics without a substantial increase in driving voltage.

A thickness of the electron transport layer may be in a range of about 100 Å to about 1,000 Å, for example, about 100 Å to about 700 Å, about 100 Å to about 600 Å, about 150 Å to about 600 Å, about 150 Å to about 500 Å or about 250 Å to about 350 Å. When the thickness of the electron transport layer is within the range described above, the electron transport layer may have suitable or satisfactory electron transport characteristics without a substantial increase in driving voltage.

The electron transport region (for example, the electron transport layer in the electron transport region) may further include, in addition to the materials described above, a metal-containing material.

The metal-containing material may include at least one selected from alkali metal complex and alkaline earth-metal complex. The alkali metal complex may include a metal ion selected from a Li ion, a Na ion, a K ion, a Rb ion, and a Cs ion, and the alkaline earth-metal complex may include a metal ion selected from a Be ion, a Mg ion, a Ca ion, a Sr ion, and a Ba ion. A ligand coordinated with the metal ion of the alkali metal complex or the alkaline earth-metal complex may be selected from a hydroxy quinoline, a hydroxy isoquinoline, a hydroxy benzoquinoline, a hydroxy acridine, a hydroxy phenanthridine, a hydroxy phenyloxazole, a hydroxy phenylthiazole, a hydroxy diphenyloxadiazole, a hydroxy diphenylthiadiazole, a hydroxy phenylpyridine, a hydroxy phenylbenzimidazole, a hydroxy phenylbenzothiazole, a bipyridine, a phenanthroline, and a cyclopentadiene, but embodiments of the present disclosure are not limited thereto.

For example, the metal-containing material may include a Li complex. The Li complex may include, for example, Compound ET-D1 (lithium quinolate, LiQ) or ET-D2:

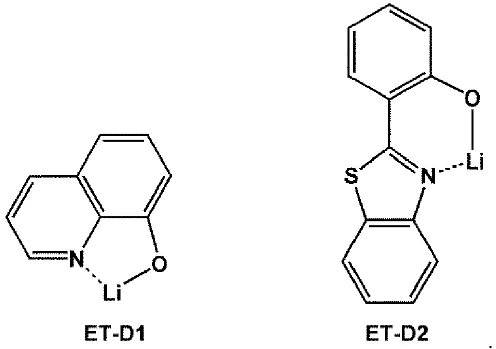

The electron transport region may include an electron injection layer that facilitates injection of electrons from the second electrode 190. The electron injection layer may directly contact the second electrode 190.

The electron injection layer may have i) a single-layered structure including a single layer including a single material, ii) a single-layered structure including a single layer including a plurality of different materials, or iii) a multi-layered structure having a plurality of layers including a plurality of different materials.

The electron injection layer may include an alkali metal, an alkaline earth metal, a rare earth metal, an alkali metal compound, an alkaline earth-metal compound, a rare earth metal compound, an alkali metal complex, an alkaline earth-metal complex, a rare earth metal complex, or any combinations thereof.

The alkali metal may be selected from Li, Na, K, Rb, and Cs. In one embodiment, the alkali metal may be Li, Na, or Cs. In one or more embodiments, the alkali metal may be Li or Cs, but embodiments of the present disclosure are not limited thereto.

The alkaline earth metal may be selected from Mg, Ca, Sr, and Ba.

The rare earth metal may be selected from Sc, Y, Ce, Tb, Yb, and Gd.

The alkali metal compound, the alkaline earth-metal compound, and the rare earth metal compound may be selected from oxides and halides (for example, fluorides, chlorides, bromides, or iodides) of the alkali metal, the alkaline earth-metal, and the rare earth metal.

The alkali metal compound may be selected from alkali metal oxides, such as Li₂O, Cs₂O, or K₂O, and alkali metal halides, such as LiF, NaF, CsF, KF, Lil, Nal, Csl, KI, or RbI. In one embodiment, the alkali metal compound may be selected from LiF, Li₂O, NaF, Lil, Nal, Csl, and KI, but embodiments of the present disclosure are not limited thereto.

The alkaline earth-metal compound may be selected from alkaline earth-metal oxides, such as BaO, SrO, CaO, BaₓSr₁₋ₓO (0<x<1), or BaₓCa₁₋ₓO (0<x<1). In one embodiment, the alkaline earth-metal compound may be selected from BaO, SrO, and CaO, but embodiments of the present disclosure are not limited thereto.

The rare earth metal compound may be selected from YbF₃, ScF₃, ScO₃, Y₂O₃, Ce₂O₃, GdF₃, and TbF₃. In one embodiment, the rare earth metal compound may be selected from YbF₃, ScF₃, TbF₃, YbI₃, ScI₃, and TbI₃, but embodiments of the present disclosure are not limited thereto.

The alkali metal complex, the alkaline earth-metal complex, and the rare earth metal complex may include an ion of alkali metal, alkaline earth-metal, and rare earth metal as described above, and a ligand coordinated with a metal ion of the alkali metal complex, the alkaline earth-metal complex, or the rare earth metal complex may be selected from hydroxy quinoline, hydroxy isoquinoline, hydroxy benzoquinoline, hydroxy acridine, hydroxy phenanthridine, hydroxy phenyloxazole, hydroxy phenylthiazole, hydroxy diphenyloxadiazole, hydroxy diphenylthiadiazole, hydroxy phenylpyridine, hydroxy phenylbenzimidazole, hydroxy phenylbenzothiazole, bipyridine, phenanthroline, and cyclopentadiene, but embodiments of the present disclosure are not limited thereto.

The electron injection layer may consist of an alkali metal, an alkaline earth metal, a rare earth metal, an alkali metal compound, an alkaline earth-metal compound, a rare earth metal compound, an alkali metal complex, an alkaline earth-metal complex, a rare earth metal complex, or any combinations thereof, as described above. In one or more embodiments, the electron injection layer may further include an organic material. When the electron injection layer further includes an organic material, an alkali metal, an alkaline earth metal, a rare earth metal, an alkali metal compound, an alkaline earth-metal compound, a rare earth metal compound, an alkali metal complex, an alkaline earth-metal complex, a rare earth metal complex, or any combinations thereof may be homogeneously or non-homogeneously dispersed in a matrix including the organic material.

A thickness of the hole injection layer may be in a range of about 1 Å to about 100 Å, for example, about 3 Å to about 90 Å or about 3 Å to about 20 Å. When the thickness of the electron injection layer is within the range described above, the electron injection layer may have suitable or satisfactory electron injection characteristics without a substantial increase in driving voltage.

### Second electrode 190

The second electrode 190 may be disposed on the organic layer 150 having such a structure. The second electrode 190 may be a cathode which is an electron injection electrode, and in this regard, a material for forming the second electrode 190 may be selected from metal, an alloy, an electrically conductive compound, and a combination thereof, which have a relatively low work function.

The second electrode 190 may include at least one selected from lithium (Li), silver (Ag), magnesium (Mg), aluminium (Al), aluminium-lithium (Al-Li), calcium (Ca), magnesium-indium (Mg-In), magnesium-silver (Mg-Ag), ITO, and IZO, but embodiments of the present disclosure are not limited thereto. The second electrode 190 may be a transmissive electrode, a semi-transmissive electrode, or a reflective electrode.

The first electrode 190 may have a single-layered structure, or a multi-layered structure including two or more layers.

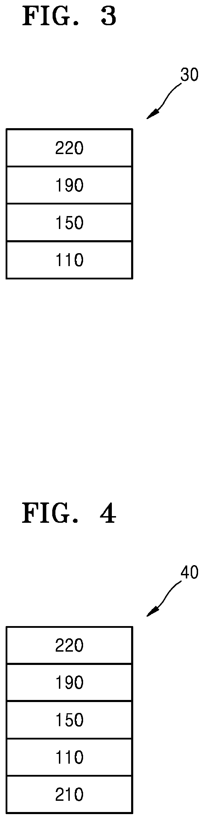

### Description of FIGS. 2 and 4

An organic light-emitting device 20 of FIG. 2 includes a first capping layer 210, a first electrode 110, an organic layer 150, and a second electrode 190 which are sequentially stacked in this stated order. An organic light-emitting device 30 of FIG. 3 includes a first electrode 110, an organic layer 150, a second electrode 190, and a second capping layer 220 which are sequentially stacked in this stated order. An organic light-emitting device 40 of FIG. 4 includes a first capping layer 210, a first electrode 110, an organic layer 150, a second electrode 190, and a second capping layer 220.

Regarding FIGS. 2-4, the first electrode 110, the organic layer 150, and the second electrode 190 may be understood by referring to the description presented in connection with FIG. 1.

In the organic layer 150 of each of the organic light-emitting devices 20 and 40, light generated in an emission layer may pass through the first electrode 110, which is a semi-transmissive electrode or a transmissive electrode, and the first capping layer 210 toward the outside, and in the organic layer 150 of each of the organic light-emitting devices 30 and 40, light generated in an emission layer may pass through the second electrode 190, which is a semi-transmissive electrode or a transmissive electrode, and the second capping layer 220 toward the outside.

The first capping layer 210 and the second capping layer 220 may increase external luminescent efficiency according to the principle of constructive interference.

The first capping layer 210 and the second capping layer 220 may each independently be an organic capping layer including an organic material, an inorganic capping layer including an inorganic material, or a composite capping layer including an organic material and an inorganic material.

At least one selected from the first capping layer 210 and the second capping layer 220 may each independently include at least one material selected from carbocyclic compounds, heterocyclic compounds, amine-based compounds, porphyrine derivatives, phthalocyanine derivatives, a naphthalocyanine derivatives, alkali metal complexes, and alkaline earth-based complexes. The carbocyclic compound, the heterocyclic compound, and the amine-based compound may be optionally substituted with a substituent containing at least one element selected from O, N, S, Se, Si, F, Cl, Br, and I. In one embodiment, at least one selected from the first capping layer 210 and the second capping layer 220 may each independently include an amine-based compound.

In one embodiment, at least one selected from the first capping layer 210 and the second capping layer 220 may each independently include the compound represented by Formula 201 or the compound represented by Formula 202.

In one or more embodiments, at least one selected from the first capping layer 210 and the second capping layer 220 may each independently include a compound selected from Compounds HT28 to HT33 and Compounds CP1 to CP5, but embodiments of the present disclosure are not limited thereto:

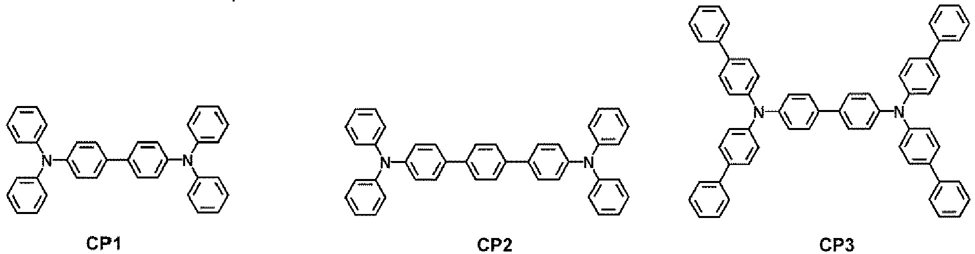

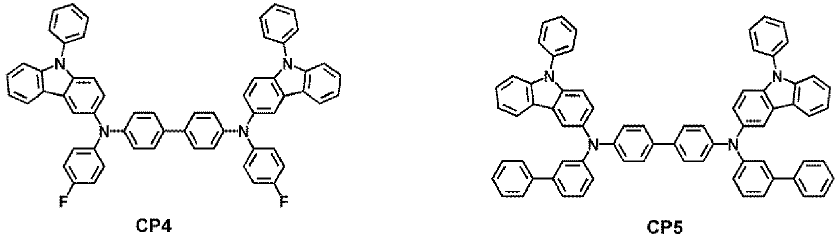

Hereinbefore, the organic light-emitting device according to an embodiment has been described in connection with FIGS. 1 and 4. However, embodiments of the present disclosure are not limited thereto.

Layers constituting the hole transport region, an emission layer, and layers constituting the electron transport region may be formed in a certain region by using one or more suitable methods selected from vacuum deposition, spin coating, casting, Langmuir-Blodgett (LB) deposition, ink-jet printing, laser-printing, and laser-induced thermal imaging.

When layers constituting the hole transport region, an emission layer, and layers constituting the electron transport region are formed by vacuum deposition, the deposition may be performed at a deposition temperature of about 100 °C to about 500 °C, a vacuum degree of about 10⁻⁸ torr to about 10⁻³ torr, and a deposition speed of about 0.01 Å/sec to about 100 Å/sec by taking into account a material to be included in a layer to be formed, and the structure of a layer to be formed.

When layers constituting the hole transport region, an emission layer, and layers constituting the electron transport region are formed by spin coating, the spin coating may be performed at a coating speed of about 2,000 rpm to about 5,000 rpm and at a heat treatment temperature of about 80 °C to about 200 °C by taking into account a material to be included in a layer to be formed, and the structure of a layer to be formed.

### General definition of some of the substituents

The term "C₁-C₆₀ alkyl group," as used herein, refers to a linear or branched saturated aliphatic hydrocarbon monovalent group having 1 to 60 carbon atoms, and examples thereof include a methyl group, an ethyl group, a propyl group, an isobutyl group, a sec-butyl group, a tert-butyl group, a pentyl group, an isoamyl group, and a hexyl group. The term "C₁-C₆₀ alkylene group," as used herein, refers to a divalent group having substantially the same structure as the C₁-C₆₀ alkyl group. Corresponding definitions apply to other ranges given for the number of carbon atoms in an alkyl/alkylene group.

The term "C₂-C₆₀ alkenyl group," as used herein, refers to a hydrocarbon group having at least one carbon-carbon double bond at a main chain (e.g., in the middle) or at a terminus of the C₂-C₆₀ alkyl group, and examples thereof include an ethenyl group, a propenyl group, and a butenyl group. The term "C₂-C₆₀ alkenylene group," as used herein, refers to a divalent group having substantially the same structure as the C₂-C₆₀ alkenyl group. Corresponding definitions apply to other ranges given for the number of carbon atoms in an alkenyl/alkenylene group.

The term "C₂-C₆₀ alkynyl group," as used herein, refers to a hydrocarbon group having at least one carbon-carbon triple bond at a main chain (e.g., in the middle) or at a terminus of the C₂-C₆₀ alkyl group, and examples thereof include an ethynyl group, and a propynyl group. The term "C₂-C₆₀ alkynylene group," as used herein, refers to a divalent group having substantially the same structure as the C₂-C₆₀ alkynyl group. Corresponding definitions apply to other ranges given for the number of carbon atoms in an alkynyl/alkynylene group.

The term "C₁-C₆₀ alkoxy group," as used herein, refers to a monovalent group represented by -OA₁₀₁ (wherein A₁₀₁ is the C₁-C₆₀ alkyl group), and examples thereof include a methoxy group, an ethoxy group, and an isopropyloxy group. Corresponding definitions apply to other ranges given for the number of carbon atoms in an alkoxy group.

The term "C₃-C₁₀ cycloalkyl group," as used herein, refers to a monovalent saturated hydrocarbon monocyclic group having 3 to 10 carbon atoms, and examples thereof include a cyclopropyl group, a cyclobutyl group, a cyclopentyl group, a cyclohexyl group, and a cycloheptyl group. The term "C₃-C₁₀ cycloalkylene group," as used herein, refers to a divalent group having substantially the same structure as the C₃-C₁₀ cycloalkyl group.

The term "C1-C10 heterocycloalkyl group," as used herein, refers to a monovalent monocyclic group having at least one heteroatom selected from N, O, Si, P, and S as a ring-forming atom and 1 to 10 carbon atoms, and examples thereof include a 1,2,3,4-oxatriazolidinyl group, a tetrahydrofuranyl group, and a tetrahydrothiophenyl group. The term "C1-C10 heterocycloalkylene group," as used herein, refers to a divalent group having substantially the same structure as the C1-C10 heterocycloalkyl group.

The term "C₃-C₁₀ cycloalkenyl group," as used herein, refers to a monovalent monocyclic group that has 3 to 10 carbon atoms and at least one double bond in the ring thereof and no aromaticity (e.g., the entire ring, group, and/or molecule is not aromatic), and examples thereof include a cyclopentenyl group, a cyclohexenyl group, and a cycloheptenyl group. The term "C₃-C₁₀ cycloalkenylene group," as used herein, refers to a divalent group having substantially the same structure as the C₃-C₁₀ cycloalkenyl group.

The term "C₁-C₁₀ heterocycloalkenyl group," as used herein, refers to a monovalent monocyclic group that has at least one heteroatom selected from N, O, Si, P, and S as a ring-forming atom, 1 to 10 carbon atoms, and at least one double bond in its ring. Non-limiting examples of the C₁-C₁₀ heterocycloalkenyl group include a 4,5-dihydro-1 ,2,3,4-oxatriazolyl group, a 2,3-dihydrofuranyl group, and a 2,3-dihydrothiophenyl group. The term "C₁-C₁₀ heterocycloalkenylene group," as used herein, refers to a divalent group having substantially the same structure as the C₁-C₁₀ heterocycloalkenyl group.

The term "C₆-C₆₀ aryl group," as used herein, refers to a monovalent group having a carbocyclic aromatic system having 6 to 60 carbon atoms, and a C₆-C₆₀ arylene group used herein refers to a divalent group having a carbocyclic aromatic system having 6 to 60 carbon atoms. Examples of the C₆-C₆₀ aryl group are a phenyl group, a naphthyl group, an anthracenyl group, a phenanthrenyl group, a pyrenyl group, and a chrysenyl group. When the C₆-C₆₀ aryl group and the C₆-C₆₀ arylene group each include two or more rings, the rings may be fused to each other (e.g., combined together). Corresponding definitions apply to other ranges given for the number of carbon atoms in an aryl/arylene group.

The term "C₁-C₆₀ heteroaryl group," as used herein, refers to a monovalent group having a carbocyclic aromatic system that has at least one heteroatom selected from N, O, Si, P, and S as a ring-forming atom, in addition to 1 to 1 carbon atoms. The term "C₁-C₆₀ heteroarylene group," as used herein, refers to a divalent group having a carbocyclic aromatic system that has at least one heteroatom selected from N, O, Si, P, and S as a ring-forming atom, in addition to 1 to 60 carbon atoms. Examples of the C₁-C₆₀ heteroaryl group are a pyridinyl group, a pyrimidinyl group, a pyrazinyl group, a pyridazinyl group, a triazinyl group, a quinolinyl group, and an isoquinolinyl group. When the C₁-C₆₀ heteroaryl group and the C₁-C₆₀ heteroarylene group each include two or more rings, the rings may be fused to each other (e.g., combined together). Corresponding definitions apply to other ranges given for the number of carbon atoms in an heteroaryl/heteroarylene group.

The term "C₆-C₆₀ aryloxy group," as used herein, indicates -OA₁₀₂ (wherein A₁₀₂ is the C₆-C₆₀ aryl group), and a C₆-C₆₀ arylthio group indicates -SA₁₀₃ (wherein A₁₀₃ is the C₆-C₆₀ aryl group). Corresponding definitions apply to other ranges given for the number of carbon atoms in an aryloxy group and an arylthio group.

The term "C₁-C₆₀ heteroaryloxy group," as used herein, indicates -OA₁₀₄ (wherein A₁₀₄ is the C₁-C₆₀ heteroaryl group), and the term " C₆-C₆₀ heteroarylthio group" as used herein indicates -SA₁₀₅ (wherein A₁₀₅ is the C₁-C₆₀ heteroaryl group). Corresponding definitions apply to other ranges given for the number of carbon atoms in a heteroaryloxy group and a heteroarylthio group.

The term "monovalent non-aromatic condensed polycyclic group," as used herein, refers to a monovalent group (for example, having 8 to 60 carbon atoms) having two or more rings condensed with each other (e.g., combined together), only carbon atoms as ring-forming atoms, and no aromaticity in its entire molecular structure (e.g., the entire group and/or molecule is not aromatic). An example of the monovalent non-aromatic condensed polycyclic group is a fluorenyl group. The term "divalent non-aromatic condensed polycyclic group," as used herein, refers to a divalent group having substantially the same structure as the monovalent non-aromatic condensed polycyclic group.

The term "monovalent non-aromatic condensed heteropolycyclic group," as used herein, refers to a monovalent group (for example, having 1 to 60 carbon atoms) having two or more rings condensed to each other (e.g., combined together), at least one heteroatom selected from N, O, Si, P, and S, other than carbon atoms, as a ring-forming atom, and no aromaticity in its entire molecular structure (e.g., the entire group and/or molecule is not aromatic). An example of the monovalent non-aromatic condensed heteropolycyclic group is a carbazolyl group. The term "divalent non-aromatic condensed heteropolycyclic group," as used herein, refers to a divalent group having substantially the same structure as the monovalent non-aromatic condensed heteropolycyclic group.

The term "C₅-C₆₀ carbocyclic group," as used herein, refers to a monocyclic or polycyclic group having 5 to 60 carbon atoms in which a ring-forming atom is a carbon atom only. The C₆-C₆₀ carbocyclic group may be an aromatic carbocyclic group or a non-aromatic carbocyclic group. The C₆-C₆₀ carbocyclic group may be a ring, such as benzene, a monovalent group, such as a phenyl group, or a divalent group, such as a phenylene group. In one or more embodiments, depending on the number of substituents connected to the C₆-C₆₀ carbocyclic group, the C₆-C₆₀ carbocyclic group may be a trivalent group or a quadrivalent group. Corresponding definitions apply to other ranges given for the number of carbon atoms in a carbocyclic group.

The term "C₁-C₆₀ heterocyclic group," as used herein, refers to a group having substantially the same structure as the C₆-C₆₀ carbocyclic group, except that as a ring-forming atom, at least one heteroatom selected from N, O, Si, P, and S is used in addition to carbon (the number of carbon atoms may be in a range of 1 to 60). Corresponding definitions apply to other ranges given for the number of carbon atoms in a heterocyclic group.

At least one substituent of the substituted C₅-C₆₀ (*e.g.* C₅-C₃₀) carbocyclic group, the substituted C₁-C₆₀ (*e.g.* C₁-C₂₀) heterocyclic group, the substituted C₃-C₁₀ cycloalkylene group, the substituted C₁-C₁₀ heterocycloalkylene group, the substituted C₃-C₁₀ cycloalkenylene group, the substituted C₁-C₁₀ heterocycloalkenylene group, the substituted C₆-C₆₀ (*e.g.* C₆-C₃₀) arylene group, the substituted C₁-C₆₀ (*e.g.* C₁-C₂₀) heteroarylene group, the substituted divalent non-aromatic condensed polycyclic group, the substituted divalent non-aromatic condensed heteropolycyclic group, the substituted C₁-C₆₀ (*e.g.* C₁-C₂₀) alkyl group, the substituted C₂-C₆₀ (*e.g.* C₂-C₂₀) alkenyl group, the substituted C₂-C₆₀ (*e.g.* C₂-C₂₀) alkynyl group, the substituted C₁-C₆₀ (*e.g.* C₁-C₂₀) alkoxy group, the substituted C₃-C₁₀ cycloalkyl group, the substituted C₁-C₁₀ heterocycloalkyl group, the substituted C₃-C₁₀ cycloalkenyl group, the substituted C₁-C₁₀ heterocycloalkenyl group, the substituted C₆-C₆₀ (*e.g.* C₆-C₃₀) aryl group, the substituted C₆-C₆₀ (*e.g.* C₆-C₃₀) aryloxy group, the substituted C₆-C₆₀ (*e.g.* C₆-C₃₀) arylthio group, the substituted C₁-C₆₀ (*e.g.* C₁-C₂₀) heteroaryl group, the substituted C₁-C₆₀ (*e.g.* C₁-C₂₀) heteroaryloxy group, the substituted C₁-C₆₀ (*e.g.* C₁-C₂₀) heteroarylthio group, the substituted monovalent non-aromatic condensed polycyclic group, and the substituted monovalent non-aromatic condensed heteropolycyclic group may be selected from:
deuterium, -F, -Cl, -Br, -I, a hydroxyl group, a cyano group, a nitro group, an amidino group, a hydrazino group, a hydrazono group, a C₁-C₆₀ (*e.g.* C₁-C₂₀) alkyl group, a C₂-C₆₀ (*e.g.* C₂-C₂₀) alkenyl group, a C₂-C₆₀ (*e.g.* C₂-C₂₀) alkynyl group, and a C₁-C₆₀ (*e.g.* C₁-C₂₀) alkoxy group;
a C₁-C₆₀ (e.g. C₁-C₂₀) alkyl group, a C₂-C₆₀ (*e.g.* C₂-C₂₀) alkenyl group, a C₂-C₆₀ (*e.g.* C₂-C₂₀) alkynyl group, and a C₁-C₆₀ (*e.g.* C₁-C₂₀) alkoxy group, each substituted with at least one selected from deuterium, -F, -Cl, -Br, -I, a hydroxyl group, a cyano group, a nitro group, an amidino group, a hydrazino group, a hydrazono group, a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ (*e.g.* C₆-C₃₀) aryl group, a C₆-C₆₀ (*e.g.* C₆-C₃₀) aryloxy group, a C₆-C₆₀ (*e.g.* C₆-C₃₀) arylthio group, a C₁-C₆₀ (*e.g.* C₁-C₂₀) heteroaryl group, a C₁-C₆₀ (*e.g.* C₁-C₂₀) heteroaryloxy group, a C₁-C₆₀ (*e.g.* C₁-C₂₀) heteroarylthio group, a monovalent non-aromatic condensed polycyclic group, a monovalent non-aromatic condensed heteropolycyclic group, -Si(Q₁₁)(Q₁₂)(Q₁₃), - N(Q₁₁)(Q₁₂), -B(Q₁₁)(Q₁₂), -C(=O)(Q₁₁), -S(=O)₂(Q₁₁), and -P(=O)(Q₁₁)(Q₁₂);
a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ (*e.g.* C₆-C₃₀) aryl group, a C₆-C₆₀ (*e.g.* C₆-C₃₀) aryloxy group, a C₆-C₆₀ (*e.g.* C₆-C₃₀) arylthio group, a C₁-C₆₀ (*e.g.* C₁-C₂₀) heteroaryl group, a C₁-C₆₀ (*e.g.* C₁-C₂₀) heteroaryloxy group, a C₁-C₆₀ (*e.g.* C₁-C₂₀) heteroarylthio group, a monovalent non-aromatic condensed polycyclic group, and a monovalent non-aromatic condensed heteropolycyclic group;
a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ (*e.g.* C₆-C₃₀) aryl group, a C₆-C₆₀ (*e.g.* C₆-C₃₀) aryloxy group, a C₆-C₆₀ (*e.g.* C₆-C₃₀) arylthio group, a C₁-C₆₀ (*e.g.* C₁-C₂₀) heteroaryl group, a C₁-C₆₀ (*e.g.* C₁-C₂₀) heteroaryloxy group, a C₁-C₆₀ (*e.g.* C₁-C₂₀) heteroarylthio group, a monovalent non-aromatic condensed polycyclic group, and a monovalent non-aromatic condensed heteropolycyclic group, each substituted with at least one selected from deuterium, -F, -Cl, -Br, -I, a hydroxyl group, a cyano group, a nitro group, an amidino group, a hydrazino group, a hydrazono group, a C₁-C₆₀ (*e.g.* C₁-C₂₀) alkyl group, a C₂-C₆₀ (*e.g.* C₂-C₂₀) alkenyl group, a C₂-C₆₀ (*e.g.* C₂-C₂₀) alkynyl group, a C₁-C₆₀ (*e.g.* C₁-C₂₀) alkoxy group, a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ (*e.g.* C₆-C₃₀) aryl group, a C₆-C₆₀ (*e.g.* C₆-C₃₀) aryloxy group, a C₆-C₆₀ (*e.g.* C₆-C₃₀) arylthio group, a C₁-C₆₀ (*e.g.* C₁-C₂₀) heteroaryl group, a C₁-C₆₀ (*e.g.* C₁-C₂₀) heteroaryloxy group, a C₁-C₆₀ (*e.g.* C₁-C₂₀) heteroarylthio group, a monovalent non-aromatic condensed polycyclic group, a monovalent non-aromatic condensed heteropolycyclic group, -Si(Q₂₁)(Q₂₂)(Q₂₃), -N(Q₂₁)(Q₂₂), - B(Q₂₁)(Q₂₂), -C(=O)(Q₂₁), -S(=O)₂(Q₂₁), and -P(=O)(Q₂₁)(Q₂₂); and
-Si(Q₃₁)(Q₃₂)(Q₃₃), -N(Q₃₁)(Q₃₂), -B(Q₃₁)(Q₃₂), -C(=O)(Q₃₁), -S(=O)₂(Q₃₁), and -P(=O)(Q₃₁)(Q₃₂), and
Q₁₁ to Q₁₃, Q₂₁ to Q₂₃, and Q₃₁ to Q₃₃ may each independently be selected from hydrogen, deuterium, -F, -Cl, -Br, -I, a hydroxyl group, a cyano group, a nitro group, an amidino group, a hydrazino group, a hydrazono group, a C₁-C₆₀ (*e.g.* C₁-C₂₀) alkyl group, a C₂-C₆₀ (*e.g.* C₂-C₂₀) alkenyl group, a C₂-C₆₀ (*e.g.* C₂-C₂₀) alkynyl group, a C₁-C₆₀ (*e.g.* C₁-C₂₀) alkoxy group, a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ (*e.g.* C₆-C₃₀) aryl group, a C₁-C₆₀ (*e.g.* C₁-C₂₀) heteroaryl group, a C₁-C₆₀ (*e.g.* C₁-C₂₀) heteroaryloxy group, a C₁-C₆₀ (*e.g.* C₁-C₂₀) heteroarylthio group, a monovalent non-aromatic condensed polycyclic group, a monovalent non-aromatic condensed heteropolycyclic group, a C₁-C₆₀ (*e.g.* C₁-C₂₀) alkyl group substituted with at least one selected from deuterium, -F, and a cyano group, a C₆-C₆₀ (*e.g*. C₆-C₃₀) aryl group substituted with at least one selected from deuterium, -F, and a cyano group, a biphenyl group, and a terphenyl group.

The term "Ph," as used herein, refers to a phenyl group, the term "Me," as used herein, refers to a methyl group, the term "Et," as used herein, refers to an ethyl group, the term "ter-Bu" or "Bu^{t}," as used herein, refers to a tert-butyl group, and the term "OMe," as used herein, refers to a methoxy group.

The term "biphenyl group," as used herein, refers to "a phenyl group substituted with a phenyl group." In other words, the "biphenyl group" is a substituted phenyl group having a C₆-C₆₀ aryl group as a substituent.

The term "terphenyl group," as used herein, refers to "a phenyl group substituted with a biphenyl group." In other words, the "terphenyl group" is a phenyl group having, as a substituent, a C₆-C₆₀ aryl group substituted with a C₆-C₆₀ aryl group.

* and *' as used herein, unless defined otherwise, each refer to a binding site to a neighboring atom in a corresponding formula.

Hereinafter, a compound according to embodiments and an organic light-emitting device according to embodiments will be described in more detail with reference to Synthesis Examples and Examples. The wording "B was used instead of A" used in describing Synthesis Examples refers to that an identical molar equivalent of B was used in place of A.

### Examples

### Example 1

As an anode, an ITO substrate, on which ITO/Ag/ITO were deposited, was cut to a size of 50 mm x 50 mm x 0.7 mm, sonicated with isopropyl alcohol and pure water each for 5 minutes, and then cleaned by exposure to ultraviolet rays and ozone for 30 minutes. Then, the ITO substrate was provided to a vacuum deposition apparatus.

Compound HT3 and F4-TCNQ were co-deposited on the ITO substrate at a weight ratio of 98:2 to form a hole injection layer having a thickness of 100 Å, Compound HT3 was vacuum-deposited on the hole injection layer to form a first hole transport layer having a thickness of 1,200 Å, and Compound HT18 was deposited on the first hole transport layer to form a second hole transport layer having a thickness of 550 Å.

Compound H-2 and Compound PD27 (weight ratio of 400:3) were co-deposited on the second hole transport layer to form an emission layer having a thickness of 400 Å. Compound HB-1 was deposited on the emission layer to form a buffer layer having a thickness of 50 Å, and Compound ET-1 and LiQ were co-deposited at a weight ratio of 50:50 to form an electron transport layer having a thickness of 310 Å. LiQ was deposited on the electron transport layer to form an electron injection layer having a thickness of 10 Å, and Mg and Ag (weight ratio of 130:10) were co-deposited on the electron injection layer to form a cathode having a thickness of 130 Å, thereby completing the manufacture of an organic light-emitting device.

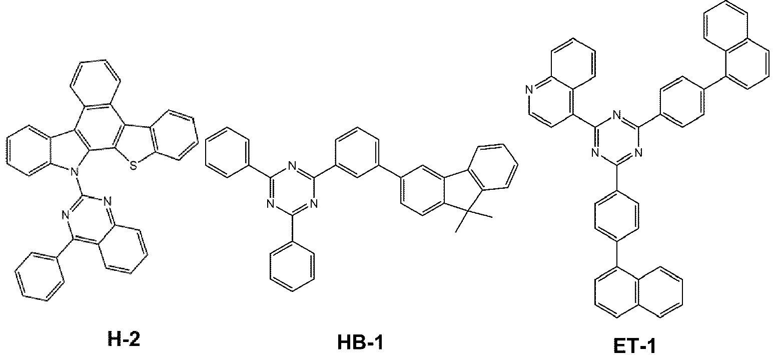

### Examples 2 to 7

Organic light-emitting devices were manufactured in substantially the same manner as in Example 1, except that Compounds shown in Table 1 were each used instead of Compounds H-2, HB-1, and ET-1.

### Example 8

As an anode, an ITO substrate, on which ITO/Ag/ITO were deposited, was cut to a size of 50 mm x 50 mm x 0.7 mm, sonicated with isopropyl alcohol and pure water each for 5 minutes, and then cleaned by exposure to ultraviolet rays and ozone for 30 minutes. Then, the ITO substrate was provided to a vacuum deposition apparatus.

Compound HT3 and F4-TCNQ were vacuum-deposited on the ITO substrate at a weight ratio of 98:2 to form a hole injection layer having a thickness of 100 Å, Compound HT3 was vacuum-deposited on the hole injection layer to form a first hole transport layer having a thickness of 1,200 Å, and Compound HT18 was deposited on the first hole transport layer to form a second hole transport layer having a thickness of 250 Å.

Compound H-7 and Compound PD26 (weight ratio of 400:10) were co-deposited on the second hole transport layer to form an emission layer having a thickness of 400 Å. Compound HB-1 was deposited on the emission layer to form a buffer layer having a thickness of 50 Å, and Compound ET-1 and LiQ were co-deposited at a weight ratio of 50:50 to form an electron transport layer having a thickness of 310 Å. LiQ was deposited on the electron transport layer to form an electron injection layer having a thickness of 10 Å, and Mg and Ag (weight ratio of 130:10) were co-deposited on the electron injection layer to form a cathode having a thickness of 130 Å, thereby completing the manufacture of an organic light-emitting device.

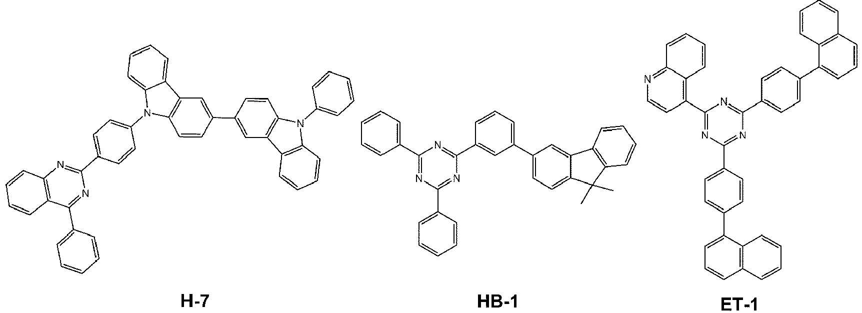

### Examples 9 to 11

Organic light-emitting devices were manufactured in substantially the same manner as in Example 8, except that Compounds shown in Table 1 were each used instead of Compounds H-7, HB-1, and ET-1.

### Comparative Examples 1 to 4

Organic light-emitting devices were manufactured in substantially the same manner as in Example 1, except that Compounds shown in Table 1 were each used instead of Compounds H-2, HB-1, and ET-1.

### Comparative Examples 5 to 7

Organic light-emitting devices were manufactured in substantially the same manner as in Example 8, except that Compounds shown in Table 1 were each used instead of Compounds H-7, HB-1, and ET-1.

### Evaluation Example

The driving voltage, luminance, efficiency, and lifespan of the organic light-emitting devices manufactured according to Examples 1 to 11 and Comparative Examples 1 to 7 were measured at a current density of 50 mA/cm² by using a Keithley SMU 236 and a luminance meter PR650, and results thereof are shown in Table 1. The lifespan T₉₇ indicates an amount of time that elapsed, during continuous operation of the organic light-emitting device, from initial luminance of the organic light-emitting device until when the luminance of the organic light-emitting device was 97% of initial luminance (100 %).

**Table 1**

| | Emission layer | Buffer layer | Electron transport layer | Driving voltage (V) | Luminance (cd/m²) | Efficiency (cd/A) | x coordinate | Emission color | T₉₇ (hr) |
|---|---|---|---|---|---|---|---|---|---|
| Example 1 | H-2 | HB-1 | ET-1 | 3.8 | 5500 | 50 | 0.665 | Red | 900 |
| Example 2 | H-4 | HB-1 | ET-1 | 3.7 | 5500 | 54 | 0.664 | Red | 700 |
| Example 3 | H-5 | HB-1 | ET-1 | 3.7 | 5500 | 55 | 0.666 | Red | 750 |
| Example 4 | H-6 | HB-1 | ET-1 | 3.7 | 5500 | 55 | 0.665 | Red | 720 |
| Example 5 | H-2 | HB-2 | ET-1 | 3.9 | 5500 | 50 | 0.666 | Red | 900 |
| Example 6 | H-4 | HB-2 | ET-1 | 3.8 | 5500 | 54 | 0.665 | Red | 700 |
| Example 7 | H-5 | HB-2 | ET-1 | 3.8 | 5500 | 55 | 0.667 | Red | 750 |
| Example 8 | H-7 | HB-1 | ET-1 | 3.7 | 15000 | 120 | 0.250 | Green | 150 |
| Example 9 | H-8 | HB-3 | ET-1 | 3.8 | 15000 | 100 | 0.252 | Green | 100 |
| Example 10 | H-9 | HB-3 | ET-1 | 3.7 | 15000 | 110 | 0.249 | Green | 100 |
| Example 11 | H-10 | HB-3 | ET-1 | 3.7 | 15000 | 100 | 0.258 | Green | 110 |
| Comparative Example 1 | H-10 | HB-2 | ET-2 | 3.8 | 5500 | 48 | 0663 | Red | 100 |
| Comparative Example 2 | H-4 | X4 | ET-1 | 4.5 | 5500 | 45 | 0.665 | Red | 650 |
| Comparative Example 3 | H-4 | HB-1 | X4 | 4.8 | 5500 | 47 | 0.665 | Red | 600 |
| Comparative Example 4 | H-10 | HB-1 | ET-1 | 3.7 | 5500 | 47 | 0.664 | Red | 90 |
| Comparative Example 5 | X5 | HB-1 | ET-1 | 6.0 | 15000 | 70 | 0.245 | Green | 50 |
| Comparative | H-11 | X4 | ET-1 | 4.1 | 15000 | 100 | 0.249 | Green | 70 |
| Example 6 | | | | | | | | | |
| Comparative Example 7 | H-9 | HB-2 | ET-1 | 3.5 | 15000 | 97 | 0.258 | Green | 20 |

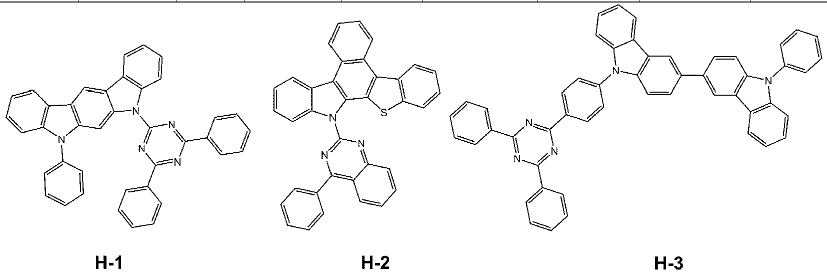

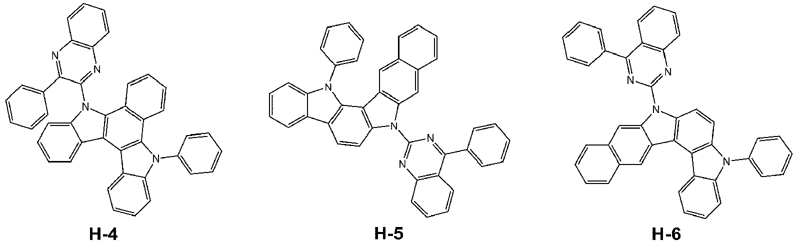

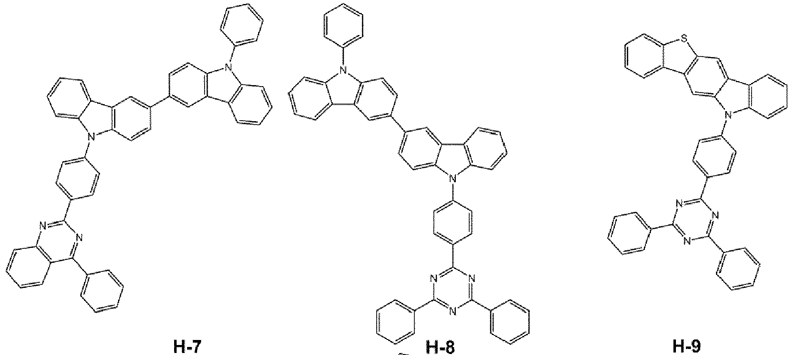

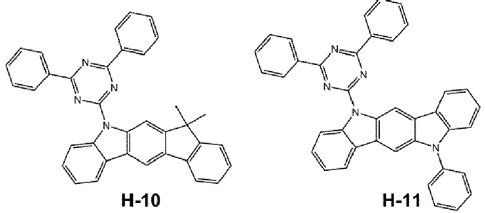

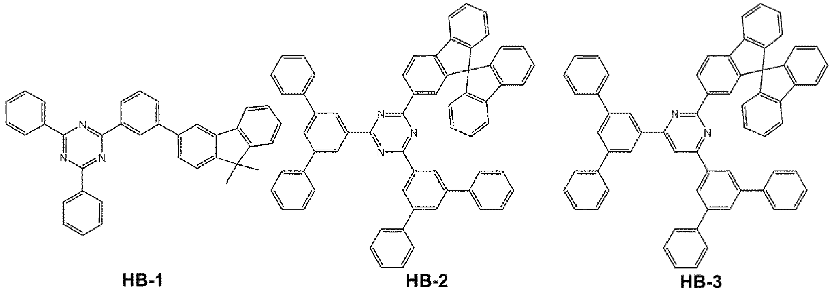

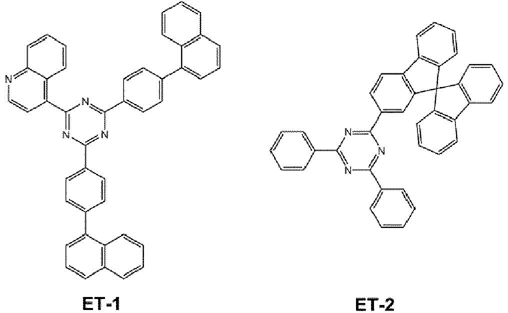

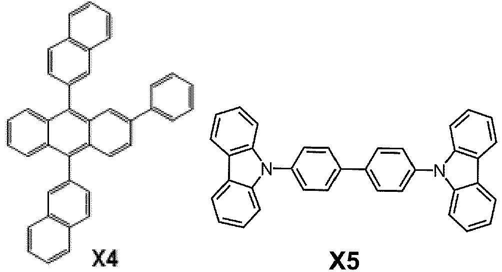

Referring to Table 1, it is confirmed that the organic light-emitting devices of Examples 1 to 11 have excellent driving voltage, luminance, efficiency, and lifespan, as compared with those of the organic light-emitting devices of Comparative Examples 1 to 7.

In view of the above, it is confirmed that the lifespan of the organic light-emitting devices of Examples 1 to 11 were remarkably improved, as compared with the organic light-emitting devices of Comparative Examples 1 and 7 in which each of the first compound, the second compound, and the third compound has a triazine structure.

The organic light-emitting device may have a low driving voltage, high efficiency, and a long lifespan.

## Claims

1. An organic light-emitting device comprising:
a first electrode;
a second electrode; and
an organic layer between the first electrode and the second electrode and comprising an emission layer,
wherein the organic layer comprises a first compound, a second compound, and a third compound, and
the first compound, the second compound, and the third compound are each independently an azine-based compound represented by one selected from Formulae 1-1 to 1-5, except for a case where the first compound, the second compound, and the third compound concurrently include an azine-based compound represented by Formula 1-3: 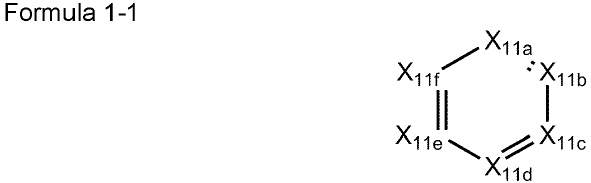 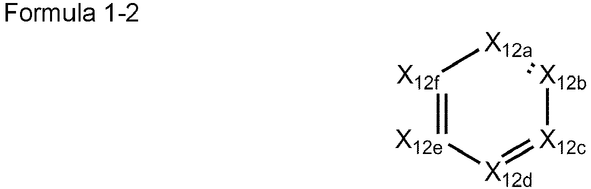 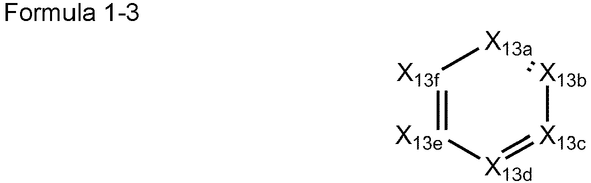 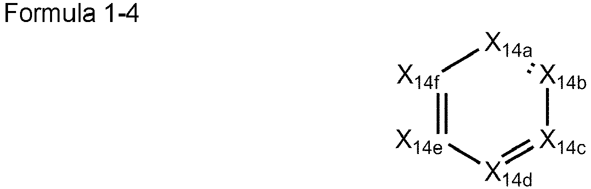 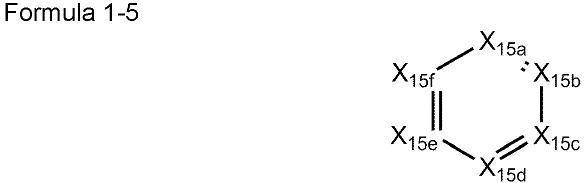
wherein, in Formulae 1-1 to 1-5,
X₁₁ₐ is selected from N and C[(L₁₁ₐ)ₐ₁₁ₐ-R₁₁ₐ]; X_{11b} is selected from N and C[(L_{11b})_{a11b}-R_{11b]}; X_{11c} is selected from N and C[(L_{11c})_{a11c}-R_{11c}]; X_{11d} is selected from N and C[(L_{11d})_{a11d}-R_{11d}]; X₁₁ₑ is selected from N and C[(L₁₁ₑ)ₐ₁₁ₑ-R₁₁ₑ]; X_{11f} is selected from N and C[(L_{11f})_{a11f}-R_{11f}]; and one of X₁₁ₐ to X_{11f} is N,
X₁₂ₐ is selected from N and C[(L₁₂ₐ)ₐ₁₂ₐ-R₁₂ₐ]; X_{12b} is selected from N and C[(L_{12b})_{a12b}-R_{12b}]; X_{12c} is selected from N and C[(L_{12c})_{a12c}-R_{12c}]; X_{12d} is selected from N and C[(L_{12d})_{a12d}-R_{12d}]; X₁₂ₑ is selected from N and C[(L₁₂ₑ)ₐ₁₂ₑ-R₁₂ₑ]; X_{12f} is selected from N and C[(L_{12f})_{a12f}-R_{12f}]; and two of X₁₂ₐ to X_{12f} are N,
X₁₃ₐ is selected from N and C[(L₁₃ₐ)ₐ₁₃ₐ-R₁₃ₐ]; X_{13b} is selected from N and C[(L_{13b})_{a13b}-R_{13b}]; X_{13c} is selected from N and C[(L_{13c})_{a13c}-R_{13c}]; X_{13d} is selected from N and C[(L_{13d})_{a13d}-R_{13d}]; X₁₃ₑ is selected from N and C[(L₁₃ₑ)ₐ₁₃ₑ-R₁₃ₑ]; X_{13f} is selected from N and C[(L_{13f})_{a13f}-R_{13f}]; and three of X₁₃ₐ to X_{13f} are N,
X₁₄ₐ is selected from N and C[(L₁₄ₐ)ₐ₁₄ₐ-R₁₄ₐ]; X_{14b} is selected from N and C[(L_{14b})_{a14b}-R_{14b}]; X_{14c} is selected from N and C[(L_{14c})_{a14c}-R_{14c}]; X_{14d} is selected from N and C[(L_{14d})_{a14d}-R_{14d}]; X₁₄ₑ is selected from N and C[(L₁₄ₑ)ₐ₁₄ₑ-R₁₄ₑ]; X_{14f} is selected from N and C[(L_{14f})_{a14f}-R_{14f}]; and four of X₁₄ₐ to X_{14f} are N,
X₁₅ₐ is selected from N and C[(L₁₅ₐ)ₐ₁₅ₐ-R₁₅ₐ]; X_{15b} is selected from N and C[(L_{15b})_{a15b}-R_{15b}]; X_{15c} is selected from N and C[(L_{15c})_{a15c}-R_{15c}]; X_{15d} is selected from N and C[(L_{15d})_{a15d}-R_{15d}]; X₁₅ₑ is selected from N and C[(L₁₅ₑ)ₐ₁₅ₑ-R₁₅ₑ]; X_{15f} is selected from N and C[(L_{15f})_{a15f}-R_{15f}]; and five of X₁₅ₐ to X_{15f} are N,
L₁₁ₐ to L_{11f}, L₁₂ₐ to L_{12f}, L₁₃ₐ to L_{13f}, L₁₄ₐ to L_{14f}, and L₁₅ₐ to L_{15f} are each independently selected from:
a C₃-C₁₀ cycloalkenylene group, a C₁-C₁₀ heterocycloalkenylene group, a C₆-C₆₀ arylene group, a C₁-C₆₀ heteroarylene group, a divalent non-aromatic condensed polycyclic group, and a divalent non-aromatic condensed heteropolycyclic group; and
a C₃-C₁₀ cycloalkenylene group, a C₁-C₁₀ heterocycloalkenylene group, a C₆-C₆₀ arylene group, a C₁-C₆₀ heteroarylene group, a divalent non-aromatic condensed polycyclic group, and a divalent non-aromatic condensed heteropolycyclic group, each substituted with at least one selected from deuterium, -F, -Cl, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₂₀ alkyl group, a C₁-C₂₀ alkoxy group, a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₃₀ aryl group, a C₆-C₃₀ aryloxy group, a C₆-C₃₀ arylthio group, a C₁-C₃₀ heteroaryloxy group, a C₁-C₃₀ heteroarylthio group, a monovalent non-aromatic condensed polycyclic group, a monovalent non-aromatic condensed heteropolycyclic group, a pyrrolyl group, a thiophenyl group, a furanyl group, an imidazolyl group, a pyrazolyl group, a thiazolyl group, an isothiazolyl group, an oxazolyl group, an isoxazolyl group, a pyridinyl group, a pyrazinyl group, a pyrimidinyl group, a pyridazinyl group, an isoindolyl group, an indolyl group, an indazolyl group, a purinyl group, a quinolinyl group, an isoquinolinyl group, a benzoquinolinyl group, a phthalazinyl group, a naphthyridinyl group, a quinoxalinyl group, a quinazolinyl group, a cinnolinyl group, a carbazolyl group, a phenanthridinyl group, an acridinyl group, a phenanthrolinyl group, a phenazinyl group, a benzimidazolyl group, a benzofuranyl group, a benzothiophenyl group, an isobenzothiazolyl group, a benzoxazolyl group, an isobenzoxazolyl group, a triazolyl group, a tetrazolyl group, an oxadiazolyl group, a dibenzofuranyl group, a dibenzothiophenyl group, a benzocarbazolyl group, a dibenzocarbazolyl group, a thiadiazolyl group, and an imidazopyridinyl group, wherein L₁₁ₐ to L_{11f}, L₁₂ₐ to L_{12f}, L₁₃ₐ to L_{13f}, L₁₄ₐ to L_{14f}, and L₁₅ₐ to L_{15f} are not each independently selected from: a triazinylene group; and a triazinylene group substituted with at least one selected from deuterium, -F, -Cl, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₂₀ alkyl group, a C₁-C₂₀ alkoxy group, a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₃₀ aryl group, a C₆-C₃₀ aryloxy group, a C₆-C₃₀ arylthio group, a C₁-C₃₀ heteroaryloxy group, a C₁-C₃₀ heteroarylthio group, a monovalent non-aromatic condensed polycyclic group, a monovalent non-aromatic condensed heteropolycyclic group, a pyrrolyl group, a thiophenyl group, a furanyl group, an imidazolyl group, a pyrazolyl group, a thiazolyl group, an isothiazolyl group, an oxazolyl group, an isoxazolyl group, a pyridinyl group, a pyrazinyl group, a pyrimidinyl group, a pyridazinyl group, an isoindolyl group, an indolyl group, an indazolyl group, a purinyl group, a quinolinyl group, an isoquinolinyl group, a benzoquinolinyl group, a phthalazinyl group, a naphthyridinyl group, a quinoxalinyl group, a quinazolinyl group, a cinnolinyl group, a carbazolyl group, a phenanthridinyl group, an acridinyl group, a phenanthrolinyl group, a phenazinyl group, a benzimidazolyl group, a benzofuranyl group, a benzothiophenyl group, an isobenzothiazolyl group, a benzoxazolyl group, an isobenzoxazolyl group, a triazolyl group, a tetrazolyl group, an oxadiazolyl group, a dibenzofuranyl group, a dibenzothiophenyl group, a benzocarbazolyl group, a dibenzocarbazolyl group, a thiadiazolyl group, and an imidazopyridinyl group,
a11a to a11f, a12a to a12f, a13a to a13f, a14a to a14f, and a15a to a15f are each independently selected from 0, 1, 2, 3, and 4,
R₁₁ₐ to R_{11f}, R₁₂ₐ to R_{12f}, R₁₃ₐ to R_{13f}, R₁₄ₐ to R_{14f}, and R₁₅ₐ to R_{15f} are each independently selected from:
hydrogen, deuterium, -F, -Cl, -Br, -I, a methyl group, an ethyl group, an n-propyl group, an isopropyl group, an n-butyl group, an isobutyl group, a see-butyl group, a tert-butyl group, a methoxy group, an ethoxy group, an n-propoxy group, an isopropoxy group, an n-butoxy group, an isobutoxy group, a sec-butoxy group, and a tert-butoxy group;
a methyl group, an ethyl group, an n-propyl group, an isopropyl group, an n-butyl group, an isobutyl group, a see-butyl group, and a tert-butyl group, each substituted with at least one selected from deuterium, -F, -Cl, -Br, and -I;
a phenyl group, a biphenyl group, a terphenyl group, a naphthyl group, a fluorenyl group, a carbazolyl group, a dibenzofuranyl group, a dibenzothiophenyl group, a pyridinyl group, a pyrimidinyl group, a quinolinyl group, an isoquinolinyl group, a quinoxalinyl group, a quinazolinyl group, a benzofuropyridinyl group, a benzothienopyridinyl group, a benzofuropyrimidinyl group, a benzothienopyrimidinyl group, a monovalent non-aromatic condensed polycyclic group, and a monovalent non-aromatic condensed heteropolycyclic group;
a phenyl group, a biphenyl group, a terphenyl group, a naphthyl group, a fluorenyl group, a carbazolyl group, a dibenzofuranyl group, a dibenzothiophenyl group, a pyridinyl group, a pyrimidinyl group, a quinolinyl group, an isoquinolinyl group, a quinoxalinyl group, a quinazolinyl group, a benzofuropyridinyl group, a benzothienopyridinyl group, a benzofuropyrimidinyl group, a benzothienopyrimidinyl group, a monovalent non-aromatic condensed polycyclic group, and a monovalent non-aromatic condensed heteropolycyclic group, each substituted with at least one selected from deuterium, -F, -Cl, -Br, -I, a methyl group, an ethyl group, an n-propyl group, an isopropyl group, an n-butyl group, an isobutyl group, a see-butyl group, a tert-butyl group, a methoxy group, an ethoxy group, an n-propoxy group, an isopropoxy group, an n-butoxy group, an isobutoxy group, a sec-butoxy group, a tert-butoxy group, a phenyl group, a biphenyl group, a terphenyl group, a naphthyl group, a fluorenyl group, a carbazolyl group, a dibenzofuranyl group, a dibenzothiophenyl group, a pyridinyl group, a pyrimidinyl group, a quinolinyl group, an isoquinolinyl group, a quinoxalinyl group, a quinazolinyl group, a benzofuropyridinyl group, a benzothienopyridinyl group, a benzofuropyrimidinyl group, and a benzothienopyrimidinyl group; and
a phenyl group, a biphenyl group, a terphenyl group, a naphthyl group, a fluorenyl group, a carbazolyl group, a dibenzofuranyl group, a dibenzothiophenyl group, a pyridinyl group, a pyrimidinyl group, a quinolinyl group, an isoquinolinyl group, a quinoxalinyl group, a quinazolinyl group, a benzofuropyridinyl group, a benzothienopyridinyl group, a benzofuropyrimidinyl group, a benzothienopyrimidinyl group, a monovalent non-aromatic condensed polycyclic group, and a monovalent non-aromatic condensed heteropolycyclic group, each substituted with at least one selected from a methyl group, an ethyl group, an n-propyl group, an isopropyl group, an n-butyl group, an isobutyl group, a see-butyl group, a tert-butyl group, a phenyl group, a biphenyl group, a terphenyl group, a naphthyl group, a fluorenyl group, a carbazolyl group, a dibenzofuranyl group, a dibenzothiophenyl group, a pyridinyl group, a pyrimidinyl group, a quinolinyl group, an isoquinolinyl group, a quinoxalinyl group, a quinazolinyl group, a benzofuropyridinyl group, a benzothienopyridinyl group, a benzofuropyrimidinyl group, and a benzothienopyrimidinyl group that are each substituted with at least one selected from deuterium, -F, -Cl, -Br, and -I, and
neighboring atoms of R₁₁ₐ to R_{11f}, neighboring atoms of R₁₂ₐ to R_{12f}, neighboring atoms of R₁₃ₐ to R_{13f}, neighboring atoms of R₁₄ₐ to R_{14f}, and/or neighboring atoms of R₁₅ₐ to R_{15f} optionally form a saturated ring or an unsaturated ring.

2. An organic light-emitting device according to claim 1, wherein:
L₁₁ₐ to L_{11f}, L₁₂ₐ to L_{12f}, L₁₃ₐ to L_{13f}, L₁₄ₐ to L_{14f}, and L₁₅ₐ to L_{15f} are each independently selected from:
a phenylene group, a pentalenylene group, an indenylene group, a naphthylene group, an azulenylene group, a heptalenylene group, an indacenylene group, an acenaphthylene group, a fluorenylene group, a spiro-fluorenylene group, a benzofluorenylene group, a dibenzofluorenylene group, a phenalenylene group, a phenanthrenylene group, an anthracenylene group, a fluoranthenylene group, a triphenylenylene group, a pyrenylene group, a chrysenylene group, a naphthacenylene group, a picenylene group, a perylenylene group, a pentaphenylene group, a hexacenylene group, a pentacenylene group, a rubicenylene group, a coronenylene group, an ovalenylene group, a pyrrolylene group, a thiophenylene group, a furanylene group, an imidazolylene group, a pyrazolylene group, a thiazolylene group, an isothiazolylene group, an oxazolylene group, an isoxazolylene group, a pyridinylene group, a pyrazinylene group, a pyrimidinylene group, a pyridazinylene group, an isoindolylene group, an indolylene group, an indazolylene group, a purinylene group, a quinolinylene group, an isoquinolinylene group, a benzoquinolinylene group, a phthalazinylene group, a naphthyridinylene group, a quinoxalinylene group, a quinazolinylene group, a cinnolinylene group, a carbazolylene group, a phenanthridinylene group, an acridinylene group, a phenanthrolinylene group, a phenazinylene group, a benzimidazolylene group, a benzofuranylene group, a benzothiophenylene group, an isobenzothiazolylene group, a benzoxazolylene group, an isobenzoxazolylene group, a triazolylene group, a tetrazolylene group, an oxadiazolylene group, a dibenzofuranylene group, a dibenzothiophenylene group, a benzofuropyridinylene group, a benzothienopyridinylene group, a benzofuropyrimidinylene group, a benzothienopyrimidinylene group, a benzocarbazolylene group, and a dibenzocarbazolylene group; and
a phenylene group, a pentalenylene group, an indenylene group, a naphthylene group, an azulenylene group, a heptalenylene group, an indacenylene group, an acenaphthylene group, a fluorenylene group, a spiro-fluorenylene group, a benzofluorenylene group, a dibenzofluorenylene group, a phenalenylene group, a phenanthrenylene group, an anthracenylene group, a fluoranthenylene group, a triphenylenylene group, a pyrenylene group, a chrysenylene group, a naphthacenylene group, a picenylene group, a perylenylene group, a pentaphenylene group, a hexacenylene group, a pentacenylene group, a rubicenylene group, a coronenylene group, an ovalenylene group, a pyrrolylene group, a thiophenylene group, a furanylene group, an imidazolylene group, a pyrazolylene group, a thiazolylene group, an isothiazolylene group, an oxazolylene group, an isoxazolylene group, a pyridinylene group, a pyrazinylene group, a pyrimidinylene group, a pyridazinylene group, an isoindolylene group, an indolylene group, an indazolylene group, a purinylene group, a quinolinylene group, an isoquinolinylene group, a benzoquinolinylene group, a phthalazinylene group, a naphthyridinylene group, a quinoxalinylene group, a quinazolinylene group, a cinnolinylene group, a carbazolylene group, a phenanthridinylene group, an acridinylene group, a phenanthrolinylene group, a phenazinylene group, a benzimidazolylene group, a benzofuranylene group, a benzothiophenylene group, an isobenzothiazolylene group, a benzoxazolylene group, an isobenzoxazolylene group, a triazolylene group, a tetrazolylene group, an oxadiazolylene group, a dibenzofuranylene group, a dibenzothiophenylene group, a benzofuropyridinylene group, a benzothienopyridinylene group, a benzofuropyrimidinylene group, a benzothienopyrimidinylene group, a benzocarbazolylene group, and a dibenzocarbazolylene group, each substituted with at least one selected from deuterium, -F, -Cl, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₂₀ alkyl group, a C₁-C₂₀ alkoxy group, a cyclopentyl group, a cyclohexyl group, a cycloheptyl group, a cyclopentenyl group, a cyclohexenyl group, a phenyl group, a pentalenyl group, an indenyl group, a naphthyl group, an azulenyl group, a heptalenyl group, an indacenyl group, an acenaphthyl group, a fluorenyl group, a spiro-fluorenyl group, a benzofluorenyl group, a dibenzofluorenyl group, a phenalenyl group, a phenanthrenyl group, an anthracenyl group, a fluoranthenyl group, a triphenylenyl group, a pyrenyl group, a chrysenyl group, a naphthacenyl group, a picenyl group, a perylenyl group, a pentaphenyl group, a hexacenyl group, a pentacenyl group, a rubicenyl group, a coronenyl group, an ovalenyl group, a pyrrolyl group, a thiophenyl group, a furanyl group, an imidazolyl group, a pyrazolyl group, a thiazolyl group, an isothiazolyl group, an oxazolyl group, an isoxazolyl group, a pyridinyl group, a pyrazinyl group, a pyrimidinyl group, a pyridazinyl group, an isoindolyl group, an indolyl group, an indazolyl group, a purinyl group, a quinolinyl group, an isoquinolinyl group, a benzoquinolinyl group, a phthalazinyl group, a naphthyridinyl group, a quinoxalinyl group, a quinazolinyl group, a cinnolinyl group, a carbazolyl group, a phenanthridinyl group, an acridinyl group, a phenanthrolinyl group, a phenazinyl group, a benzimidazolyl group, a benzofuranyl group, a benzothiophenyl group, an isobenzothiazolyl group, a benzoxazolyl group, an isobenzoxazolyl group, a triazolyl group, a tetrazolyl group, an oxadiazolyl group, a dibenzofuranyl group, a dibenzothiophenyl group, a benzocarbazolyl group, a dibenzocarbazolyl group, a thiadiazolyl group, and an imidazopyridinyl group.

3. An organic light-emitting device according to claim 1 or claim 2, wherein:
R₁₁ₐ to R_{11f}, R₁₂ₐ to R_{12f}, R₁₃ₐ to R_{13f}, R₁₄ₐ to R_{14f}, and R₁₅ₐ to R_{15f} are each independently selected from:
hydrogen, deuterium, -F, -Cl, -Br, -I, a methyl group, an ethyl group, an n-propyl group, an isopropyl group, an n-butyl group, an isobutyl group, a see-butyl group, a tert-butyl group, a methoxy group, an ethoxy group, an n-propoxy group, an isopropoxy group, an n-butoxy group, an isobutoxy group, a sec-butoxy group, and a tert-butoxy group;
a methyl group, an ethyl group, an n-propyl group, an isopropyl group, an n-butyl group, an isobutyl group, a see-butyl group, and a tert-butyl group, each substituted with at least one selected from deuterium, -F, -Cl, -Br, and -I;
a phenyl group, a biphenyl group, a terphenyl group, a naphthyl group, a fluorenyl group, a carbazolyl group, a dibenzofuranyl group, a dibenzothiophenyl group, a pyridinyl group, a pyrimidinyl group, a quinolinyl group, an isoquinolinyl group, a quinoxalinyl group, a quinazolinyl group, a benzofuropyridinyl group, a benzothienopyridinyl group, a benzofuropyrimidinyl group, and a benzothienopyrimidinyl group;
a phenyl group, a biphenyl group, a terphenyl group, a naphthyl group, a fluorenyl group, a carbazolyl group, a dibenzofuranyl group, a dibenzothiophenyl group, a pyridinyl group, a pyrimidinyl group, a quinolinyl group, an isoquinolinyl group, a quinoxalinyl group, a quinazolinyl group, a benzofuropyridinyl group, a benzothienopyridinyl group, a benzofuropyrimidinyl group, and a benzothienopyrimidinyl group, each substituted with at least one selected from deuterium, -F, -Cl, -Br, -I, a methyl group, an ethyl group, an n-propyl group, an isopropyl group, an n-butyl group, an isobutyl group, a see-butyl group, a tert-butyl group, a methoxy group, an ethoxy group, an n-propoxy group, an isopropoxy group, an n-butoxy group, an isobutoxy group, a sec-butoxy group, a tert-butoxy group, a phenyl group, a biphenyl group, a terphenyl group, a naphthyl group, a fluorenyl group, a carbazolyl group, a dibenzofuranyl group, a dibenzothiophenyl group, a pyridinyl group, a pyrimidinyl group, a quinolinyl group, an isoquinolinyl group, a quinoxalinyl group, a quinazolinyl group, a benzofuropyridinyl group, a benzothienopyridinyl group, a benzofuropyrimidinyl group, and a benzothienopyrimidinyl group;
a phenyl group, a biphenyl group, a terphenyl group, a naphthyl group, a fluorenyl group, a carbazolyl group, a dibenzofuranyl group, a dibenzothiophenyl group, a pyridinyl group, a pyrimidinyl group, a quinolinyl group, an isoquinolinyl group, a quinoxalinyl group, a quinazolinyl group, a benzofuropyridinyl group, a benzothienopyridinyl group, a benzofuropyrimidinyl group, and a benzothienopyrimidinyl group, each substituted with at least one selected from a methyl group, an ethyl group, an n-propyl group, an isopropyl group, an n-butyl group, an isobutyl group, a see-butyl group, a tert-butyl group, a phenyl group, a biphenyl group, a terphenyl group, a naphthyl group, a fluorenyl group, a carbazolyl group, a dibenzofuranyl group, a dibenzothiophenyl group, a pyridinyl group, a pyrimidinyl group, a quinolinyl group, an isoquinolinyl group, a quinoxalinyl group, a quinazolinyl group, a benzofuropyridinyl group, a benzothienopyridinyl group, a benzofuropyrimidinyl group, and a benzothienopyrimidinyl group that are each substituted with at least one selected from deuterium, -F, -Cl, -Br, and -I; and
a partial structure represented by one selected from Formulae 2-1 to 2-17: 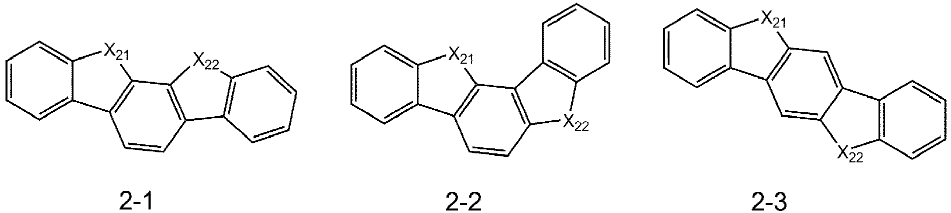 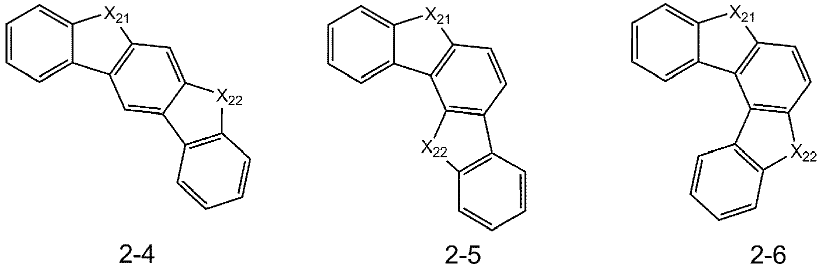 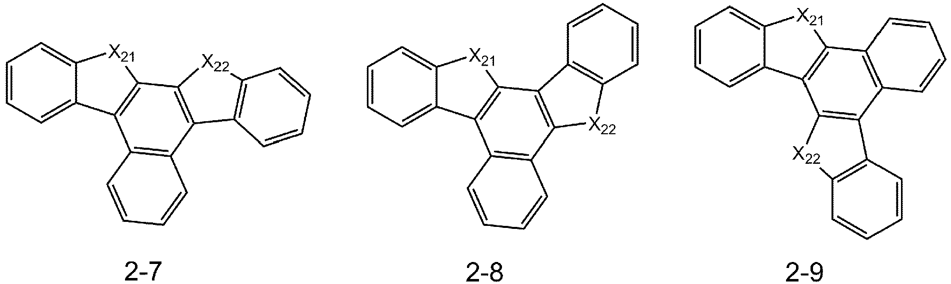 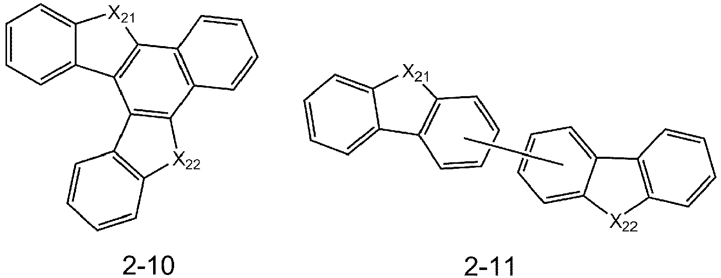 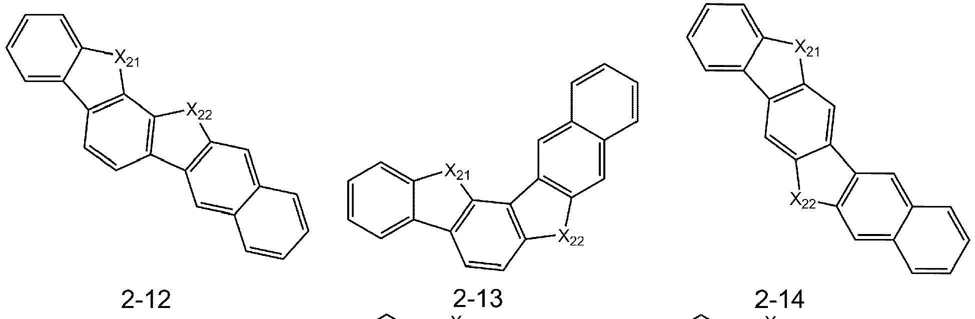 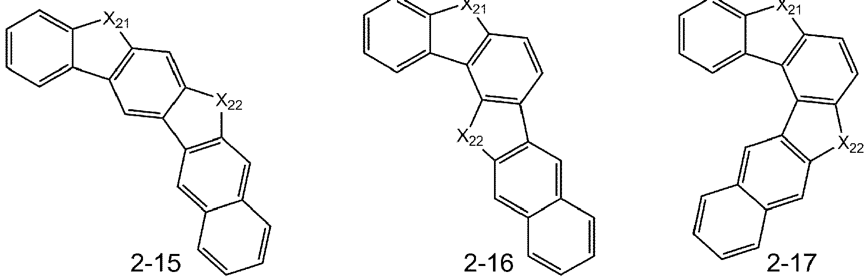
wherein, in Formulae 2-1 to 2-17,
X₂₁ is selected from C(R₂₁)(R₂₂), N(R₂₁), O, and S,
X₂₂ is selected from C(R₂₃)(R₂₄), N(R₂₃), O, and S,
R₂₁ to R₂₄ are each independently selected from:
a methyl group, an ethyl group, an n-propyl group, an isopropyl group, an n-butyl group, an isobutyl group, a see-butyl group, and a tert-butyl group;
a methyl group, an ethyl group, an n-propyl group, an isopropyl group, an n-butyl group, an isobutyl group, a see-butyl group, and a tert-butyl group, each substituted with at least one selected from deuterium, -F, -Cl, -Br, and -I;
a phenyl group, a biphenyl group, a terphenyl group, a naphthyl group, a fluorenyl group, a carbazolyl group, a dibenzofuranyl group, a dibenzothiophenyl group, a pyridinyl group, a pyrimidinyl group, a quinolinyl group, an isoquinolinyl group, a quinoxalinyl group, a quinazolinyl group, a benzofuropyridinyl group, a benzothienopyridinyl group, a benzofuropyrimidinyl group, and a benzothienopyrimidinyl group; and
a phenyl group, a biphenyl group, a terphenyl group, a naphthyl group, a fluorenyl group, a carbazolyl group, a dibenzofuranyl group, a dibenzothiophenyl group, a pyridinyl group, a pyrimidinyl group, a quinolinyl group, an isoquinolinyl group, a quinoxalinyl group, a quinazolinyl group, a benzofuropyridinyl group, a benzothienopyridinyl group, a benzofuropyrimidinyl group, and a benzothienopyrimidinyl group, each substituted with at least one selected from deuterium, -F, -Cl, -Br, -I, a methyl group, an ethyl group, an n-propyl group, an isopropyl group, an n-butyl group, an isobutyl group, a see-butyl group, a tert-butyl group, a methoxy group, an ethoxy group, an n-propoxy group, an isopropoxy group, an n-butoxy group, an isobutoxy group, a sec-butoxy group, a tert-butoxy group, a phenyl group, a biphenyl group, a terphenyl group, a naphthyl group, a fluorenyl group, a carbazolyl group, a dibenzofuranyl group, a dibenzothiophenyl group, a pyridinyl group, a pyrimidinyl group, a quinolinyl group, an isoquinolinyl group, a quinoxalinyl group, a quinazolinyl group, a benzofuropyridinyl group, a benzothienopyridinyl group, a benzofuropyrimidinyl group, and a benzothienopyrimidinyl group, and
one selected from a carbon atom and a nitrogen atom that constitute the partial structure represented by one selected from Formulae 2-1 to 2-17 is a binding site to a neighboring atom selected from Formula 1-1 to 1-5;
preferably wherein:
R₁₁ₐ to R_{11f}, R₁₂ₐ to R_{12f}, R₁₃ₐ to R_{13f}, R₁₄ₐ to R_{14f}, and R₁₅ₐ to R_{15f} are each independently selected from:
hydrogen, deuterium, -F, -Cl, -Br, -I, a methyl group, an ethyl group, an n-propyl group, an isopropyl group, an n-butyl group, an isobutyl group, a see-butyl group, and a tert-butyl group;
a phenyl group, a biphenyl group, a terphenyl group, a naphthyl group, a fluorenyl group, a carbazolyl group, a dibenzofuranyl group, a dibenzothiophenyl group, a pyridinyl group, a pyrimidinyl group, a quinolinyl group, an isoquinolinyl group, a quinoxalinyl group, and a quinazolinyl group;
a phenyl group, a biphenyl group, a terphenyl group, a naphthyl group, a fluorenyl group, a carbazolyl group, a dibenzofuranyl group, a dibenzothiophenyl group, a pyridinyl group, a pyrimidinyl group, a quinolinyl group, an isoquinolinyl group, a quinoxalinyl group, and a quinazolinyl group, each substituted with at least one selected from deuterium, -F, -Cl, -Br, -I, a methyl group, an ethyl group, an n-propyl group, an isopropyl group, an n-butyl group, an isobutyl group, a see-butyl group, a tert-butyl group, a phenyl group, a biphenyl group, a terphenyl group, a naphthyl group, a fluorenyl group, a carbazolyl group, a dibenzofuranyl group, a dibenzothiophenyl group, a pyridinyl group, a pyrimidinyl group, a quinolinyl group, an isoquinolinyl group, a quinoxalinyl group, and a quinazolinyl group; and
a partial structure represented by one selected from Formulae 2-1 to 2-17: 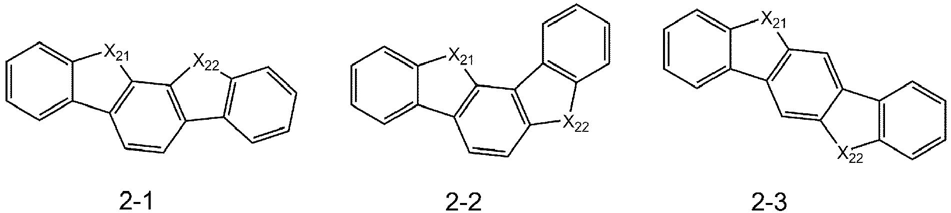 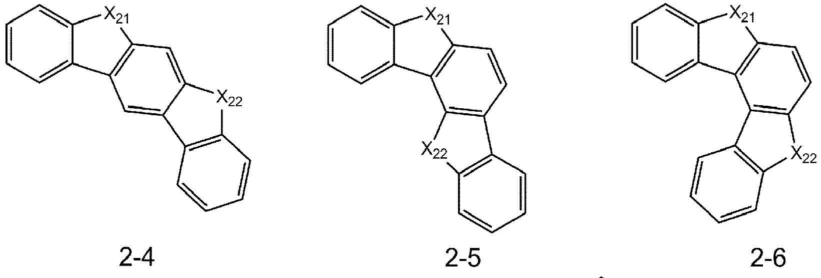 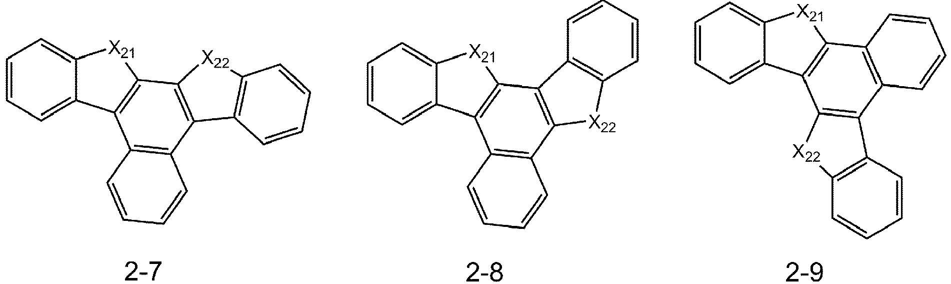 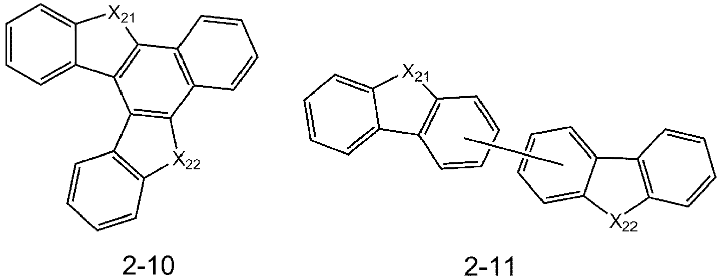 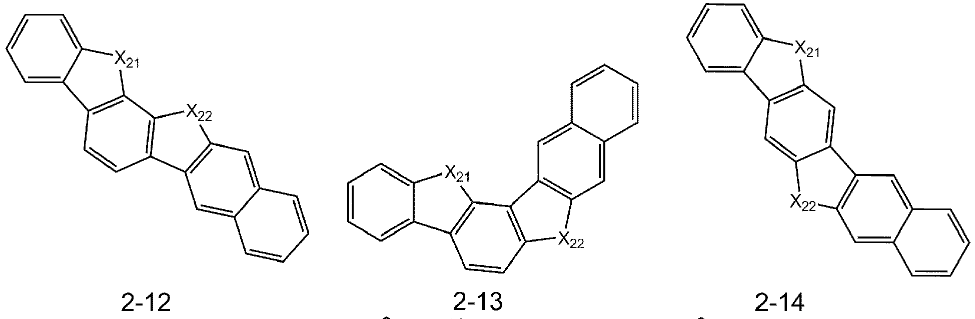 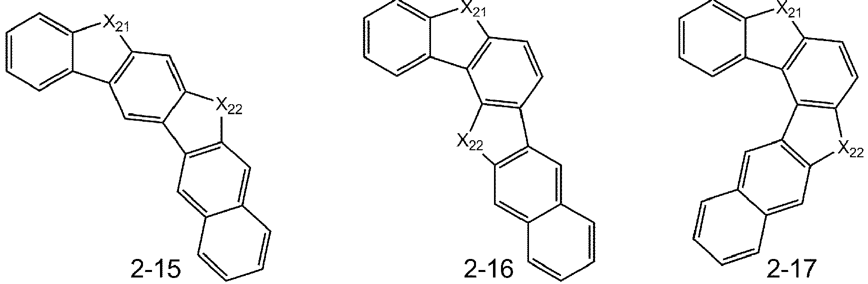
wherein, in Formulae 2-1 to 2-17,
X₂₁ is selected from C(R₂₁)(R₂₂), N(R₂₁), O, and S,
X₂₂ is selected from C(R₂₃)(R₂₄), N(R₂₃), O, and S,
R₂₁ to R₂₄ are each independently selected from:
a methyl group, an ethyl group, an n-propyl group, an isopropyl group, an n-butyl group, an isobutyl group, a see-butyl group, and a tert-butyl group;
a methyl group, an ethyl group, an n-propyl group, an isopropyl group, an n-butyl group, an isobutyl group, a see-butyl group, and a tert-butyl group, each substituted with at least one selected from deuterium, -F, -Cl, -Br, and -I; and
a phenyl group, a biphenyl group, a terphenyl group, a naphthyl group, a fluorenyl group, a carbazolyl group, a dibenzofuranyl group, a dibenzothiophenyl group, a pyridinyl group, a pyrimidinyl group, a quinolinyl group, an isoquinolinyl group, a quinoxalinyl group, and a quinazolinyl group, and
one selected from a carbon atom and a nitrogen atom that constitute the partial structure represented by one selected from Formulae 2-1 to 2-17 is a binding site to a neighboring atom selected from Formula 1-1 to 1-5.

4. An organic light-emitting device according to any one of claims 1 to 3, wherein:
the first compound, the second compound, and the third compound are each independently an azine-based compound represented by one selected from Formulae 1-11, 1-21 to 1-25, 1-31 to 1-33, 1-41, 1-42, and 1-51, except for a case where the first compound, the second compound, and the third compound concurrently include an azine-based compound respectively represented by one selected from Formulae 1-31 to 1-33: 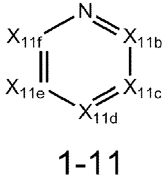 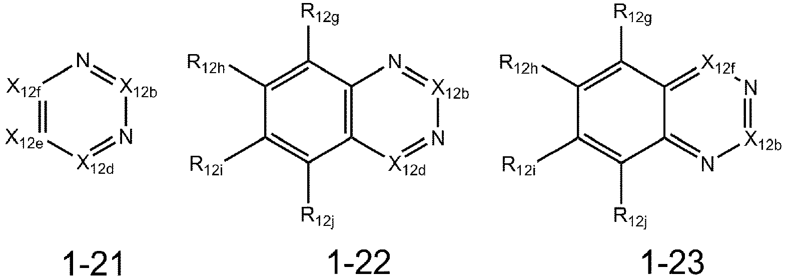 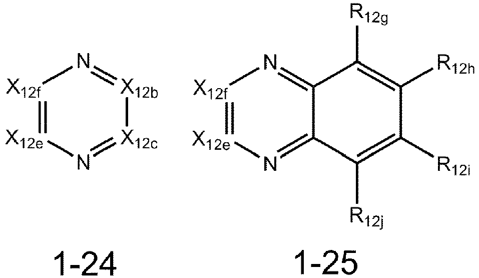 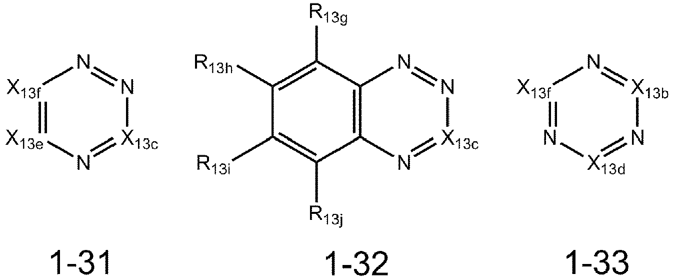 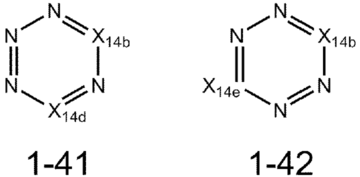 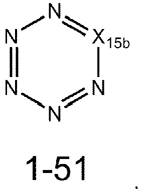
wherein, in Formulae 1-11, 1-21 to 1-25, 1-31 to 1-33, 1-41, 1-42, and 1-51,
X_{11b} to X_{11f} are each independently defined the same as Formula 1-1,
X_{12b} to X_{12f} are each independently defined the same as Formula 1-2,
X_{13b} to X_{13f} are each independently defined the same as Formula 1-3,
X_{14b}, X_{14d}, and X₁₄ₑ are each independently defined the same as Formula 1-4,
X_{15b} is defined the same as Formula 1-5, and
R_{12g} to R₁₂ⱼ and R_{13g} to R₁₃ⱼ are each independently selected from:
hydrogen, deuterium, -F, -Cl, -Br, -I, a methyl group, an ethyl group, an n-propyl group, an isopropyl group, an n-butyl group, an isobutyl group, a see-butyl group, a tert-butyl group, a methoxy group, an ethoxy group, an n-propoxy group, an isopropoxy group, an n-butoxy group, an isobutoxy group, a sec-butoxy group, a tert-butoxy group, a phenyl group, a biphenyl group, a terphenyl group, a naphthyl group, a fluorenyl group, a carbazolyl group, a dibenzofuranyl group, a dibenzothiophenyl group, a pyridinyl group, a pyrimidinyl group, a quinolinyl group, an isoquinolinyl group, a quinoxalinyl group, a quinazolinyl group, a benzofuropyridinyl group, a benzothienopyridinyl group, a benzofuropyrimidinyl group, and a benzothienopyrimidinyl group; and
a methyl group, an ethyl group, an n-propyl group, an isopropyl group, an n-butyl group, an isobutyl group, a see-butyl group, a tert-butyl group, a phenyl group, a biphenyl group, a terphenyl group, a naphthyl group, a fluorenyl group, a carbazolyl group, a dibenzofuranyl group, a dibenzothiophenyl group, a pyridinyl group, a pyrimidinyl group, a quinolinyl group, an isoquinolinyl group, a quinoxalinyl group, a quinazolinyl group, a benzofuropyridinyl group, a benzothienopyridinyl group, a benzofuropyrimidinyl group, and a benzothienopyrimidinyl group, each substituted with at least one selected from deuterium, -F, -Cl, -Br, and -I.

5. An organic light-emitting device according to any one of claims 1 to 4, wherein:
the first compound comprises a partial structure represented by one selected from Formulae 2-1 to 2-17: 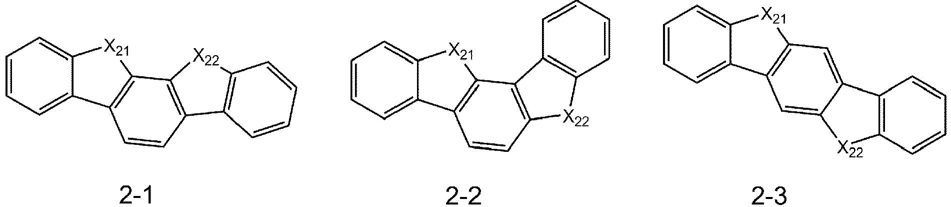 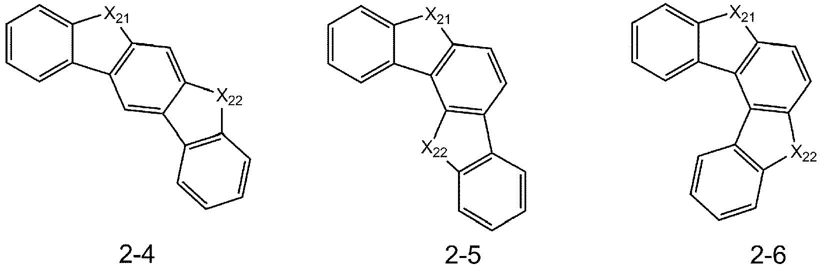 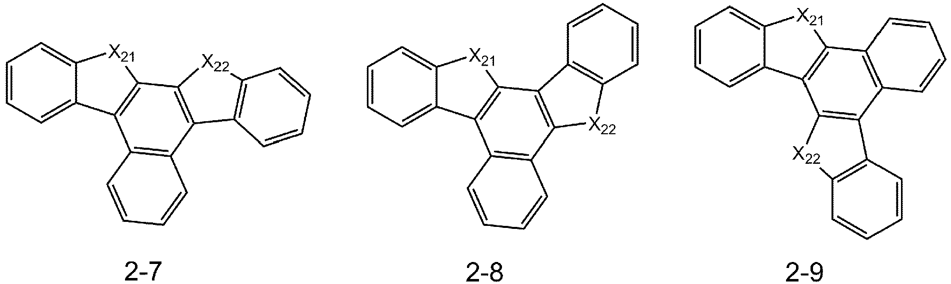 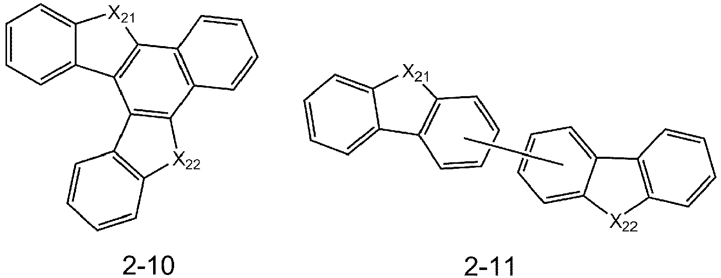 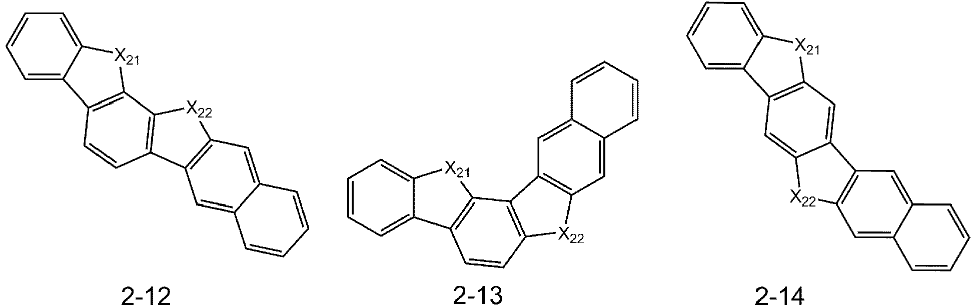 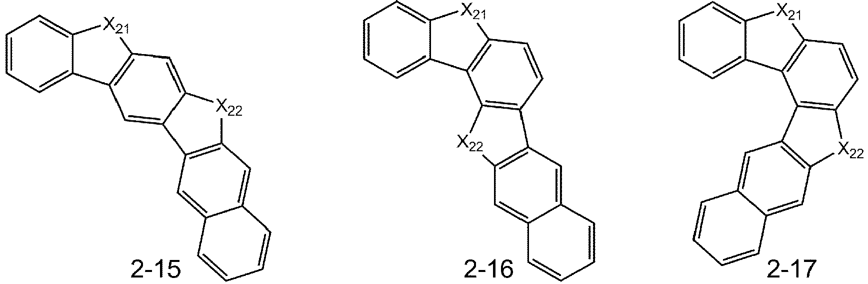
wherein, in Formulae 2-1 to 2-17,
X₂₁ is selected from C(R₂₁)(R₂₂), N(R₂₁), O, and S,
X₂₂ is selected from C(R₂₃)(R₂₄), N(R₂₃), O, and S,
R₂₁ to R₂₄ are each independently selected from:
a methyl group, an ethyl group, an n-propyl group, an isopropyl group, an n-butyl group, an isobutyl group, a see-butyl group, and a tert-butyl group;
a methyl group, an ethyl group, an n-propyl group, an isopropyl group, an n-butyl group, an isobutyl group, a see-butyl group, and a tert-butyl group, each substituted with at least one selected from deuterium, -F, -Cl, -Br, and -I;
a phenyl group, a naphthyl group, a fluorenyl group, a carbazolyl group, a dibenzofuranyl group, a dibenzothiophenyl group, a pyridinyl group, a pyrimidinyl group, a quinolinyl group, an isoquinolinyl group, a quinoxalinyl group, a quinazolinyl group, a benzofuropyridinyl group, a benzothienopyridinyl group, a benzofuropyrimidinyl group, and a benzothienopyrimidinyl group; and
a phenyl group, a biphenyl group, a terphenyl group, a naphthyl group, a fluorenyl group, a carbazolyl group, a dibenzofuranyl group, a dibenzothiophenyl group, a pyridinyl group, a pyrimidinyl group, a quinolinyl group, an isoquinolinyl group, a quinoxalinyl group, a quinazolinyl group, a benzofuropyridinyl group, a benzothienopyridinyl group, a benzofuropyrimidinyl group, and a benzothienopyrimidinyl group, each substituted with at least one selected from deuterium, -F, -Cl, -Br, -I, a methyl group, an ethyl group, an n-propyl group, an isopropyl group, an n-butyl group, an isobutyl group, a see-butyl group, a tert-butyl group, a methoxy group, an ethoxy group, an n-propoxy group, an isopropoxy group, an n-butoxy group, an isobutoxy group, a sec-butoxy group, a tert-butoxy group, a phenyl group, a biphenyl group, a terphenyl group, a naphthyl group, a fluorenyl group, a carbazolyl group, a dibenzofuranyl group, a dibenzothiophenyl group, a pyridinyl group, a pyrimidinyl group, a quinolinyl group, an isoquinolinyl group, a quinoxalinyl group, a quinazolinyl group, a benzofuropyridinyl group, a benzothienopyridinyl group, a benzofuropyrimidinyl group, and a benzothienopyrimidinyl group, and
one selected from a carbon atom and a nitrogen atom that constitute the partial structure represented by one selected from Formulae 2-1 to 2-17 is a binding site to a neighboring atom selected from Formula 1-1 to 1-5.

6. An organic light-emitting device according to any one of claims 1 to 5, wherein:
the first compound is selected from Compounds H-1 to H-11,
the second compound is selected from Compounds HB-1 to HB-3, and
the third compound is selected from Compounds ET-1 and ET-2:

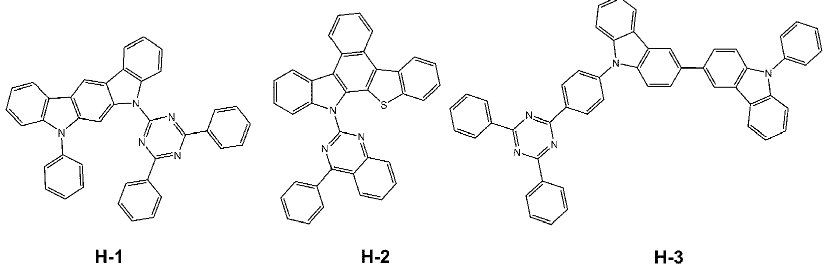

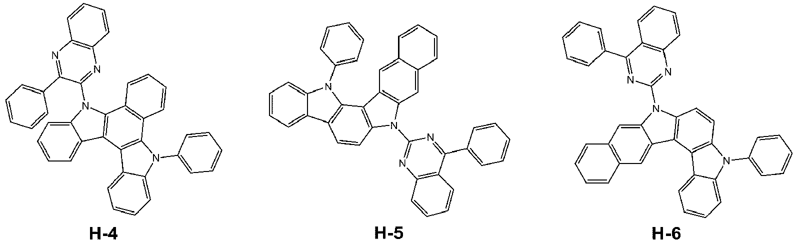

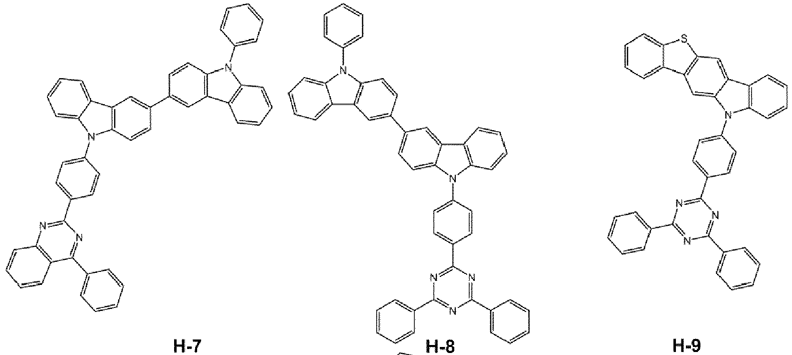

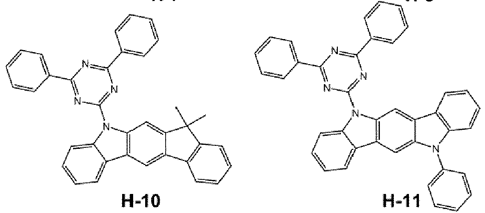

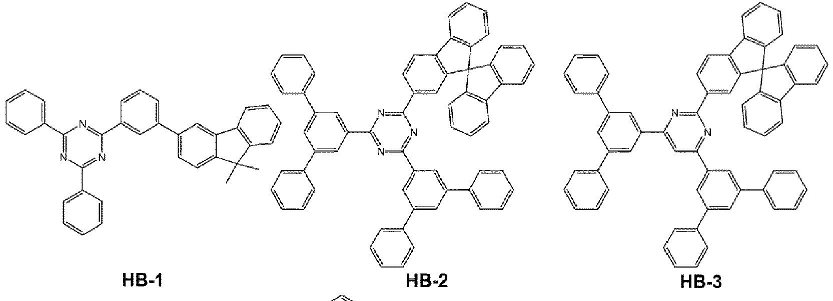

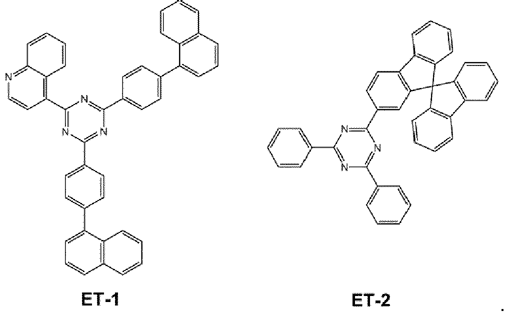

7. An organic light-emitting device according to any one of claims 1 to 6, wherein: the first compound, the second compound, and the third compound are each independently an azine-based compound represented by one selected from Formulae 1-1 to 1-3.

8. An organic light-emitting device according to any one of claims 1 to 7, wherein:
i) the first compound is an azine-based compound represented by one selected from Formulae 1-1 and 1-2, and the second compound and the third compound are each independently an azine-based compound represented by one selected from Formulae 1-2 and 1-3;
ii) the second compound is an azine-based compound represented by one selected from Formulae 1-1 and 1-2, and the first compound and the third compound are each independently an azine-based compound represented by one selected from Formulae 1-2 and 1-3; or
iii) the third compound is an azine-based compound represented by one selected from Formulae 1-1 and 1-2, and the first compound and the second compound are each independently an azine-based compound represented by one selected from Formulae 1-2 and 1-3.

9. An organic light-emitting device according to any one of claims 1 to 8, wherein:
the organic layer comprises an electron transport region between the emission layer and the second electrode,
the electron transport region comprises a buffer layer and an electron transport layer,
the buffer layer is between the emission layer and the electron transport layer,
the emission layer comprises the first compound,
the buffer layer comprises the second compound, and
the electron transport layer comprises the third compound;
preferably wherein:
the emission layer is in direct contact with the buffer layer, and
the buffer layer is in direct contact with the electron transport layer.

10. An organic light-emitting device according to any one of claims 1 to 9, wherein:
the first electrode is an anode, and the second electrode is a cathode;
and/or wherein:
the emission layer comprises an organometallic compound.

11. An organic light-emitting device according to any one of claims 1 to 10, wherein:
the organic light-emitting device further comprises a substrate divided into a first sub-pixel region, a second sub-pixel region, and a third sub-pixel region;
the first electrode comprises a plurality of first electrodes respectively arranged in the first sub-pixel region, the second sub-pixel region, and the third sub-pixel region of the substrate;
the second electrode faces the plurality of first electrodes; and
the organic layer is between the plurality of first electrodes and the second electrode.

12. An organic light-emitting device according to claim 11, wherein:
the emission layer comprises a first emission layer, a second emission layer, and a third emission layer respectively arranged in the first sub-pixel region, the second sub-pixel region, and the third sub-pixel region of the substrate, and
at least one of the first emission layer and the second emission layer comprises the first compound and an organometallic compound.

## Patentansprüche

1. Organische lichtemittierende Vorrichtung, die Folgendes umfasst:
eine erste Elektrode;
eine zweite Elektrode; und
eine organische Schicht zwischen der ersten Elektrode und der zweiten Elektrode, die eine Emissionsschicht umfasst,
wobei die organische Schicht eine erste Verbindung, eine zweite Verbindung und eine dritte Verbindung umfasst, und
die erste Verbindung, die zweite Verbindung und die dritte Verbindung jeweils unabhängig voneinander eine Verbindung auf Azinbasis sind, die durch eine aus den Formeln 1-1 bis 1-5 ausgewählte Verbindung dargestellt wird, außer in dem Fall, in dem die erste Verbindung, die zweite Verbindung und die dritte Verbindung gleichzeitig eine Verbindung auf Azinbasis einschließen, die durch die Formel 1-3 dargestellt wird: 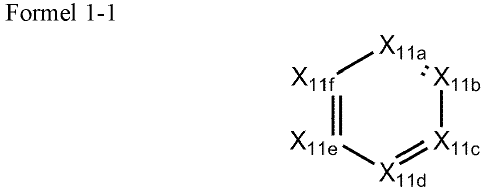 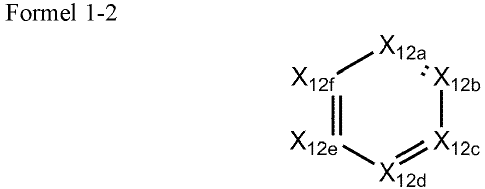 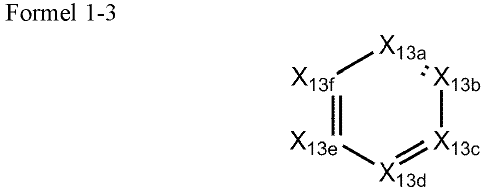 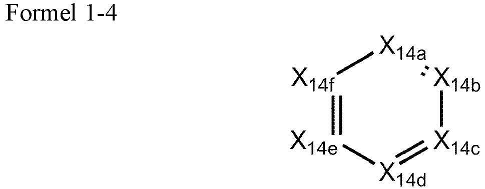 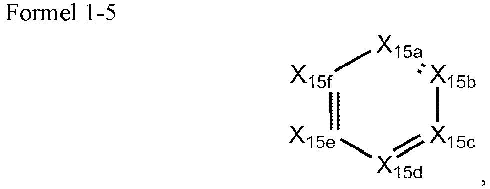
wobei in den Formeln 1-1 bis 1-5,
X₁₁ₐ ausgewählt ist aus N und C[(L₁₁ₐ)ₐ₁₁ₐ-R_{11a]}; X_{11b} ausgewählt ist aus N und C[(L_{11b})_{a11b}-R_{11b}]; X_{11c} ausgewählt ist aus N und C[(L_{11c})_{a11c}-R_{11c}], X_{11d} ausgewählt ist aus N und C[(L_{11d})_{a11d}-R_{11d}]; X₁₁ₑ ausgewählt ist aus N und C[(L₁₁ₑ)ₐ₁₁ₑ-R₁₁ₑ]; X_{11f} ausgewählt ist aus N und C[(L_{11f})_{a11f}-R_{11f}]; und eines von X₁₁ₐ bis X_{11f} N ist,
X₁₂ₐ ausgewählt ist aus N und C[(L₁₂ₐ)ₐ₁₂ₐ-R₁₂ₐ], X_{12b} ausgewählt ist aus N und C[(L_{12b})_{a12b}-R_{12b}]; X_{12c} ausgewählt ist aus N und C[(L_{12c})_{a12c}-R_{12c}]; X_{12d} ausgewählt ist aus N und C[(L_{12d})_{a12d}-R_{12d}]; X₁₂ₑ ausgewählt ist aus N und C[(L₁₂ₑ)ₐ₁₂ₑ-R₁₂ₑ]; X_{12f} ausgewählt ist aus N und C[(L_{12f})_{a12f}-R_{12f}]; und zwei von X₁₂ₐ bis X_{12f} N sind,
X₁₃ₐ ausgewählt ist aus N und C[(L₁₃ₐ)ₐ₁₃ₐ-R₁₃ₐ]; X_{13b} ausgewählt ist aus N und C[(L_{13b})_{a13b}-R_{13b}]; X_{13c} ausgewählt ist aus N und C[(L_{13c})_{a13c}-R_{13c}]; X_{13d} ausgewählt ist aus N und C[(L_{13d})_{a13d}-R_{13d}]; X₁₃ₑ ausgewählt ist aus N und C[(L₁₃ₑ)ₐ₁₃ₑ-R₁₃ₑ]; X_{13f} ausgewählt ist aus N und C[(L_{13f})_{a13f}-R_{13f}]; und drei von X₁₃ₐ bis X_{13f} N sind,
X₁₄ₐ ausgewählt ist aus N und C[(L₁₄ₐ)ₐ₁₄ₐ-R₁₄ₐ]; X_{14b} ausgewählt ist aus N und C[(L_{14b})_{a14b}-R_{14b}]; X_{14c} ausgewählt ist aus N und C[(L_{14c})_{a14c}-R_{14c}]; X_{14d} ausgewählt ist aus N und C[(L_{14d})_{a14d}-R_{14d}]; X₁₄ₑ ausgewählt ist aus N und C[(L₁₄ₑ)ₐ₁₄ₑ-R₁₄ₑ]; X_{14f} ausgewählt ist aus N und C[(L_{14f})_{a14f}-R_{14f}]; und vier von X₁₄ₐ bis X_{14f} N sind,
X₁₅ₐ ausgewählt ist aus N und C[(L₁₅ₐ)ₐ₁₅ₐ-R₁₅ₐ]; X_{15b} ausgewählt ist aus N und C[(L_{15b})_{a15b}-R_{15b}]; X_{15c} ausgewählt ist aus N und C[(L_{15c})_{a15c}-R_{15c}]; X_{15d} ausgewählt ist aus N und C[(L_{15d})_{a15d}-R_{15d}]; X₁₅ₑ ausgewählt ist aus N und C[(L₁₅ₑ)ₐ₁₅ₑ-R₁₅ₑ]; X_{15f} ausgewählt ist aus N und C[(L_{15f})_{a15f}-R_{15f}]; und fünf von X₁₅ₐ bis X_{15f} N sind,
L₁₁ₐ bis L_{11f}, L₁₂ₐ bis L_{12f}, L₁₃ₐ bis L_{13f}, L₁₄ₐ bis L_{14f}, und L₁₅ₐ bis L_{15f} sind jeweils unabhängig voneinander ausgewählt aus:
einer C₃-C₁₀-Cycloalkenylengruppe, einer C₁-C₁₀-Heterocycloalkenylengruppe, einer C₆-C₆₀-Arylengruppe, einer C₁-C₆₀-Heteroarylengruppe, einer zweiwertigen nichtaromatischen kondensierten polyzyklischen Gruppe und einer zweiwertige nichtaromatischen kondensierten heteropolyzyklischen Gruppe; und
einer C₃-C₁₀-Cycloalkenylengruppe, einer C₁-C₁₀-Heterocycloalkenylengruppe, einer C₆-C₆₀-Arylengruppe, einer C₁-C₆₀-Heteroarylengruppe, einer zweiwertigen nicht-aromatischen kondensierten polyzyklischen Gruppe und einer zweiwertigen nicht-aromatischen kondensierten heteropolyzyklischen Gruppe, jeweils substituiert mit mindestens einer Gruppe, ausgewählt aus Deuterium, -F, -Cl, -Br, -I, einer Hydroxylgruppe, einer Cyanogruppe, einer Nitrogruppe, einer Aminogruppe, einer Amidinogruppe, einer Hydrazingruppe, einer Hydrazongruppe, einer Carbonsäuregruppe oder einem Salz davon, einer Sulfonsäuregruppe oder einem Salz davon, einer Phosphorsäuregruppe oder einem Salz davon, einer C₁-C₂₀-Alkylgruppe, einer C₁-C₂₀-Alkoxygruppe, einer C₃-C₁₀-Cycloalkylgruppe, einer C₁-C₁₀-Heterocycloalkylgruppe, einer C₃-C₁₀-Cycloalkenylgruppe, einer C₁-C₁₀-Heterocycloalkenylgruppe, einer C₆-C₃₀-Arylgruppe, einer C₆-C₃₀-Aryloxygruppe, einer C₆-C₃₀ Arylthiogruppe, einer C₁-C₃₀-Heteroaryloxygruppe, einer C₁-C₃₀-Heteroarylthiogruppe, einer monovalenten nicht-aromatischen kondensierten polyzyklischen Gruppe, einer monovalenten nicht-aromatischen kondensierten heteropolyzyklischen Gruppe, einer Pyrrolylgruppe, einer Thiophenylgruppe, einer Furanylgruppe, einer Imidazolylgruppe, einer Pyrazolylgruppe, einer Thiazolylgruppe, einer Isothiazolylgruppe, einer Oxazolylgruppe, einer Isoxazolylgruppe, einer Pyridinylgruppe, einer Pyrazinylgruppe, einer Pyrimidinylgruppe, einer Pyridazinylgruppe, einer Isoindolylgruppe, einer Indolylgruppe, einer Indazolylgruppe, einer Purinylgruppe, einer Chinolinylgruppe, einer Isochinolinylgruppe, einer Benzochinolinylgruppe, einer Phthalazinylgruppe, einer Naphthyridinylgruppe, einer Chinoxalinylgruppe, einer Chinazolinylgruppe, einer Cinnolinylgruppe, einer Carbazolylgruppe, einer Phenanthridinylgruppe, einer Acridinylgruppe, einer Phenanthrolinylgruppe, einer Phenazinylgruppe, einer Benzimidazolylgruppe, einer Benzofuranylgruppe, einer Benzothiophenylgruppe, einer Isobenzothiazolylgruppe, einer Benzoxazolylgruppe, einer Isobenzoxazolylgruppe, einer Triazolylgruppe, einer Tetrazolylgruppe, einer Oxadiazolylgruppe, einer Dibenzofuranylgruppe, einer Dibenzothiophenylgruppe, einer Benzocarbazolylgruppe, einer Dibenzocarbazolylgruppe, einer Thiadiazolylgruppe und einer Imidazopyridinylgruppe, wobei L₁₁ₐ bis L_{11f}, L₁₂, bis L_{12f}, L₁₅ₐ bis L_{13f}, L₁₄ₐ bis L_{14f} und L₁₅ₐ bis Lisf nicht jeweils unabhängig voneinander ausgewählt sind aus: einer Triazinylengruppe; und einer Triazinylengruppe, die mit mindestens einer der folgenden Gruppen substituiert ist: Deuterium, -F, -Cl, -Br, -I, einer Hydroxylgruppe, einer Cyanogruppe, einer Nitrogruppe, einer Aminogruppe, einer Amidinogruppe, einer Hydrazingruppe, einer Hydrazongruppe, einer Carbonsäuregruppe oder einem Salz davon, einer Sulfonsäuregruppe oder einem Salz davon, einer Phosphorsäuregruppe oder einem Salz davon, einer C₁-C₂₀-Alkylgruppe, einer C₁-C₂₀-Alkoxygruppe, einer C₃-C₁₀-Cycloalkylgruppe, einer C₁-C₁₀-Heterocycloalkylgruppe, einer C₃-C₁₀-Cycloalkenylgruppe, einer C₁-C₁₀-Heterocycloalkenylgruppe, einer C₆-C₃₀-Arylgruppe, einer C₆-C₃₀-Aryloxygruppe, einer C₆-C₃₀-Arylthiogruppe, einer C₁-C₃₀-Heteroaryloxygruppe, einer C₁-C₃₀-Heteroarylthiogruppe, einer monovalenten nicht-aromatischen kondensierten polycyclischen Gruppe, einer monovalenten nicht-aromatischen kondensierten heteropolyzyklischen Gruppe, einer Pyrrolylgruppe, einer Thiophenylgruppe, einer Furanylgruppe, einer Imidazolylgruppe, einer Pyrazolylgruppe, einer Thiazolylgruppe, einer Isothiazolylgruppe, einer Oxazolylgruppe, einer Isoxazolylgruppe, einer Pyridinylgruppe, einer Pyrazinylgruppe, einer Pyrimidinylgruppe, einer Pyridazinylgruppe, einer Isoindolylgruppe, einer Indolylgruppe, einer Indazolylgruppe, einer Purinylgruppe, einer Chinolinylgruppe, einer Isochinolinylgruppe, einer Benzochinolinylgruppe, einer Phthalazinylgruppe, einer Naphthyridinylgruppe, einer Chinoxalinylgruppe, einer Chinazolinylgruppe, einer Cinnolinylgruppe, einer Carbazolylgruppe, einer Phenanthridinylgruppe, einer Acridinylgruppe, einer Phenanthrolinylgruppe, einer Phenazinylgruppe, einer Benzimidazolylgruppe, einer Benzofuranylgruppe, einer Benzothiophenylgruppe, einer Isobenzothiazolylgruppe, einer Benzoxazolylgruppe, einer Isobenzoxazolylgruppe, einer Triazolylgruppe, einer Tetrazolylgruppe, einer Oxadiazolylgruppe, einer Dibenzofuranylgruppe, einer Dibenzothiophenylgruppe, einer Benzocarbazolylgruppe, einer Dibenzocarbazolylgruppe, einer Thiadiazolylgruppe und einer Imidazopyridinylgruppe,
a11a bis a11f, a12a bis a12f, a13a bis a13f, a14a bis a14f und a15a bis a15f jeweils unabhängig voneinander aus 0, 1, 2, 3 und 4 ausgewählt sind,
R₁₁ₐ bis R_{11f}, R₁₂ₐ bis R_{12f}, R₁₃ₐ bis R_{13f}, R₁₄ₐ bis R_{14f}, und R₁₅ₐ bis R_{15f} jeweils unabhängig voneinander ausgewählt sind aus:
Wasserstoff, Deuterium, -F, -Cl, -Br, -I, einer Methylgruppe, einer Ethylgruppe, einer n-Propylgruppe, einer Isopropylgruppe, einer n-Butylgruppe, einer Isobutylgruppe, einer sec-Butylgruppe, einer tert-Butylgruppe, einer Methoxygruppe, einer Ethoxygruppe, einer n-Propoxygruppe, einer Isopropoxygruppe, einer n-Butoxygruppe, einer Isobutoxygruppe, einer sec-Butoxygruppe und einer tert-Butoxygruppe;
einer Methylgruppe, einer Ethylgruppe, einer n-Propylgruppe, einer Isopropylgruppe, einer n-Butylgruppe, einer Isobutylgruppe, einer sec-Butylgruppe und einer tert-Butylgruppe, jeweils substituiert mit mindestens einer Gruppe, ausgewählt aus Deuterium, -F, -Cl, -Br und -I;
einer Phenylgruppe, einer Biphenylgruppe, einer Terphenylgruppe, einer Naphthylgruppe, einer Fluorenylgruppe, einer Carbazolylgruppe, einer Dibenzofuranylgruppe, einer Dibenzothiophenylgruppe, einer Pyridinylgruppe, einer Pyrimidinylgruppe, einer Chinolinylgruppe, einer Isochinolinylgruppe, einer Chinoxalinylgruppe, einer Chinazolinylgruppe, einer Benzofuropyridinylgruppe, einer Benzothienopyridinylgruppe, einer Benzofuropyrimidinylgruppe, einer Benzothienopyrimidinylgruppe, einer monovalenten nicht-aromatischen kondensierten polyzyklischen Gruppe und einer monovalenten nicht-aromatischen kondensierten heteropolyzyklischen Gruppe;
einer Phenylgruppe, einer Biphenylgruppe, einer Terphenylgruppe, einer Naphthylgruppe, einer Fluorenylgruppe, einer Carbazolylgruppe, einer Dibenzofuranylgruppe, einer Dibenzothiophenylgruppe, einer Pyridinylgruppe, einer Pyrimidinylgruppe, einer Chinolinylgruppe, einer Isochinolinylgruppe, einer Chinoxalinylgruppe, einer Chinazolinylgruppe, einer Benzofuropyridinylgruppe, einer Benzothienopyridinylgruppe, einer Benzofuropyrimidinylgruppe, einer Benzothienopyrimidinylgruppe, einer einwertigen nichtaromatischen kondensierten polyzyklischen Gruppe und einer einwertigen nichtaromatischen kondensierten heteropolyzyklischen Gruppe, jeweils substituiert mit mindestens einer Gruppe, ausgewählt aus Deuterium, -F, -Cl, -Br, -I, einer Methylgruppe, einer Ethylgruppe, einer n-Propylgruppe, einer Isopropylgruppe, einer n-Butylgruppe, einer Isobutylgruppe, einer sec-Butylgruppe, einer tert-Butylgruppe, einer Methoxygruppe, einer Ethoxygruppe, einer n-Propoxygruppe, einer Isopropoxygruppe, einer n-Butoxygruppe, einer Isobutoxygruppe, einer sec-Butoxygruppe, einer tert-Butoxygruppe, einer Phenylgruppe, einer Biphenylgruppe, einer Terphenylgruppe, einer Naphthylgruppe, einer Fluorenylgruppe, einer Carbazolylgruppe, einer Dibenzofuranylgruppe, einer Dibenzothiophenylgruppe, einer Pyridinylgruppe, einer Pyrimidinylgruppe, einer Chinolinylgruppe, einer Isochinolinylgruppe, einer Chinoxalinylgruppe, einer Chinazolinylgruppe, einer Benzofuropyridinylgruppe, einer Benzothienopyridinylgruppe, einer Benzofuropyrimidinylgruppe, und einer Benzothienopyrimidinylgruppe, und
einer Phenylgruppe, einer Biphenylgruppe, einer Terphenylgruppe, einer Naphthylgruppe, einer Fluorenylgruppe, einer Carbazolylgruppe, einer Dibenzofuranylgruppe, einer Dibenzothiophenylgruppe, einer Pyridinylgruppe, einer Pyrimidinylgruppe, einer Chinolinylgruppe, einer Isochinolinylgruppe, einer Chinoxalinylgruppe, einer Chinazolinylgruppe, einer Benzofuropyridinylgruppe, einer Benzothienopyridinylgruppe, einer Benzofuropyrimidinylgruppe, einer Benzothienopyrimidinylgruppe, einer einwertigen nichtaromatischen kondensierten polyzyklischen Gruppe und einer einwertigen nichtaromatischen kondensierten heteropolyzyklischen Gruppe, jeweils substituiert mit mindestens einer Gruppe, ausgewählt aus einer Methylgruppe, einer Ethylgruppe, einer n-Propylgruppe, einer Isopropylgruppe, einer n-Butylgruppe, einer Isobutylgruppe, einer sec-Butylgruppe, einer tert-Butylgruppe, einer Phenylgruppe, einer Biphenylgruppe, einer Terphenylgruppe, einer Naphthylgruppe, einer Fluorenylgruppe, einer Carbazolylgruppe, einer Dibenzofuranylgruppe, einer Dibenzothiophenylgruppe, einer Pyridinylgruppe, einer Pyrimidinylgruppe, einer Chinolinylgruppe, einer Isochinolinylgruppe, einer Chinoxalinylgruppe, einer Chinazolinylgruppe, einer Benzofuropyridinylgruppe, einer Benzothienopyridinylgruppe, einer Benzofuropyrimidinylgruppe und einer Benzothienopyrimidinylgruppe, die jeweils mit mindestens einem aus Deuterium, -F, -Cl, -Br und -I substituiert sind, und
benachbarte Atome von R₁₁ₐ bis R_{11f}, benachbarte Atome von R₁₁ₐ bis R_{12f}, benachbarte Atome von R₁₃ₐ bis R_{13f}, benachbarte Atome von R₁₄ₐ bis R_{14f} und/oder benachbarte Atome von R₁₅ₐ bis R_{15f} wahlweise einen gesättigten Ring oder einen ungesättigten Ring bilden.

2. Organische lichtemittierende Vorrichtung nach Anspruch 1, wobei:
L₁₁ₐ bis L_{11f}, L₁₂ₐ bis L_{12f}, L₁₅ₐ bis L_{13f}, L₁₄ₐ bis L_{14f}, und L₁₅ₐ bis L_{15f} sind jeweils unabhängig voneinander ausgewählt aus:
einer Phenylengruppe, einer Pentalenylengruppe, einer Indenylengruppe, einer Naphthylengruppe, einer Azulenylengruppe, einer Heptalenylengruppe, einer Indacenylengruppe, einer Acenaphthylengruppe, einer Fluorenylengruppe, einer Spirofluorenylengruppe, einer Benzofluorenylengruppe, einer Dibenzofluorenylengruppe, einer Phenalenylengruppe, einer Phenanthrenylengruppe, einer Anthracenylengruppe, einer Fluoranthenylengruppe, einer Triphenylenylengruppe, einer Pyrenylengruppe, einer Chrysenylengruppe, einer Naphthacenylengruppe, einer Pikenylengruppe, einer Perylenylengruppe, einer Pentaphenylengruppe, einer Hexacenylengruppe, einer Pentacenylengruppe, einer Rubicenylengruppe, einer Coronenylengruppe, einer Ovalenylengruppe, einer Pyrrolylengruppe, einer Thiophenylengruppe, einer Furanylengruppe, einer Imidazolylengruppe, einer Pyrazolylengruppe, einer Thiazolylengruppe, einer Isothiazolylengruppe, einer Oxazolylengruppe, einer Isoxazolylengruppe, einer Pyridinylengruppe, einer Pyrazinylengruppe, einer Pyrimidinylengruppe, einer Pyridazinylengruppe, einer Isoindolylengruppe, einer Indolylengruppe, einer Indazolylengruppe, einer Purinylengruppe, einer Chinolinylengruppe, einer Isochinolinylengruppe, einer Benzochinolinylengruppe, einer Phthalazinylengruppe, einer Naphthyridinylengruppe, einer Chinoxalinylengruppe, einer Chinazolinylengruppe, einer Cinnolinylengruppe, einer Carbazolylengruppe, einer Phenanthridinylengruppe, einer Acridinylengruppe, einer Phenanthrolinylengruppe, einer Phenazinylengruppe, einer Benzimidazolylengruppe, einer Benzofuranylengruppe, einer Benzothiophenylengruppe, einer Isobenzothiazolylengruppe, einer Benzoxazolylengruppe, einer Isobenzoxazolylengruppe, einer Triazolylengruppe, einer Tetrazolylengruppe, einer Oxadiazolylengruppe, einer Dibenzofuranylengruppe, einer Dibenzothiophenylengruppe, einer Benzofuropyridinylengruppe, einer Benzothienopyridinylengruppe, einer Benzofuropyrimidinylengruppe, einer Benzothienopyrimidinylengruppe, einer Benzocarbazolylengruppe und einer Dibenzocarbazolylengruppe; und
einer Phenylengruppe, einer Pentalenylengruppe, einer Indenylengruppe, einer Naphthylengruppe, einer Azulenylengruppe, einer Heptalenylengruppe, einer Indacenylengruppe, einer Acenaphthylengruppe, einer Fluorenylengruppe, einer Spirofluorenylengruppe, einer Benzofluorenylengruppe, einer Dibenzofluorenylengruppe, einer Phenalenylengruppe, einer Phenanthrenylengruppe, einer Anthracenylengruppe, einer Fluoranthenylengruppe, einer Triphenylenylengruppe, einer Pyrenylengruppe, einer Chrysenylengruppe, einer Naphthacenylengruppe, einer Picenylengruppe, einer Perylengruppe, einer Pentaphenylengruppe, einer Hexacenylengruppe, einer Pentacenylengruppe, einer Rubicenylengruppe, einer Coronenylengruppe, einer Ovalenylengruppe, einer Pyrrolylengruppe, einer Thiophenylengruppe, einer Furanylengruppe, einer Imidazolylengruppe, einer Pyrazolylengruppe, einer Thiazolylengruppe, einer Isothiazolylengruppe, einer Oxazolylengruppe, einer Isoxazolylengruppe, einer Pyridinylengruppe, einer Pyrazinylengruppe, einer Pyrimidinylengruppe, einer Pyridazinylengruppe, einer Isoindolylengruppe, einer Indolylengruppe, einer Indazolylengruppe, einer Purinylengruppe, einer Chinolinylengruppe, einer Isochinolinylengruppe, einer Benzochinolinylengruppe, einer Phthalazinylengruppe, einer Naphthyridinylengruppe, einer Chinoxalinylengruppe, einer Chinazolinylengruppe, einer Cinnolinylengruppe, einer Carbazolylengruppe, einer Phenanthridinylengruppe, einer Acridinylengruppe, einer Phenanthrolinylengruppe, einer Phenazinylengruppe, einer Benzimidazolylengruppe, einer Benzofuranylengruppe, einer Benzothiophenylengruppe, einer Isobenzothiazolylengruppe, einer Benzoxazolylengruppe, einer Isobenzoxazolylengruppe, einer Triazolylengruppe, einer Tetrazolylengruppe, einer Oxadiazolylengruppe, einer Dibenzofuranylengruppe, einer Dibenzothiophenylengruppe, einer Benzofuropyridinylengruppe, einer Benzothienopyridinylengruppe, einer Benzofuropyrimidinylengruppe, einer Benzothienopyrimidinylengruppe, einer Benzocarbazolylengruppe und einer Dibenzocarbazolylengruppe, jeweils substituiert mit mindestens einer Gruppe, ausgewählt aus Deuterium, -F, -Cl, -Br, -I, einer Hydroxylgruppe, einer Cyanogruppe, einer Nitrogruppe, einer Aminogruppe, einer Amidinogruppe, einer Hydrazingruppe, einer Hydrazongruppe, einer Carbonsäuregruppe oder einem Salz davon, einer Sulfonsäuregruppe oder einem Salz davon, einer Phosphorsäuregruppe oder einem Salz davon, einer C₁-C₂₀-Alkylgruppe, einer C₁-C₂₀-Alkoxygruppe, einer Cyclopentylgruppe, einer Cyclohexylgruppe, einer Cycloheptylgruppe, einer Cyclopentenylgruppe, einer Cyclohexenylgruppe, einer Phenylgruppe, einer Pentalenylgruppe, einer Indenylgruppe, einer Naphthylgruppe, einer Azulenylgruppe, einer Heptalenylgruppe, einer Indacenylgruppe, einer Acenaphthylgruppe, einer Fluorenylgruppe, einer Spirofluorenylgruppe, einer Benzofluorenylgruppe, einer Dibenzofluorenylgruppe, einer Phenalenylgruppe, einer Phenanthrenylgruppe, einer Anthracenylgruppe, einer Fluoranthenylgruppe, einer Triphenylenylgruppe, einer Pyrenylgruppe, einer Chrysenylgruppe, einer Naphthacenylgruppe, einer Pikenylgruppe, einer Perylenylgruppe, einer Pentaphenylgruppe, einer Hexacenylgruppe, einer Pentacenylgruppe, einer Rubicenylgruppe, einer Coronenylgruppe, einer Ovalenylgruppe, einer Pyrrolylgruppe, einer Thiophenylgruppe, einer Furanylgruppe, einer Imidazolylgruppe, einer Pyrazolylgruppe, einer Thiazolylgruppe, einer Isothiazolylgruppe, einer Oxazolylgruppe, einer Isoxazolylgruppe, einer Pyridinylgruppe, einer Pyrazinylgruppe, einer Pyrimidinylgruppe, einer Pyridazinylgruppe, einer Isoindolylgruppe, einer Indolylgruppe, einer Indazolylgruppe, einer Purinylgruppe, einer Chinolinylgruppe, einer Isochinolinylgruppe, einer Benzochinolinylgruppe, einer Phthalazinylgruppe, einer Naphthyridinylgruppe, einer Chinoxalinylgruppe, einer Chinazolinylgruppe, einer Cinnolinylgruppe, einer Carbazolylgruppe, einer Phenanthridinylgruppe, einer Acridinylgruppe, einer Phenanthrolinylgruppe, einer Phenazinylgruppe, einer Benzimidazolylgruppe, einer Benzofuranylgruppe, einer Benzothiophenylgruppe, einer Isobenzothiazolylgruppe, einer Benzoxazolylgruppe, einer Isobenzoxazolylgruppe, einer Triazolylgruppe, einer Tetrazolylgruppe, einer Oxadiazolylgruppe, einer Dibenzofuranylgruppe, einer Dibenzothiophenylgruppe, einer Benzocarbazolylgruppe, einer Dibenzocarbazolylgruppe, einer Thiadiazolylgruppe und einer Imidazopyridinylgruppe.

3. Organische lichtemittierende Vorrichtung nach Anspruch 1 oder Anspruch 2, wobei:
R₁₁ₐ bis Ruf, R₁₂ₐ bis R_{12f}, R₁₃ₐ bis R_{13f}, R₁₄ₐ bis R_{14f}, und R₁₅ₐ bis R_{15f} jeweils unabhängig voneinander ausgewählt sind aus:
Wasserstoff, Deuterium, -F, -Cl, -Br, -I, einer Methylgruppe, einer Ethylgruppe, einer n-Propylgruppe, einer Isopropylgruppe, einer n-Butylgruppe, einer Isobutylgruppe, einer sec-Butylgruppe, einer tert-Butylgruppe, einer Methoxygruppe, einer Ethoxygruppe, einer n-Propoxygruppe, einer Isopropoxygruppe, einer n-Butoxygruppe, einer Isobutoxygruppe, einer sec-Butoxygruppe und einer tert-Butoxygruppe;
einer Methylgruppe, einer Ethylgruppe, einer n-Propylgruppe, einer Isopropylgruppe, einer n-Butylgruppe, einer Isobutylgruppe, einer sec-Butylgruppe und einer tert-Butylgruppe, jeweils substituiert mit mindestens einer Gruppe, ausgewählt aus Deuterium, -F, -Cl, -Br und -I;
einer Phenylgruppe, einer Biphenylgruppe, einer Terphenylgruppe, einer Naphthylgruppe, einer Fluorenylgruppe, einer Carbazolylgruppe, einer Dibenzofuranylgruppe, einer Dibenzothiophenylgruppe, einer Pyridinylgruppe, einer Pyrimidinylgruppe, einer Chinolinylgruppe, einer Isochinolinylgruppe, einer Chinoxalinylgruppe, einer Chinazolinylgruppe, einer Benzofuropyridinylgruppe, einer Benzothienopyridinylgruppe, einer Benzofuropyrimidinylgruppe und einer Benzothienopyrimidinylgruppe,
einer Phenylgruppe, einer Biphenylgruppe, einer Terphenylgruppe, einer Naphthylgruppe, einer Fluorenylgruppe, einer Carbazolylgruppe, einer Dibenzofuranylgruppe, einer Dibenzothiophenylgruppe, einer Pyridinylgruppe, einer Pyrimidinylgruppe, einer Chinolinylgruppe, einer Isochinolinylgruppe, einer Chinoxalinylgruppe, einer Chinazolinylgruppe, einer Benzofuropyridinylgruppe, einer Benzothienopyridinylgruppe, einer Benzofuropyrimidinylgruppe und einer Benzothienopyrimidinylgruppe, jeweils substituiert mit mindestens einer Gruppe, ausgewählt aus Deuterium, -F, -Cl, -Br, -I, einer Methylgruppe, einer Ethylgruppe, einer n-Propylgruppe, einer Isopropylgruppe, einer n-Butylgruppe, einer Isobutylgruppe, einer sec-Butylgruppe, einer tert-Butylgruppe, einer Methoxygruppe, einer Ethoxygruppe, einer n-Propoxygruppe, einer Isopropoxygruppe, einer n-Butoxygruppe, einer Isobutoxygruppe, einer sec-Butoxygruppe, einer tert-Butoxygruppe, einer Phenylgruppe, einer Biphenylgruppe, einer Terphenylgruppe, einer Naphthylgruppe, einer Fluorenylgruppe, einer Carbazolylgruppe, einer Dibenzofuranylgruppe, einer Dibenzothiophenylgruppe, einer Pyridinylgruppe, einer Pyrimidinylgruppe, einer Chinolinylgruppe, einer Isochinolinylgruppe, einer Chinoxalinylgruppe, einer Chinazolinylgruppe, einer Benzofuropyridinylgruppe, einer Benzothienopyridinylgruppe, einer Benzofuropyrimidinylgruppe und einer Benzothienopyrimidinylgruppe,
einer Phenylgruppe, einer Biphenylgruppe, einer Terphenylgruppe, einer Naphthylgruppe, einer Fluorenylgruppe, einer Carbazolylgruppe, einer Dibenzofuranylgruppe, einer Dibenzothiophenylgruppe, einer Pyridinylgruppe, einer Pyrimidinylgruppe, einer Chinolinylgruppe, einer Isochinolinylgruppe, einer Chinoxalinylgruppe, einer Chinazolinylgruppe, einer Benzofuropyridinylgruppe, einer Benzothienopyridinylgruppe, einer Benzofuropyrimidinylgruppe und einer Benzothienopyrimidinylgruppe, jeweils substituiert mit mindestens einer Gruppe, ausgewählt aus einer Methylgruppe, einer Ethylgruppe, einer n-Propylgruppe, einer Isopropylgruppe, einer n-Butylgruppe, einer Isobutylgruppe, einer sec-Butylgruppe, einer tert-Butylgruppe, einer Phenylgruppe, einer Biphenylgruppe, einer Terphenylgruppe, einer Naphthylgruppe, einer Fluorenylgruppe, einer Carbazolylgruppe, einer Dibenzofuranylgruppe, einer Dibenzothiophenylgruppe, einer Pyridinylgruppe, einer Pyrimidinylgruppe, einer Chinolinylgruppe, einer Isochinolinylgruppe, einer Chinoxalinylgruppe, einer Chinazolinylgruppe, einer Benzofuropyridinylgruppe, einer Benzothienopyridinylgruppe, einer Benzofuropyrimidinylgruppe und einer Benzothienopyrimidinylgruppe, die jeweils mit mindestens einer Gruppe, ausgewählt aus Deuterium, -F, -Cl, -Br und -I, substituiert sind und
einer Teilstruktur, die durch eine aus den Formeln 2-1 bis 2-17 ausgewählte Formel dargestellt wird: 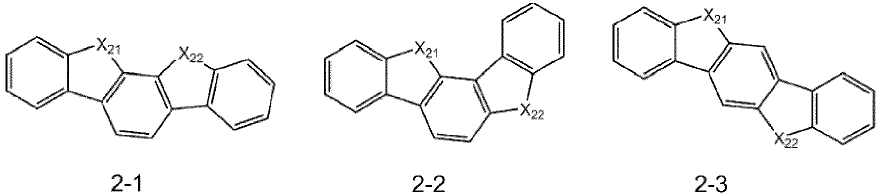 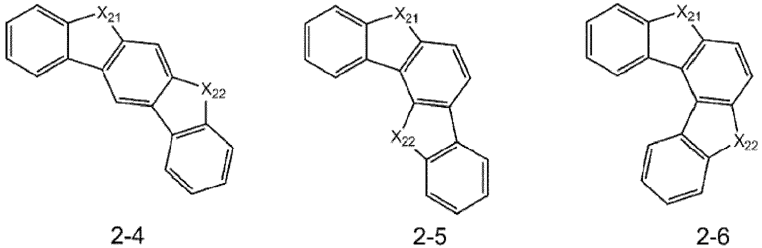 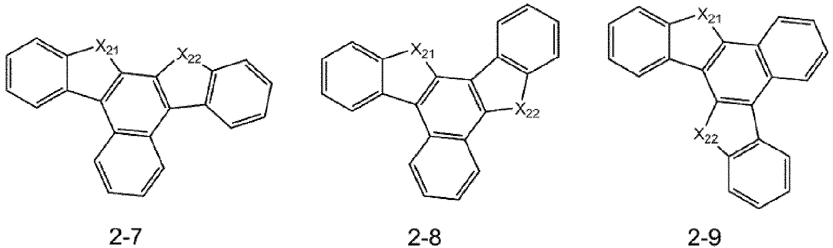 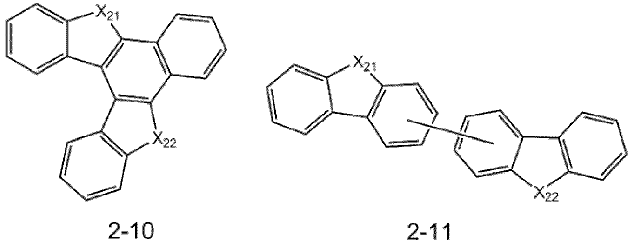 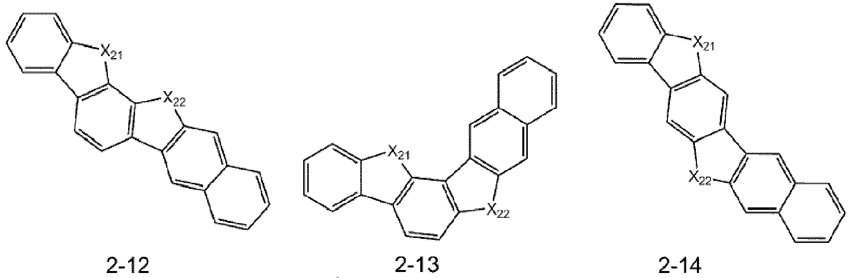 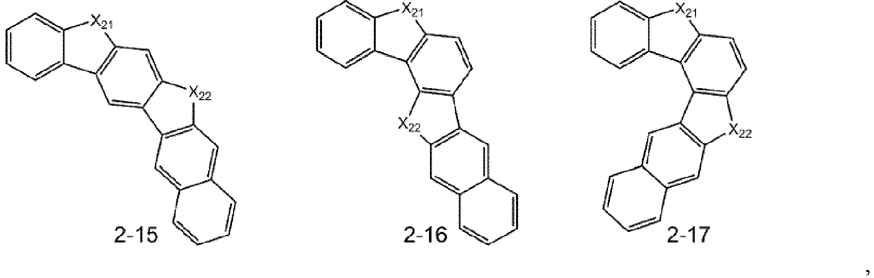
wobei in den Formeln 2-1 bis 2-17,
X₂₁ ausgewählt ist aus C(R₂₁)(R₂₂), N(R₂₁), O und S,
X₂₂ ausgewählt ist aus C(R₂₃)(R₂₄), N(R₂₃), O und S,
R₂₁ bis R₂₄ sind jeweils unabhängig voneinander ausgewählt aus:
einer Methylgruppe, einer Ethylgruppe, einer n-Propylgruppe, einer Isopropylgruppe, einer n-Butylgruppe, einer Isobutylgruppe, einer sec-Butylgruppe und einer tert-Butylgruppe;
einer Methylgruppe, einer Ethylgruppe, einer n-Propylgruppe, einer Isopropylgruppe, einer n-Butylgruppe, einer Isobutylgruppe, einer sec-Butylgruppe und einer tert-Butylgruppe, jeweils substituiert mit mindestens einer Gruppe, ausgewählt aus Deuterium, -F, -Cl, -Br und -I;
einer Phenylgruppe, einer Biphenylgruppe, einer Terphenylgruppe, einer Naphthylgruppe, einer Fluorenylgruppe, einer Carbazolylgruppe, einer Dibenzofuranylgruppe, einer Dibenzothiophenylgruppe, einer Pyridinylgruppe, einer Pyrimidinylgruppe, einer Chinolinylgruppe, einer Isochinolinylgruppe, einer Chinoxalinylgruppe, einer Chinazolinylgruppe, einer Benzofuropyridinylgruppe, einer Benzothienopyridinylgruppe, einer Benzofuropyrimidinylgruppe, und einer Benzothienopyrimidinylgruppe, und
einer Phenylgruppe, einer Biphenylgruppe, einer Terphenylgruppe, einer Naphthylgruppe, einer Fluorenylgruppe, einer Carbazolylgruppe, einer Dibenzofuranylgruppe, einer Dibenzothiophenylgruppe, einer Pyridinylgruppe, einer Pyrimidinylgruppe, einer Chinolinylgruppe, einer Isochinolinylgruppe, einer Chinoxalinylgruppe, einer Chinazolinylgruppe, einer Benzofuropyridinylgruppe, einer Benzothienopyridinylgruppe, einer Benzofuropyrimidinylgruppe und einer Benzothienopyrimidinylgruppe, jeweils substituiert mit mindestens einer Gruppe, ausgewählt aus Deuterium, -F, -Cl, -Br, -I, einer Methylgruppe, einer Ethylgruppe, einer n-Propylgruppe, einer Isopropylgruppe, einer n-Butylgruppe, einer Isobutylgruppe, einer sec-Butylgruppe, einer tert-Butylgruppe, einer Methoxygruppe, einer Ethoxygruppe, einer n-Propoxygruppe, einer Isopropoxygruppe, einer n-Butoxygruppe, einer Isobutoxygruppe, einer sec-Butoxygruppe, einer tert-Butoxygruppe, einer Phenylgruppe, einer Biphenylgruppe, einer Terphenylgruppe, einer Naphthylgruppe, einer Fluorenylgruppe, einer Carbazolylgruppe, einer Dibenzofuranylgruppe, einer Dibenzothiophenylgruppe, einer Pyridinylgruppe, einer Pyrimidinylgruppe, einer Chinolinylgruppe, einer Isochinolinylgruppe, einer Chinoxalinylgruppe, einer Chinazolinylgruppe, einer Benzofuropyridinylgruppe, einer Benzothienopyridinylgruppe, einer Benzofuropyrimidinylgruppe und einer Benzothienopyrimidinylgruppe, und
eines, ausgewählt aus einem Kohlenstoffatom und einem Stickstoffatom, die die durch eine aus den Formeln 2-1 bis 2-17 ausgewählte Formel dargestellte Teilstruktur bilden, eine Bindungsstelle zu einem benachbarten Atom, ausgewählt aus den Formeln 1-1 bis 1-5, ist;
vorzugsweise wobei:
R₁₁ₐ bis R_{11f}, R₁₂ₐ bis R_{12f}, R₁₃ₐ bis R_{13f}, R₁₄ₐ bis R_{14f}, und R₁₅ₐ bis R_{15f} jeweils unabhängig voneinander ausgewählt sind aus:
Wasserstoff, Deuterium, -F, -Cl, -Br, -I, einer Methylgruppe, einer Ethylgruppe, einer n-Propylgruppe, einer Isopropylgruppe, einer n-Butylgruppe, einer Isobutylgruppe, einer sec-Butylgruppe und einer tert-Butylgruppe;
einer Phenylgruppe, einer Biphenylgruppe, einer Terphenylgruppe, einer Naphthylgruppe, einer Fluorenylgruppe, einer Carbazolylgruppe, einer Dibenzofuranylgruppe, einer Dibenzothiophenylgruppe, einer Pyridinylgruppe, einer Pyrimidinylgruppe, einer Chinolinylgruppe, einer Isochinolinylgruppe, einer Chinoxalinylgruppe und einer Chinazolinylgruppe,
einer Phenylgruppe, einer Biphenylgruppe, einer Terphenylgruppe, einer Naphthylgruppe, einer Fluorenylgruppe, einer Carbazolylgruppe, einer Dibenzofuranylgruppe, einer Dibenzothiophenylgruppe, einer Pyridinylgruppe, einer Pyrimidinylgruppe, einer Chinolinylgruppe, einer Isochinolinylgruppe, einer Chinoxalinylgruppe und einer Chinazolinylgruppe, jeweils substituiert mit mindestens einer Gruppe, ausgewählt aus Deuterium, -F, -Cl, -Br, -I, einer Methylgruppe, einer Ethylgruppe, einer n-Propylgruppe, einer Isopropylgruppe, einer n-Butylgruppe, einer Isobutylgruppe, einer sec-Butylgruppe, einer tert-Butylgruppe, einer Phenylgruppe, einer Biphenylgruppe, einer Terphenylgruppe, einer Naphthylgruppe, einer Fluorenylgruppe, einer Carbazolylgruppe, einer Dibenzofuranylgruppe, einer Dibenzothiophenylgruppe, einer Pyridinylgruppe, einer Pyrimidinylgruppe, einer Chinolinylgruppe, einer Isochinolinylgruppe, einer Chinoxalinylgruppe, und einer Chinazolinylgruppe, und
einer Teilstruktur, die durch eine aus den Formeln 2-1 bis 2-17 ausgewählte Formel dargestellt wird: 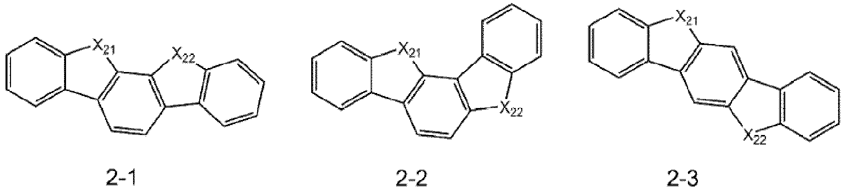 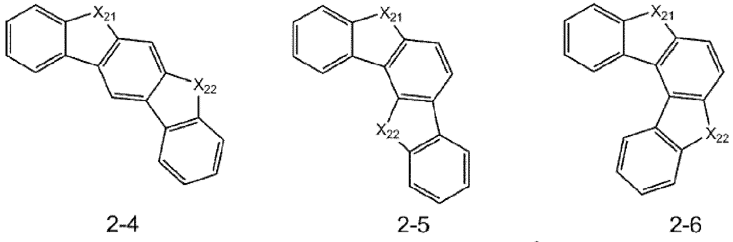 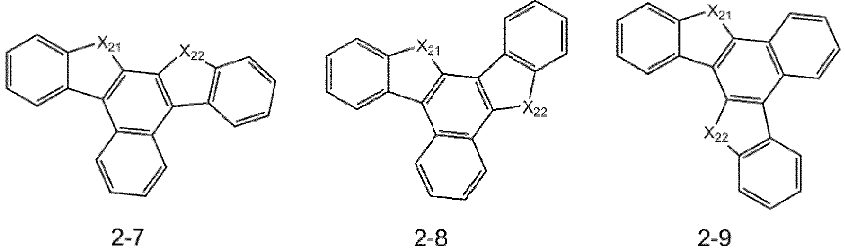 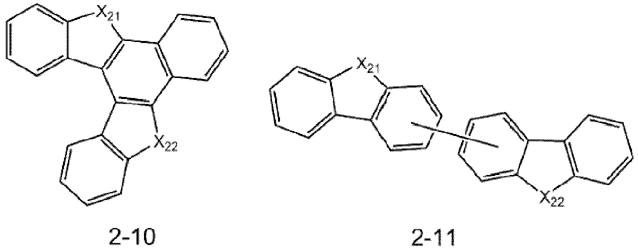 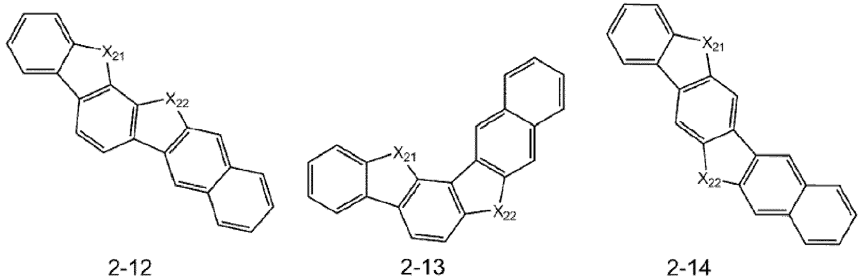 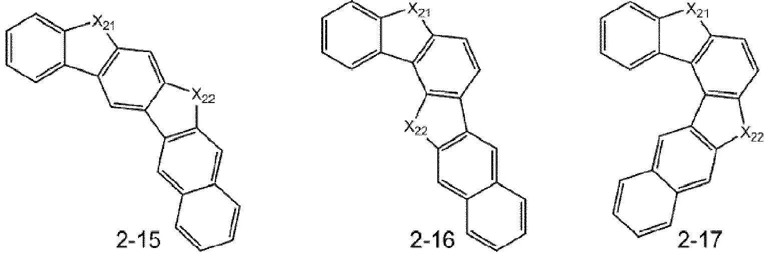
wobei in den Formeln 2-1 bis 2-17,
X₂₁ ausgewählt ist aus C(R₂₁)(R₂₂), N(R₂₁), O und S,
X₂₂ ausgewählt ist aus C(R₂₃)(R₂₄), N(R₂₃), O und S,
R₂₁ bis R₂₄ sind jeweils unabhängig voneinander ausgewählt aus:
einer Methylgruppe, einer Ethylgruppe, einer n-Propylgruppe, einer Isopropylgruppe, einer n-Butylgruppe, einer Isobutylgruppe, einer sec-Butylgruppe und einer tert-Butylgruppe;
einer Methylgruppe, einer Ethylgruppe, einer n-Propylgruppe, einer Isopropylgruppe, einer n-Butylgruppe, einer Isobutylgruppe, einer sec-Butylgruppe und einer tert-Butylgruppe, jeweils substituiert mit mindestens einer Gruppe, ausgewählt aus Deuterium, -F, -Cl, -Br und -I; und
einer Phenylgruppe, einer Biphenylgruppe, einer Terphenylgruppe, einer Naphthylgruppe, einer Fluorenylgruppe, einer Carbazolylgruppe, einer Dibenzofuranylgruppe, einer Dibenzothiophenylgruppe, einer Pyridinylgruppe, einer Pyrimidinylgruppe, einer Chinolinylgruppe, einer Isochinolinylgruppe, einer Chinoxalinylgruppe und einer Chinazolinylgruppe, und
eines, ausgewählt aus einem Kohlenstoffatom und einem Stickstoffatom, die die durch eine aus den Formeln 2-1 bis 2-17 ausgewählte Formel dargestellte Teilstruktur bilden, eine Bindungsstelle zu einem benachbarten Atom, ausgewählt aus den Formeln 1-1 bis 1-5, ist.

4. Organische lichtemittierende Vorrichtung nach einem der Ansprüche 1 bis 3, wobei:
die erste Verbindung, die zweite Verbindung und die dritte Verbindung jeweils unabhängig voneinander eine Verbindung auf Azinbasis sind, die durch eine aus den Formeln 1-11, 1-21 bis 1-25, 1-31 bis 1-33, 1-41, 1-42 und 1-51 ausgewählte Formel dargestellt wird, mit Ausnahme des Falles, in dem die erste Verbindung, die zweite Verbindung und die dritte Verbindung gleichzeitig eine Verbindung auf Azinbasis einschließen, die jeweils durch eine aus den Formeln 1-31 bis 1-33 ausgewählte Formel dargestellt wird: 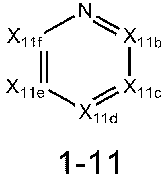 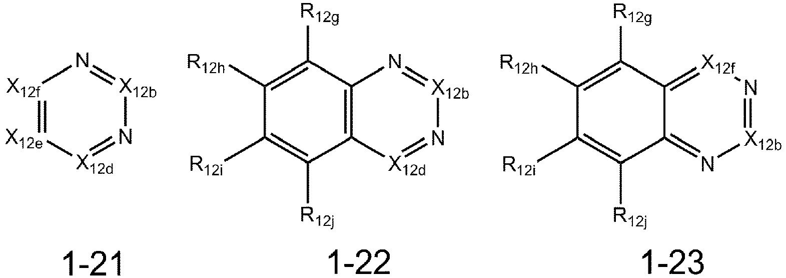 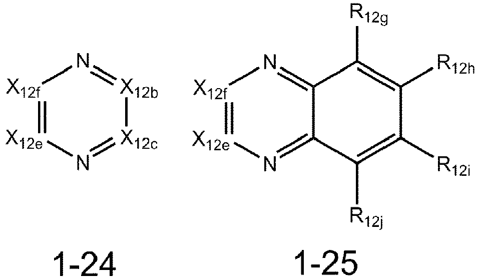 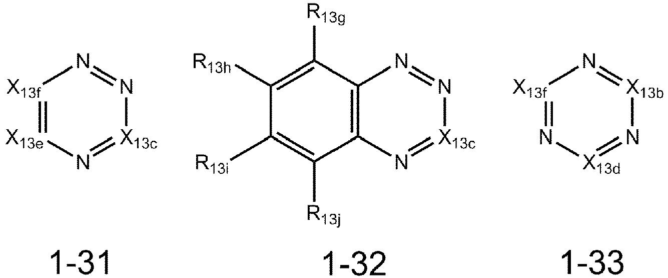 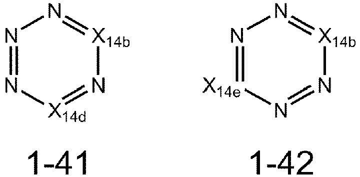 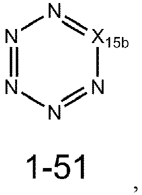
wobei in den Formeln 1-11, 1-21 bis 1-25, 1-31 bis 1-33, 1-41, 1-42 und 1-51,
X_{11b} bis X_{11f} sind jeweils unabhängig voneinander wie in Formel 1-1 definiert,
X_{12b} bis X_{12f} sind jeweils unabhängig voneinander wie in Formel 1-2 definiert,
X_{13b} bis X_{13f} sind jeweils unabhängig voneinander wie in Formel 1-3 definiert,
X_{14b}, X_{14d} und X₁₄ₑ sind jeweils unabhängig voneinander wie in Formel 1-4 definiert,
X_{15b} ist wie in Formel 1-5 definiert, und
R_{12g} bis R₁₂ⱼ und R_{13g} bis R₁₃ⱼ sind jeweils unabhängig voneinander ausgewählt aus:
Wasserstoff, Deuterium, -F, -Cl, -Br, -I, einer Methylgruppe, einer Ethylgruppe, einer n-Propylgruppe, einer Isopropylgruppe, einer n-Butylgruppe, einer Isobutylgruppe, einer sec-Butylgruppe, einer tert-Butylgruppe, einer Methoxygruppe, einer Ethoxygruppe, einer n-Propoxygruppe, einer Isopropoxygruppe, einer n-Butoxygruppe, einer Isobutoxygruppe, einer sec-Butoxygruppe, einer tert-Butoxygruppe, einer Phenylgruppe, einer Biphenylgruppe, einer Terphenylgruppe, einer Naphthylgruppe, einer Fluorenylgruppe, einer Carbazolylgruppe, einer Dibenzofuranylgruppe, einer Dibenzothiophenylgruppe, einer Pyridinylgruppe, einer Pyrimidinylgruppe, einer Chinolinylgruppe, einer Isochinolinylgruppe, einer Chinoxalinylgruppe, einer Chinazolinylgruppe, einer Benzofuropyridinylgruppe, einer Benzothienopyridinylgruppe, einer Benzofuropyrimidinylgruppe und einer Benzothienopyrimidinylgruppe, und
einer Methylgruppe, einer Ethylgruppe, einer n-Propylgruppe, einer Isopropylgruppe, einer n-Butylgruppe, einer Isobutylgruppe, einer sec-Butylgruppe, einer tert-Butylgruppe, einer Phenylgruppe, einer Biphenylgruppe, einer Terphenylgruppe, einer Naphthylgruppe, einer Fluorenylgruppe, einer Carbazolylgruppe, einer Dibenzofuranylgruppe, einer Dibenzothiophenylgruppe, einer Pyridinylgruppe, einer Pyrimidinylgruppe, einer Chinolinylgruppe, einer Isochinolinylgruppe, einer Chinoxalinylgruppe, einer Chinazolinylgruppe, einer Benzofuropyridinylgruppe, einer Benzothienopyridinylgruppe, einer Benzofuropyrimidinylgruppe und einer Benzothienopyrimidinylgruppe, jeweils substituiert mit mindestens einem aus Deuterium, -F, -Cl, -Br und -I.

5. Organische lichtemittierende Vorrichtung nach einem der Ansprüche 1 bis 4, wobei:
die erste Verbindung eine Teilstruktur aufweist, die durch eine aus den Formeln 2-1 bis 2-17 ausgewählte Formel dargestellt wird: 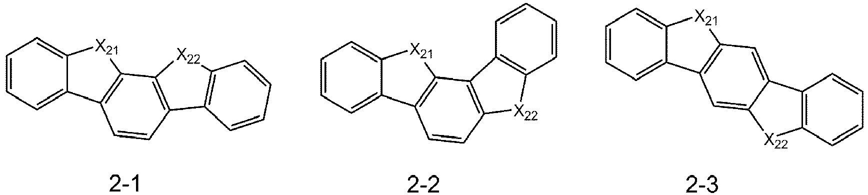 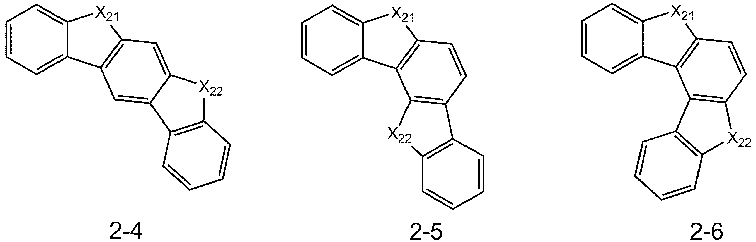 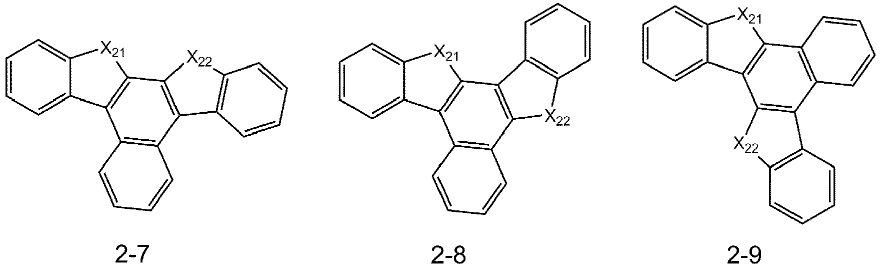 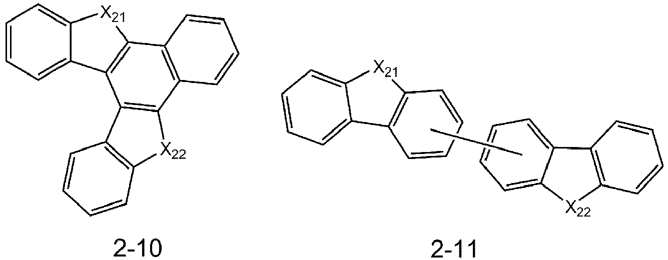 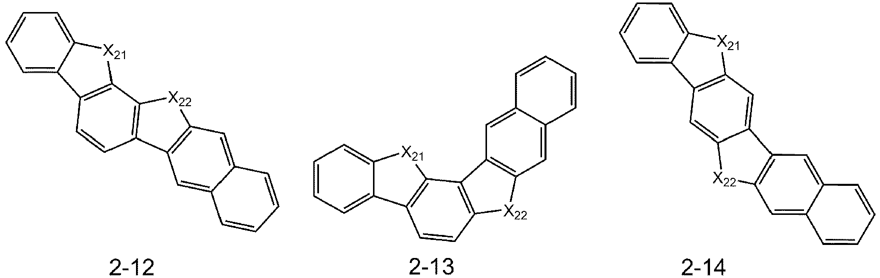 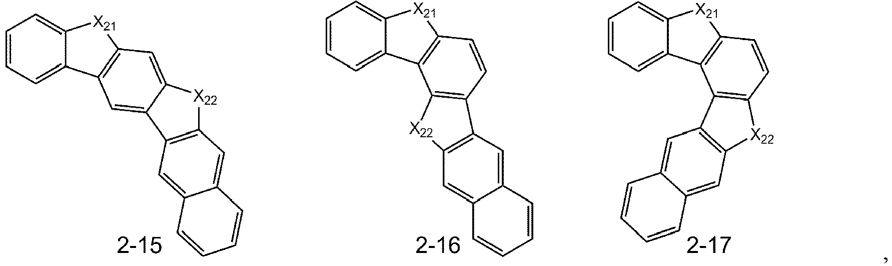
wobei in den Formeln 2-1 bis 2-17,
X₂₁ ausgewählt ist aus C(R₂₁)(R₂₂), N(R₂₁), O und S,
X₂₂ ausgewählt ist aus C(R₂₃)(R₂₄), N(R₂₃), O und S,
R₂₁ bis R₂₄ sind jeweils unabhängig voneinander ausgewählt aus:
einer Methylgruppe, einer Ethylgruppe, einer n-Propylgruppe, einer Isopropylgruppe, einer n-Butylgruppe, einer Isobutylgruppe, einer sec-Butylgruppe und einer tert-Butylgruppe;
einer Methylgruppe, einer Ethylgruppe, einer n-Propylgruppe, einer Isopropylgruppe, einer n-Butylgruppe, einer Isobutylgruppe, einer sec-Butylgruppe und einer tert-Butylgruppe, jeweils substituiert mit mindestens einer Gruppe, ausgewählt aus Deuterium, -F, -Cl, -Br und -I;
einer Phenylgruppe, einer Naphthylgruppe, einer Fluorenylgruppe, einer Carbazolylgruppe, einer Dibenzofuranylgruppe, einer Dibenzothiophenylgruppe, einer Pyridinylgruppe, einer Pyrimidinylgruppe, einer Chinolinylgruppe, einer Isochinolinylgruppe, einer Chinoxalinylgruppe, einer Chinazolinylgruppe, einer Benzofuropyridinylgruppe, einer Benzothienopyridinylgruppe, einer Benzofuropyrimidinylgruppe und einer Benzothienopyrimidinylgruppe, und
einer Phenylgruppe, einer Biphenylgruppe, einer Terphenylgruppe, einer Naphthylgruppe, einer Fluorenylgruppe, einer Carbazolylgruppe, einer Dibenzofuranylgruppe, einer Dibenzothiophenylgruppe, einer Pyridinylgruppe, einer Pyrimidinylgruppe, einer Chinolinylgruppe, einer Isochinolinylgruppe, einer Chinoxalinylgruppe, einer Chinazolinylgruppe, einer Benzofuropyridinylgruppe, einer Benzothienopyridinylgruppe, einer Benzofuropyrimidinylgruppe und einer Benzothienopyrimidinylgruppe, jeweils substituiert mit mindestens einer Gruppe, ausgewählt aus Deuterium, -F, -Cl, -Br, -I, einer Methylgruppe, einer Ethylgruppe, einer n-Propylgruppe, einer Isopropylgruppe, einer n-Butylgruppe, einer Isobutylgruppe, einer sec-Butylgruppe, einer tert-Butylgruppe, einer Methoxygruppe, einer Ethoxygruppe, einer n-Propoxygruppe, einer Isopropoxygruppe, einer n-Butoxygruppe, einer Isobutoxygruppe, einer sec-Butoxygruppe, einer tert-Butoxygruppe, einer Phenylgruppe, einer Biphenylgruppe, einer Terphenylgruppe, einer Naphthylgruppe, einer Fluorenylgruppe, einer Carbazolylgruppe, einer Dibenzofuranylgruppe, einer Dibenzothiophenylgruppe, einer Pyridinylgruppe, einer Pyrimidinylgruppe, einer Chinolinylgruppe, einer Isochinolinylgruppe, einer Chinoxalinylgruppe, einer Chinazolinylgruppe, einer Benzofuropyridinylgruppe, einer Benzothienopyridinylgruppe, einer Benzofuropyrimidinylgruppe und einer Benzothienopyrimidinylgruppe, und
eines, ausgewählt aus einem Kohlenstoffatom und einem Stickstoffatom, die die durch eine aus den Formeln 2-1 bis 2-17 ausgewählte Formel dargestellte Teilstruktur bilden, eine Bindungsstelle zu einem benachbarten Atom, ausgewählt aus den Formeln 1-1 bis 1-5, ist.

6. Organische lichtemittierende Vorrichtung nach einem der Ansprüche 1 bis 5, wobei:
die erste Verbindung ausgewählt ist aus den Verbindungen H-1 bis H-1 1,
die zweite Verbindung ausgewählt ist aus den Verbindungen HB-1 bis HB-3 und
die dritte Verbindung ausgewählt ist aus den Verbindungen ET-1 und ET-2:

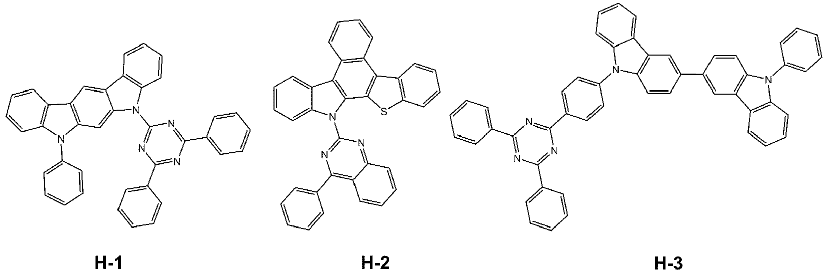

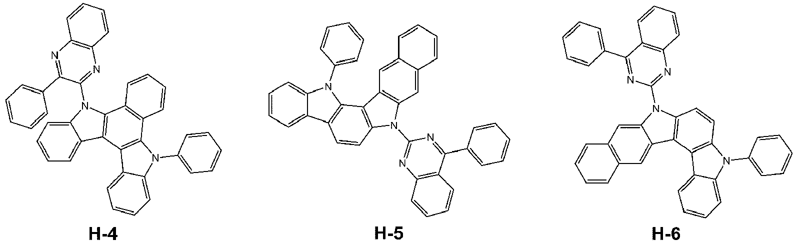

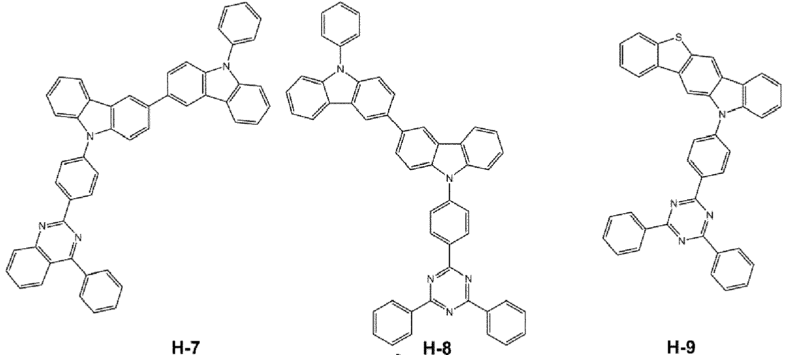

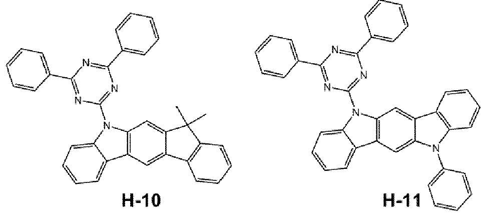

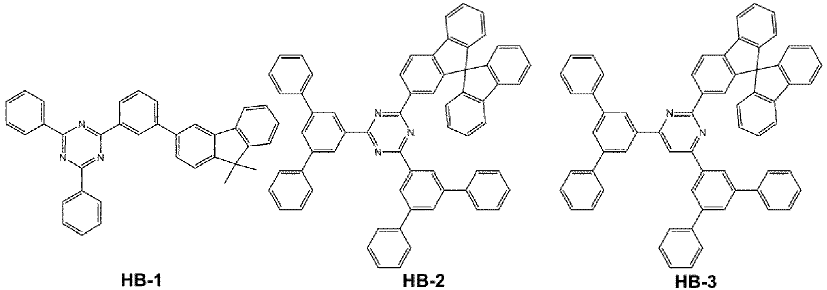

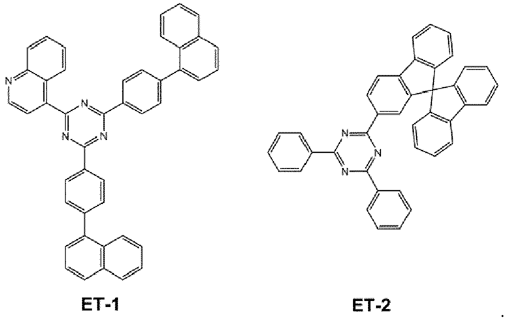

7. Organische lichtemittierende Vorrichtung nach einem der Ansprüche 1 bis 6, wobei:
die erste Verbindung, die zweite Verbindung und die dritte Verbindung jeweils unabhängig voneinander eine Verbindung auf Azinbasis sind, die durch eine aus den Formeln 1-1 bis 1-3 ausgewählte Verbindung dargestellt wird.

8. Organische lichtemittierende Vorrichtung nach einem der Ansprüche 1 bis 7, wobei:
i) die erste Verbindung eine Verbindung auf Azinbasis ist, die durch eine aus den Formeln 1-1 und 1-2 ausgewählte Formel dargestellt wird, und die zweite Verbindung und die dritte Verbindung jeweils unabhängig voneinander eine Verbindung auf Azinbasis sind, die durch eine aus den Formeln 1-2 und 1-3 ausgewählte Formel dargestellt wird;
ii) die zweite Verbindung eine Verbindung auf Azinbasis ist, die durch eine aus den Formeln 1-1 und 1-2 ausgewählte Formel dargestellt wird, und die erste Verbindung und die dritte Verbindung jeweils unabhängig voneinander eine Verbindung auf Azinbasis sind, die durch eine aus den Formeln 1-2 und 1-3 ausgewählte Formel dargestellt wird; oder
iii) die dritte Verbindung eine Verbindung auf Azinbasis ist, die durch eine aus den Formeln 1-1 und 1-2 ausgewählte Formel dargestellt wird, und die erste Verbindung und die zweite Verbindung jeweils unabhängig voneinander eine Verbindung auf Azinbasis sind, die durch eine aus den Formeln 1-2 und 1-3 ausgewählte Formel dargestellt wird.

9. Organische lichtemittierende Vorrichtung nach einem der Ansprüche 1 bis 8, wobei:
die organische Schicht einen Elektronentransportbereich zwischen der Emissionsschicht und der zweiten Elektrode umfasst,
der Elektronentransportbereich eine Pufferschicht und eine Elektronentransportschicht umfasst,
die Pufferschicht zwischen der Emissionsschicht und der Elektronentransportschicht liegt,
die Emissionsschicht die erste Verbindung umfasst,
die Pufferschicht die zweite Verbindung umfasst, und
die Elektronentransportschicht die dritte Verbindung umfasst;
vorzugsweise wobei:
die Emissionsschicht in direktem Kontakt mit der Pufferschicht steht, und
die Pufferschicht in direktem Kontakt mit der Elektronentransportschicht steht.

10. Organische lichtemittierende Vorrichtung nach einem der Ansprüche 1 bis 9, wobei:
die erste Elektrode eine Anode ist und die zweite Elektrode eine Kathode ist;
und/oder wobei:
die Emissionsschicht eine metallorganische Verbindung umfasst.

11. Organische lichtemittierende Vorrichtung nach einem der Ansprüche 1 bis 10, wobei:
die organische lichtemittierende Vorrichtung ferner ein Substrat umfasst, das in einen ersten Subpixelbereich, einen zweiten Subpixelbereich und einen dritten Subpixelbereich unterteilt ist;
die erste Elektrode eine Vielzahl von ersten Elektroden umfasst, die jeweils in dem ersten Subpixelbereich, dem zweiten Subpixelbereich und dem dritten Subpixelbereich des Substrats angeordnet sind;
die zweite Elektrode der Vielzahl der ersten Elektroden gegenüberliegt; und
die organische Schicht sich zwischen der Vielzahl von ersten Elektroden und der zweiten Elektrode befindet.

12. Organische lichtemittierende Vorrichtung nach Anspruch 11, wobei:
die Emissionsschicht eine erste Emissionsschicht, eine zweite Emissionsschicht und eine dritte Emissionsschicht umfasst, die jeweils in dem ersten Subpixelbereich, dem zweiten Subpixelbereich und dem dritten Subpixelbereich des Substrats angeordnet sind, und
mindestens eine der ersten Emissionsschicht und der zweiten Emissionsschicht die erste Verbindung und eine metallorganische Verbindung umfasst.

## Revendications

1. Dispositif électroluminescent organique comprenant :
une première électrode ;
une seconde électrode ; et
une couche organique entre la première électrode et la seconde électrode et qui comprend une couche d'émission,
dans lequel la couche organique comprend un premier composé, un deuxième composé, et un troisième composé, et
le premier composé, le deuxième composé, et le troisième composé sont chacun indépendamment un composé à base d'azine représenté par l'une choisie parmi les Formules 1-1 à 1-5, sauf dans un cas où le premier composé, le deuxième composé, et le troisième composé incluent simultanément un composé à base d'azine représenté par la Formule 1-3 : 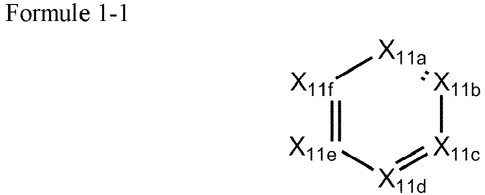 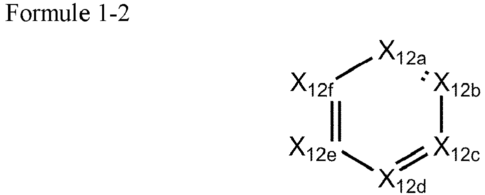 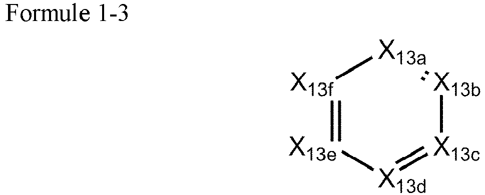 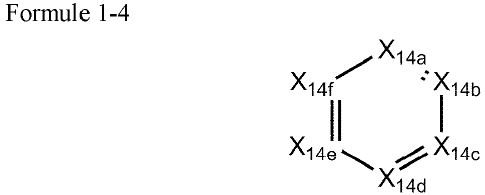 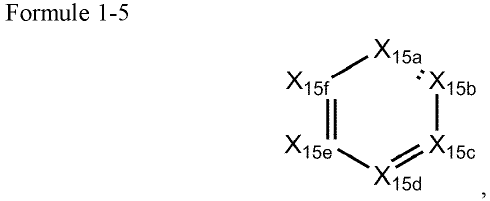
dans lequel, dans les Formules 1-1 à 1-5,
X₁₁ₐ est choisi parmi N et C[(L₁₁ₐ)ₐ₁₁ₐ-R₁₁ₐ] ; X_{11b} est choisi parmi N et C[(L_{11b})_{a11b}-R_{11b}] ; X_{11c} est choisi parmi N et C[(L_{11c})_{a11c}-R_{11c}] ; X_{11d} est choisi parmi N et C[(L₁₁ₐ)ₐ₁₁ₐ-R_{11d}] ; X₁₁ₑ est choisi parmi N et C[(L₁₁ₑ)ₐ₁₁ₑ-R₁₁ₑ] ; X_{11f} est choisi parmi N et C[(L_{11f})_{a11f}-R_{11f}] ; et l'un de X₁₁ₐ à X_{11f} est N,
X₁₂ₐ est choisi parmi N et C[(L₁₂ₐ)ₐ₁₂ₐ-R₁₂ₐ] ; X_{12b} est choisi parmi N et C[(L_{12b})_{a12b}-R_{12b}] ; X_{12c} est choisi parmi N et C[(L_{12c})_{a12c}-R_{12c}] ; X_{12d} est choisi parmi N et C[(L_{12d})_{a12d}-R_{12d}] ; X₁₂ₑ est choisi parmi N et C[(L₁₂ₑ)ₐ₁₂ₑ-R₁₂ₑ] ; X_{12f} est choisi parmi N et C[(L_{12f})_{a12f}-R_{12f}] ; et deux de X₁₂ₐ à X_{12f} sont N,
X₁₃ₐ est choisi parmi N et C[(L₁₃ₐ)ₐ₁₃ₐ-R₁₃ₐ] ; X_{13b} est choisi parmi N et C[(L_{13b})_{a13b}-R_{13b}] ; X_{13c} est choisi parmi N et C[(L_{13c})_{a13c}-R_{13c}] ; X_{13d} est choisi parmi N et C[(L_{13d})_{a13d}-R_{13d}] ; X₁₃ₑ est choisi parmi N et C[(L₁₃ₑ)ₐ₁₃ₑ-R₁₃ₑ] ; X_{13f} est choisi parmi N et C[(L_{13f})_{a13f}-R_{13f}] ; et trois de X₁₃ₐ à X_{13f} sont N,
X₁₄ₐ est choisi parmi N et C[(L₁₄ₐ)ₐ₁₄ₐ-R₁₄ₐ] ; X_{14b} est choisi parmi N et C[(L_{14b})_{a14b}-R_{14b}] ; X_{14c} est choisi parmi N et C[(L_{14c})_{a14c}-R_{14c}] ; X_{14d} est choisi parmi N et C[(L_{14d})_{a14d}-R_{14d}] ; X₁₄ₑ est choisi parmi N et C[(L₁₄ₑ)ₐ₁₄ₑ-R₁₄ₑ] ; X_{14f} est choisi parmi N et C[(L_{14f})_{a14f}-R_{14f}] ; et quatre de X₁₄ₐ à X_{14f} sont N,
X₁₅ₐ est choisi parmi N et C[(L₁₅ₐ)ₐ₁₅ₐ-R₁₅ₐ] ; X_{15b} est choisi parmi N et C[(L_{15b})_{a15b}-R_{15b}] ; X_{15c} est choisi parmi N et C[(L_{15c})a_{15c}-R_{15c}] ; X_{15d} est choisi parmi N et C[(L_{15d})_{a15d}-R_{15d}] ; X₁₅ₑ est choisi parmi N et C[(L₁₅ₑ)ₐ₁₅ₑ-R₁₅ₑ] ; X_{15f} est choisi parmi N et C[(L_{15f})_{a15f}-R_{15f}] ; et cinq de X₁₅ₐ à X_{15f} sont N,
L₁₁ₐ à L_{11f}, L₁₂ₐ à L_{12f}, L₁₃ₐ à L_{13f}, L₁₄ₐ à L_{14f}, et L₁₅ₐ à L_{15f} sont chacun choisis indépendamment parmi :
un groupe cycloalcénylène en C₃-C₁₀, un groupe hétérocycloalcénylène en C₁-C₁₀, un groupe arylène en C₆-C₆₀, un groupe hétéroarylène en C₁-C₆₀, un groupe polycyclique condensé non aromatique divalent, et un groupe hétéropolycyclique condensé non aromatique divalent ; et
un groupe cycloalcénylène en C₃-C₁₀, un groupe hétérocycloalcénylène en C₁-C₁₀, un groupe arylène en C₆-C₆₀, un groupe hétéroarylène en C₁-C₆₀, un groupe polycyclique condensé non aromatique divalent, et un groupe hétéropolycyclique condensé non aromatique divalent, chacun substitué par au moins l'un choisi parmi du deutérium, -F,-Cl, -Br, -I, un groupe hydroxyle, un groupe cyano, un groupe nitro, un groupe amino, un groupe amidino, un groupe hydrazine, un groupe hydrazone, un groupe acide carboxylique ou un sel de celui-ci, un groupe acide sulfonique ou un sel de celui-ci, un groupe acide phosphorique ou un sel de celui-ci, un groupe alkyle en C₁-C₂₀, un groupe alcoxy en C₁-C₂₀, un groupe cycloalkyle en C₃-C₁₀, un groupe hétérocycloalkyle en C₁-C₁₀, un groupe cycloalcényle en C₃-C₁₀, un groupe hétérocycloalcényle en C₁-C₁₀, un groupe aryle en C₆-C₃₀, un groupe aryloxy en C₆-C₃₀, un groupe arylthio en C₆-C₃₀, un groupe hétéroaryloxy en C₁-C₃₀, un groupe hétéroarylthio en C₁-C₃₀, un groupe polycyclique condensé non aromatique monovalent, un groupe hétéropolycyclique condensé non aromatique monovalent, un groupe pyrrolyle, un groupe thiophényle, un groupe furanyle, un groupe imidazolyle, un groupe pyrazolyle, un groupe thiazolyle, un groupe thiazolyle, un groupe isothiazolyle, un groupe oxazolyle, un groupe isoxazolyle, un groupe pyridinyle, un groupe pyrazinyle, un groupe pyrimidinyle, un groupe pyridazinyle, un groupe isoindolyle, un groupe indolyle, un groupe indazolyle, un groupe purinyle, un groupe quinolinyle, un groupe isoquinolinyle, un groupe benzoquinolinyle, un groupe phtalazinyle, un groupe naphtyridinyle, un groupe quinoxalinyle, un groupe quinazolinyle, un groupe cinnolinyle, un groupe carbazolyle, un groupe phénanthridinyle, un groupe acridinyle, un groupe phénanthrolinyle, un groupe phénazinyle, un groupe benzimidazolyle, un groupe benzofuranyle, un groupe benzothiophényle, un groupe isobenzothiazolyle, un groupe benzoxazolyle, un groupe isobenzoxazolyle, un groupe triazolyle, un groupe tétrazolyle, un groupe oxadiazolyle, un groupe dibenzofuranyle, un groupe dibenzothiophényle, un groupe benzocarbazolyle, un groupe dibenzocarbazolyle, un groupe thiadiazolyle, et un groupe imidazopyridinyle, dans lequel L₁₁ₐ à L_{11f}, L₁₂ₐ à L_{12f}, L₁₃ₐ à L_{13f}, L₁₄ₐ à L_{14f}, et L₁₅ₐ à L_{15f} ne sont pas chacun choisis indépendamment parmi : un groupe triazinylène ; et un groupe triazinylène substitué par au moins l'un choisi parmi du deutérium, -F, -Cl, -Br, -I, un groupe hydroxyle, un groupe cyano, un groupe nitro, un groupe amino, un groupe amidino, un groupe hydrazine, un groupe hydrazone, un groupe acide carboxylique ou un sel de celui-ci, un groupe acide sulfonique ou un sel de celui-ci, un groupe acide phosphorique ou un sel de celui-ci, un groupe alkyle en C₁-C₂₀, un groupe alcoxy en C₁-C₂₀, un groupe cycloalkyle en C₃-C₁₀, un groupe hétérocycloalkyle en C₁-C₁₀, un groupe cycloalcényle en C₃-C₁₀, un groupe hétérocycloalcényle en C₁-C₁₀, un groupe aryle en C₆-C₃₀, un groupe aryloxy en C₆-C₃₀, un groupe arylthio en C₆-C₃₀, un groupe hétéroaryloxy en C₁-C₃₀, un groupe hétéroarylthio en C₁-C₃₀, un groupe polycyclique condensé non aromatique monovalent, un groupe hétéropolycyclique condensé non aromatique monovalent, un groupe pyrrolyle, un groupe thiophényle, un groupe furanyle, un groupe imidazolyle, un groupe pyrazolyle, un groupe thiazolyle, un groupe isothiazolyle, un groupe oxazolyle, un groupe isoxazolyle, un groupe pyridinyle, un groupe pyrazinyle, un groupe pyrimidinyle, un groupe pyridazinyle, un groupe isoindolyle, un groupe indolyle, un groupe indazolyle, un groupe purinyle, un groupe quinolinyle, un groupe isoquinolinyle, un groupe benzoquinolinyle, un groupe phtalazinyle, un groupe naphthyridinyle, un groupe quinoxalinyle, un groupe quinazolinyle, un groupe cinnolinyle, un groupe carbazolyle, un groupe phénanthridinyle, un groupe acridinyle, un groupe phénanthrolinyle, un groupe phénazinyle, un groupe benzimidazolyle, un groupe benzofuranyle, un groupe benzothiophényle, un groupe isobenzothiazolyle, un groupe benzoxazolyle, un groupe isobenzoxazolyle, un groupe triazolyle, un groupe tétrazolyle, un groupe oxadiazolyle, un groupe dibenzofuranyle, un groupe dibenzothiophényle, un groupe benzocarbazolyle, un groupe dibenzocarbazolyle, un groupe thiadiazolyle, et un groupe imidazopyridinyle,
a11a à a11f, a12a à a12f, a13a à a13f, a14a à a14f, et a15a à a15f sont chacun choisis indépendamment parmi 0, 1, 2, 3 et 4,
R₁₁ₐ à R_{11f}, R₁₂ₐ à R_{12f}, R₁₃ₐ à R_{13f}, R₁₄ₐ à R_{14f}, et R₁₅ₐ à R_{15f} sont chacun choisis indépendamment parmi :
hydrogène, deutérium, -F, -Cl, -Br, -I, un groupe méthyle, un groupe éthyle, un groupe n-propyle, un groupe isopropyle, un groupe n-butyle, un groupe isobutyle, un groupe sec-butyle, un groupe tert-butyle, un groupe méthoxy, un groupe éthoxy, un groupe n-propoxy, un groupe isopropoxy, un groupe n-butoxy, un groupe isobutoxy, un groupe sec-butoxy, et un groupe tert-butoxy ;
un groupe méthyle, un groupe éthyle, un groupe n-propyle, un groupe isopropyle, un groupe n-butyle, un groupe isobutyle, un groupe sec-butyle, et un groupe tert-butyle, chacun substitué par au moins l'un choisi parmi du deutérium, -F, -Cl, -Br, et -I ;
un groupe phényle, un groupe biphényle, un groupe terphényle, un groupe naphtyle, un groupe fluorényle, un groupe carbazolyle, un groupe dibenzofuranyle, un groupe dibenzothiophényle, un groupe pyridinyle, un groupe pyrimidinyle, un groupe quinolinyle, un groupe isoquinolinyle, un groupe quinoxalinyle, un groupe quinazolinyle, un groupe benzofuropyridinyle, un groupe benzothiénopyridinyle, un groupe benzofuropyrimidinyle, un groupe benzothiénopyrimidinyle, un groupe polycyclique condensé non aromatique monovalent, et un groupe hétéropolycyclique condensé non aromatique monovalent ;
un groupe phényle, un groupe biphényle, un groupe terphényle, un groupe naphtyle, un groupe fluorényle, un groupe carbazolyle, un groupe dibenzofuranyle, un groupe dibenzothiophényle, un groupe pyridinyle, un groupe pyrimidinyle, un groupe quinolinyle, un groupe isoquinolinyle, un groupe quinoxalinyle, un groupe quinazolinyle, un groupe benzofuropyridinyle, un groupe benzothiénopyridinyle, un groupe benzofuropyrimidinyle, un groupe benzothiénopyrimidinyle, un groupe polycyclique condensé non aromatique monovalent et un groupe hétéropolycyclique condensé non aromatique monovalent, chacun substitué par au moins l'un choisi parmi du deutérium, -F, -Cl, -Br, -I, un groupe méthyle, un groupe éthyle, un groupe n-propyle, un groupe isopropyle, un groupe n-butyle, un groupe isobutyle, un groupe sec-butyle, un groupe tert-butyle, un groupe méthoxy, un groupe éthoxy, un groupe n-propoxy, un groupe isopropoxy, un groupe n-butoxy, un groupe isobutoxy, un groupe sec-butoxy, un groupe tert-butoxy, un groupe phényle, un groupe biphényle, un groupe terphényle, un groupe naphtyle, un groupe fluorényle, un groupe carbazolyle, un groupe dibenzofuranyle, un groupe dibenzothiophényle, un groupe pyridinyle, un groupe pyrimidinyle, un groupe quinolinyle, un groupe isoquinolinyle, un groupe quinoxalinyle, un groupe quinazolinyle, un groupe benzofuropyridinyle, un groupe benzothiénopyridinyle, un groupe benzofuropyrimidinyle, et un groupe benzothiénopyrimidinyle ; et
un groupe phényle, un groupe biphényle, un groupe terphényle, un groupe naphtyle, un groupe fluorényle, un groupe carbazolyle, un groupe dibenzofuranyle, un groupe dibenzothiophényle, un groupe pyridinyle, un groupe pyrimidinyle, un groupe quinolinyle, un groupe isoquinolinyle, un groupe quinoxalinyle, un groupe quinazolinyle, un groupe benzofuropyridinyle, un groupe benzothiénopyridinyle, un groupe benzofuropyrimidinyle, un groupe benzothiénopyrimidinyle, un groupe polycyclique condensé non aromatique monovalent, et un groupe hétéropolycyclique condensé non aromatique monovalent, chacun substitué par au moins l'un choisi parmi un groupe méthyle, un groupe éthyle, un groupe n-propyle, un groupe isopropyle, un groupe n-butyle, un groupe isobutyle, un groupe sec-butyle, un groupe tert-butyle, un groupe phényle, un groupe biphényle, un groupe terphényle, un groupe naphtyle, un groupe fluorényle, un groupe carbazolyle, un groupe dibenzofuranyle, un groupe dibenzothiophényle, un groupe pyridinyle, un groupe pyrimidinyle, un groupe quinolinyle, un groupe isoquinolinyle, un groupe quinoxalinyle, un groupe quinazolinyle, un groupe benzofuropyridinyle, un groupe benzothiénopyridinyle, un groupe benzofuropyrimidinyle et un groupe benzothiénopyrimidinyle qui sont chacun substitués par au moins l'un choisi parmi du deutérium, -F, -Cl, -Br, et -I, et
des atomes voisins de R₁₁ₐ à R_{11f}, des atomes voisins de R₁₂ₐ à R_{12f}, des atomes voisins de R₁₃ₐ à R_{13f}, des atomes voisins de R₁₄ₐ à R_{14f}, et/ou des atomes voisins de R₁₅ₐ à R_{15f} forment facultativement un anneau saturé ou un anneau insaturé.

2. Dispositif électroluminescent organique selon la revendication 1, dans lequel :
L₁₁ₐ à L_{11f}, L₁₂ₐ à L_{12f}, L₁₃ₐ à L_{13f}, L₁₄ₐ à L_{14f}, et L₁₅ₐ à L_{15f} sont chacun choisis indépendamment parmi :
un groupe phénylène, un groupe pentalénylène, un groupe indénylène, un groupe naphtylène, un groupe azulénylène, un groupe heptalenylène, un groupe indacénylène, un groupe acénaphtylène, un groupe fluorénylène, un groupe spiro-fluorénylène, un groupe benzofluorénylène, un groupe dibenzofluorénylène, un groupe phénalénylène, un groupe phénanthrénylène, un groupe anthracénylène, un groupe fluoranthénylène, un groupe triphénylénylène, un groupe pyrénylène, un groupe chrysénylène, un groupe naphtacénylène, un groupe picenylène, un groupe pérylénylène, un groupe pentaphénylène, un groupe hexacénylène, un groupe pentacénylène, un groupe rubicenylène, un groupe coronenylène, un groupe ovalénylène, un groupe pyrrolylène, un groupe thiophénylène, un groupe furanylène, un groupe imidazolylène, un groupe pyrazolylène, un groupe thiazolylène, un groupe isothiazolylène, un groupe oxazolylène, un groupe isoxazolylène, un groupe pyridinylène, un groupe pyrazinylène, un groupe pyrimidinylène, un groupe pyridazinylène, un groupe isoindolylène, un groupe indolylène,un groupe indazolylène, un groupe purinylène, un groupe quinolinylène, un groupe isoquinolinylène, un groupe benzoquinolinylène, un groupe phtalazinylène, un groupe naphtyridinylène, un groupe quinoxalinylène, un groupe quinazolinylène, un groupe cinnolinylène, un groupe carbazolylène, un groupe phénanthridinylène, un groupe acridinylène, un groupe phénanthrolinylène, un groupe phénazinylène, un groupe benzimidazolylène, un groupe benzofuranylène, un groupe benzothiophénylène, un groupe isobenzothiazolylène, un groupe benzoxazolylène, un groupe isobenzoxazolylène, un groupe triazolylène, un groupe tétrazolylène, un groupe oxadiazolylène, un groupe dibenzofuranylène, un groupe dibenzothiophénylène, un groupe benzofuropyridinylène, un groupe benzothiénopyridinylène, un groupe benzofuropyrimidinylène, un groupe benzothiénopyrimidinylène, un groupe benzocarbazolylène et un groupe dibenzocarbazolylène ; et
un groupe phénylène, un groupe pentalénylène, un groupe indénylène, un groupe naphtylène, un groupe azulénylène, un groupe heptalenylène, un groupe indacénylène, un groupe acénaphtylène, un groupe fluorénylène, un groupe spiro-fluorénylène, un groupe benzofluorénylène, un groupe dibenzofluorénylène, un groupe phénalénylène, un groupe phénanthrénylène, un groupe anthracénylène, un groupe fluoranthénylène, un groupe triphénylénylène, un groupe pyrénylène, un groupe chrysénylène, un groupe naphtacénylène, un groupe picenylène, un groupe pérylénylène, un groupe pentaphénylène, un groupe hexacénylène, un groupe pentacénylène, un groupe rubicenylène, un groupe coronenylène, un groupe ovalénylène, un groupe pyrrolylène, un groupe thiophénylène, un groupe furanylène, un groupe imidazolylène, un groupe pyrazolylène, un groupe thiazolylène, un groupe isothiazolylène, un groupe oxazolylène, un groupe isoxazolylène, un groupe pyridinylène, un groupe pyrazinylène, un groupe pyrimidinylène, un groupe pyridazinylène, un groupe isoindolylène, un groupe indolylène, un groupe indazolylène, un groupe purinylène, un groupe quinolinylène, un groupe isoquinolinylène, un groupe benzoquinolinylène, un groupe phtalazinylène, un groupe naphtyridinylène, un groupe quinoxalinylène, un groupe quinazolinylène, un groupe cinnolinylène, un groupe carbazolylène, un groupe phénanthridinylène, un groupe acridinylène, un groupe phénanthrolinylène, un groupe phénazinylène, un groupe benzimidazolylène, un groupe benzofuranylène, un groupe benzothiophénylène, un groupe isobenzothiazolylène, un groupe benzoxazolylène, un groupe isobenzoxazolylène, un groupe triazolylène, un groupe tétrazolylène, un groupe oxadiazolylène, un groupe dibenzofuranylène, un groupe dibenzothiophénylène, un groupe benzofuropyridinylène, un groupe benzothiénopyridinylène, un groupe benzofuropyrimidinylène, un groupe benzothiénopyrimidinylène, un groupe benzocarbazolylène et un groupe dibenzocarbazolylène, chacun substitué par au moins l'un choisi parmi du deutérium, - F, -Cl, -Br, -I, un groupe hydroxyle, un groupe cyano, un groupe nitro, un groupe amino, un groupe amidino, un groupe hydrazine, un groupe hydrazone, un groupe acide carboxylique ou un sel de celui-ci, un groupe acide sulfonique ou un sel de celui-ci, un groupe acide phosphorique ou un sel de celui-ci, un groupe alkyle en C₁-C₂₀, un groupe alcoxy en C₁-C₂₀, un groupe cyclopentyle, un groupe cyclohexyle, un groupe cycloheptyle, un groupe cyclopentényle, un groupe cyclohexényle, un groupe phényle, un groupe pentalényle, un groupe indényle, un groupe naphtyle, un groupe azulenyle, un groupe heptalenyle, un groupe indacenyle, un groupe acénaphtyle, un groupe fluorényle, un groupe spiro-fluorényle, un groupe benzofluorényle, un groupe dibenzofluorényle, un groupe phénalényle, un groupe phénanthrényle, un groupe anthracényle, un groupe fluoranthényle, un groupe triphénylènyle, un groupe pyrényle, un groupe chrysényle, un groupe naphtacényle, un groupe picényle, un groupe pérylényle, un groupe pentaphényle, un groupe hexacényle, un groupe pentacényle, un groupe rubicenyle, un groupe coronenyle, un groupe ovalenyle, un groupe pyrrolyle, un groupe thiophényle, un groupe furanyle, un groupe imidazolyle, un groupe pyrazolyle, un groupe thiazolyle, un groupe isothiazolyle, un groupe oxazolyle, un groupe isoxazolyle, un groupe pyridinyle, un groupe pyrazinyle, un groupe pyrimidinyle, un groupe pyridazinyle, un groupe isoindolyle, un groupe indolyle, un groupe indazolyle, un groupe purinyle, un groupe quinolinyle, un groupe isoquinolinyle, un groupe benzoquinolinyle, un groupe phtalazinyle, un groupe naphtyridinyle, un groupe quinoxalinyle, un groupe quinazolinyle, un groupe cinnolinyle, un groupe carbazolyle, un groupe phénanthridinyle, un groupe acridinyle, un groupe phénanthrolinyle, un groupe phénazinyle, un groupe benzimidazolyle, un groupe benzofuranyle, un groupe benzothiophényle, un groupe isobenzothiazolyle, un groupe benzoxazolyle, un groupe isobenzoxazolyle, un groupe triazolyle, un groupe tétrazolyle, un groupe oxadiazolyle, un groupe oxadiazolyle, un groupe dibenzofuranyle, un groupe dibenzothiophényle, un groupe benzocarbazolyle, un groupe dibenzocarbazolyle, un groupe thiadiazolyle, et un groupe imidazopyridinyle.

3. Dispositif électroluminescent organique selon la revendication 1 ou la revendication 2, dans lequel :
R₁₁ₐ à R_{11f}, R₁₂ₐ à R_{12f}, R₁₃ₐ à R_{13f}, R₁₄ₐ à R_{14f}, et R₁₅ₐ à R_{15f} sont chacun choisis indépendamment parmi :
hydrogène, deutérium, -F, -Cl, -Br, -I, un groupe méthyle, un groupe éthyle, un groupe n-propyle, un groupe isopropyle, un groupe n-butyle, un groupe isobutyle, un groupe sec-butyle, un groupe tert-butyle, un groupe méthoxy, un groupe éthoxy, un groupe n-propoxy, un groupe isopropoxy, un groupe n-butoxy, un groupe isobutoxy, un groupe sec-butoxy et un groupe tert-butoxy ;
un groupe méthyle, un groupe éthyle, un groupe n-propyle, un groupe isopropyle, un groupe n-butyle, un groupe isobutyle, un groupe sec-butyle, et un groupe tert-butyle, chacun substitué par au moins l'un choisi parmi du deutérium, -F, -Cl, -Br, et -I ;
un groupe phényle, un groupe biphényle, un groupe terphényle, un groupe naphtyle, un groupe fluorényle, un groupe carbazolyle, un groupe dibenzofuranyle, un groupe dibenzothiophényle, un groupe pyridinyle, un groupe pyrimidinyle, un groupe quinolinyle, un groupe isoquinolinyle, un groupe quinoxalinyle, un groupe quinazolinyle, un groupe benzofuropyridinyle, un groupe benzothiénopyridinyle, un groupe benzofuropyrimidinyle, et un groupe benzothiénopyrimidinyle ;
un groupe phényle, un groupe biphényle, un groupe terphényle, un groupe naphtyle, un groupe fluorényle, un groupe carbazolyle, un groupe dibenzofuranyle, un groupe dibenzothiophényle, un groupe pyridinyle, un groupe pyrimidinyle, un groupe quinolinyle, un groupe isoquinolinyle, un groupe quinoxalinyle, un groupe quinazolinyle, un groupe benzofuropyridinyle, un groupe benzothiénopyridinyle, un groupe benzofuropyrimidinyle, et un groupe benzothiénopyrimidinyle, chacun substitué par au moins l'un parmi du deutérium, -F, -Cl, -Br, -I, un groupe méthyle, un groupe éthyle, un groupe n-propyle, un groupe isopropyle, un groupe n-butyle, un groupe isobutyle, un groupe sec-butyle, un groupe tert-butyle, un groupe méthoxy, un groupe éthoxy, un groupe n-propoxy, un groupe isopropoxy, un groupe n-butoxy, un groupe isobutoxy, un groupe sec-butoxy, un groupe tert-butoxy, un groupe phényle, un groupe biphényle, un groupe terphényle, un groupe naphtyle, un groupe fluorényle, un groupe carbazolyle, un groupe dibenzofuranyle, un groupe dibenzothiophényle, un groupe pyridinyle, un groupe pyrimidinyle, un groupe quinoléinyle, un groupe isoquinolinyle, un groupe quinoxalinyle, un groupe quinazolinyle, un groupe benzofuropyridinyle, un groupe benzothiénopyridinyle, un groupe benzofuropyrimidinyle, et un groupe benzothiénopyrimidinyle ;
un groupe phényle, un groupe biphényle, un groupe terphényle, un groupe naphtyle, un groupe fluorényle, un groupe carbazolyle, un groupe dibenzofuranyle, un groupe dibenzothiophényle, un groupe pyridinyle, un groupe pyrimidinyle, un groupe quinolinyle, un groupe isoquinolinyle, un groupe quinoxalinyle, un groupe quinazolinyle, un groupe benzofuropyridinyle, un groupe benzothiénopyridinyle, un groupe benzofuropyrimidinyle, et un groupe benzothiénopyrimidinyle, chacun substitué par au moins l'un choisi parmi un groupe méthyle, un groupe éthyle, un groupe n-propyle, un groupe isopropyle, un groupe n-butyle, un groupe isobutyle, un groupe sec-butyle, un groupe tert-butyle, un groupe phényle, un groupe biphényle, un groupe terphényle, un groupe naphtyle, un groupe fluorényle, un groupe carbazolyle, un groupe dibenzofuranyle, un groupe dibenzothiophényle, un groupe pyridinyle, un groupe pyrimidinyle, un groupe quinolinyle, un groupe isoquinolinyle, un groupe quinoxalinyle, un groupe quinazolinyle, un groupe benzofuropyridinyle, un groupe benzothiénopyridinyle, un groupe benzofuropyrimidinyle, et un groupe benzothiénopyrimidinyle qui sont chacun substitués par au moins l'un choisi parmi du deutérium, -F, -Cl, -Br, et -I ; et
une structure partielle représentée par l'une choisie parmi les Formules 2-1 à 2-17 : 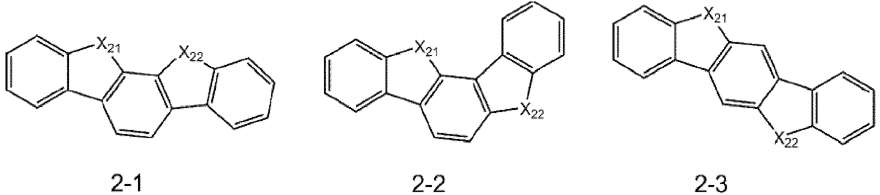 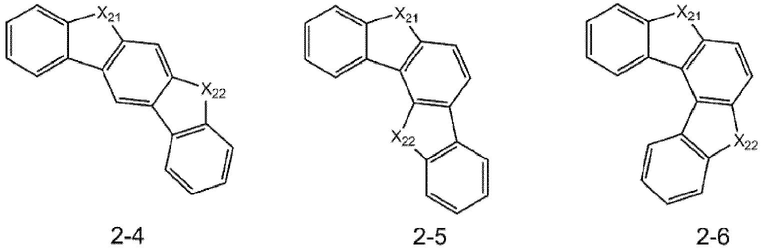 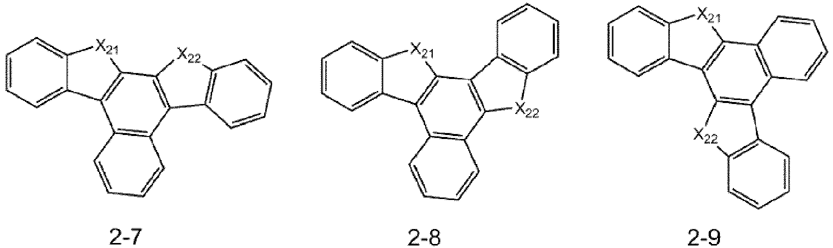 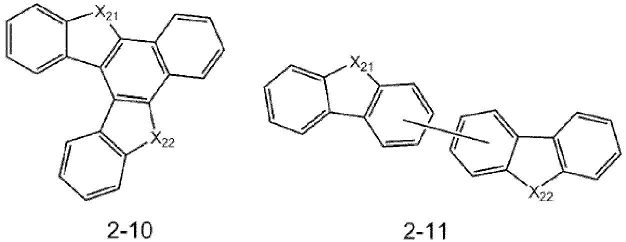 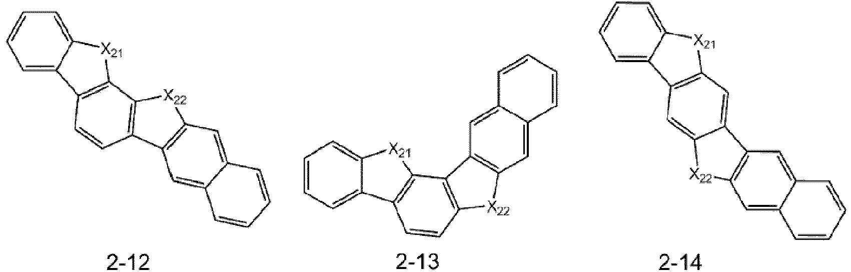 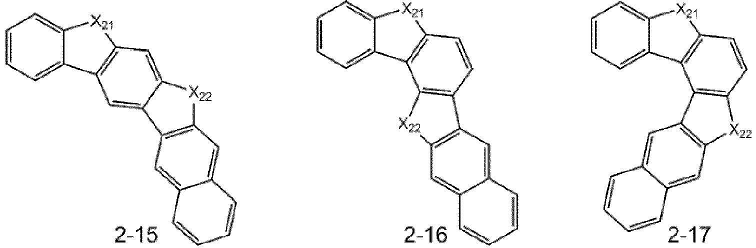
dans lequel, dans les Formules 2-1 à 2-17,
X₂₁ est choisi parmi C(R₂₁)(R₂₂), N(R₂₁), O, et S,
X₂₂ est choisi parmi C(R₂₃)(R₂₄), N(R₂₃), O, et S,
R₂₁ à R₂₄ sont chacun choisis indépendamment parmi :
un groupe méthyle, un groupe éthyle, un groupe n-propyle, un groupe isopropyle, un groupe n-butyle, un groupe isobutyle, un groupe sec-butyle, et un groupe tert-butyle ;
un groupe méthyle, un groupe éthyle, un groupe n-propyle, un groupe isopropyle, un groupe n-butyle, un groupe isobutyle, un groupe sec-butyle, et un groupe tert-butyle, chacun substitué par au moins l'un choisi parmi du deutérium, -F, -Cl, -Br, et -I ;
un groupe phényle, un groupe biphényle, un groupe terphényle, un groupe naphtyle, un groupe fluorényle, un groupe carbazolyle, un groupe dibenzofuranyle, un groupe dibenzothiophényle, un groupe pyridinyle, un groupe pyrimidinyle, un groupe quinolinyle, un groupe isoquinolinyle, un groupe quinoxalinyle, un groupe quinazolinyle, un groupe benzofuropyridinyle, un groupe benzothiénopyridinyle, un groupe benzofuropyrimidinyle, et un groupe benzothiénopyrimidinyle ; et
un groupe phényle, un groupe biphényle, un groupe terphényle, un groupe naphtyle, un groupe fluorényle, un groupe carbazolyle, un groupe dibenzofuranyle, un groupe dibenzothiophényle, un groupe pyridinyle, un groupe pyrimidinyle, un groupe quinolinyle, un groupe isoquinolinyle, un groupe quinoxalinyle, un groupe quinazolinyle, un groupe benzofuropyridinyle, un groupe benzothiénopyridinyle, un groupe benzofuropyrimidinyle, et un groupe benzothiénopyrimidinyle, chacun substitué par au moins l'un choisi parmi du deutérium, -F, -Cl, -Br, -I, un groupe méthyle, un groupe éthyle, un groupe n-propyle, un groupe isopropyle, un groupe n-butyle, un groupe isobutyle, un groupe sec-butyle, un groupe tert-butyle, un groupe méthoxy, un groupe éthoxy, un groupe n-propoxy, un groupe isopropoxy, un groupe n-butoxy, un groupe isobutoxy, un groupe sec-butoxy groupe, un groupe tert-butoxy, un groupe phényle, un groupe biphényle, un groupe terphényle, un groupe naphtyle, un groupe fluorényle, un groupe carbazolyle, un groupe dibenzofuranyle, un groupe dibenzothiophényle, un groupe pyridinyle, un groupe pyrimidinyle, un groupe quinolinyle, un groupe isoquinolinyle, un groupe quinoxalinyle, un groupe quinazolinyle, un groupe benzofuropyridinyle, un groupe benzothiénopyridinyle, un groupe benzofuropyrimidinyle, et un groupe benzothiénopyrimidinyle, et
l'un choisi parmi un atome de carbone et un atome d'azote qui constituent la structure partielle représentée par l'une choisie parmi les Formules 2-1 à 2-17 est un site de liaison à un atome voisin choisi parmi les Formules 1-1 à 1-5 ;
de préférence dans lequel :
R₁₁ₐ à R_{11f}, R₁₂ₐ à R_{12f}, R₁₃ₐ à R_{13f}, R₁₄ₐ à R_{14f}, et R₁₅ₐ à R_{15f} sont chacun choisis indépendamment parmi :
hydrogène, deutérium, -F, -Cl, -Br, -I, un groupe méthyle, un groupe éthyle, un groupe n-propyle, un groupe isopropyle, un groupe n-butyle, un groupe isobutyle, un groupe sec-butyle, et un groupe tert-butyle ;
un groupe phényle, un groupe biphényle, un groupe terphényle, un groupe naphtyle, un groupe fluorényle, un groupe carbazolyle, un groupe dibenzofuranyle, un groupe dibenzothiophényle, un groupe pyridinyle, un groupe pyrimidinyle, un groupe quinolinyle, un groupe isoquinolinyle, un groupe quinoxalinyle, et un groupe quinazolinyle ;
un groupe phényle, un groupe biphényle, un groupe terphényle, un groupe naphtyle, un groupe fluorényle, un groupe carbazolyle, un groupe dibenzofuranyle, un groupe dibenzothiophényle, un groupe pyridinyle, un groupe pyrimidinyle, un groupe quinolinyle, un groupe isoquinolinyle, un groupe quinoxalinyle, et un groupe quinazolinyle, chacun substitué par au moins l'un choisi parmi du deutérium, -F, -Cl, -Br, -I, un groupe méthyle, un groupe éthyle, un groupe n-propyle, un groupe isopropyle, un groupe n-butyle, un groupe isobutyle, un groupe sec-butyle, un groupe tert-butyle, un groupe phényle, un groupe biphényle, un groupe terphényle, un groupe naphtyle, un groupe fluorényle, un groupe carbazolyle, un groupe dibenzofuranyle, un groupe dibenzothiophényle, un groupe pyridinyle, un groupe pyrimidinyle, un groupe quinolinyle, un groupe isoquinolinyle, un groupe quinoxalinyle, et un groupe quinazolinyle ; et
une structure partielle représentée par une structure choisie parmi les Formules 2-1 à 2-17 : 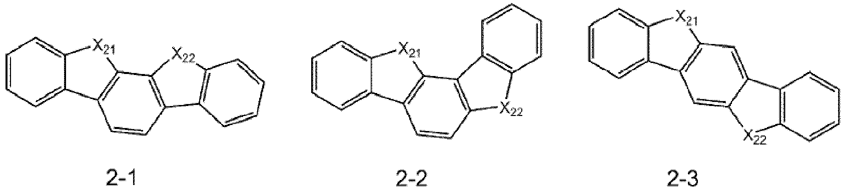 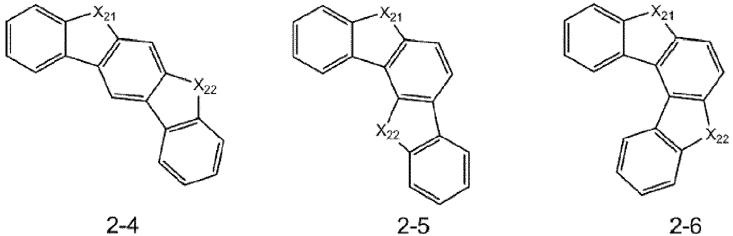 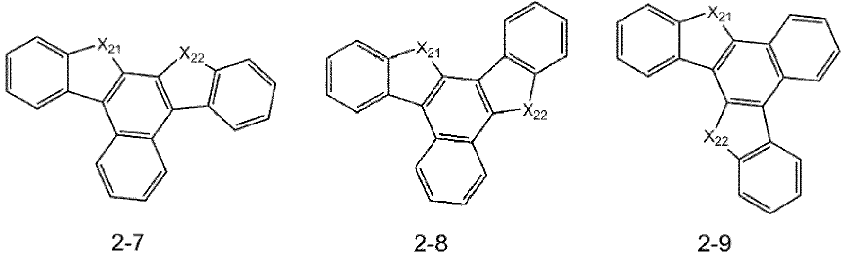 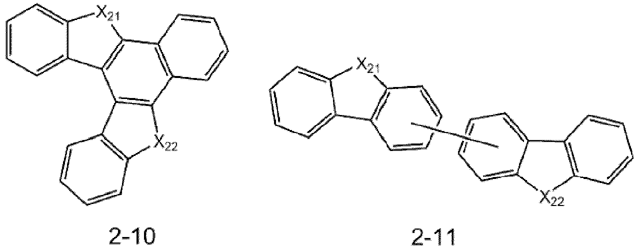 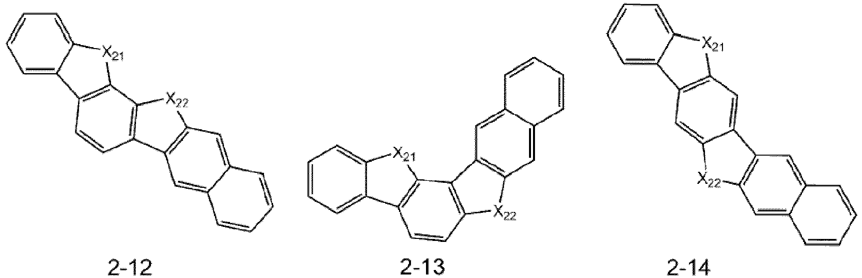 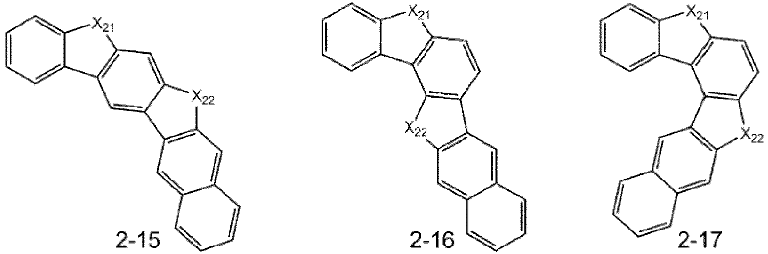
dans lequel, dans les Formules 2-1 à 2-17,
X₂₁ est choisi parmi C(R₂₁)(R₂₂), N(R₂₁), O, et S,
X₂₂ est choisi parmi C(R₂₃)(R₂₄), N(R₂₃), O, et S,
R₂₁ à R₂₄ sont chacun choisis indépendamment parmi :
un groupe méthyle, un groupe éthyle, un groupe n-propyle, un groupe isopropyle, un groupe n-butyle, un groupe isobutyle, un groupe sec-butyle, et un groupe tert-butyle ;
un groupe méthyle, un groupe éthyle, un groupe n-propyle, un groupe isopropyle, un groupe n-butyle, un groupe isobutyle, un groupe sec-butyle, et un groupe tert-butyle, chacun substitué par au moins l'un choisi parmi du deutérium, -F, -Cl, -Br, et -I ; et
un groupe phényle, un groupe biphényle, un groupe terphényle, un groupe naphtyle, un groupe fluorényle, un groupe carbazolyle, un groupe dibenzofuranyle, un groupe dibenzothiophényle, un groupe pyridinyle, un groupe pyrimidinyle, un groupe quinolinyle, un groupe isoquinolinyle, un groupe quinoxalinyle, et un groupe quinazolinyle, et
l'un choisi parmi un atome de carbone et un atome d'azote qui constituent la structure partielle représentée par l'une choisie parmi les Formules 2-1 à 2-17 est un site de liaison à un atome voisin choisi parmi les Formules 1-1 à 1-5.

4. Dispositif électroluminescent organique selon l'une quelconque des revendications 1 à 3, dans lequel :
le premier composé, le deuxième composé, et le troisième composé sont chacun indépendamment un composé à base d'azine représenté par l'une choisie parmi les Formules 1-11, 1-21 à 1-25, 1-31 à 1-33, 1-41, 1-42 et 1-51, sauf dans un cas où le premier composé, le deuxième composé, et le troisième composé incluent simultanément un composé à base d'azine représenté respectivement par l'une choisie parmi les Formules 1-31 à 1-33 : 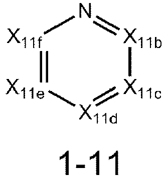 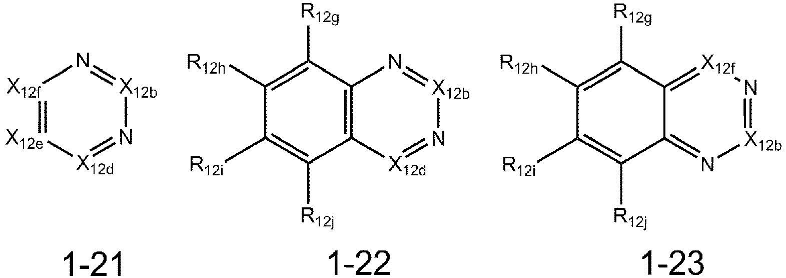 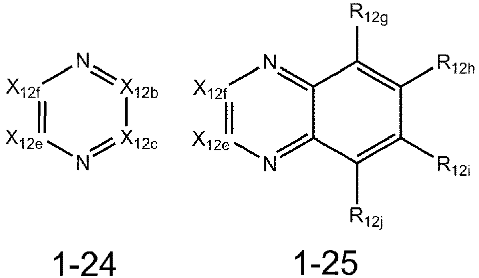 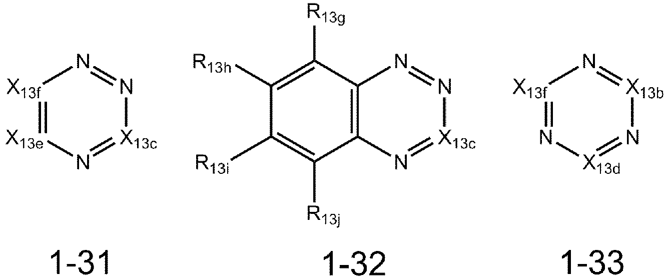 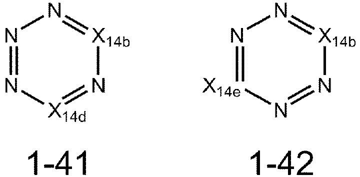 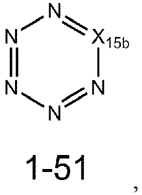
dans lequel, dans les Formules 1-11, 1-21 à 1-25, 1-31 à 1-33, 1-41, 1-42 et 1-51,
X_{11b} à X_{11f} sont chacun définis indépendamment comme dans la Formule 1-1,
X_{12b} à X_{12f} sont chacun définis indépendamment comme dans la Formule 1-2,
X_{13b} à X_{13f} sont chacun définis indépendamment comme dans la Formule 1-3,
X_{14b}, X_{14d}, et X₁₄ₑ sont chacun définis indépendamment comme dans la Formule 1-4,
X_{15b} est défini comme dans la Formule 1-5, et
R_{12g} à R₁₂ⱼ et R_{13g} à R₁₃ⱼ sont chacun choisis indépendamment parmi :
hydrogène, deutérium, -F, -Cl, -Br, -I, un groupe méthyle, un groupe éthyle, un groupe n-propyle, un groupe isopropyle, un groupe n-butyle, un groupe isobutyle, un groupe sec-butyle, un groupe tert-butyle, un groupe méthoxy, un groupe éthoxy, un groupe n-propoxy, un groupe isopropoxy, un groupe n-butoxy, un groupe isobutoxy, un groupe sec-butoxy, un groupe tert-butoxy, un groupe phényle, un groupe biphényle, un groupe terphényle, un groupe naphtyle, un groupe fluorényle, un groupe carbazolyle, un groupe dibenzofuranyle, un groupe dibenzothiophényle, un groupe pyridinyle, un groupe pyrimidinyle, un groupe quinoléinyle, un groupe isoquinolinyle, un groupe quinoxalinyle, un groupe quinazolinyle, un groupe benzofuropyridinyle, un groupe benzothiénopyridinyle, un groupe benzofuropyrimidinyle, et un groupe benzothiénopyrimidinyle ; et
un groupe méthyle, un groupe éthyle, un groupe n-propyle, un groupe isopropyle, un groupe n-butyle, un groupe isobutyle, un groupe sec-butyle, un groupe tert-butyle, un groupe phényle, un groupe biphényle, un groupe terphényle, un groupe naphtyle, un groupe fluorényle, un groupe carbazolyle, un groupe dibenzofuranyle, un groupe dibenzothiophényle, un groupe pyridinyle, un groupe pyrimidinyle, un groupe quinolinyle, un groupe isoquinolinyle, un groupe quinoxalinyle, un groupe quinazolinyle, un groupe benzofuropyridinyle, un groupe benzothiénopyridinyle, un groupe benzofuropyrimidinyle, et un groupe benzothiénopyrimidinyle, chacun substitué par au moins l'un choisi parmi du deutérium, -F, -Cl, -Br, et -I.

5. Dispositif électroluminescent organique selon l'une quelconque des revendications 1 à 4, dans lequel :
le premier composé comprend une structure partielle représentée par l'une choisie parmi les Formules 2-1 à 2-17 : 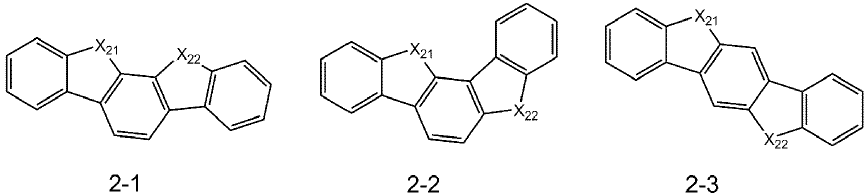 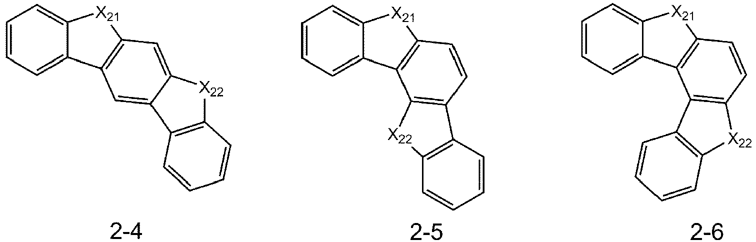 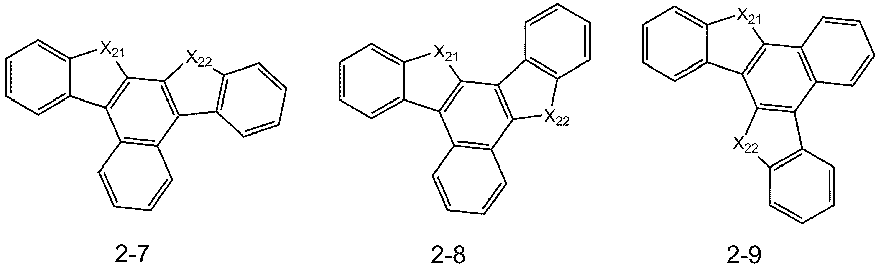 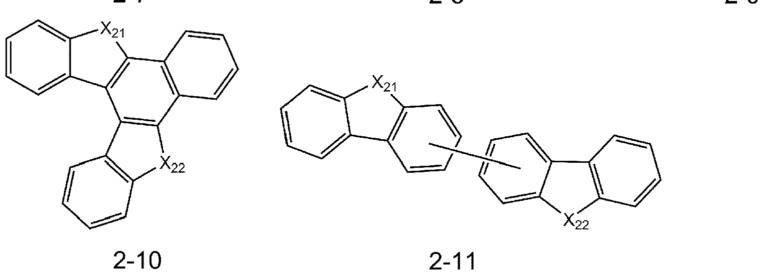 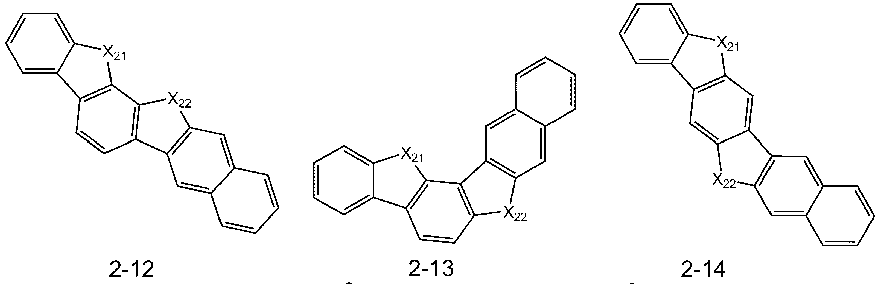 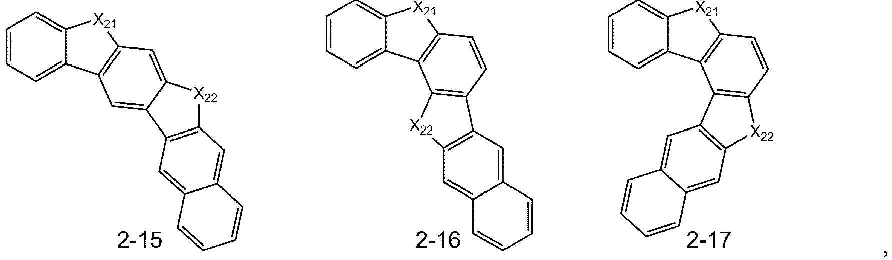
dans lequel, dans les Formules 2-1 à 2-17,
X₂₁ est choisi parmi C(R₂₁)(R₂₂), N(R₂₁), O, et S,
X₂₂ est choisi parmi C(R₂₃)(R₂₄), N(R₂₃), O, et S,
R₂₁ à R₂₄ sont chacun choisis indépendamment parmi :
un groupe méthyle, un groupe éthyle, un groupe n-propyle, un groupe isopropyle, un groupe n-butyle, un groupe isobutyle, un groupe sec-butyle, et un groupe tert-butyle ;
un groupe méthyle, un groupe éthyle, un groupe n-propyle, un groupe isopropyle, un groupe n-butyle, un groupe isobutyle, un groupe sec-butyle, et un groupe tert-butyle, chacun substitué par au moins l'un choisi parmi du deutérium, -F, -Cl, -Br, et -I ;
un groupe phényle, un groupe naphtyle, un groupe fluorényle, un groupe carbazolyle, un groupe dibenzofuranyle, un groupe dibenzothiophényle, un groupe pyridinyle, un groupe pyrimidinyle, un groupe quinolinyle, un groupe isoquinolinyle, un groupe quinoxalinyle, un groupe quinazolinyle, un groupe benzofuropyridinyle, un groupe benzothiénopyridinyle, un groupe benzofuropyrimidinyle, et un groupe benzothiénopyrimidinyle ; et
un groupe phényle, un groupe biphényle, un groupe terphényle, un groupe naphtyle, un groupe fluorényle, un groupe carbazolyle, un groupe dibenzofuranyle, un groupe dibenzothiophényle, un groupe pyridinyle, un groupe pyrimidinyle, un groupe quinolinyle, un groupe isoquinolinyle, un groupe quinoxalinyle, un groupe quinazolinyle, un groupe benzofuropyridinyle, un groupe benzothiénopyridinyle, un groupe benzofuropyrimidinyle, et un groupe benzothiénopyrimidinyle, chacun substitué par au moins l'un choisi parmi du deutérium, -F, -Cl, -Br, -I, un groupe méthyle, un groupe éthyle, un groupe n-propyle, un groupe isopropyle, un groupe n-butyle, un groupe isobutyle, un groupe sec-butyle, un groupe tert-butyle, un groupe méthoxy, un groupe éthoxy, un groupe n-propoxy, un groupe isopropoxy, un groupe n-butoxy, un groupe isobutoxy, un groupe sec-butoxy, un groupe tert-butoxy, un groupe phényle, un groupe biphényle, un groupe terphényle, un groupe naphtyle, un groupe fluorényle, un groupe carbazolyle, un groupe dibenzofuranyle, un groupe dibenzothiophényle, un groupe pyridinyle, un groupe pyrimidinyle, un groupe quinolinyle, un groupe isoquinolinyle, un groupe quinoxalinyle, un groupe quinazolinyle, un groupe benzofuropyridinyle, un groupe benzothiénopyridinyle, un groupe benzofuropyrimidinyle, et un groupe benzothiénopyrimidinyle, et
l'un choisi parmi un atome de carbone et un atome d'azote qui constituent la structure partielle représentée par l'une choisie parmi les Formules 2-1 à 2-17 est un site de liaison à un atome voisin choisi parmi les Formules 1-1 à 1-5.

6. Dispositif électroluminescent organique selon l'une quelconque des revendications 1 à 5, dans lequel :
le premier composé est choisi parmi les Composés H-1 à H-1 1,
le deuxième composé est choisi parmi les Composés HB-1 à HB-3, et
le troisième composé est choisi parmi les Composés ET-1 et ET-2 :

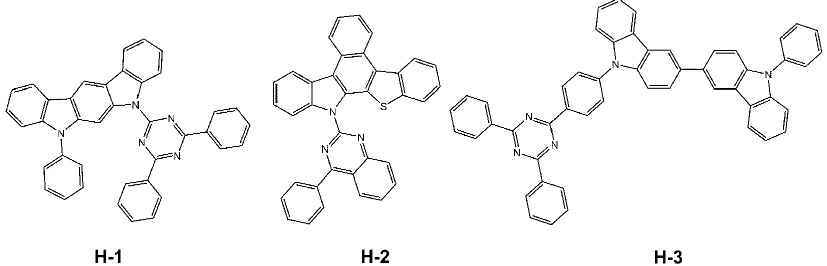

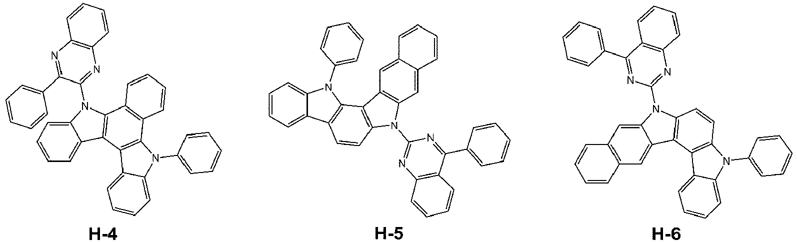

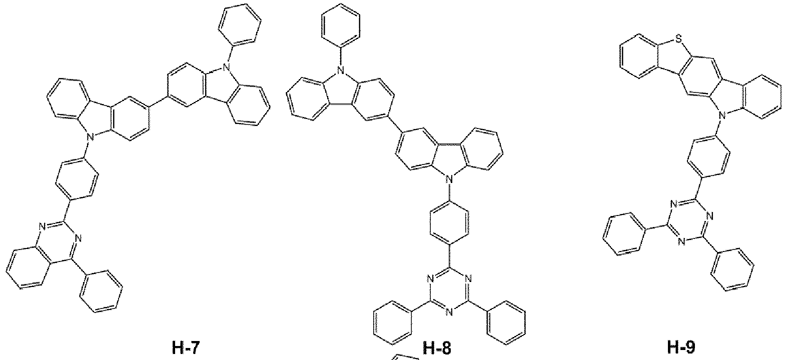

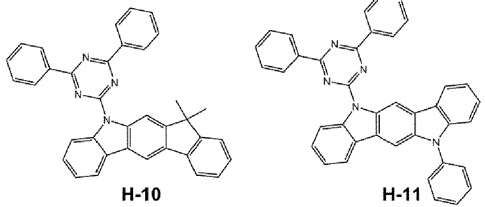

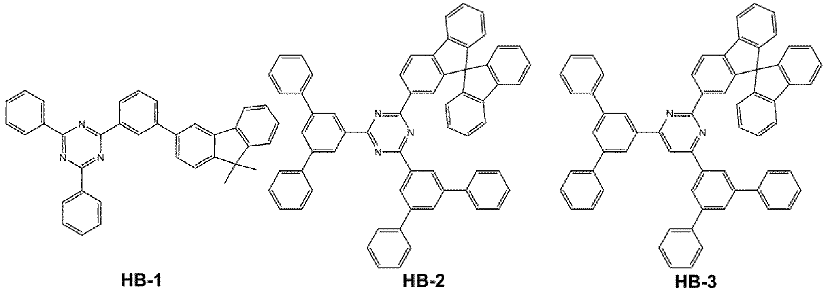

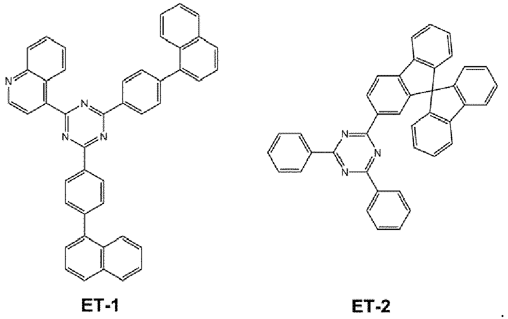

7. Dispositif électroluminescent organique selon l'une quelconque des revendications 1 à 6, dans lequel :
le premier composé, le deuxième composé, et le troisième composé sont chacun indépendamment un composé à base d'azine représenté par l'une choisie parmi les Formules 1-1 à 1-3.

8. Dispositif électroluminescent organique selon l'une quelconque des revendications 1 à 7, dans lequel :
i) le premier composé est un composé à base d'azine représenté par l'une choisie parmi les Formules 1-1 et 1-2, et le deuxième composé et le troisième composé sont chacun indépendamment un composé à base d'azine représenté par l'une choisie parmi les Formules 1-2 et 1-3 ;
ii) le deuxième composé est un composé à base d'azine représenté par l'une choisie parmi les Formules 1-1 et 1-2, et le premier composé et le troisième composé sont chacun indépendamment un composé à base d'azine représenté par l'une choisie parmi les Formules 1-2 et 1-3 ; ou
iii) le troisième composé est un composé à base d'azine représenté par l'une choisie parmi les Formules 1-1 et 1-2, et le premier composé et le deuxième composé sont chacun indépendamment un composé à base d'azine représenté par l'une choisie parmi les Formules 1-2 et 1-3.

9. Dispositif électroluminescent organique selon l'une quelconque des revendications 1 à 8, dans lequel :
la couche organique comprend une région de transport d'électrons entre la couche d'émission et la seconde électrode,
la région de transport d'électrons comprend une couche tampon et une couche de transport d'électrons,
la couche tampon est située entre la couche d'émission et la couche de transport d'électrons,
la couche d'émission comprend le premier composé,
la couche tampon comprend le deuxième composé, et
la couche de transport d'électrons comprend le troisième composé ;
de préférence dans lequel :
la couche d'émission est en contact direct avec la couche tampon, et
la couche tampon est en contact direct avec la couche de transport d'électrons.

10. Dispositif électroluminescent organique selon l'une quelconque des revendications 1 à 9, dans lequel :
la première électrode est une anode, et la seconde électrode est une cathode ;
et/ou dans lequel :
la couche d'émission comprend un composé organométallique.

11. Dispositif électroluminescent organique selon l'une quelconque des revendications 1 à 10, dans lequel :
le dispositif électroluminescent organique comprend en outre un substrat divisé en une première région de sous-pixels, une deuxième région de sous-pixels, et une troisième région de sous-pixels ;
la première électrode comprend une pluralité de premières électrodes disposées respectivement dans la première région de sous-pixels, la deuxième région de sous-pixels, et la troisième région de sous-pixels du substrat ;
la seconde électrode fait face à la pluralité de premières électrodes ; et
la couche organique est située entre la pluralité de premières électrodes et la seconde électrode.

12. Dispositif électroluminescent organique selon la revendication 11, dans lequel :
la couche d'émission comprend une première couche d'émission, une deuxième couche d'émission, et une troisième couche d'émission disposées respectivement dans la première région de sous-pixels, la deuxième région de sous-pixels, et la troisième région de sous-pixels du substrat, et
au moins l'une de la première couche d'émission et de la deuxième couche d'émission comprend le premier composé et un composé organométallique.
